# EUROPEAN PATENT SPECIFICATION

(11) **EP 2 833 907 B1**
(45) Date of publication and mention of the grant of the patent: **28.02.2018**
(21) Application number: 13798222.9
(22) Date of filing: 05.04.2013
(51) Int. Cl.: A61K 39/00, C07K 16/40

(54) **COMPOSITIONS AND METHODS OF INHIBTING MASP-1 AND/OR MASP-3 FOR THE TREATMENT OF PAROXYSMAL NOCTURNAL HEMOGLOBINURIA**
ZUSAMMENSETZUNGEN UND VERFAHREN ZUR HEMMUNG VON MASP-1 UND/ODER MASP-3 ZUR BEHANDLUNG VON PAROXYSMALER NÄCHTLICHER HÄMOGLOBINURIE
COMPOSITIONS ET PROCÉDÉS D'INHIBITION DE MASP-1 ET/OU MASP-3 POUR LE TRAITEMENT L'HÉMOGLOBINURIE PAROXYSMIQUE NOCTURNE

(30) Priority: 06.04.2012 US 201261621461 P
(43) Date of publication of application: 11.02.2015
(73) Proprietor: Omeros Corporation, Seattle, WA 98119 (US); University of Leicester, Leicester LE1 7RH (GB)
(72) Inventor: SCHWAEBLE, Hans-Wilhelm, Mountsorrel Leicestershire LE12 7AF (GB); DEMOPULOS, Gregory, A., Mercer Island, WA 98040 (US)
(74) Representative: Avidity IP
(86) International application number: PCT/US2013/035488
(87) International publication number: WO 2013/180834

(56) References cited:
- WO-A1-2011/057158
- WO-A2-2011/006982
- US-A1- 2009 017 031
- US-A1- 2009 042 248
- US-A1- 2011 311 549
- H. JIANG ET AL: "Complement 1 Inhibitor Is a Regulator of the Alternative Complement Pathway", THE JOURNAL OF EXPERIMENTAL MEDICINE, vol. 194, no. 11, 3 December 2001 (2001-12-03), pages 1609-1616, XP055246972, US ISSN: 0022-1007, DOI: 10.1084/jem.194.11.1609
- WONG N K H ET AL: "ACTIVITIES OF THE MBL-ASSOCIATED SERINE PROTEASES (MASPS) AND THEIR REGULATION BY NATURAL INHIBITORS", MOLECULAR IMMUNOLOGY, PERGAMON, GB, vol. 36, no. 13/14, 1 January 1999 (1999-01-01), pages 853-861, XP001037853, ISSN: 0161-5890, DOI: 10.1016/S0161-5890(99)00106-6
- M. FRIDKIS-HARELI ET AL: "Design and development of TT30, a novel C3d-targeted C3/C5 convertase inhibitor for treatment of human complement alternative pathway-mediated diseases", BLOOD, vol. 118, no. 17, 27 October 2011 (2011-10-27), pages 4705-4713, XP055072079, ISSN: 0006-4971, DOI: 10.1182/blood-2011-06-359646
- RISITANO A M ET AL: "The human complement receptor type 2/factor H fusion protein TT30, an alternative pathway specific complement inhibitor, prevents hemolysis of erythrocytes from patients with paroxysmal nocturnal hemoglobinuria by membrane-targeting of factor H activity", MOLECULAR IMMUNOLOGY, PERGAMON, GB, vol. 47, no. 13, 1 August 2010 (2010-08-01), page 2215, XP027120165, ISSN: 0161-5890 [retrieved on 2010-07-03]
- NIRMAL K BANDA ET AL: "Mechanisms of mannose-binding lectin-associated serine proteases-1/3 activation of the alternative pathway of complement", MOLECULAR IMMUNOLOGY, PERGAMON, GB, vol. 49, no. 1, 27 August 2011 (2011-08-27), pages 281-289, XP028328168, ISSN: 0161-5890, DOI: 10.1016/J.MOLIMM.2011.08.021 [retrieved on 2011-09-05]
- BANDA ET AL.: 'Essential Role of Complement Mannose-Hinding Lectin-Associa!ed Serine Prateases-1/3 in the Murine Collagen Anybody-induced Model of Inflammatory' ARTHRITIS THE JOURNAL OF IMMUNOLOGY vol. 185, no. 9, 01 November 2010, pages 5598 - 5606, XP055172413
- S. Zundel ET AL: "Characterization of Recombinant Mannan-Binding Lectin-Associated Serine Protease (MASP)-3 Suggests an Activation Mechanism Different from That of MASP-1 and MASP-2", THE JOURNAL OF IMMUNOLOGY, vol. 172, no. 7, 19 March 2004 (2004-03-19), pages 4342-4350, XP055246991, US ISSN: 0022-1767, DOI: 10.4049/jimmunol.172.7.4342

### SEQUENCE LISTING

<110> Omeros Corporation
   University of Leicester
<120> Compositions and Methods of Inhibiting MASP-1 and/or MASP-2 and/or MASP-3 for the Treatment of Paroxysmal Nocturnal Hemoglobinuria
<130> MP.1.0146.PCT
<150> US 61/621,461
   <151> 2012-04-06
<160> 42
<170> PatentIn version 3.5
<210> 1
   <211> 725
   <212> DNA
   <213> homo sapiens
<400> 1
   ggccaggcca gctggacggg cacaccatga ggctgctgac cctcctgggc cttctgtgtg        60
   gctcggtggc cacccccttg ggcccgaagt ggcctgaacc tgtgttcggg cgcctggcat       120
   cccccggctt tccaggggag tatgccaatg accaggagcg gcgctggacc ctgactgcac     180
   cccccggcta ccgcctgcgc ctctacttca cccacttcga cctggagctc tcccacctct     240
   gcgagtacga cttcgtcaag ctgagctcgg gggccaaggt gctggccacg ctgtgcgggc     300
   aggagagcac agacacggag cgggcccctg gcaaggacac tttctactcg ctgggctcca     360
   gcctggacat taccttccgc tccgactact ccaacgagaa gccgttcacg gggttcgagg     420
   ccttctatgc agccgaggac attgacgagt gccaggtggc cccgggagag gcgcccacct     480
   gcgaccacca ctgccacaac cacctgggcg gtttctactg ctcctgccgc gcaggctacg     540
   tcctgcaccg taacaagcgc acctgctcag agcagagcct ctagcctccc ctggagctcc     600
   ggcctgccca gcaggtcaga agccagagcc agcctgctgg cctcagctcc gggttgggct     660
   gagatggctg tgccccaact cccattcacc caccatggac ccaataataa acctggcccc     720
   acccc                                                                 725
<210> 2
   <211> 185
   <212> PRT
   <213> homo sapiens
<400> 2
   Met Arg Leu Leu Thr Leu Leu Gly Leu Leu Cys Gly Ser Val Ala Thr
   1               5                   10                  15
   Pro Leu Gly Pro Lys Trp Pro Glu Pro Val Phe Gly Arg Leu Ala Ser
   20 25 30
   Pro Gly Phe Pro Gly Glu Tyr Ala Asn Asp Gln Glu Arg Arg Trp Thr
   35 40 45
   Leu Thr Ala Pro Pro Gly Tyr Arg Leu Arg Leu Tyr Phe Thr His Phe
   50 55 60
   Asp Leu Glu Leu Ser His Leu Cys Glu Tyr Asp Phe Val Lys Leu Ser
   65 70 75 80
   Ser Gly Ala Lys Val Leu Ala Thr Leu Cys Gly Gln Glu Ser Thr Asp
   85 90 95
   Thr Glu Arg Ala Pro Gly Lys Asp Thr Phe Tyr Ser Leu Gly Ser Ser
   100 105 110
   Leu Asp Ile Thr Phe Arg Ser Asp Tyr Ser Asn Glu Lys Pro Phe Thr
   115 120 125
   Gly Phe Glu Ala Phe Tyr Ala Ala Glu Asp Ile Asp Glu Cys Gln Val
   130 135 140
   Ala Pro Gly Glu Ala Pro Thr Cys Asp His His Cys His Asn His Leu
   145 150 155 160
   Gly Gly Phe Tyr Cys Ser Cys Arg Ala Gly Tyr Val Leu His Arg Asn
   165 170 175
   Lys Arg Thr Cys Ser Glu Gln Ser Leu
   180 185
<210> 3
   <211> 170
   <212> PRT
   <213> homo sapiens
<400> 3
   Thr Pro Leu Gly Pro Lys Trp Pro Glu Pro Val Phe Gly Arg Leu Ala
   1               5                   10                  15
   Ser Pro Gly Phe Pro Gly Glu Tyr Ala Asn Asp Gln Glu Arg Arg Trp
               20                  25                  30
   Thr Leu Thr Ala Pro Pro Gly Tyr Arg Leu Arg Leu Tyr Phe Thr His
           35                  40                  45
   Phe Asp Leu Glu Leu Ser His Leu Cys Glu Tyr Asp Phe Val Lys Leu
       50                  55                  60
   Ser Ser Gly Ala Lys Val Leu Ala Thr Leu Cys Gly Gln Glu Ser Thr
   65                  70                  75                  80
   Asp Thr Glu Arg Ala Pro Gly Lys Asp Thr Phe Tyr Ser Leu Gly Ser
                   85                  90                  95
   Ser Leu Asp Ile Thr Phe Arg Ser Asp Tyr Ser Asn Glu Lys Pro Phe
               100                 105                 110
   Thr Gly Phe Glu Ala Phe Tyr Ala Ala Glu Asp Ile Asp Glu Cys Gln
           115                 120                 125
   Val Ala Pro Gly Glu Ala Pro Thr Cys Asp His His Cys His Asn His
       130                 135                 140
   Leu Gly Gly Phe Tyr Cys Ser Cys Arg Ala Gly Tyr Val Leu His Arg
   145                 150                 155                 160
   Asn Lys Arg Thr Cys Ser Glu Gln Ser Leu
                   165                 170
<210> 4
   <211> 2460
   <212> DNA
   <213> homo sapiens
<400> 4
   ggccagctgg acgggcacac catgaggctg ctgaccctcc tgggccttct gtgtggctcg      60
   gtggccaccc ccttgggccc gaagtggcct gaacctgtgt tcgggcgcct ggcatccccc     120
   ggctttccag gggagtatgc caatgaccag gagcggcgct ggaccctgac tgcacccccc     180
   ggctaccgcc tgcgcctcta cttcacccac ttcgacctgg agctctccca cctctgcgag     240
   tacgacttcg tcaagctgag ctcgggggcc aaggtgctgg ccacgctgtg cgggcaggag     300
   agcacagaca cggagcgggc ccctggcaag gacactttct actcgctggg ctccagcctg     360
   gacattacct tccgctccga ctactccaac gagaagccgt tcacggggtt cgaggccttc     420
   tatgcagccg aggacattga cgagtgccag gtggccccgg gagaggcgcc cacctgcgac     480
   caccactgcc acaaccacct gggcggtttc tactgctcct gccgcgcagg ctacgtcctg     540
   caccgtaaca agcgcacctg ctcagccctg tgctccggcc aggtcttcac ccagaggtct     600
   ggggagctca gcagccctga atacccacgg ccgtatccca aactctccag ttgcacttac     660
   agcatcagcc tggaggaggg gttcagtgtc attctggact ttgtggagtc cttcgatgtg     720
   gagacacacc ctgaaaccct gtgtccctac gactttctca agattcaaac agacagagaa     780
   gaacatggcc cattctgtgg gaagacattg ccccacagga ttgaaacaaa aagcaacacg 840
   gtgaccatca cctttgtcac agatgaatca ggagaccaca caggctggaa gatccactac 900
   acgagcacag cgcagccttg cccttatccg atggcgccac ctaatggcca cgtttcacct 960
   gtgcaagcca aatacatcct gaaagacagc ttctccatct tttgcgagac tggctatgag 1020
   cttctgcaag gtcacttgcc cctgaaatcc tttactgcag tttgtcagaa agatggatct 1080
   tgggaccggc caatgcccgc gtgcagcatt gttgactgtg gccctcctga tgatctaccc 1140
   agtggccgag tggagtacat cacaggtcct ggagtgacca cctacaaagc tgtgattcag 1200
   tacagctgtg aagagacctt ctacacaatg aaagtgaatg atggtaaata tgtgtgtgag 1260
   gctgatggat tctggacgag ctccaaagga gaaaaatcac tcccagtctg tgagcctgtt 1320
   tgtggactat cagcccgcac aacaggaggg cgtatatatg gagggcaaaa ggcaaaacct 1380
   ggtgattttc cttggcaagt cctgatatta ggtggaacca cagcagcagg tgcactttta 1440
   tatgacaact gggtcctaac agctgctcat gccgtctatg agcaaaaaca tgatgcatcc 1500
   gccctggaca ttcgaatggg caccctgaaa agactatcac ctcattatac acaagcctgg 1560
   tctgaagctg tttttataca tgaaggttat actcatgatg ctggctttga caatgacata 1620
   gcactgatta aattgaataa caaagttgta atcaatagca acatcacgcc tatttgtctg 1680
   ccaagaaaag aagctgaatc ctttatgagg acagatgaca ttggaactgc atctggatgg 1740
   ggattaaccc aaaggggttt tcttgctaga aatctaatgt atgtcgacat accgattgtt 1800
   gaccatcaaa aatgtactgc tgcatatgaa aagccaccct atccaagggg aagtgtaact 1860
   gctaacatgc tttgtgctgg cttagaaagt gggggcaagg acagctgcag aggtgacagc 1920
   ggaggggcac tggtgtttct agatagtgaa acagagaggt ggtttgtggg aggaatagtg 1980
   tcctggggtt ccatgaattg tggggaagca ggtcagtatg gagtctacac aaaagttatt 2040
   aactatattc cctggatcga gaacataatt agtgattttt aacttgcgtg tctgcagtca 2100
   aggattcttc atttttagaa atgcctgtga agaccttggc agcgacgtgg ctcgagaagc 2160
   attcatcatt actgtggaca tggcagttgt tgctccaccc aaaaaaacag actccaggtg 2220
   aggctgctgt catttctcca cttgccagtt taattccagc cttacccatt gactcaaggg 2280
   gacataaacc acgagagtga cagtcatctt tgcccaccca gtgtaatgtc actgctcaaa 2340
   ttacatttca ttaccttaaa aagccagtct cttttcatac tggctgttgg catttctgta 2400
   aactgcctgt ccatgctctt tgtttttaaa cttgttctta ttgaaaaaaa aaaaaaaaaa 2460
<210> 5
   <211> 686
   <212> PRT
   <213> homo sapiens
<400> 5
   Met Arg Leu Leu Thr Leu Leu Gly Leu Leu Cys Gly Ser Val Ala Thr
   1               5                   10                  15
   Pro Leu Gly Pro Lys Trp Pro Glu Pro Val Phe Gly Arg Leu Ala Ser
               20                  25                  30
   Pro Gly Phe Pro Gly Glu Tyr Ala Asn Asp Gln Glu Arg Arg Trp Thr
           35                  40                  45
   Leu Thr Ala Pro Pro Gly Tyr Arg Leu Arg Leu Tyr Phe Thr His Phe
       50                  55                  60
   Asp Leu Glu Leu Ser His Leu Cys Glu Tyr Asp Phe Val Lys Leu Ser
   65                  70                  75                  80
   Ser Gly Ala Lys Val Leu Ala Thr Leu Cys Gly Gln Glu Ser Thr Asp
                   85                  90                  95
   Thr Glu Arg Ala Pro Gly Lys Asp Thr Phe Tyr Ser Leu Gly Ser Ser
               100                 105                 110
   Leu Asp Ile Thr Phe Arg Ser Asp Tyr Ser Asn Glu Lys Pro Phe Thr
           115                 120                 125
   Gly Phe Glu Ala Phe Tyr Ala Ala Glu Asp Ile Asp Glu Cys Gln Val
       130                 135                 140
   Ala Pro Gly Glu Ala Pro Thr Cys Asp His His Cys His Asn His Leu
   145                 150                 155                 160
   Gly Gly Phe Tyr Cys Ser Cys Arg Ala Gly Tyr Val Leu His Arg Asn
                   165                 170                 175
   Lys Arg Thr Cys Ser Ala Leu Cys Ser Gly Gln Val Phe Thr Gln Arg
               180                 185                 190
   Ser Gly Glu Leu Ser Ser Pro Glu Tyr Pro Arg Pro Tyr Pro Lys Leu
           195                 200                 205
   Ser Ser Cys Thr Tyr Ser Ile Ser Leu Glu Glu Gly Phe Ser Val Ile
       210                 215                 220
   Leu Asp Phe Val Glu Ser Phe Asp Val Glu Thr His Pro Glu Thr Leu
   225                 230                 235                 240
   Cys Pro Tyr Asp Phe Leu Lys Ile Gln Thr Asp Arg Glu Glu His Gly
                   245                 250                 255
   Pro Phe Cys Gly Lys Thr Leu Pro His Arg Ile Glu Thr Lys Ser Asn
   260 265 270
   Thr Val Thr Ile Thr Phe Val Thr Asp Glu Ser Gly Asp His Thr Gly
   275 280 285
   Trp Lys Ile His Tyr Thr Ser Thr Ala Gln Pro Cys Pro Tyr Pro Met
   290 295 300
   Ala Pro Pro Asn Gly His Val Ser Pro Val Gln Ala Lys Tyr Ile Leu
   305 310 315 320
   Lys Asp Ser Phe Ser Ile Phe Cys Glu Thr Gly Tyr Glu Leu Leu Gln
   325 330 335
   Gly His Leu Pro Leu Lys Ser Phe Thr Ala Val Cys Gln Lys Asp Gly
   340 345 350
   Ser Trp Asp Arg Pro Met Pro Ala Cys Ser Ile Val Asp Cys Gly Pro
   355 360 365
   Pro Asp Asp Leu Pro Ser Gly Arg Val Glu Tyr Ile Thr Gly Pro Gly
   370 375 380
   Val Thr Thr Tyr Lys Ala Val Ile Gln Tyr Ser Cys Glu Glu Thr Phe
   385 390 395 400
   Tyr Thr Met Lys Val Asn Asp Gly Lys Tyr Val Cys Glu Ala Asp Gly
   405 410 415
   Phe Trp Thr Ser Ser Lys Gly Glu Lys Ser Leu Pro Val Cys Glu Pro
   420 425 430
   Val Cys Gly Leu Ser Ala Arg Thr Thr Gly Gly Arg Ile Tyr Gly Gly
   435 440 445
   Gln Lys Ala Lys Pro Gly Asp Phe Pro Trp Gln Val Leu Ile Leu Gly
   450 455 460
   Gly Thr Thr Ala Ala Gly Ala Leu Leu Tyr Asp Asn Trp Val Leu Thr
   465 470 475 480
   Ala Ala His Ala Val Tyr Glu Gln Lys His Asp Ala Ser Ala Leu Asp
   485 490 495
   Ile Arg Met Gly Thr Leu Lys Arg Leu Ser Pro His Tyr Thr Gln Ala
               500                 505                 510
   Trp Ser Glu Ala Val Phe Ile His Glu Gly Tyr Thr His Asp Ala Gly
           515                 520                 525
   Phe Asp Asn Asp Ile Ala Leu Ile Lys Leu Asn Asn Lys Val Val Ile
       530                 535                 540
   Asn Ser Asn Ile Thr Pro Ile Cys Leu Pro Arg Lys Glu Ala Glu Ser
   545                 550                 555                 560
   Phe Met Arg Thr Asp Asp Ile Gly Thr Ala Ser Gly Trp Gly Leu Thr
                   565                 570                 575
   Gln Arg Gly Phe Leu Ala Arg Asn Leu Met Tyr Val Asp Ile Pro Ile
               580                 585                 590
   Val Asp His Gln Lys Cys Thr Ala Ala Tyr Glu Lys Pro Pro Tyr Pro
           595                 600                 605
   Arg Gly Ser Val Thr Ala Asn Met Leu Cys Ala Gly Leu Glu Ser Gly
       610                 615                 620
   Gly Lys Asp Ser Cys Arg Gly Asp Ser Gly Gly Ala Leu Val Phe Leu
   625                 630                 635                 640
   Asp Ser Glu Thr Glu Arg Trp Phe Val Gly Gly Ile Val Ser Trp Gly
                   645                 650                 655
   Ser Met Asn Cys Gly Glu Ala Gly Gln Tyr Gly Val Tyr Thr Lys Val
               660                 665                 670
   Ile Asn Tyr Ile Pro Trp Ile Glu Asn Ile Ile Ser Asp Phe
           675                 680                 685
<210> 6
   <211> 671
   <212> PRT
   <213> homo sapiens
<400> 6
   Thr Pro Leu Gly Pro Lys Trp Pro Glu Pro Val Phe Gly Arg Leu Ala
   1               5                   10                  15
   Ser Pro Gly Phe Pro Gly Glu Tyr Ala Asn Asp Gln Glu Arg Arg Trp
               20                  25                  30
   Thr Leu Thr Ala Pro Pro Gly Tyr Arg Leu Arg Leu Tyr Phe Thr His
           35                  40                  45
   Phe Asp Leu Glu Leu Ser His Leu Cys Glu Tyr Asp Phe Val Lys Leu
       50                  55                  60
   Ser Ser Gly Ala Lys Val Leu Ala Thr Leu Cys Gly Gln Glu Ser Thr
   65                  70                  75                  80
   Asp Thr Glu Arg Ala Pro Gly Lys Asp Thr Phe Tyr Ser Leu Gly Ser
                   85                  90                  95
   Ser Leu Asp Ile Thr Phe Arg Ser Asp Tyr Ser Asn Glu Lys Pro Phe
               100                 105                 110
   Thr Gly Phe Glu Ala Phe Tyr Ala Ala Glu Asp Ile Asp Glu Cys Gln
           115                 120                 125
   Val Ala Pro Gly Glu Ala Pro Thr Cys Asp His His Cys His Asn His
       130                 135                 140
   Leu Gly Gly Phe Tyr Cys Ser Cys Arg Ala Gly Tyr Val Leu His Arg
   145                 150                 155                 160
   Asn Lys Arg Thr Cys Ser Ala Leu Cys Ser Gly Gln Val Phe Thr Gln
                   165                 170                 175
   Arg Ser Gly Glu Leu Ser Ser Pro Glu Tyr Pro Arg Pro Tyr Pro Lys
               180                 185                 190
   Leu Ser Ser Cys Thr Tyr Ser Ile Ser Leu Glu Glu Gly Phe Ser Val
           195                 200                 205
   Ile Leu Asp Phe Val Glu Ser Phe Asp Val Glu Thr His Pro Glu Thr
       210                 215                 220
   Leu Cys Pro Tyr Asp Phe Leu Lys Ile Gln Thr Asp Arg Glu Glu His
   225                 230                 235                 240
   Gly Pro Phe Cys Gly Lys Thr Leu Pro His Arg Ile Glu Thr Lys Ser
                   245                 250                 255
   Asn Thr Val Thr Ile Thr Phe Val Thr Asp Glu Ser Gly Asp His Thr
               260                 265                 270
   Gly Trp Lys Ile His Tyr Thr Ser Thr Ala Gln Pro Cys Pro Tyr Pro
           275                 280                 285
   Met Ala Pro Pro Asn Gly His Val Ser Pro Val Gln Ala Lys Tyr Ile
       290                 295                 300
   Leu Lys Asp Ser Phe Ser Ile Phe Cys Glu Thr Gly Tyr Glu Leu Leu
   305                 310                 315                 320
   Gln Gly His Leu Pro Leu Lys Ser Phe Thr Ala Val Cys Gln Lys Asp
                   325                 330                 335
   Gly Ser Trp Asp Arg Pro Met Pro Ala Cys Ser Ile Val Asp Cys Gly
               340                 345                 350
   Pro Pro Asp Asp Leu Pro Ser Gly Arg Val Glu Tyr Ile Thr Gly Pro
           355                 360                 365
   Gly Val Thr Thr Tyr Lys Ala Val Ile Gln Tyr Ser Cys Glu Glu Thr
       370                 375                 380
   Phe Tyr Thr Met Lys Val Asn Asp Gly Lys Tyr Val Cys Glu Ala Asp
   385                 390                 395                 400
   Gly Phe Trp Thr Ser Ser Lys Gly Glu Lys Ser Leu Pro Val Cys Glu
                   405                 410                 415
   Pro Val Cys Gly Leu Ser Ala Arg Thr Thr Gly Gly Arg Ile Tyr Gly
               420                 425                 430
   Gly Gln Lys Ala Lys Pro Gly Asp Phe Pro Trp Gln Val Leu Ile Leu
           435                 440                 445
   Gly Gly Thr Thr Ala Ala Gly Ala Leu Leu Tyr Asp Asn Trp Val Leu
       450                 455                 460
   Thr Ala Ala His Ala Val Tyr Glu Gln Lys His Asp Ala Ser Ala Leu
   465                 470                 475                 480
   Asp Ile Arg Met Gly Thr Leu Lys Arg Leu Ser Pro His Tyr Thr Gln
                   485                 490                 495
   Ala Trp Ser Glu Ala Val Phe Ile His Glu Gly Tyr Thr His Asp Ala
               500                 505                 510
   Gly Phe Asp Asn Asp Ile Ala Leu Ile Lys Leu Asn Asn Lys Val Val
           515                 520                 525
   Ile Asn Ser Asn Ile Thr Pro Ile Cys Leu Pro Arg Lys Glu Ala Glu
       530                 535                 540
   Ser Phe Met Arg Thr Asp Asp Ile Gly Thr Ala Ser Gly Trp Gly Leu
   545                 550                 555                 560
   Thr Gln Arg Gly Phe Leu Ala Arg Asn Leu Met Tyr Val Asp Ile Pro
                   565                 570                 575
   Ile Val Asp His Gln Lys Cys Thr Ala Ala Tyr Glu Lys Pro Pro Tyr
               580                 585                 590
   Pro Arg Gly Ser Val Thr Ala Asn Met Leu Cys Ala Gly Leu Glu Ser
           595                 600                 605
   Gly Gly Lys Asp Ser Cys Arg Gly Asp Ser Gly Gly Ala Leu Val Phe
       610                 615                 620
   Leu Asp Ser Glu Thr Glu Arg Trp Phe Val Gly Gly Ile Val Ser Trp
   625                 630                 635                 640
   Gly Ser Met Asn Cys Gly Glu Ala Gly Gln Tyr Gly Val Tyr Thr Lys
                   645                 650                 655
   Val Ile Asn Tyr Ile Pro Trp Ile Glu Asn Ile Ile Ser Asp Phe
               660                 665                 670
<210> 7
   <211> 3895
   <212> DNA
   <213> homo sapiens
<400> 7
   attccggcac agggacacaa acaagctcac ccaacaaagc caagctggga ggaccaaggc      60
   cgggcagccg ggagcaccca aggcaggaaa atgaggtggc tgcttctcta ttatgctctg     120
   tgcttctccc tgtcaaaggc ttcagcccac accgtggagc taaacaatat gtttggccag     180
   atccagtcgc ctggttatcc agactcctat cccagtgatt cagaggtgac ttggaatatc     240
   actgtcccag atgggtttcg gatcaagctt tacttcatgc acttcaactt ggaatcctcc     300
   tacctttgtg aatatgacta tgtgaaggta gaaactgagg accaggtgct ggcaaccttc     360
   tgtggcaggg agaccacaga cacagagcag actcccggcc aggaggtggt cctctcccct     420
   ggctccttca tgtccatcac tttccggtca gatttctcca atgaggagcg tttcacaggc     480
   tttgatgccc actacatggc tgtggatgtg gacgagtgca aggagaggga ggacgaggag     540
   ctgtcctgtg accactactg ccacaactac attggcggct actactgctc ctgccgcttc     600
   ggctacatcc tccacacaga caacaggacc tgccgagtgg agtgcagtga caacctcttc     660
   actcaaagga ctggggtgat caccagccct gacttcccaa acccttaccc caagagctct     720
   gaatgcctgt ataccatcga gctggaggag ggtttcatgg tcaacctgca gtttgaggac 780
   atatttgaca ttgaggacca tcctgaggtg ccctgcccct atgactacat caagatcaaa 840
   gttggtccaa aagttttggg gcctttctgt ggagagaaag ccccagaacc catcagcacc 900
   cagagccaca gtgtcctgat cctgttccat agtgacaact cgggagagaa ccggggctgg 960
   aggctctcat acagggctgc aggaaatgag tgcccagagc tacagcctcc tgtccatggg 1020
   aaaatcgagc cctcccaagc caagtatttc ttcaaagacc aagtgctcgt cagctgtgac 1080
   acaggctaca aagtgctgaa ggataatgtg gagatggaca cattccagat tgagtgtctg 1140
   aaggatggga cgtggagtaa caagattccc acctgtaaaa ttgtagactg tagagcccca 1200
   ggagagctgg aacacgggct gatcaccttc tctacaagga acaacctcac cacatacaag 1260
   tctgagatca aatactcctg tcaggagccc tattacaaga tgctcaacaa taacacaggt 1320
   atatatacct gttctgccca aggagtctgg atgaataaag tattggggag aagcctaccc 1380
   acctgccttc cagagtgtgg tcagccctcc cgctccctgc caagcctggt caagaggatc 1440
   attgggggcc gaaatgctga gcctggcctc ttcccgtggc aggccctgat agtggtggag 1500
   gacacttcga gagtgccaaa tgacaagtgg tttgggagtg gggccctgct ctctgcgtcc 1560
   tggatcctca cagcagctca tgtgctgcgc tcccagcgta gagacaccac ggtgatacca 1620
   gtctccaagg agcatgtcac cgtctacctg ggcttgcatg atgtgcgaga caaatcgggg 1680
   gcagtcaaca gctcagctgc ccgagtggtg ctccacccag acttcaacat ccaaaactac 1740
   aaccacgata tagctctggt gcagctgcag gagcctgtgc ccctgggacc ccacgttatg 1800
   cctgtctgcc tgccaaggct tgagcctgaa ggcccggccc cccacatgct gggcctggtg 1860
   gccggctggg gcatctccaa tcccaatgtg acagtggatg agatcatcag cagtggcaca 1920
   cggaccttgt cagatgtcct gcagtatgtc aagttacccg tggtgcctca cgctgagtgc 1980
   aaaactagct atgagtcccg ctcgggcaat tacagcgtca cggagaacat gttctgtgct 2040
   ggctactacg agggcggcaa agacacgtgc cttggagata gcggtggggc ctttgtcatc 2100
   tttgatgact tgagccagcg ctgggtggtg caaggcctgg tgtcctgggg gggacctgaa 2160
   gaatgcggca gcaagcaggt ctatggagtc tacacaaagg tctccaatta cgtggactgg 2220
   gtgtgggagc agatgggctt accacaaagt gttgtggagc cccaggtgga acggtgagct 2280
   gacttacttc ctcggggcct gcctcccctg agcgaagcta caccgcactt ccgacagcac 2340
   actccacatt acttatcaga ccatatggaa tggaacacac tgacctagcg gtggcttctc 2400
   ctaccgagac agcccccagg accctgagag gcagagtgtg gtatagggaa aaggctccag 2460
   gcaggagacc tgtgttcctg agcttgtcca agtctctttc cctgtctggg cctcactcta 2520
   ccgagtaata caatgcagga gctcaaccaa ggcctctgtg ccaatcccag cactcctttc 2580
   caggccatgc ttcttacccc agtggccttt attcactcct gaccacttat caaacccatc 2640
   ggtcctactg ttggtataac tgagcttgga cctgactatt agaaaatggt ttctaacatt 2700
   gaactgaatg ccgcatctgt atattttcct gctctgcctt ctgggactag ccttggccta 2760
   atccttcctc taggagaaga gcattcaggt tttgggagat ggctcatagc caagcccctc 2820
   tctcttagtg tgatcccttg gagcaccttc atgcctgggg tttctctccc aaaagcttct 2880
   tgcagtctaa gccttatccc ttatgttccc cattaaagga atttcaaaag acatggagaa 2940
   agttgggaag gtttgtgctg actgctggga gcagaatagc cgtgggaggc ccaccaagcc 3000
   cttaaattcc cattgtcaac tcagaacaca tttgggccca tatgccaccc tggaacacca 3060
   gctgacacca tgggcgtcca cacctgctgc tccagacaag cacaaagcaa tctttcagcc 3120
   ttgaaatgta ttatctgaaa ggctacctga agcccaggcc cgaatatggg gacttagtcg 3180
   attacctgga aaaagaaaag acccacactg tgtcctgctg tgcttttggg caggaaaatg 3240
   gaagaaagag tggggtgggc acattagaag tcacccaaat cctgccaggc tgcctggcat 3300
   ccctggggca tgagctgggc ggagaatcca ccccgcagga tgttcagagg gacccactcc 3360
   ttcatttttc agagtcaaag gaatcagagg ctcacccatg gcaggcagtg aaaagagcca 3420
   ggagtcctgg gttctagtcc ctgctctgcc cccaactggc tgtataacct ttgaaaaatc 3480
   attttctttg tctgagtctc tggttctccg tcagcaacag gctggcataa ggtcccctgc 3540
   aggttccttc tagctggagc actcagagct tccctgactg ctagcagcct ctctggccct 3600
   cacagggctg attgttctcc ttctccctgg agctctctct cctgaaaatc tccatcagag 3660
   caaggcagcc agagaagccc ctgagaggga atgattggga agtgtccact ttctcaaccg 3720
   gctcatcaaa cacactcctt tgtctatgaa tggcacatgt aaatgatgtt atattttgta 3780
   tcttttatat catatgcttc accattctgt aaagggcctc tgcattgttg ctcccatcag 3840
   gggtctcaag tggaaataaa ccctcgtgga taaccaaaaa aaaaaaaaaa aaaaa 3895
<210> 8
   <211> 728
   <212> PRT
   <213> homo sapiens
<400> 8
   Met Arg Trp Leu Leu Leu Tyr Tyr Ala Leu Cys Phe Ser Leu Ser Lys
   1 5 10 15
   Ala Ser Ala His Thr Val Glu Leu Asn Asn Met Phe Gly Gln Ile Gln
   20 25 30
   Ser Pro Gly Tyr Pro Asp Ser Tyr Pro Ser Asp Ser Glu Val Thr Trp
   35 40 45
   Asn Ile Thr Val Pro Asp Gly Phe Arg Ile Lys Leu Tyr Phe Met His
       50                  55                  60
   Phe Asn Leu Glu Ser Ser Tyr Leu Cys Glu Tyr Asp Tyr Val Lys Val
   65                  70                  75                  80
   Glu Thr Glu Asp Gln Val Leu Ala Thr Phe Cys Gly Arg Glu Thr Thr
                   85                  90                  95
   Asp Thr Glu Gln Thr Pro Gly Gln Glu Val Val Leu Ser Pro Gly Ser
               100                 105                 110
   Phe Met Ser Ile Thr Phe Arg Ser Asp Phe Ser Asn Glu Glu Arg Phe
           115                 120                 125
   Thr Gly Phe Asp Ala His Tyr Met Ala Val Asp Val Asp Glu Cys Lys
       130                 135                 140
   Glu Arg Glu Asp Glu Glu Leu Ser Cys Asp His Tyr Cys His Asn Tyr
   145                 150                 155                 160
   Ile Gly Gly Tyr Tyr Cys Ser Cys Arg Phe Gly Tyr Ile Leu His Thr
                   165                 170                 175
   Asp Asn Arg Thr Cys Arg Val Glu Cys Ser Asp Asn Leu Phe Thr Gln
               180                 185                 190
   Arg Thr Gly Val Ile Thr Ser Pro Asp Phe Pro Asn Pro Tyr Pro Lys
           195                 200                 205
   Ser Ser Glu Cys Leu Tyr Thr Ile Glu Leu Glu Glu Gly Phe Met Val
       210                 215                 220
   Asn Leu Gln Phe Glu Asp Ile Phe Asp Ile Glu Asp His Pro Glu Val
   225                 230                 235                 240
   Pro Cys Pro Tyr Asp Tyr Ile Lys Ile Lys Val Gly Pro Lys Val Leu
                   245                 250                 255
   Gly Pro Phe Cys Gly Glu Lys Ala Pro Glu Pro Ile Ser Thr Gln Ser
               260                 265                 270
   His Ser Val Leu Ile Leu Phe His Ser Asp Asn Ser Gly Glu Asn Arg
           275                 280                 285
   Gly Trp Arg Leu Ser Tyr Arg Ala Ala Gly Asn Glu Cys Pro Glu Leu
       290                 295                 300
   Gln Pro Pro Val His Gly Lys Ile Glu Pro Ser Gln Ala Lys Tyr Phe
   305                 310                 315                 320
   Phe Lys Asp Gln Val Leu Val Ser Cys Asp Thr Gly Tyr Lys Val Leu
                   325                 330                 335
   Lys Asp Asn Val Glu Met Asp Thr Phe Gln Ile Glu Cys Leu Lys Asp
               340                 345                 350
   Gly Thr Trp Ser Asn Lys Ile Pro Thr Cys Lys Ile Val Asp Cys Arg
           355                 360                 365
   Ala Pro Gly Glu Leu Glu His Gly Leu Ile Thr Phe Ser Thr Arg Asn
       370                 375                 380
   Asn Leu Thr Thr Tyr Lys Ser Glu Ile Lys Tyr Ser Cys Gln Glu Pro
   385                 390                 395                 400
   Tyr Tyr Lys Met Leu Asn Asn Asn Thr Gly Ile Tyr Thr Cys Ser Ala
                   405                 410                 415
   Gln Gly Val Trp Met Asn Lys Val Leu Gly Arg Ser Leu Pro Thr Cys
               420                 425                 430
   Leu Pro Glu Cys Gly Gln Pro Ser Arg Ser Leu Pro Ser Leu Val Lys
           435                 440                 445
   Arg Ile Ile Gly Gly Arg Asn Ala Glu Pro Gly Leu Phe Pro Trp Gln
       450                 455                 460
   Ala Leu Ile Val Val Glu Asp Thr Ser Arg Val Pro Asn Asp Lys Trp
   465                 470                 475                 480
   Phe Gly Ser Gly Ala Leu Leu Ser Ala Ser Trp Ile Leu Thr Ala Ala
                   485                 490                 495
   His Val Leu Arg Ser Gln Arg Arg Asp Thr Thr Val Ile Pro Val Ser
               500                 505                 510
   Lys Glu His Val Thr Val Tyr Leu Gly Leu His Asp Val Arg Asp Lys
           515                 520                 525
   Ser Gly Ala Val Asn Ser Ser Ala Ala Arg Val Val Leu His Pro Asp
       530                 535                 540
   Phe Asn Ile Gln Asn Tyr Asn His Asp Ile Ala Leu Val Gln Leu Gln
   545                 550                 555                 560
   Glu Pro Val Pro Leu Gly Pro His Val Met Pro Val Cys Leu Pro Arg
                   565                 570                 575
   Leu Glu Pro Glu Gly Pro Ala Pro His Met Leu Gly Leu Val Ala Gly
               580                 585                 590
   Trp Gly Ile Ser Asn Pro Asn Val Thr Val Asp Glu Ile Ile Ser Ser
           595                 600                 605
   Gly Thr Arg Thr Leu Ser Asp Val Leu Gln Tyr Val Lys Leu Pro Val
       610                 615                 620
   Val Pro His Ala Glu Cys Lys Thr Ser Tyr Glu Ser Arg Ser Gly Asn
   625                 630                 635                 640
   Tyr Ser Val Thr Glu Asn Met Phe Cys Ala Gly Tyr Tyr Glu Gly Gly
                   645                 650                 655
   Lys Asp Thr Cys Leu Gly Asp Ser Gly Gly Ala Phe Val Ile Phe Asp
               660                 665                 670
   Asp Leu Ser Gln Arg Trp Val Val Gln Gly Leu Val Ser Trp Gly Gly
           675                 680                 685
   Pro Glu Glu Cys Gly Ser Lys Gln Val Tyr Gly Val Tyr Thr Lys Val
       690                 695                 700
   Ser Asn Tyr Val Asp Trp Val Trp Glu Gln Met Gly Leu Pro Gln Ser
   705                 710                 715                 720
   Val Val Glu Pro Gln Val Glu Arg
                   725
<210> 9
   <211> 2852
   <212> DNA
   <213> homo sapiens
<400> 9
   tggcgataca ttcacacagg aacagctatg ccatgtttac gaattccggt tttgaaaaaa      60
   ctttcgttga cagttacaca aagggtcact tcctccccag cgacacatgg gcctctcaaa     120
   ggagaggagg gagtaagtcc cacggtaggg ccagtggttg ctccctgggt tttggaatca     180
   tttctgcgga gctttcaagg ccagaccctg ggcttagggt cgagacttta tagcagtgac     240
   agccagaccc agcaagatgg ctgcgaccgt gaaaccctgg gcggcgatcc gggtgcgcat     300
   catgagctga gagcgctggc tgttgccccg gtggaaggag tagaggccgt aggtgagggc     360
   ggccgccgtg gccaggcaac ctatgggtac caccgggttc tcgcgggcaa gtcaagctgg      420
   gaggaccaag gccgggcagc cgggagcacc caaggcagga aaatgaggtg gctgcttctc      480
   tattatgctc tgtgcttctc cctgtcaaag gcttcagccc acaccgtgga gctaaacaat      540
   atgtttggcc agatccagtc gcctggttat ccagactcct atcccagtga ttcagaggtg      600
   acttggaata tcactgtccc agatgggttt cggatcaagc tttacttcat gcacttcaac      660
   ttggaatcct cctacctttg tgaatatgac tatgtgaagg tagaaactga ggaccaggtg      720
   ctggcaacct tctgtggcag ggagaccaca gacacagagc agactcccgg ccaggaggtg      780
   gtcctctccc ctggctcctt catgtccatc actttccggt cagatttctc caatgaggag      840
   cgtttcacag gctttgatgc ccactacatg gctgtggatg tggacgagtg caaggagagg      900
   gaggacgagg agctgtcctg tgaccactac tgccacaact acattggcgg ctactactgc      960
   tcctgccgct tcggctacat cctccacaca gacaacagga cctgccgagt ggagtgcagt     1020
   gacaacctct tcactcaaag gactggggtg atcaccagcc ctgacttccc aaacccttac     1080
   cccaagagct ctgaatgcct gtataccatc gagctggagg agggtttcat ggtcaacctg     1140
   cagtttgagg acatatttga cattgaggac catcctgagg tgccctgccc ctatgactac     1200
   atcaagatca aagttggtcc aaaagttttg ggcctttctg gtggagagaa agccccagaa      1260
   cccatcagca cccagagcca cagtgtcctg atcctgttcc atagtgacaa ctcgggagag     1320
   aaccggggct ggaggctctc atacagggct gcaggaaatg agtgcccaga gctacagcct     1380
   cctgtccatg ggaaaatcga gccctcccaa gccaagtatt tcttcaaaga ccaagtgctc     1440
   gtcagctgtg acacaggcta caaagtgctg aaggataatg tggagatgga cacattccag     1500
   attgagtgtc tgaaggatgg gacgtggagt aacaagattc ccacctgtaa aattgtagac     1560
   tgtagagccc caggagagct ggaacacggg ctgatcacct tctctacaag gaacaacctc     1620
   accacataca agtctgagat caaatactcc tgtcaggagc cctattacaa gatgctcaac     1680
   aataacacag gtatatatac ctgttctgcc caaggagtct ggatgaataa agtattgggg     1740
   agaagcctac ccacctgcct tccagtgtgt gggctcccca agttctcccg gaagctgatg     1800
   gccaggatct tcaatggacg cccagcccag aaaggcacca ctccctggat tgccatgctg     1860
   tcacacctga atgggcagcc cttctgcgga ggctcccttc taggctccag ctggatcgtg     1920
   accgccgcac actgcctcca ccagtcactc gatccgaaag atccgaccct acgtgattca     1980
   gacttgctca gcccttctga cttcaaaatc atcctgggca agcattggag gctccggtca     2040
   gatgaaaatg aacagcatct cggcgtaaaa cacaccactc tccaccccaa gtatgatccc     2100
   aacacattcg agaatgacgt ggctctggtg gagctgttgg agagcccagt gctgaatgcc     2160
   ttcgtgatgc ccatctgtct gcctgaggga ccccagcagg aaggagccat ggtcatcgtc     2220
   agcggctggg gaaagcagtt cttgcaaagg ttcccagaga ccctgatgga gattgaaatc 2280
   ccgattgttg accacagcac ctgccagaag gcttatgccc cgctgaagaa gaaagtgacc 2340
   agggacatga tctgtgctgg ggagaaggaa gggggaaagg acgcctgttc gggtgactct 2400
   ggaggcccca tggtgaccct gaatagagaa agaggccagt ggtacctggt gggcactgtg 2460
   tcctggggtg atgactgtgg gaagaaggac cgctacggag tatactctta catccaccac 2520
   aacaaggact ggatccagag ggtcaccgga gtgaggaact gaatttggct cctcagcccc 2580
   agcaccacca gctgtgggca gtcagtagca gaggacgatc ctccgatgaa agcagccatt 2640
   tctcctttcc ttcctcccat cccccctcct tcggcctatc cattactggg caatagagca 2700
   ggtatcttca cccccttttc actctcttta aagagatgga gcaagagagt ggtcagaaca 2760
   caggccgaat ccaggctcta tcacttacta gttttcagtt ctgggcaggt gacttcatct 2820
   cttcgaactt cagtttcttc ataagatgga aa 2852
<210> 10
   <211> 699
   <212> PRT
   <213> homo sapiens
<400> 10
   Met Arg Trp Leu Leu Leu Tyr Tyr Ala Leu Cys Phe Ser Leu Ser Lys
   1               5                   10                  15
   Ala Ser Ala His Thr Val Glu Leu Asn Asn Met Phe Gly Gln Ile Gln
               20                  25                  30
   Ser Pro Gly Tyr Pro Asp Ser Tyr Pro Ser Asp Ser Glu Val Thr Trp
           35                  40                  45
   Asn Ile Thr Val Pro Asp Gly Phe Arg Ile Lys Leu Tyr Phe Met His
       50                  55                  60
   Phe Asn Leu Glu Ser Ser Tyr Leu Cys Glu Tyr Asp Tyr Val Lys Val
   65                  70                  75                  80
   Glu Thr Glu Asp Gln Val Leu Ala Thr Phe Cys Gly Arg Glu Thr Thr
                   85                  90                  95
   Asp Thr Glu Gln Thr Pro Gly Gln Glu Val Val Leu Ser Pro Gly Ser
               100                 105                 110
   Phe Met Ser Ile Thr Phe Arg Ser Asp Phe Ser Asn Glu Glu Arg Phe
           115                 120                 125
   Thr Gly Phe Asp Ala His Tyr Met Ala Val Asp Val Asp Glu Cys Lys
       130                 135                 140
   Glu Arg Glu Asp Glu Glu Leu Ser Cys Asp His Tyr Cys His Asn Tyr
   145                 150                 155                 160
   Ile Gly Gly Tyr Tyr Cys Ser Cys Arg Phe Gly Tyr Ile Leu His Thr
                   165                 170                 175
   Asp Asn Arg Thr Cys Arg Val Glu Cys Ser Asp Asn Leu Phe Thr Gln
               180                 185                 190
   Arg Thr Gly Val Ile Thr Ser Pro Asp Phe Pro Asn Pro Tyr Pro Lys
           195                 200                 205
   Ser Ser Glu Cys Leu Tyr Thr Ile Glu Leu Glu Glu Gly Phe Met Val
       210                 215                 220
   Asn Leu Gln Phe Glu Asp Ile Phe Asp Ile Glu Asp His Pro Glu Val
   225                 230                 235                 240
   Pro Cys Pro Tyr Asp Tyr Ile Lys Ile Lys Val Gly Pro Lys Val Leu
                   245                 250                 255
   Gly Pro Phe Cys Gly Glu Lys Ala Pro Glu Pro Ile Ser Thr Gln Ser
               260                 265                 270
   His Ser Val Leu Ile Leu Phe His Ser Asp Asn Ser Gly Glu Asn Arg
           275                 280                 285
   Gly Trp Arg Leu Ser Tyr Arg Ala Ala Gly Asn Glu Cys Pro Glu Leu
       290                 295                 300
   Gln Pro Pro Val His Gly Lys Ile Glu Pro Ser Gln Ala Lys Tyr Phe
   305                 310                 315                 320
   Phe Lys Asp Gln Val Leu Val Ser Cys Asp Thr Gly Tyr Lys Val Leu
                   325                 330                 335
   Lys Asp Asn Val Glu Met Asp Thr Phe Gln Ile Glu Cys Leu Lys Asp
               340                 345                 350
   Gly Thr Trp Ser Asn Lys Ile Pro Thr Cys Lys Ile Val Asp Cys Arg
           355                 360                 365
   Ala Pro Gly Glu Leu Glu His Gly Leu Ile Thr Phe Ser Thr Arg Asn
       370                 375                 380
   Asn Leu Thr Thr Tyr Lys Ser Glu Ile Lys Tyr Ser Cys Gln Glu Pro
   385                 390                 395                 400
   Tyr Tyr Lys Met Leu Asn Asn Asn Thr Gly Ile Tyr Thr Cys Ser Ala
                   405                 410                 415
   Gln Gly Val Trp Met Asn Lys Val Leu Gly Arg Ser Leu Pro Thr Cys
               420                 425                 430
   Leu Pro Val Cys Gly Leu Pro Lys Phe Ser Arg Lys Leu Met Ala Arg
           435                 440                 445
   Ile Phe Asn Gly Arg Pro Ala Gln Lys Gly Thr Thr Pro Trp Ile Ala
       450                 455                 460
   Met Leu Ser His Leu Asn Gly Gln Pro Phe Cys Gly Gly Ser Leu Leu
   465                 470                 475                 480
   Gly Ser Ser Trp Ile Val Thr Ala Ala His Cys Leu His Gln Ser Leu
                   485                 490                 495
   Asp Pro Lys Asp Pro Thr Leu Arg Asp Ser Asp Leu Leu Ser Pro Ser
               500                 505                 510
   Asp Phe Lys Ile Ile Leu Gly Lys His Trp Arg Leu Arg Ser Asp Glu
           515                 520                 525
   Asn Glu Gln His Leu Gly Val Lys His Thr Thr Leu His Pro Lys Tyr
       530                 535                 540
   Asp Pro Asn Thr Phe Glu Asn Asp Val Ala Leu Val Glu Leu Leu Glu
   545                 550                 555                 560
   Ser Pro Val Leu Asn Ala Phe Val Met Pro Ile Cys Leu Pro Glu Gly
                   565                 570                 575
   Pro Gln Gln Glu Gly Ala Met Val Ile Val Ser Gly Trp Gly Lys Gln
               580                 585                 590
   Phe Leu Gln Arg Phe Pro Glu Thr Leu Met Glu Ile Glu Ile Pro Ile
           595                 600                 605
   Val Asp His Ser Thr Cys Gln Lys Ala Tyr Ala Pro Leu Lys Lys Lys
       610                 615                 620
   Val Thr Arg Asp Met Ile Cys Ala Gly Glu Lys Glu Gly Gly Lys Asp
   625                 630                 635                 640
   Ala Cys Ser Gly Asp Ser Gly Gly Pro Met Val Thr Leu Asn Arg Glu
                   645                 650                 655
   Arg Gly Gln Trp Tyr Leu Val Gly Thr Val Ser Trp Gly Asp Asp Cys
               660                 665                 670
   Gly Lys Lys Asp Arg Tyr Gly Val Tyr Ser Tyr Ile His His Asn Lys
           675                 680                 685
   Asp Trp Ile Gln Arg Val Thr Gly Val Arg Asn
       690                 695
<210> 11
   <211> 380
   <212> PRT
   <213> homo sapiens
<400> 11
   Met Arg Trp Leu Leu Leu Tyr Tyr Ala Leu Cys Phe Ser Leu Ser Lys
   1               5                   10                  15
   Ala Ser Ala His Thr Val Glu Leu Asn Asn Met Phe Gly Gln Ile Gln
               20                  25                  30
   Ser Pro Gly Tyr Pro Asp Ser Tyr Pro Ser Asp Ser Glu Val Thr Trp
           35                  40                  45
   Asn Ile Thr Val Pro Asp Gly Phe Arg Ile Lys Leu Tyr Phe Met His
       50                  55                  60
   Phe Asn Leu Glu Ser Ser Tyr Leu Cys Glu Tyr Asp Tyr Val Lys Val
   65                  70                  75                  80
   Glu Thr Glu Asp Gln Val Leu Ala Thr Phe Cys Gly Arg Glu Thr Thr
                   85                  90                  95
   Asp Thr Glu Gln Thr Pro Gly Gln Glu Val Val Leu Ser Pro Gly Ser
               100                 105                 110
   Phe Met Ser Ile Thr Phe Arg Ser Asp Phe Ser Asn Glu Glu Arg Phe
           115                 120                 125
   Thr Gly Phe Asp Ala His Tyr Met Ala Val Asp Val Asp Glu Cys Lys
       130                 135                 140
   Glu Arg Glu Asp Glu Glu Leu Ser Cys Asp His Tyr Cys His Asn Tyr
   145                 150                 155                 160
   Ile Gly Gly Tyr Tyr Cys Ser Cys Arg Phe Gly Tyr Ile Leu His Thr
                   165                 170                 175
   Asp Asn Arg Thr Cys Arg Val Glu Cys Ser Asp Asn Leu Phe Thr Gln
               180                 185                 190
   Arg Thr Gly Val Ile Thr Ser Pro Asp Phe Pro Asn Pro Tyr Pro Lys
           195                 200                 205
   Ser Ser Glu Cys Leu Tyr Thr Ile Glu Leu Glu Glu Gly Phe Met Val
       210                 215                 220
   Asn Leu Gln Phe Glu Asp Ile Phe Asp Ile Glu Asp His Pro Glu Val
   225                 230                 235                 240
   Pro Cys Pro Tyr Asp Tyr Ile Lys Ile Lys Val Gly Pro Lys Val Leu
                   245                 250                 255
   Gly Pro Phe Cys Gly Glu Lys Ala Pro Glu Pro Ile Ser Thr Gln Ser
               260                 265                 270
   His Ser Val Leu Ile Leu Phe His Ser Asp Asn Ser Gly Glu Asn Arg
           275                 280                 285
   Gly Trp Arg Leu Ser Tyr Arg Ala Ala Gly Asn Glu Cys Pro Glu Leu
       290                 295                 300
   Gln Pro Pro Val His Gly Lys Ile Glu Pro Ser Gln Ala Lys Tyr Phe
   305                 310                 315                 320
   Phe Lys Asp Gln Val Leu Val Ser Cys Asp Thr Gly Tyr Lys Val Leu
                   325                 330                 335
   Lys Asp Asn Val Glu Met Asp Thr Phe Gln Ile Glu Cys Leu Lys Asp
               340                 345                 350
   Gly Thr Trp Ser Asn Lys Ile Pro Thr Cys Lys Lys Asn Glu Ile Asp
           355                 360                 365
   Leu Glu Ser Glu Leu Lys Ser Glu Gln Val Thr Glu
       370                 375                 380
<210> 12
   <211> 2091
   <212> DNA
   <213> rattus
<400> 12
   tggcacacaa tgaggctact gatcgtcctg ggtctgcttt ggagtttggt ggccacactt      60
   ttgggctcca agtggcctga gcctgtattc gggcgcctgg tgtccctggc cttcccagag     120
   aagtatggca accatcagga tcgatcctgg acgctgactg caccccctgg cttccgcctg      180
   cgcctctact tcacccactt caacctggaa ctctcttacc gctgcgagta tgactttgtc     240
   aagttgacct cagggaccaa ggtgctagcc acgctgtgtg ggcaggagag tacagatact     300
   gagcgggcac ctggcaatga caccttctac tcactgggtc ccagcctaaa ggtcaccttc     360
   cactccgact actccaatga gaagccattc acaggatttg aggccttcta tgcagcggag     420
   gatgtggatg aatgcagaac atccctggga gactcagtcc cttgtgacca ttattgccac     480
   aactacctgg gcggctacta ctgctcctgc cgagtgggct acattctgca ccagaacaag     540
   catacctgct cagccctttg ttcaggccag gtgttcactg ggaggtctgg ctttctcagt     600
   agccctgagt acccacagcc ataccccaaa ctctccagct gcgcctacaa catccgcctg     660
   gaggaaggct tcagtatcac cctggacttc gtggagtcct ttgatgtgga gatgcaccct     720
   gaagcccagt gcccctacga ctccctcaag attcaaacag acaagaggga atacggcccg     780
   ttttgtggga agacgctgcc ccccaggatt gaaactgaca gcaacaaggt gaccattacc     840
   tttaccaccg acgagtcagg gaaccacaca ggctggaaga tacactacac aagcacagca     900
   cagccctgcc ctgatccaac ggcgccacct aatggtcaca tttcacctgt gcaagccacg     960
   tatgtcctga aggacagctt ttctgtcttc tgcaagactg gcttcgagct tctgcaaggt    1020
   tctgtccccc tgaagtcatt cactgctgtc tgtcagaaag atggatcttg ggaccggccg    1080
   ataccagagt gcagcattat tgactgtggc cctcccgatg acctacccaa tggccacgtg    1140
   gactatatca caggccctga agtgaccacc tacaaagctg tgattcagta cagctgtgaa    1200
   gagactttct acacaatgag cagcaatggt aaatatgtgt gtgaggctga tggattctgg    1260
   acgagctcca aaggagaaaa atccctcccg gtttgcaagc ctgtctgtgg actgtccaca    1320
   cacacttcag gaggccgtat aattggagga cagcctgcaa agcctggtga ctttccttgg    1380
   caagtcttgt tactgggtga aactacagca gcaggtgctc ttatacatga cgactgggtc    1440
   ctaacagcgg ctcatgctgt atatgggaaa acagaggcga tgtcctccct ggacatccgc    1500
   atgggcatcc tcaaaaggct ctccctcatt tacactcaag cctggccaga ggctgtcttt    1560
   atccatgaag gctacactca cggagctggt tttgacaatg atatagcact gattaaactc    1620
   aagaacaaag tcacaatcaa cagaaacatc atgccgattt gtctaccaag aaaagaagct    1680
   gcatccttaa tgaaaacaga cttcgttgga actgtggctg gctgggggtt aacccagaag    1740
   gggtttcttg ctagaaacct aatgtttgtg gacataccaa ttgttgacca ccaaaaatgt    1800
   gctactgcgt atacaaagca gccctaccca ggagcaaaag tgactgttaa catgctctgt    1860
   gctggcctag accgcggtgg caaggacagc tgcagaggtg acagcggagg ggcattagtg    1920
   tttctagaca atgaaacaca gagatggttt gtgggaggaa tagtttcctg gggttctatt 1980
   aactgtgggg ggtcagaaca gtatggggtc tacacgaaag tcacgaacta tattccctgg 2040
   attgagaaca taataaataa tttctaattt gcaaaaaaaa aaaaaaaaaa a 2091
<210> 13
   <211> 685
   <212> PRT
   <213> rattus
<400> 13
   Met Arg Leu Leu Ile Val Leu Gly Leu Leu Trp Ser Leu Val Ala Thr
   1               5                   10                  15
   Leu Leu Gly Ser Lys Trp Pro Glu Pro Val Phe Gly Arg Leu Val Ser
               20                  25                  30
   Leu Ala Phe Pro Glu Lys Tyr Gly Asn His Gln Asp Arg Ser Trp Thr
           35                  40                  45
   Leu Thr Ala Pro Pro Gly Phe Arg Leu Arg Leu Tyr Phe Thr His Phe
       50                  55                  60
   Asn Leu Glu Leu Ser Tyr Arg Cys Glu Tyr Asp Phe Val Lys Leu Thr
   65                  70                  75                  80
   Ser Gly Thr Lys Val Leu Ala Thr Leu Cys Gly Gln Glu Ser Thr Asp
                   85                  90                  95
   Thr Glu Arg Ala Pro Gly Asn Asp Thr Phe Tyr Ser Leu Gly Pro Ser
               100                 105                 110
   Leu Lys Val Thr Phe His Ser Asp Tyr Ser Asn Glu Lys Pro Phe Thr
           115                 120                 125
   Gly Phe Glu Ala Phe Tyr Ala Ala Glu Asp Val Asp Glu Cys Arg Thr
       130                 135                 140
   Ser Leu Gly Asp Ser Val Pro Cys Asp His Tyr Cys His Asn Tyr Leu
   145                 150                 155                 160
   Gly Gly Tyr Tyr Cys Ser Cys Arg Val Gly Tyr Ile Leu His Gln Asn
                   165                 170                 175
   Lys His Thr Cys Ser Ala Leu Cys Ser Gly Gln Val Phe Thr Gly Arg
               180                 185                 190
   Ser Gly Phe Leu Ser Ser Pro Glu Tyr Pro Gln Pro Tyr Pro Lys Leu
           195                 200                 205
   Ser Ser Cys Ala Tyr Asn Ile Arg Leu Glu Glu Gly Phe Ser Ile Thr
       210                 215                 220
   Leu Asp Phe Val Glu Ser Phe Asp Val Glu Met His Pro Glu Ala Gln
   225                 230                 235                 240
   Cys Pro Tyr Asp Ser Leu Lys Ile Gln Thr Asp Lys Arg Glu Tyr Gly
                   245                 250                 255
   Pro Phe Cys Gly Lys Thr Leu Pro Pro Arg Ile Glu Thr Asp Ser Asn
               260                 265                 270
   Lys Val Thr Ile Thr Phe Thr Thr Asp Glu Ser Gly Asn His Thr Gly
           275                 280                 285
   Trp Lys Ile His Tyr Thr Ser Thr Ala Gln Pro Cys Pro Asp Pro Thr
       290                 295                 300
   Ala Pro Pro Asn Gly His Ile Ser Pro Val Gln Ala Thr Tyr Val Leu
   305                 310                 315                 320
   Lys Asp Ser Phe Ser Val Phe Cys Lys Thr Gly Phe Glu Leu Leu Gln
                   325                 330                 335
   Gly Ser Val Pro Leu Lys Ser Phe Thr Ala Val Cys Gln Lys Asp Gly
               340                 345                 350
   Ser Trp Asp Arg Pro Ile Pro Glu Cys Ser Ile Ile Asp Cys Gly Pro
           355                 360                 365
   Pro Asp Asp Leu Pro Asn Gly His Val Asp Tyr Ile Thr Gly Pro Glu
       370                 375                 380
   Val Thr Thr Tyr Lys Ala Val Ile Gln Tyr Ser Cys Glu Glu Thr Phe
   385                 390                 395                 400
   Tyr Thr Met Ser Ser Asn Gly Lys Tyr Val Cys Glu Ala Asp Gly Phe
                   405                 410                 415
   Trp Thr Ser Ser Lys Gly Glu Lys Ser Leu Pro Val Cys Lys Pro Val
               420                 425                 430
   Cys Gly Leu Ser Thr His Thr Ser Gly Gly Arg Ile Ile Gly Gly Gln
           435                 440                 445
   Pro Ala Lys Pro Gly Asp Phe Pro Trp Gln Val Leu Leu Leu Gly Glu
       450                 455                 460
   Thr Thr Ala Ala Gly Ala Leu Ile His Asp Asp Trp Val Leu Thr Ala
   465                 470                 475                 480
   Ala His Ala Val Tyr Gly Lys Thr Glu Ala Met Ser Ser Leu Asp Ile
                   485                 490                 495
   Arg Met Gly Ile Leu Lys Arg Leu Ser Leu Ile Tyr Thr Gln Ala Trp
               500                 505                 510
   Pro Glu Ala Val Phe Ile His Glu Gly Tyr Thr His Gly Ala Gly Phe
           515                 520                 525
   Asp Asn Asp Ile Ala Leu Ile Lys Leu Lys Asn Lys Val Thr Ile Asn
       530                 535                 540
   Arg Asn Ile Met Pro Ile Cys Leu Pro Arg Lys Glu Ala Ala Ser Leu
   545                 550                 555                 560
   Met Lys Thr Asp Phe Val Gly Thr Val Ala Gly Trp Gly Leu Thr Gln
                   565                 570                 575
   Lys Gly Phe Leu Ala Arg Asn Leu Met Phe Val Asp Ile Pro Ile Val
               580                 585                 590
   Asp His Gln Lys Cys Ala Thr Ala Tyr Thr Lys Gln Pro Tyr Pro Gly
           595                 600                 605
   Ala Lys Val Thr Val Asn Met Leu Cys Ala Gly Leu Asp Arg Gly Gly
       610                 615                 620
   Lys Asp Ser Cys Arg Gly Asp Ser Gly Gly Ala Leu Val Phe Leu Asp
   625                 630                 635                 640
   Asn Glu Thr Gln Arg Trp Phe Val Gly Gly Ile Val Ser Trp Gly Ser
                   645                 650                 655
   Ile Asn Cys Gly Gly Ser Glu Gln Tyr Gly Val Tyr Thr Lys Val Thr
               660                 665                 670
   Asn Tyr Ile Pro Trp Ile Glu Asn Ile Ile Asn Asn Phe
           675                 680                 685
<210> 14
   <211> 354
   <212> DNA
   <213> artificial sequence
<220>
   <223> Synthetic
<400> 14
   caggtcacct tgaaggagtc tggtcctgtg ctggtgaaac ccacagagac cctcacgctg       60
   acctgcaccg tctctgggtt ctcactcagc aggggtaaaa tgggtgtgag ctggatccgt      120
   cagcccccag ggaaggccct ggagtggctt gcacacattt tttcgagtga cgaaaaatcc      180
   tacaggacat cgctgaagag caggctcacc atctccaagg acacctccaa aaaccaggtg      240
   gtccttacaa tgaccaacat ggaccctgtg gacacagcca cgtattactg tgcacggata      300
   cgacgtggag gaattgacta ctggggccag ggaaccctgg tcactgtctc ctca            354
<210> 15
   <211> 118
   <212> PRT
   <213> artificial sequence
<220>
   <223> Synthetic
<400> 15
   Gln Val Thr Leu Lys Glu Ser Gly Pro Val Leu Val Lys Pro Thr Glu
   1               5                   10                  15
   Thr Leu Thr Leu Thr Cys Thr Val Ser Gly Phe Ser Leu Ser Arg Gly
               20                  25                  30
   Lys Met Gly Val Ser Trp Ile Arg Gln Pro Pro Gly Lys Ala Leu Glu
           35                  40                  45
   Trp Leu Ala His Ile Phe Ser Ser Asp Glu Lys Ser Tyr Arg Thr Ser
       50                  55                  60
   Leu Lys Ser Arg Leu Thr Ile Ser Lys Asp Thr Ser Lys Asn Gln Val
   65                  70                  75                  80
   Val Leu Thr Met Thr Asn Met Asp Pro Val Asp Thr Ala Thr Tyr Tyr
                   85                  90                  95
   Cys Ala Arg Ile Arg Arg Gly Gly Ile Asp Tyr Trp Gly Gln Gly Thr
               100                 105                 110
   Leu Val Thr Val Ser Ser
           115
<210> 16
   <211> 121
   <212> PRT
   <213> artificial sequence
<220>
   <223> Synthetic
<400> 16
   Gln Val Gln Leu Gln Gln Ser Gly Pro Gly Leu Val Lys Pro Ser Gln
   1               5                   10                  15
   Thr Leu Ser Leu Thr Cys Ala Ile Ser Gly Asp Ser Val Ser Ser Thr
               20                  25                  30
   Ser Ala Ala Trp Asn Trp Ile Arg Gln Ser Pro Ser Arg Gly Leu Glu
           35                  40                  45
   Trp Leu Gly Arg Thr Tyr Tyr Arg Ser Lys Trp Tyr Asn Asp Tyr Ala
       50                  55                  60
   Val Ser Val Lys Ser Arg Ile Thr Ile Asn Pro Asp Thr Ser Lys Asn
   65                  70                  75                  80
   Gln Phe Ser Leu Gln Leu Asn Ser Val Thr Pro Glu Asp Thr Ala Val
                   85                  90                  95
   Tyr Tyr Cys Ala Arg Asp Pro Phe Gly Val Pro Phe Asp Ile Trp Gly
               100                 105                 110
   Gln Gly Thr Met Val Thr Val Ser Ser
           115                 120
<210> 17
   <211> 106
   <212> PRT
   <213> artificial sequence
<220>
   <223> Synthetic
<400> 17
   Gln Pro Val Leu Thr Gln Pro Pro Ser Leu Ser Val Ser Pro Gly Gln
   1               5                   10                  15
   Thr Ala Ser Ile Thr Cys Ser Gly Glu Lys Leu Gly Asp Lys Tyr Ala
               20                  25                  30
   Tyr Trp Tyr Gln Gln Lys Pro Gly Gln Ser Pro Val Leu Val Met Tyr
           35                  40                  45
   Gln Asp Lys Gln Arg Pro Ser Gly Ile Pro Glu Arg Phe Ser Gly Ser
   50 55 60
   Asn Ser Gly Asn Thr Ala Thr Leu Thr Ile Ser Gly Thr Gln Ala Met
   65 70 75 80
   Asp Glu Ala Asp Tyr Tyr Cys Gln Ala Trp Asp Ser Ser Thr Ala Val
   85 90 95
   Phe Gly Gly Gly Thr Lys Leu Thr Val Leu
   100 105
<210> 18
   <211> 324
   <212> DNA
   <213> artificial sequence
<220>
   <223> Synthetic
<400> 18
   tcctatgagc tgatacagcc accctcggtg tcagtggccc caggacagac ggccaccatt 60
   acctgtgcgg gagacaacct tgggaagaaa cgtgtgcact ggtaccagca gaggccaggc 120
   caggcccctg tgttggtcat ctatgatgat agcgaccggc cctcagggat ccctgaccga 180
   ttctctgcct ccaactctgg gaacacggcc accctgacca tcactagggg cgaagccggg 240
   gatgaggccg actattattg tcaggtgtgg gacattgcta ctgatcatgt ggtcttcggc 300
   ggagggacca agctcaccgt ccta 324
<210> 19
   <211> 120
   <212> PRT
   <213> artificial sequence
<220>
   <223> Synthetic
<400> 19
   Ser Tyr Glu Leu Ile Gln Pro Pro Ser Val Ser Val Ala Pro Gly Gln
   1               5                   10                  15
   Thr Ala Thr Ile Thr Cys Ala Gly Asp Asn Leu Gly Lys Lys Arg Val
               20                  25                  30
   His Trp Tyr Gln Gln Arg Pro Gly Gln Ala Pro Val Leu Val Ile Tyr
           35                  40                  45
   Asp Asp Ser Asp Arg Pro Ser Gly Ile Pro Asp Arg Phe Ser Ala Ser
       50                  55                  60
   Asn Ser Gly Asn Thr Ala Thr Leu Thr Ile Thr Arg Gly Glu Ala Gly
   65                  70                  75                  80
   Asp Glu Ala Asp Tyr Tyr Cys Gln Val Trp Asp Ile Ala Thr Asp His
                   85                  90                  95
   Val Val Phe Gly Gly Gly Thr Lys Leu Thr Val Leu Ala Ala Ala Gly
               100                 105                 110
   Ser Glu Gln Lys Leu Ile Ser Glu
           115                 120
<210> 20
   <211> 262
   <212> PRT
   <213> artificial sequence
<220>
   <223> Synthetic
<400> 20
   Gln Val Gln Leu Gln Gln Ser Gly Pro Gly Leu Val Lys Pro Ser Gln
   1               5                   10                  15
   Thr Leu Ser Leu Thr Cys Ala Ile Ser Gly Asp Ser Val Ser Ser Thr
               20                  25                  30
   Ser Ala Ala Trp Asn Trp Ile Arg Gln Ser Pro Ser Arg Gly Leu Glu
           35                  40                  45
   Trp Leu Gly Arg Thr Tyr Tyr Arg Ser Lys Trp Tyr Asn Asp Tyr Ala
       50                  55                  60
   Val Ser Val Lys Ser Arg Ile Thr Ile Asn Pro Asp Thr Ser Lys Asn
   65                  70                  75                  80
   Gln Phe Ser Leu Gln Leu Asn Ser Val Thr Pro Glu Asp Thr Ala Val
                   85                  90                  95
   Tyr Tyr Cys Ala Arg Asp Pro Phe Gly Val Pro Phe Asp Ile Trp Gly
               100                 105                 110
   Gln Gly Thr Met Val Thr Val Ser Ser Lys Leu Ser Gly Ser Ala Ser
           115                 120                 125
   Ala Pro Lys Leu Glu Glu Gly Glu Phe Ser Glu Ala Arg Val Ser Tyr
       130                 135                 140
   Glu Leu Ile Gln Pro Pro Ser Val Ser Val Ala Pro Gly Gln Thr Ala
   145                 150                 155                 160
   Thr Ile Thr Cys Ala Gly Asp Asn Leu Gly Lys Lys Arg Val His Trp
                   165                 170                 175
   Tyr Gln Gln Arg Pro Gly Gln Ala Pro Val Leu Val Ile Tyr Asp Asp
               180                 185                 190
   Ser Asp Arg Pro Ser Gly Ile Pro Asp Arg Phe Ser Ala Ser Asn Ser
           195                 200                 205
   Gly Asn Thr Ala Thr Leu Thr Ile Thr Arg Gly Glu Ala Gly Asp Glu
       210                 215                 220
   Ala Asp Tyr Tyr Cys Gln Val Trp Asp Ile Ala Thr Asp His Val Val
   225                 230                 235                 240
   Phe Gly Gly Gly Thr Lys Leu Thr Val Leu Ala Ala Ala Gly Ser Glu
                   245                 250                 255
   Gln Lys Leu Ile Ser Glu
               260
<210> 21
   <211> 245
   <212> PRT
   <213> artificial sequence
<220>
   <223> Synthetic
<400> 21
   Gln Val Thr Leu Lys Glu Ser Gly Pro Val Leu Val Lys Pro Thr Glu
   1               5                   10                  15
   Thr Leu Thr Leu Thr Cys Thr Val Ser Gly Phe Ser Leu Ser Arg Gly
               20                  25                  30
   Lys Met Gly Val Ser Trp Ile Arg Gln Pro Pro Gly Lys Ala Leu Glu
           35                  40                  45
   Trp Leu Ala His Ile Phe Ser Ser Asp Glu Lys Ser Tyr Arg Thr Ser
       50                  55                  60
   Leu Lys Ser Arg Leu Thr Ile Ser Lys Asp Thr Ser Lys Asn Gln Val
   65                  70                  75                  80
   Val Leu Thr Met Thr Asn Met Asp Pro Val Asp Thr Ala Thr Tyr Tyr
                   85                  90                  95
   Cys Ala Arg Ile Arg Arg Gly Gly Ile Asp Tyr Trp Gly Gln Gly Thr
               100                 105                 110
   Leu Val Thr Val Ser Ser Lys Leu Ser Gly Ser Ala Ser Ala Pro Lys
           115                 120                 125
   Leu Glu Glu Gly Glu Phe Ser Glu Ala Arg Val Gln Pro Val Leu Thr
       130                 135                 140
   Gln Pro Pro Ser Leu Ser Val Ser Pro Gly Gln Thr Ala Ser Ile Thr
   145                 150                 155                 160
   Cys Ser Gly Glu Lys Leu Gly Asp Lys Tyr Ala Tyr Trp Tyr Gln Gln
                   165                 170                 175
   Lys Pro Gly Gln Ser Pro Val Leu Val Met Tyr Gln Asp Lys Gln Arg
               180                 185                 190
   Pro Ser Gly Ile Pro Glu Arg Phe Ser Gly Ser Asn Ser Gly Asn Thr
           195                 200                 205
   Ala Thr Leu Thr Ile Ser Gly Thr Gln Ala Met Asp Glu Ala Asp Tyr
       210                 215                 220
   Tyr Cys Gln Ala Trp Asp Ser Ser Thr Ala Val Phe Gly Gly Gly Thr
   225                 230                 235                 240
   Lys Leu Thr Val Leu
                   245
<210> 22
   <211> 750
   <212> DNA
   <213> artificial sequence
<220>
   <223> Synthetic
<400> 22
   caggtacagc tgcagcagtc aggtccagga ctggtgaagc cctcgcagac cctctcactc        60
   acctgtgcca tctccgggga cagtgtctct agcaccagtg ctgcttggaa ctggatcagg       120
   cagtccccat cgagaggcct tgagtggctg ggaaggacat actacaggtc caagtggtat       180
   aatgattatg cagtatctgt gaaaagtcga ataaccatca acccagacac atccaagaac       240
   cagttctccc tgcagctgaa ctctgtgact cccgaggaca cggctgtgta ttactgtgca       300
   agagatcctt tcggggtacc ttttgatatc tggggccaag ggacaatggt caccgtctct       360
   tcaaagcttt cagggagtgc atccgcccca aaacttgaag aaggtgaatt ttcagaagca       420
   cgcgtatcct atgagctgat acagccaccc tcggtgtcag tggccccagg acagacggcc       480
   accattacct gtgcgggaga caaccttggg aagaaacgtg tgcactggta ccagcagagg       540
   ccaggccagg cccctgtgtt ggtcatctat gatgatagcg accggccctc agggatccct       600
   gaccgattct ctgcctccaa ctctgggaac acggccaccc tgaccatcac taggggcgaa       660
   gccggggatg aggccgacta ttattgtcag gtgtgggaca ttgctactga tcatgtggtc       720
   ttcggcggag ggaccaagct caccgtccta                                        750
<210> 23
   <211> 735
   <212> DNA
   <213> artificial sequence
<220>
   <223> Synthetic
<220>
   <221> misc_feature
   <222> (663)..(663)
   <223> n is a, c, g, or t
<400> 23
   caggtcacct tgaaggagtc tggtcctgtg ctggtgaaac ccacagagac cctcacgctg      60
   acctgcaccg tctctgggtt ctcactcagc aggggtaaaa tgggtgtgag ctggatccgt     120
   cagcccccag ggaaggccct ggagtggctt gcacacattt tttcgagtga cgaaaaatcc     180
   tacaggacat cgctgaagag caggctcacc atctccaagg acacctccaa aaaccaggtg     240
   gtccttacaa tgaccaacat ggaccctgtg gacacagcca cgtattactg tgcacggata     300
   cgacgtggag gaattgacta ctggggccag ggaaccctgg tcactgtctc ctcaaagctt     360
   tcagggagtg catccgcccc aaaacttgaa gaaggtgaat tttcagaagc acgcgtacag     420
   ccagtgctga ctcagccccc ctcactgtcc gtgtccccag gacagacagc cagcatcacc     480
   tgctctggag agaaattggg ggataaatat gcttactggt atcagcagaa gccaggccag     540
   tcccctgtgt tggtcatgta tcaagataaa cagcggccct cagggatccc tgagcgattc     600
   tctggctcca actctgggaa cacagccact ctgaccatca gcgggaccca ggctatggat     660
   gangctgact attactgtca ggcgtgggac agcagcactg cggtattcgg cggagggacc     720
   aagctgaccg tccta                                                      735
<210> 24
   <211> 125
   <212> PRT
   <213> artificial sequence
<220>
   <223> Synthetic
<400> 24
   Ala Val Thr Leu Asp Glu Ser Gly Gly Gly Leu Gln Thr Pro Gly Gly
   1               5                   10                  15
   Ala Leu Ser Leu Val Cys Lys Ala Ser Gly Phe Thr Phe Ser Ser Asn
               20                  25                  30
   Ala Met Gly Trp Val Arg Gln Ala Pro Gly Lys Gly Leu Glu Trp Val
           35                  40                  45
   Ala Gly Ile Asp Asp Asp Gly Ser Gly Thr Arg Tyr Ala Pro Ala Val
       50                  55                  60
   Lys Gly Arg Ala Thr Ile Ser Arg Asp Asn Gly Gln Ser Thr Leu Arg
   65                  70                  75                  80
   Leu Gln Leu Asn Asn Leu Arg Ala Glu Asp Thr Gly Thr Tyr Tyr Cys
                   85                  90                  95
   Thr Lys Cys Ala Tyr Ser Ser Gly Cys Asp Tyr Glu Gly Gly Tyr Ile
               100                 105                 110
   Asp Ala Trp Gly His Gly Thr Glu Val Ile Val Ser Ser
           115                 120                 125
<210> 25
   <211> 118
   <212> PRT
   <213> artificial sequence
<220>
   <223> Synthetic
<400> 25
   Ala Val Thr Leu Asp Glu Ser Gly Gly Gly Leu Gln Thr Pro Gly Gly
   1               5                   10                  15
   Gly Leu Ser Leu Val Cys Lys Ala Ser Gly Phe Thr Phe Ser Ser Tyr
               20                  25                  30
   Ala Met Gly Trp Met Arg Gln Ala Pro Gly Lys Gly Leu Glu Tyr Val
           35                  40                  45
   Ala Gly Ile Arg Ser Asp Gly Ser Phe Thr Leu Tyr Ala Thr Ala Val
       50                  55                  60
   Lys Gly Arg Ala Thr Ile Ser Arg Asp Asn Gly Gln Ser Thr Val Arg
   65 70 75 80
   Leu Gln Leu Asn Asn Leu Arg Ala Glu Asp Thr Ala Thr Tyr Phe Cys
   85 90 95
   Thr Arg Ser Gly Asn Val Gly Asp Ile Asp Ala Trp Gly His Gly Thr
   100 105 110
   Glu Val Ile Val Ser Ser
   115
<210> 26
   <211> 128
   <212> PRT
   <213> artificial sequence
<220>
   <223> Synthetic
<400> 26
   Ala Val Thr Leu Asp Glu Ser Gly Gly Gly Leu Gln Thr Pro Gly Gly
   1 5 10 15
   Gly Leu Ser Leu Val Cys Lys Ala Ser Gly Phe Asp Phe Ser Ser Tyr
   20 25 30
   Gln Met Asn Trp Ile Arg Gln Ala Pro Gly Lys Gly Leu Glu Phe Val
   35 40 45
   Ala Ala Ile Asn Arg Phe Gly Asn Ser Thr Gly His Gly Ala Ala Val
   50 55 60
   Lys Gly Arg Val Thr Ile Ser Arg Asp Asp Gly Gln Ser Thr Val Arg
   65 70 75 80
   Leu Gln Leu Ser Asn Leu Arg Ala Glu Asp Thr Ala Thr Tyr Tyr Cys
   85 90 95
   Ala Lys Gly Val Tyr Gly Tyr Cys Gly Ser Tyr Ser Cys Cys Gly Val
   100 105 110
   Asp Thr Ile Asp Ala Trp Gly His Gly Thr Glu Val Ile Val Ser Ser
   115 120 125
<210> 27
   <211> 107
   <212> PRT
   <213> artificial sequence
<220>
   <223> Synthetic
<400> 27
   Ala Leu Thr Gln Pro Ala Ser Val Ser Ala Asn Leu Gly Gly Thr Val
   1               5                   10                  15
   Lys Ile Thr Cys Ser Gly Gly Gly Ser Tyr Ala Gly Ser Tyr Tyr Tyr
               20                  25                  30
   Gly Trp Tyr Gln Gln Lys Ser Pro Gly Ser Ala Pro Val Thr Val Ile
           35                  40                  45
   Tyr Asp Asn Asp Lys Arg Pro Ser Asp Ile Pro Ser Arg Phe Ser Gly
       50                  55                  60
   Ser Leu Ser Gly Ser Thr Asn Thr Leu Thr Ile Thr Gly Val Arg Ala
   65                  70                  75                  80
   Asp Asp Glu Ala Val Tyr Phe Cys Gly Ser Ala Asp Asn Ser Gly Ala
                   85                  90                  95
   Ala Phe Gly Ala Gly Thr Thr Leu Thr Val Leu
               100                 105
<210> 28
   <211> 108
   <212> PRT
   <213> artificial sequence
<220>
   <223> Synthetic
<400> 28
   Ala Leu Thr Gln Pro Ala Ser Val Ser Ala Asn Pro Gly Glu Thr Val
   1 5 10 15
   Lys Ile Thr Cys Ser Gly Gly Tyr Ser Gly Tyr Ala Gly Ser Tyr Tyr
   20 25 30
   Tyr Gly Trp Tyr Gln Gln Lys Ala Pro Gly Ser Ala Pro Val Thr Leu
   35 40 45
   Ile Tyr Tyr Asn Asn Lys Arg Pro Ser Asp Ile Pro Ser Arg Phe Ser
   50 55 60
   Gly Ser Leu Ser Gly Ser Thr Asn Thr Leu Thr Ile Thr Gly Val Arg
   65 70 75 80
   Ala Asp Asp Glu Ala Val Tyr Phe Cys Gly Ser Ala Asp Asn Ser Gly
   85 90 95
   Ala Ala Phe Gly Ala Gly Thr Thr Leu Thr Val Leu
   100 105
<210> 29
   <211> 107
   <212> PRT
   <213> artificial sequence
<220>
   <223> Synthetic
<400> 29
   Ala Leu Thr Gln Pro Ala Ser Val Ser Ala Asn Pro Gly Glu Thr Val
   1               5                   10                  15
   Lys Ile Thr Cys Ser Gly Gly Gly Ser Tyr Ala Gly Ser Tyr Tyr Tyr
               20                  25                  30
   Gly Trp Tyr Gln Gln Lys Ala Pro Gly Ser Ala Pro Val Thr Leu Ile
           35                  40                  45
   Tyr Tyr Asn Asn Lys Arg Pro Ser Asp Ile Pro Ser Arg Phe Ser Gly
       50                  55                  60
   Ser Leu Ser Gly Ser Thr Asn Thr Leu Thr Ile Thr Gly Val Arg Ala
   65                  70                  75                  80
   Asp Asp Glu Ala Val Tyr Phe Cys Gly Ser Ala Asp Asn Ser Gly Ala
                   85                  90                  95
   Ala Phe Gly Ala Gly Thr Thr Leu Thr Val Leu
               100                 105
<210> 30
   <211> 126
   <212> PRT
   <213> artificial sequence
<220>
   <223> Synthetic
<400> 30
   Ala Val Thr Leu Asp Glu Ser Gly Gly Gly Leu Gln Thr Pro Gly Gly
   1               5                   10                  15
   Ala Leu Ser Leu Val Cys Lys Ala Ser Gly Phe Thr Phe Ser Ser Tyr
               20                  25                  30
   Ala Met His Trp Val Arg Gln Ala Pro Gly Lys Gly Leu Glu Trp Val
   35 40 45
   Ala Gly Ile Tyr Lys Ser Gly Ala Gly Thr Asn Tyr Ala Pro Ala Val
   50 55 60
   Lys Gly Arg Ala Thr Ile Ser Arg Asp Asn Gly Gln Ser Thr Val Arg
   65 70 75 80
   Leu Gln Leu Asn Asn Leu Arg Ala Glu Asp Thr Gly Thr Tyr Tyr Cys
   85 90 95
   Ala Lys Thr Thr Gly Ser Gly Cys Ser Ser Gly Tyr Arg Ala Glu Tyr
   100 105 110
   Ile Asp Ala Trp Gly His Gly Thr Glu Val Ile Val Ser Ser
   115 120 125
<210> 31
   <211> 107
   <212> PRT
   <213> artificial sequence
<220>
   <223> Synthetic
<400> 31
   Ala Leu Thr Gln Pro Ala Ser Val Ser Ala Asn Pro Gly Glu Thr Val
   1 5 10 15
   Lys Ile Thr Cys Ser Gly Gly Gly Ser Tyr Ala Gly Ser Tyr Tyr Tyr
   20 25 30
   Gly Trp Tyr Gln Gln Lys Ala Pro Gly Ser Ala Pro Val Thr Leu Ile
   35 40 45
   Tyr Tyr Asn Asn Lys Arg Pro Ser Asp Ile Pro Ser Arg Phe Ser Gly
   50 55 60
   Ser Leu Ser Gly Ser Thr Asn Thr Leu Thr Ile Thr Gly Val Arg Ala
   65 70 75 80
   Asp Asp Glu Ala Val Tyr Phe Cys Gly Ser Ala Asp Asn Ser Gly Ala
   85 90 95
   Ala Phe Gly Ala Gly Thr Thr Leu Thr Val Leu
   100 105
<210> 32
   <211> 126
   <212> PRT
   <213> artificial sequence
<220>
   <223> Synthetic
<400> 32
   Ala Val Thr Leu Asp Glu Ser Gly Gly Gly Leu Gln Thr Pro Gly Gly
   1               5                   10                  15
   Ala Leu Ser Leu Val Cys Lys Ala Ser Gly Phe Thr Phe Ser Ser Tyr
               20                  25                  30
   Asp Met Val Trp Val Arg Gln Ala Pro Gly Lys Gly Leu Glu Phe Val
           35                  40                  45
   Ala Gly Ile Ser Arg Asn Asp Gly Arg Tyr Thr Glu Tyr Gly Ser Ala
       50                  55                  60
   Val Lys Gly Arg Ala Thr Ile Ser Arg Asp Asn Gly Gln Ser Thr Val
   65                  70                  75                  80
   Arg Leu Gln Leu Asn Asn Leu Arg Ala Glu Asp Thr Ala Thr Tyr Tyr
                   85                  90                  95
   Cys Ala Arg Asp Ala Gly Gly Ser Ala Tyr Trp Phe Asp Ala Gly Gln
               100                 105                 110
   Ile Asp Ala Trp Gly His Gly Thr Glu Val Ile Val Ser Ser
           115                 120                 125
<210> 33
   <211> 107
   <212> PRT
   <213> artificial sequence
<220>
   <223> Synthetic
<400> 33
   Ala Leu Thr Gln Pro Ala Ser Val Ser Ala Asn Pro Gly Glu Thr Val
   1               5                   10                  15
   Lys Ile Thr Cys Ser Gly Gly Gly Ser Tyr Ala Gly Ser Tyr Tyr Tyr
               20                  25                  30
   Gly Trp Tyr Gln Gln Lys Ala Pro Gly Ser Ala Pro Val Thr Leu Ile
           35                  40                  45
   Tyr Tyr Asn Asn Lys Arg Pro Ser Asp Ile Pro Ser Arg Phe Ser Gly
       50                  55                  60
   Ser Leu Ser Gly Ser Thr Asn Thr Leu Thr Ile Thr Gly Val Arg Ala
   65                  70                  75                  80
   Asp Asp Glu Ala Val Tyr Phe Cys Gly Ser Ala Asp Asn Ser Gly Ala
                   85                  90                  95
   Ala Phe Gly Ala Gly Thr Thr Leu Thr Val Leu
               100                 105
<210> 34
   <211> 36
   <212> PRT
   <213> artificial sequence
<220>
   <223> Synthetic
<400> 34
   Leu Glu Val Thr Cys Glu Pro Gly Thr Thr Phe Lys Asp Lys Cys Asn
   1               5                   10                  15
   Thr Cys Arg Cys Gly Ser Asp Gly Lys Ser Ala Phe Cys Thr Arg Lys
               20                  25                  30
   Leu Cys Tyr Gln
           35
<210> 35
   <211> 36
   <212> PRT
   <213> artificial sequence
<220>
   <223> Synthetic
<400> 35
   Leu Glu Val Thr Cys Glu Pro Gly Thr Thr Phe Lys Asp Lys Cys Asn
   1               5                   10                  15
   Thr Cys Arg Cys Gly Ser Asp Gly Lys Ser Ala Val Cys Thr Lys Leu
               20                  25                  30
   Trp Cys Asn Gln
           35
<210> 36
   <211> 227
   <212> PRT
   <213> homo sapiens
<400> 36
   Asp Lys Thr His Thr Cys Pro Pro Cys Pro Ala Pro Glu Leu Leu Gly
   1               5                   10                  15
   Gly Pro Ser Val Phe Leu Phe Pro Pro Lys Pro Lys Asp Thr Leu Met
               20                  25                  30
   Ile Ser Arg Thr Pro Glu Val Thr Cys Val Val Val Asp Val Ser His
           35                  40                  45
   Glu Asp Pro Glu Val Lys Phe Asn Trp Tyr Val Asp Gly Val Glu Val
       50                  55                  60
   His Asn Ala Lys Thr Lys Pro Arg Glu Glu Gln Tyr Asn Ser Thr Tyr
   65                  70                  75                  80
   Arg Val Val Ser Val Leu Thr Val Leu His Gln Asp Trp Leu Asn Gly
                   85                  90                  95
   Lys Glu Tyr Lys Cys Lys Val Ser Asn Lys Ala Leu Pro Ala Pro Ile
               100                 105                 110
   Glu Lys Thr Ile Ser Lys Ala Lys Gly Gln Pro Arg Glu Pro Gln Val
           115                 120                 125
   Tyr Thr Leu Pro Pro Ser Arg Glu Glu Met Thr Lys Asn Gln Val Ser
       130                 135                 140
   Leu Thr Cys Leu Val Lys Gly Phe Tyr Pro Ser Asp Ile Ala Val Glu
   145                 150                 155                 160
   Trp Glu Ser Asn Gly Gln Pro Glu Asn Asn Tyr Lys Thr Thr Pro Pro
                   165                 170                 175
   Val Leu Asp Ser Asp Gly Ser Phe Phe Leu Tyr Ser Lys Leu Thr Val
               180                 185                 190
   Asp Lys Ser Arg Trp Gln Gln Gly Asn Val Phe Ser Cys Ser Val Met
           195                 200                 205
   His Glu Ala Leu His Asn His Tyr Thr Gln Lys Ser Leu Ser Leu Ser
       210                 215                 220
   Pro Gly Lys
   225
<210> 37
   <211> 12
   <212> PRT
   <213> artificial sequence
<220>
   <223> Synthetic
<400> 37
   Gly Thr Gly Gly Gly Ser Gly Ser Ser Ser Arg Ser
   1 5 10
<210> 38
   <211> 10
   <212> PRT
   <213> artificial sequence
<220>
   <223> Synthetic
<400> 38
   Gly Thr Gly Gly Gly Ser Gly Ser Ser Ser
   1 5 10
<210> 39
   <211> 888
   <212> DNA
   <213> artificial sequence
<220>
   <223> Synthetic
<400> 39
   atgtacagga tgcaactcct gtcttgcatt gcactaagtc ttgcacttgt cacgaattcg 60
   ctcgaggtga catgtgaacc cggtacgacg tttaaggata agtgcaacac atgtaggtgc 120
   ggtagcgacg gcaaatcagc gttctgtacc cggaaattgt gctaccaggg aactggagga 180
   gggtcggggt cctcgtcaag atctgacaaa actcacacat gcccaccgtg cccagcacct 240
   gaactcctgg ggggaccgtc agtcttcctc ttccccccaa aacccaagga caccctcatg 300
   atctcccgga cccctgaggt cacatgcgtg gtggtggacg tgagccacga agaccctgag 360
   gtcaagttca actggtacgt ggacggcgtg gaggtgcata atgccaagac aaagccgcgg 420
   gaggagcagt acaacagcac gtaccgtgtg gtcagcgtcc tcaccgtcct gcaccaggac 480
   tggctgaatg gcaaggagta caagtgcaag gtctccaaca aagccctccc agcccccatc 540
   gagaaaacca tctccaaagc caaagggcag ccccgagaac cacaggtgta caccctgccc 600
   ccatcccggg aggagatgac caagaaccag gtcagcctga cctgcctggt caaaggcttc 660
   tatcccagcg acatcgccgt ggagtgggag agcaatgggc agccggagaa caactacaag 720
   accacgcctc ccgtgctgga ctccgacggc tccttcttcc tctacagcaa gctcaccgtg 780
   gacaagagca ggtggcagca ggggaacgtc ttctcatgct ccgtgatgca cgaggctctg 840
   cacaaccact acacgcagaa gagcctctcc ctgtctccgg gtaaatga 888
<210> 40
   <211> 275
   <212> PRT
   <213> artificial sequence
<220>
   <223> Synthetic
<400> 40
   Leu Glu Val Thr Cys Glu Pro Gly Thr Thr Phe Lys Asp Lys Cys Asn
   1               5                   10                  15
   Thr Cys Arg Cys Gly Ser Asp Gly Lys Ser Ala Phe Cys Thr Arg Lys
               20                  25                  30
   Leu Cys Tyr Gln Gly Thr Gly Gly Gly Ser Gly Ser Ser Ser Arg Ser
           35                  40                  45
   Asp Lys Thr His Thr Cys Pro Pro Cys Pro Ala Pro Glu Leu Leu Gly
       50                  55                  60
   Gly Pro Ser Val Phe Leu Phe Pro Pro Lys Pro Lys Asp Thr Leu Met
   65                  70                  75                  80
   Ile Ser Arg Thr Pro Glu Val Thr Cys Val Val Val Asp Val Ser His
                   85                  90                  95
   Glu Asp Pro Glu Val Lys Phe Asn Trp Tyr Val Asp Gly Val Glu Val
               100                 105                 110
   His Asn Ala Lys Thr Lys Pro Arg Glu Glu Gln Tyr Asn Ser Thr Tyr
           115                 120                 125
   Arg Val Val Ser Val Leu Thr Val Leu His Gln Asp Trp Leu Asn Gly
       130                 135                 140
   Lys Glu Tyr Lys Cys Lys Val Ser Asn Lys Ala Leu Pro Ala Pro Ile
   145                 150                 155                 160
   Glu Lys Thr Ile Ser Lys Ala Lys Gly Gln Pro Arg Glu Pro Gln Val
                   165                 170                 175
   Tyr Thr Leu Pro Pro Ser Arg Glu Glu Met Thr Lys Asn Gln Val Ser
               180                 185                 190
   Leu Thr Cys Leu Val Lys Gly Phe Tyr Pro Ser Asp Ile Ala Val Glu
           195                 200                 205
   Trp Glu Ser Asn Gly Gln Pro Glu Asn Asn Tyr Lys Thr Thr Pro Pro
       210                 215                 220
   Val Leu Asp Ser Asp Gly Ser Phe Phe Leu Tyr Ser Lys Leu Thr Val
   225                 230                 235                 240
   Asp Lys Ser Arg Trp Gln Gln Gly Asn Val Phe Ser Cys Ser Val Met
                   245                 250                 255
   His Glu Ala Leu His Asn His Tyr Thr Gln Lys Ser Leu Ser Leu Ser
               260                 265                 270
   Pro Gly Lys
           275
<210> 41
   <211> 888
   <212> DNA
   <213> artificial sequence
<220>
   <223> Synthetic
<400> 41
   atgtacagga tgcaactcct gtcttgcatt gcactaagtc ttgcacttgt cacgaattcg      60
   ttggaagtga cgtgtgagcc cggaacgaca ttcaaagaca agtgcaatac ttgtcggtgc     120
   ggttcagatg ggaaatcggc ggtctgcaca aagctctggt gtaaccaggg caccggtgga     180
   gggtcgggat ccagctcaag atctgacaaa actcacacat gcccaccgtg cccagcacct     240
   gaactcctgg ggggaccgtc agtcttcctc ttccccccaa aacccaagga caccctcatg     300
   atctcccgga cccctgaggt cacatgcgtg gtggtggacg tgagccacga agaccctgag     360
   gtcaagttca actggtacgt ggacggcgtg gaggtgcata atgccaagac aaagccgcgg     420
   gaggagcagt acaacagcac gtaccgtgtg gtcagcgtcc tcaccgtcct gcaccaggac     480
   tggctgaatg gcaaggagta caagtgcaag gtctccaaca aagccctccc agcccccatc     540
   gagaaaacca tctccaaagc caaagggcag ccccgagaac cacaggtgta caccctgccc     600
   ccatcccggg aggagatgac caagaaccag gtcagcctga cctgcctggt caaaggcttc     660
   tatcccagcg acatcgccgt ggagtgggag agcaatgggc agccggagaa caactacaag     720
   accacgcctc ccgtgctgga ctccgacggc tccttcttcc tctacagcaa gctcaccgtg     780
   gacaagagca ggtggcagca ggggaacgtc ttctcatgct ccgtgatgca cgaggctctg     840
   cacaaccact acacgcagaa gagcctctcc ctgtctccgg gtaaatga 888
<210> 42
   <211> 275
   <212> PRT
   <213> artificial sequence
<220>
   <223> Synthetic
<400> 42
   Leu Glu Val Thr Cys Glu Pro Gly Thr Thr Phe Lys Asp Lys Cys Asn
   1               5                   10                  15
   Thr Cys Arg Cys Gly Ser Asp Gly Lys Ser Ala Val Cys Thr Lys Leu
               20                  25                  30
   Trp Cys Asn Gln Gly Thr Gly Gly Gly Ser Gly Ser Ser Ser Arg Ser
           35                  40                  45
   Asp Lys Thr His Thr Cys Pro Pro Cys Pro Ala Pro Glu Leu Leu Gly
       50                  55                  60
   Gly Pro Ser Val Phe Leu Phe Pro Pro Lys Pro Lys Asp Thr Leu Met
   65                  70                  75                  80
   Ile Ser Arg Thr Pro Glu Val Thr Cys Val Val Val Asp Val Ser His
                   85                  90                  95
   Glu Asp Pro Glu Val Lys Phe Asn Trp Tyr Val Asp Gly Val Glu Val
               100                 105                 110
   His Asn Ala Lys Thr Lys Pro Arg Glu Glu Gln Tyr Asn Ser Thr Tyr
           115                 120                 125
   Arg Val Val Ser Val Leu Thr Val Leu His Gln Asp Trp Leu Asn Gly
       130                 135                 140
   Lys Glu Tyr Lys Cys Lys Val Ser Asn Lys Ala Leu Pro Ala Pro Ile
   145                 150                 155                 160
   Glu Lys Thr Ile Ser Lys Ala Lys Gly Gln Pro Arg Glu Pro Gln Val
                   165                 170                 175
   Tyr Thr Leu Pro Pro Ser Arg Glu Glu Met Thr Lys Asn Gln Val Ser
               180                 185                 190
   Leu Thr Cys Leu Val Lys Gly Phe Tyr Pro Ser Asp Ile Ala Val Glu
           195                 200                 205
   Trp Glu Ser Asn Gly Gln Pro Glu Asn Asn Tyr Lys Thr Thr Pro Pro
       210                 215                 220
   Val Leu Asp Ser Asp Gly Ser Phe Phe Leu Tyr Ser Lys Leu Thr Val
   225                 230                 235                 240
   Asp Lys Ser Arg Trp Gln Gln Gly Asn Val Phe Ser Cys Ser Val Met
                   245                 250                 255
   His Glu Ala Leu His Asn His Tyr Thr Gln Lys Ser Leu Ser Leu Ser
               260                 265                 270
   Pro Gly Lys
           275

## Description

### BACKGROUND

The complement system provides an early acting mechanism to initiate, amplify and orchestrate the immune response to microbial infection and other acute insults (M.K. Liszewski and J.P. Atkinson, 1993, in Fundamental Immunology, Third Edition, edited by W.E. Paul, Raven Press, Ltd., New York), in humans and other vertebrates. While complement activation provides a valuable first-line defense against potential pathogens, the activities of complement that promote a protective immune response can also represent a potential threat to the host (K.R. Kalli, et al., Springer Semin. Immunopathol. 15:417-431, 1994; B.P. Morgan, Eur. J. Clinical Investig. 24:219-228, 1994). For example, C3 and C5 proteolytic products recruit and activate neutrophils. While indispensable for host defense, activated neutrophils are indiscriminate in their release of destructive enzymes and may cause organ damage. In addition, complement activation may cause the deposition of lytic complement components on nearby host cells as well as on microbial targets, resulting in host cell lysis.

The complement system has also been implicated in the pathogenesis of numerous acute and chronic disease states, including: myocardial infarction, stroke, ARDS, reperfusion injury, septic shock, capillary leakage following thermal bums, postcardiopulmonary bypass inflammation, transplant rejection, rheumatoid arthritis, multiple sclerosis, myasthenia gravis, and Alzheimer's disease. In almost all of these conditions, complement is not the cause but is one of several factors involved in pathogenesis. Nevertheless, complement activation may be a major pathological mechanism and represents an effective point for clinical control in many of these disease states. The growing recognition of the importance of complement-mediated tissue injury in a variety of disease states underscores the need for effective complement inhibitory drugs. To date, Eculizumab (Solaris®), an antibody against C5, is the only complement-targeting drug that has been approved for human use. Yet, C5 is one of several effector molecules located "downstream" in the complement system, and blockade of C5 does not inhibit activation of the complement system. Therefore, an inhibitor of the initiation steps of complement activation would have significant advantages over a "downstream" complement inhibitor.

Currently, it is widely accepted that the complement system can be activated through three distinct pathways: the classical pathway, the lectin pathway, and the alternative pathway. The classical pathway is usually triggered by a complex composed of host antibodies bound to a foreign particle (*i.e.,* an antigen) and thus requires prior exposure to an antigen for the generation of a specific antibody response. Since activation of the classical pathway depends on a prior adaptive immune response by the host, the classical pathway is part of the acquired immune system. In contrast, both the lectin and alternative pathways are independent of adaptive immunity and are part of the innate immune system.

The activation of the complement system results in the sequential activation of serine protease zymogens. The first step in activation of the classical pathway is the binding of a specific recognition molecule, C1q, to antigen-bound IgG and IgM molecules. C1q is associated with the C1r and C1s serine protease proenzymes as a complex called C1. Upon binding of C1q to an immune complex, autoproteolytic cleavage of the Arg-Ile site of C1r is followed by Clr-mediated cleavage and activation of C1s, which thereby acquires the ability to cleave C4 and C2. C4 is cleaved into two fragments, designated C4a and C4b, and, similarly, C2 is cleaved into C2a and C2b. C4b fragments are able to form covalent bonds with adjacent hydroxyl or amino groups and generate the C3 convertase (C4b2a) through noncovalent interaction with the C2a fragment of activated C2. C3 convertase (C4b2a) activates C3 by proteolytic cleavage into C3a and C3b subcomponents leading to generation of the C5 convertase (C4b2a3b), which, by cleaving C5 leads to the formation of the membrane attack complex (C5b combined with C6, C7, C8 and C-9, also referred to as "MAC") that can disrupt cellular membranes resulting in cell lysis. The activated forms of C3 and C4 (C3b and C4b) are covalently deposited on the foreign target surfaces, which are recognized by complement receptors on multiple phagocytes.

Independently, the first step in activation of the complement system through the lectin pathway is also the binding of specific recognition molecules, which is followed by the activation of associated serine protease proenzymes. However, rather than the binding of immune complexes by C1q, the recognition molecules in the lectin pathway comprise a group of carbohydrate-binding proteins (mannan-binding lectin (MBL), H-ficolin, M-ficolin, L-ficolin and C-type lectin CL-11), collectively referred to as lectins. See J. Lu et al., Biochim. Biophys. Acta 1572:387-400, (2002); Holmskov et al., Annu. Rev. Immunol. 21:547-578 (2003); Teh et al., Immunology 101:225-232 (2000)). See also J. Luet et al., Biochim Biophys Acta 1572:387-400 (2002); Holmskov et al, Annu Rev Immunol 21:547-578 (2003); Teh et al., Immunology 101:225-232 (2000); Hansen et al, J. Immunol 185(10):6096-6104 (2010).

Ikeda et al. first demonstrated that, like C1q, MBL could activate the complement system upon binding to yeast mannan-coated erythrocytes in a C4-dependent manner (Ikeda et al., J. Biol. Chem. 262:7451-7454, (1987)). MBL, a member of the collectin protein family, is a calcium-dependent lectin that binds carbohydrates with 3-and 4-hydroxy groups oriented in the equatorial plane of the pyranose ring. Prominent ligands for MBL are thus D-mannose and N-acetyl-D-glucosamine, while carbohydrates not fitting this steric requirement have undetectable affinity for MBL (Weis et al., Nature 360:127-134, (1992)). The interaction between MBL and monovalent sugars is extremely weak, with dissociation constants typically in the single-digit millimolar range. MBL achieves tight, specific binding to glycan ligands by avidity, i.e., by interacting simultaneously with multiple monosaccharide residues located in close proximity to each other (Lee et al., Archiv. Biochem. Biophys. 299:129-136, (1992)). MBL recognizes the carbohydrate patterns that commonly decorate microorganisms such as bacteria, yeast, parasites and certain viruses. In contrast, MBL does not recognize D-galactose and sialic acid, the penultimate and ultimate sugars that usually decorate "mature" complex glycoconjugates present on mammalian plasma and cell surface glycoproteins. This binding specificity is thought to promote recognition of "foreign" surfaces and help protect from "self-activation." However, MBL does bind with high affinity to clusters of high-mannose "precursor" glycans on N-linked glycoproteins and glycolipids sequestered in the endoplasmic reticulum and Golgi of mammalian cells (Maynard et al., J. Biol. Chem. 257:3788-3794, (1982)). In addition, it has been shown that MBL can bind the polynucleotides, DNA and RNA, which may be exposed on necrotic and apoptotic cells (Palaniyar et al., Ann. N.Y. Acad. Sci., 1010:467-470 (2003); Nakamura et al., J. Leuk. Biol. 86:737-748 (2009)). Therefore, damaged cells are potential targets for lectin pathway activation via MBL binding.

The ficolins possess a different type of lectin domain than MBL, called the fibrinogen-like domain. Ficolins bind sugar residues in a Ca⁺⁺-independent manner. In humans, three kinds of ficolins (L-ficolin, M-ficolin and H-ficolin) have been identified. The two serum ficolins, L-ficolin and H-ficolin, have in common a specificity for N-acetyl-D-glucosamine; however, H-ficolin also binds N-acetyl-D-galactosamine. The difference in sugar specificity of L-ficolin, H-ficolin, CL-11, and MBL means that the different lectins may be complementary and target different, though overlapping, glycoconjugates. This concept is supported by the recent report that, of the known lectins in the lectin pathway, only L-ficolin binds specifically to lipoteichoic acid, a cell wall glycoconjugate found on all Gram-positive bacteria (Lynch et al., J. Immunol. 172:1198-1202, (2004)). In addition to acetylated sugar moieties, the ficolins can also bind acetylated amino acids and polypeptides (Thomsen et al., Mol. Immunol. 48(4):369-81 (2011)). The collectins (i.e., MBL) and the ficolins bear no significant similarity in amino acid sequence. However, the two groups of proteins have similar domain organizations and, like C1q, assemble into oligomeric structures, which maximize the possibility of multisite binding.

The serum concentrations of MBL are highly variable in healthy populations and this is genetically controlled by polymorphisms/mutations in both the promoter and coding regions of the MBL gene. As an acute phase protein, the expression of MBL is further upregulated during inflammation. L-ficolin is present in serum at concentrations similar to those of MBL. Therefore, the L-ficolin branch of the lectin pathway is potentially comparable to the MBL arm in strength. MBL and ficolins can also function as opsonins, which allow phagocytes to target MBL- and ficolin-decorated surfaces (see Jack et al., J Leukoc Biol., 77(3):328-36 (2004), Matsushita and Fujita, Immunobiology, 205(4-5):490-7 (2002), Aoyagi et al., J Immunol, 174(1):418-25(2005). This opsonization requires the interaction of these proteins with phagocyte receptors (Kuhlman et al., J. Exp. Med. 169:1733, (1989); Matsushita et al., J. Biol. Chem. 271:2448-54, (1996)), the indentity of which has not been established.

Human MBL forms a specific and high-affinity interaction through its collagen-like domain with unique C1r/C1s-like serine proteases, termed MBL-associated serine proteases (MASPs). To date, three MASPs have been described. First, a single enzyme "MASP" was identified and characterized as the enzyme responsible for the initiation of the complement cascade (i.e., cleaving C2 and C4) (Matsushita et al., JExp Med 176(6):1497-1502 (1992); Ji et al., J. Immunol. 150:571-578, (1993)). It was subsequently determined that the MASP activity was, in fact, a mixture of two proteases: MASP-1 and MASP-2 (Thiel et al., Nature 386:506-510, (1997)). However, it was demonstrated that the MBL-MASP-2 complex alone is sufficient for complement activation (Vorup-Jensen et al., J. Immunol. 165:2093-2100, (2000)). Furthermore, only MASP-2 cleaved C2 and C4 at high rates (Ambrus et al., J. Immunol. 170:1374-1382, (2003)). Therefore, MASP-2 is the protease responsible for activating C4 and C2 to generate the C3 convertase, C4b2a. This is a significant difference from the C1 complex of the classical pathway, where the coordinated action of two specific serine proteases (C1r and C1s) leads to the activation of the complement system. In addition, a third novel protease, MASP-3, has been isolated (Dahl, M.R., et al., Immunity 15:127-35, 2001). MASP-1 and MASP-3 are alternatively spliced products of the same gene.

MASPs share identical domain organizations with those of C1r and C1s, the enzymatic components of the C1 complex (Sim et al., Biochem. Soc. Trans. 28:545, (2000)). These domains include an N-terminal C1r/C1s/sea urchin VEGF/bone morphogenic protein (CUB) domain, an epidermal growth factor-like domain, a second CUB domain, a tandem of complement control protein domains, and a serine protease domain. As in the C1 proteases, activation of MASP-2 occurs through cleavage of an Arg-I1e bond adjacent to the serine protease domain, which splits the enzyme into disulfide-linked A and B chains, the latter consisting of the serine protease domain.

MBL can also associate with an alternatively spliced form of MASP-2, known as MBL-associated protein of 19 kDa (MAp19) or small MBL-associated protein (sMAP), which lacks the catalytic activity of MASP-2. (Stover, J. Immunol. 162:3481-90, (1999); Takahashi et al., Int. Immunol. 11:859-863, (1999)). MAp19 comprises the first two domains of MASP-2, followed by an extra sequence of four unique amino acids. The function of Map19 is unclear (Degn et al., J Immunol. Methods, 2011). The MASP-1 and MASP-2 genes are located on human chromosomes 3 and 1, respectively (Schwaeble et al., Immunobiology 205:455-466, (2002)).

Several lines of evidence suggest that there are different MBL-MASP complexes and a large fraction of the MASPs in serum is not complexed with MBL (Thiel,et al., J. Immunol. 165:878-887, (2000)). Both H- and L-ficolin bind to all MASPs and activate the lectin complement pathway, as does MBL (Dahl et al., Immunity 15:127-35, (2001); Matsushita et al., J. Immunol. 168:3502-3506, (2002)). Both the lectin and classical pathways form a common C3 convertase (C4b2a) and the two pathways converge at this step.

The lectin pathway is widely thought to have a major role in host defense against infection in the naive host. Strong evidence for the involvement of MBL in host defense comes from analysis of patients with decreased serum levels of functional MBL (Kilpatrick, Biochim. Biophys. Acta 1572:401-413, (2002)). Such patients display susceptibility to recurrent bacterial and fungal infections. These symptoms are usually evident early in life, during an apparent window of vulnerability as maternally derived antibody titer wanes, but before a full repertoire of antibody responses develops. This syndrome often results from mutations at several sites in the collagenous portion of MBL, which interfere with proper formation of MBL oligomers. However, since MBL can function as an opsonin independent of complement, it is not known to what extent the increased susceptibility to infection is due to impaired complement activation.

In contrast to the classical and lectin pathways, no initiators of the alternative pathway have previously been found to fulfill the recognition functions that C1q and lectins perform in the other two pathways. Currently it is widely accepted that the alternative pathway spontaneously undergoes a low level of turnover activation, which can be readily amplified on foreign or other abnormal surfaces (bacteria, yeast, virally infected cells, or damaged tissue) that lack the proper molecular elements that keep spontaneous complement activation in check. There are four plasma proteins directly involved in the activation of the alternative pathway: C3, factors B and D, and properdin.

Although there is extensive evidence implicating both the classical and alternative complement pathways in the pathogenesis of non-infectious human diseases, the role of the lectin pathway is just beginning to be evaluated. Recent studies provide evidence that activation of the lectin pathway can be responsible for complement activation and related inflammation in ischemia/reperfusion injury. Collard et al. (2000) reported that cultured endothelial cells subjected to oxidative stress bind MBL and show deposition of C3 upon exposure to human serum (Collard et al., Am. J. Pathol. 156:1549-1556, (2000)). In addition, treatment of human sera with blocking anti-MBL monoclonal antibodies inhibited MBL binding and complement activation. These findings were extended to a rat model of myocardial ischemia-reperfusion in which rats treated with a blocking antibody directed against rat MBL showed significantly less myocardial damage upon occlusion of a coronary artery than rats treated with a control antibody (Jordan et al., Circulation 104:1413-1418, (2001)). The molecular mechanism of MBL binding to the vascular endothelium after oxidative stress is unclear; a recent study suggests that activation of the lectin pathway after oxidative stress may be mediated by MBL binding to vascular endothelial cytokeratins, and not to glycoconjugates (Collard et al., Am. J. Pathol. 159:1045-1054, (2001)). Other studies have implicated the classical and alternative pathways in the pathogenesis of ischemia/reperfusion injury and the role of the lectin pathway in this disease remains controversial (Riedermann, N.C., et al., Am. J. Pathol. 162:363-367, 2003).

Recent studies have shown that MASP-1 and MASP-3 convert the alternative pathway activation enzyme factor D from its zymogen form into its enzymatically active form (see Takahashi M. et al., J Exp Med 207(1):29-37 (2010); Iwaki et al., J. Immunol. 187:3751-58 (2011)). The physiological importance of this process is underlined by the absence of alternative pathway functional activity in plasma of MASP-1/3-deficient mice. Proteolytic generation of C3b from native C3 is required for the alternative pathway to function. Since the alternative pathway C3 convertase (C3bBb) contains C3b as an essential subunit, the question regarding the origin of the first C3b via the alternative pathway has presented a puzzling problem and has stimulated considerable research.

C3 belongs to a family of proteins (along with C4 and α-2 macroglobulin) that contain a rare posttranslational modification known as a thioester bond. The thioester group is composed of a glutamine whose terminal carbonyl group forms a covalent thioester linkage with the sulfhydryl group of a cysteine three amino acids away. This bond is unstable and the electrophilic glutamyl-thioester can react with nucleophilic moieties such as hydroxyl or amino groups and thus form a covalent bond with other molecules. The thioester bond is reasonably stable when sequestered within a hydrophobic pocket of intact C3. However, proteolytic cleavage of C3 to C3a and C3b results in exposure of the highly reactive thioester bond on C3b and, following nucleophilic attack by adjacent moieties comprising hydroxyl or amino groups, C3b becomes covalently linked to a target. In addition to its well-documented role in covalent attachment of C3b to complement targets, the C3 thioester is also thought to have a pivotal role in triggering the alternative pathway. According to the widely accepted "tick-over theory", the alternative pathway is initiated by the generation of a fluid-phase convertase, iC3Bb, which is formed from C3 with hydrolyzed thioester (iC3; C3(H₂O)) and factor B (Lachmann, P.J., et al., Springer Semin. Immunopathol. 7:143-162, (1984)). The C3b-like C3(H₂O) is generated from native C3 by a slow spontaneous hydrolysis of the internal thioester in the protein (Pangburn, M.K., et al., J. Exp. Med. 154:856-867, 1981). Through the activity of the C3(H₂O)Bb convertase, C3b molecules are deposited on the target surface thereby initiating the alternative pathway.

Prior to the instant discovery described herein, very little was known about the initiators of activation of the alternative pathway. Activators were thought to include yeast cell walls (zymosan), many pure polysaccharides, rabbit erythrocytes, certain immunoglobulins, viruses, fungi, bacteria, animal tumor cells, parasites, and damaged cells. The only feature common to these activators is the presence of carbohydrate, but the complexity and variety of carbohydrate structures has made it difficult to establish the shared molecular determinants which are recognized. It has been widely accepted that alternative pathway activation is controlled through the fine balance between inhibitory regulatory components of this pathway, such as factor H, factor I, DAF, and CR1, and properdin, the latter of which is the only positive regulator of the alternative pathway (see Schwaeble W.J. and Reid K.B., Immunol Today 20(1):17-21 (1999)).

In addition to the apparently unregulated activation mechanism described above, the alternative pathway can also provide a powerful amplification loop for the lectin/classical pathway C3 convertase (C4b2a) since any C3b generated can participate with factor B in forming additional alternative pathway C3 convertase (C3bBb). The alternative pathway C3 convertase is stabilized by the binding of properdin. Properdin extends the alternative pathway C3 convertase half-life six to ten fold. Addition of C3b to the alternative pathway C3 convertase leads to the formation of the alternative pathway C5 convertase.

All three pathways (i.e., the classical, lectin and alternative) have been thought to converge at C5, which is cleaved to form products with multiple proinflammatory effects. The converged pathway has been referred to as the terminal complement pathway. C5a is the most potent anaphylatoxin, inducing alterations in smooth muscle and vascular tone, as well as vascular permeability. It is also a powerful chemotaxin and activator of both neutrophils and monocytes. C5a-mediated cellular activation can significantly amplify inflammatory responses by inducing the release of multiple additional inflammatory mediators, including cytokines, hydrolytic enzymes, arachidonic acid metabolites, and reactive oxygen species. C5 cleavage leads to the formation of C5b-9, also known as the membrane attack complex (MAC). There is now strong evidence that sublytic MAC deposition may play an important role in inflammation in addition to its role as a lytic pore-forming complex.

Jiang et al., Journal Exp Med vol 194 (11):1609-1616,2001, discusses Complement 1 inhibitor of the alternative complement pathway. Wong et al., Mol Immunol vol 36(13/14):853-861, 1999, discusses MBL-associated serine proteases regulation. Frikis-Hareli et al., Blood vol 118 (17):4705-4713, 2011; Risitano et al., Molecular Immunology vol 47(13):2215, 2010; and Holers et al. (WO2011/057158) discuss C3/C5 convertase inhibitor TT30 for for treatment of diseases mediated by the complement associated pathway. In addition to its essential role in immune defense, the complement system contributes to tissue damage in many clinical conditions. Thus, there is a pressing need to develop therapeutically effective complement inhibitors to prevent these adverse effects.

### SUMMARY

The present disclosure provides a method of inhibiting MASP-3-dependent complement activation in a subject suffering from paroxysmal nocturnal hemoglobinuria (PNH). The method includes the step of administering to the subject a composition comprising an amount of a MASP-3 inhibitory agent effective to inhibit MASP-3-dependent complement activation. In some cases, the method further comprises administering to the subject a composition comprising a MASP-2 inhibitory agent.

In one aspect, the present invention provides a composition comprising an amount of a MASP-3 inhibitory agent effective to inhibit MASP-3-dependent complement activation for use in treating a subject suffering from paroxysmal nocturnal hemoglobinuria (PNH), wherein said MASP-3 inhibitory agent is a MASP-3 monoclonal antibody or fragment thereof that specifically binds to a portion of human MASP-3 (SEQ ID NO:8).

In another aspect, the present invention provides a composition comprising an amount of a MASP-3 inhibitory agent effective to increase the survival of red blood cells in a subject suffering from paroxysmal nocturnal hemoglobinuria (PNH) for use in treating a subject suffering from PNH wherein said MASP-3 inhibitory agent is a MASP-3 monoclonal antibody or fragment thereof that specifically binds to a portion of human MASP-3 (SEQ ID NO:8).

The present disclosure provides a pharmaceutical composition comprising at least one inhibitory agent, wherein the at least one inhibitory agent comprises a MASP-2 inhibitory agent and a MASP-3 inhibitory agent and a pharmaceutically acceptable carrier.

The present disclosure provides a pharmaceutical composition comprising a MASP-3 inhibitory agent that binds to a portion of MASP-1 (SEQ ID NO: 10: full-length) and that also binds to a portion of MASP-3 (SEQ ID NO:8) and a pharmaceutical carrier.

The present disclosure provides a pharmaceutical composition comprising a MASP-3 inhibitory agent that binds to a portion of MASP-2 (SEQ ID NO: 5: full-length) and that also binds to a portion of MASP-3 (SEQ ID NO:8) and a pharmaceutical carrier.

The present disclosure provides a pharmaceutical composition comprising a MASP-3 inhibitory agent that binds to a portion of MASP-1 (SEQ ID NO: 10: full-length) and that also binds to a portion of MASP-2 (SEQ ID NO:5) and a pharmaceutical carrier.

The present disclosure provides a pharmaceutical composition comprising a MASP-3 inhibitory agent that binds to a portion of MASP-1 (SEQ ID NO: 10 full length), that binds to a portion of MASP-2 (SEQ ID NO: 5: full-length) and that also binds to a portion of MASP-3 (SEQ ID NO:8) and a pharmaceutical carrier.

As described herein, the pharmaceutical compositions of the disclosure can be used in accordance with the methods of the disclosure.

The herein described disclosure will be evident upon reference to the following detailed description and drawings.

### DESCRIPTION OF THE DRAWINGS

This disclosure will become more readily appreciated as the same become better understood by reference to the following detailed description, when taken in conjunction with the accompanying drawings, wherein:

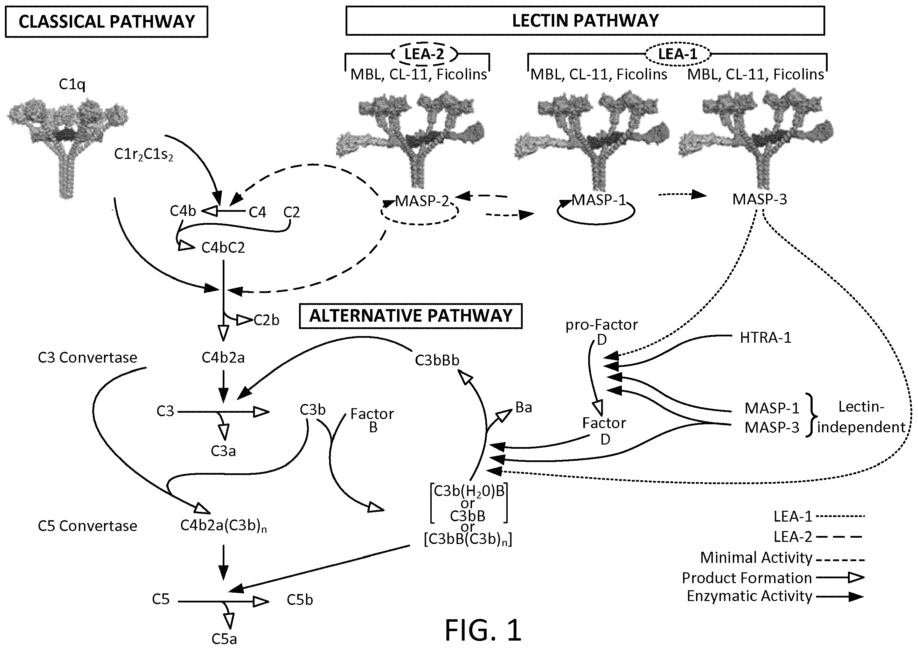

FIGURE 1 illustrates a new understanding of the lectin and alternative pathways;

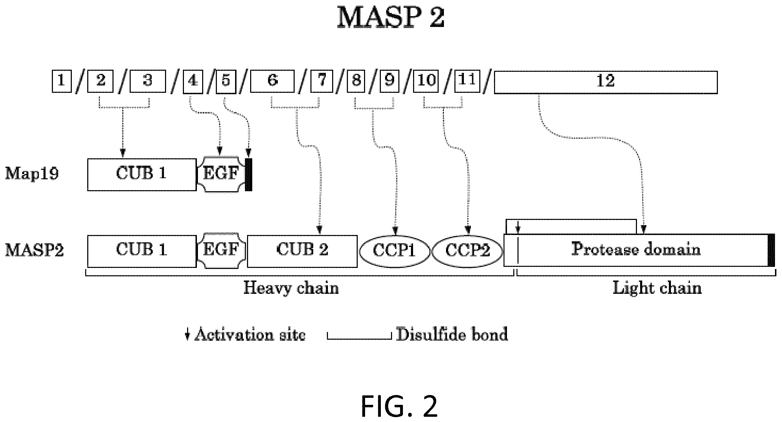

FIGURE 2 is a schematic diagram adapted from Schwaeble et al., Immunobiol 205:455-466 (2002), as modified by Yongqing et al., BBA 1824:253 (2012), illustrating the MASP-2 and MAp19 protein domains and the exons encoding the same;

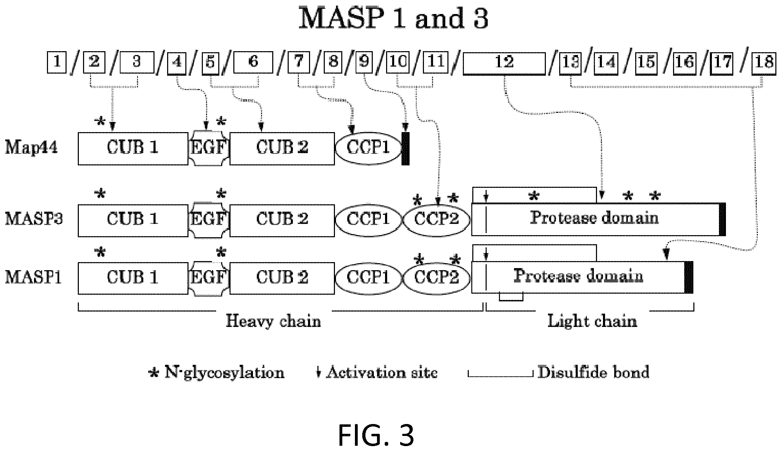

FIGURE 3 is a schematic diagram adapted from Schwaeble et al., Immunobiol 205:455-466 (2002), as modified by Yongqing et al., BBA 1824:253 (2012), illustrating the MASP-1, MASP-3 and MAp44 protein domains and the exons encoding the same;

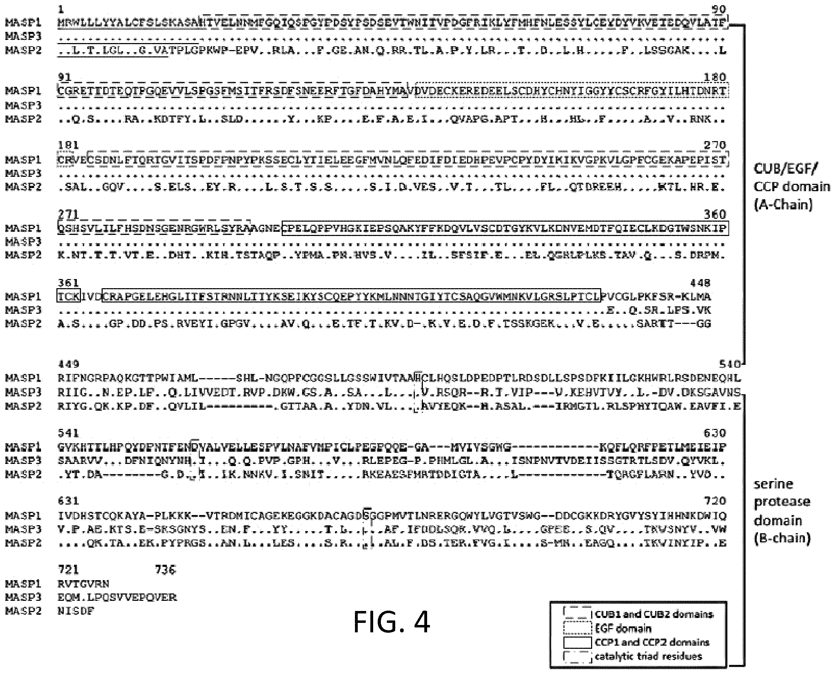

FIGURE 4 shows an alignment of the amino acid sequences of the MASP-1, MASP-2 and MASP-3 proteins and indicates consensus regions therebetween;

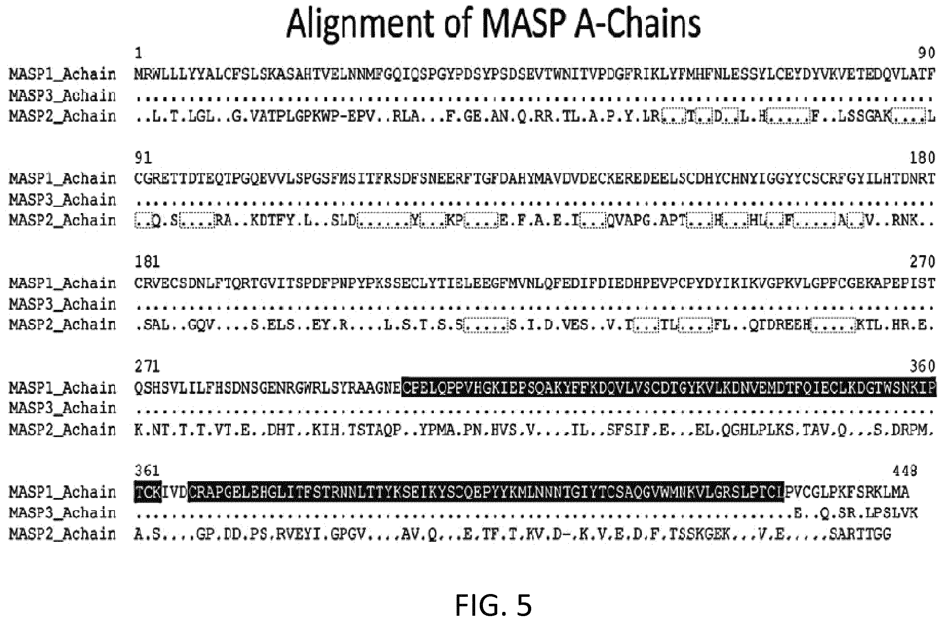

FIGURE 5 shows an alignment of the amino acid sequences of the MASP-1, MASP-2 and MASP-3 Alpha chains;

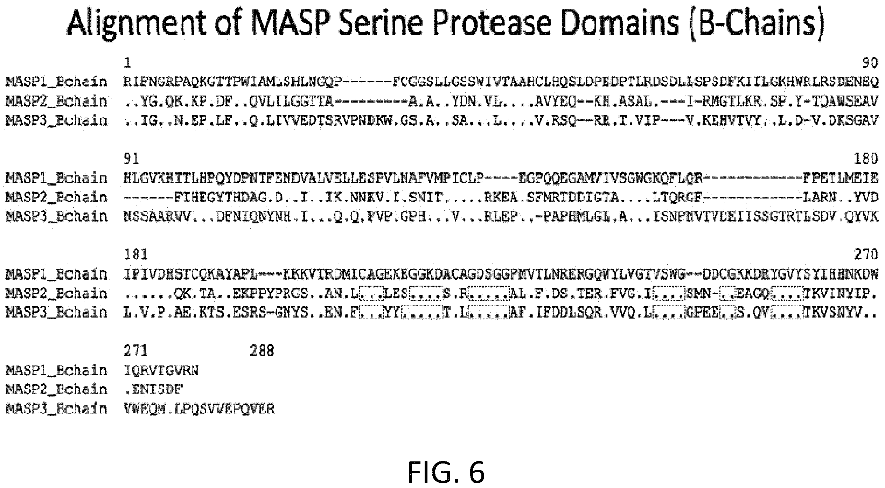

FIGURE 6 shows an alignment of the amino acid sequences of the MASP-1, MASP-2 and MASP-3 Beta Chains;

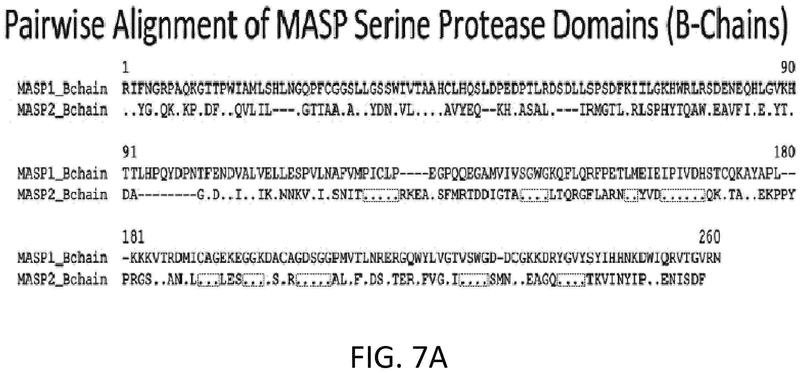

FIGURE 7A shows a pairwise alignment of the amino acid sequences of the MASP-1 and MASP-2 Protease Domains (Beta-chains);

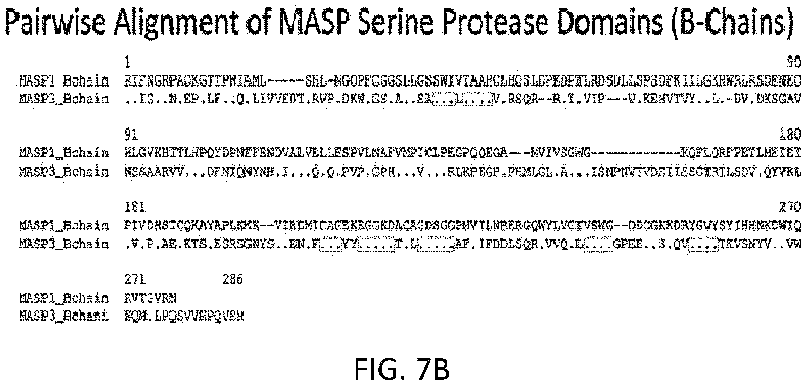

FIGURE 7B shows a pairwise alignment of the amino acid sequences of the MASP-1 and MASP-3 Protease Domains (Beta-chains);

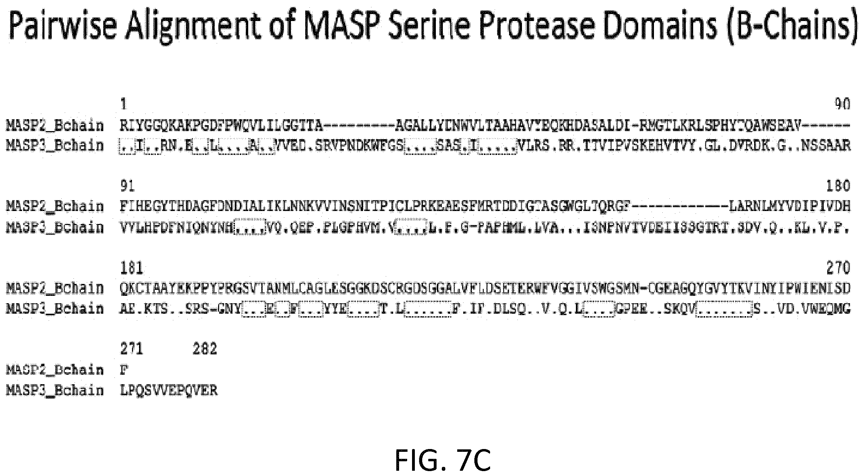

FIGURE 7C shows a pairwise alignment of the amino acid sequences of the MASP-2 and MASP-3 Protease Domains (Beta-chains);

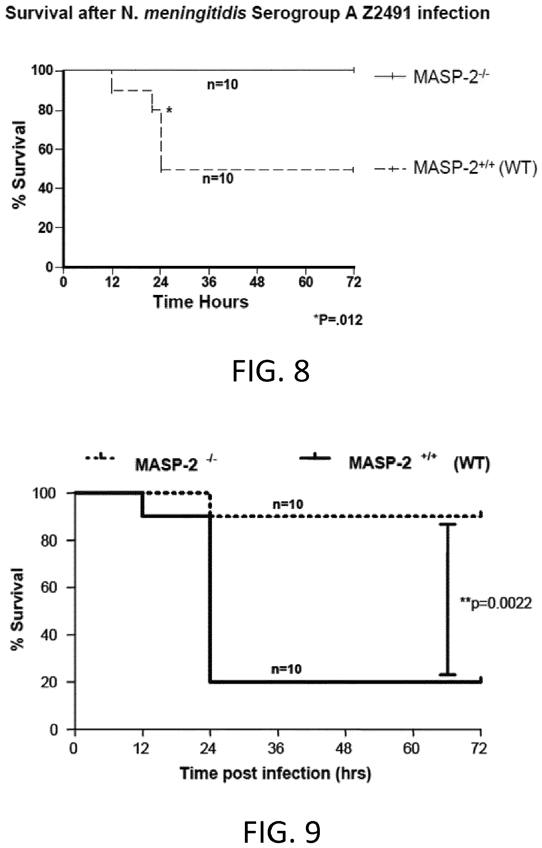

FIGURE 8 is a Kaplan-Meyer plot graphically illustrating the percent survival of MASP-2 KO and WT mice after administration of an infective dose of 2.6 x 10⁷ cfu *of N. meningitidis* serogroup A Z2491, demonstrating that MASP-2 deficient mice are protected from *N. meningitidis* induced mortality, as described in Example 1;
FIGURE 9 is a Kaplan-Meyer plot graphically illustrating the percent survival of MASP-2 KO and WT mice after administration of an infective dose of 6 x 10⁶ cfu *of N. meningitidis* serogroup B strain MC58, demonstrating that MASP-2 deficient mice are protected from *N. meningitidis* induced mortality, as described in Example 1;

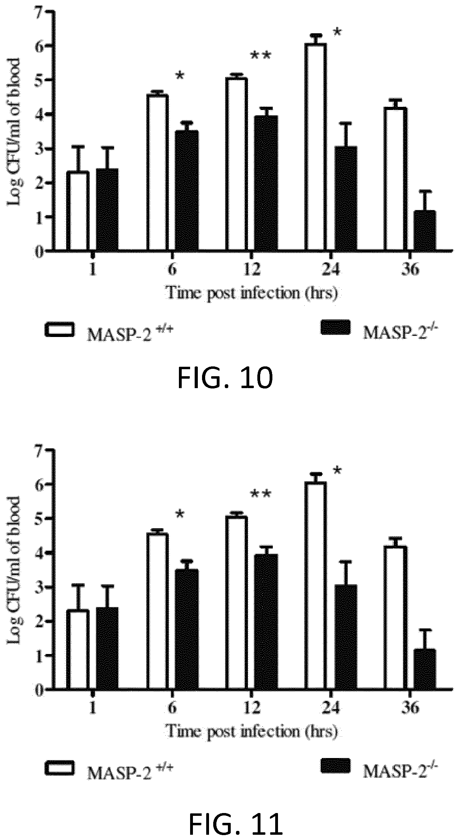

FIGURE 10 graphically illustrates the log cfu/mL *of N. meningitidis* serogroup B strain MC58 per mL of blood recovered from MASP-2 KO and WT mice at different time points after i.p. infection with 6x10⁶ cfu *of N. meningitidis* serogroup B strain MC58 (n=3 at different time points for both groups of mice), demonstrating that although the MASP-2 KO mice were infected with the same dose *of N. meningitidis* serogroup B strain MC58 as the WT mice, the MASP-2 KO mice have enhanced clearance of bacteremia as compared to WT, as described in Example 1;
FIGURE 11 graphically illustrates the average illness score of MASP-2 KO and WT mice at 3, 6, 12 and 24 hours after infection with 6x10⁶ cfu *of N. meningitidis* serogroup B strain MC58, demonstrating that the MASP-2-deficient mice showed much lower illness scores at 6 hours, 12 hours, and 24 hours after infection, as compared to WT mice, as described in Example 1;

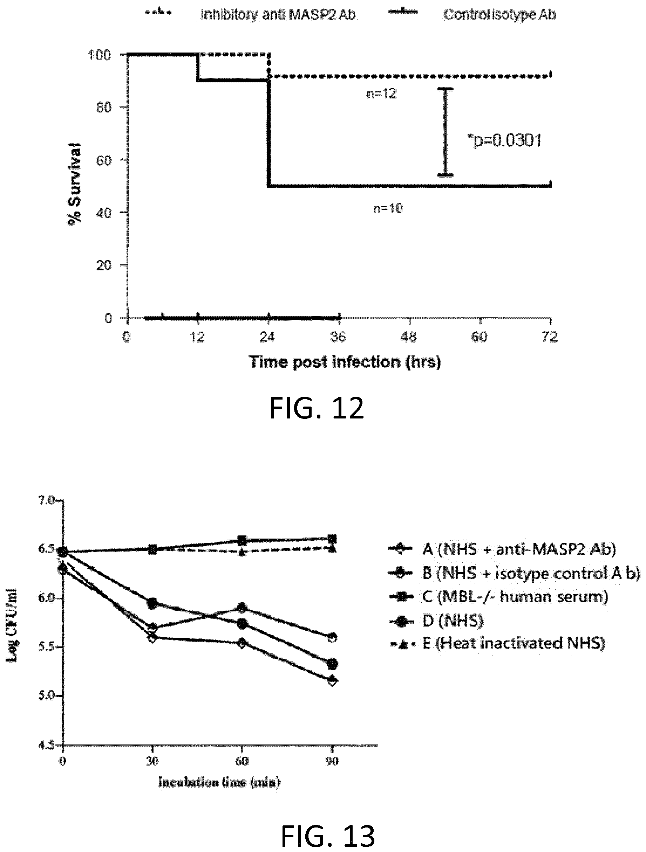

FIGURE 12 is a Kaplan-Meyer plot graphically illustrating the percent survival of mice after administration of an infective dose of 4x10⁶ cfu *of N. meningitidis* serogroup B strain MC58, followed by administration 3 hours post-infection of either inhibitory MASP-2 antibody (1 mg/kg) or control isotype antibody, demonstrating that MASP-2 antibody is effective to treat and improve survival in subjects infected with N. *meningitidis,* as described in Example 2;
FIGURE 13 graphically illustrates the log cfu/mL of viable counts of *N. meningitidis* serogroup B strain MC58 recovered at different time points in the human sera samples shown in TABLE 5 taken at various time points after incubation with N. *meningitidis* serogroup B strain MC58, as described in Example 3;

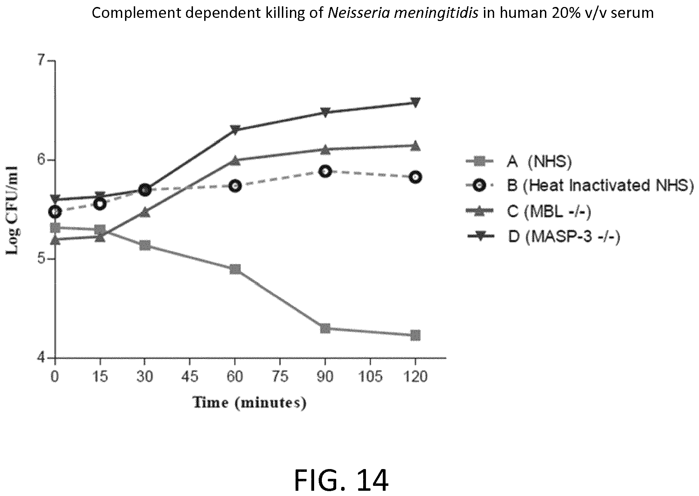

FIGURE 14 graphically illustrates the log cfu/mL of viable counts of *N. meningitidis* serogroup B-MC58 recovered at different time points in the human sera samples shown in TABLE 7, showing that complement-dependent killing of *N. meningitidis* in human 20% (v/v) serum is MASP-3 and MBL-dependent, as described in Example 3;

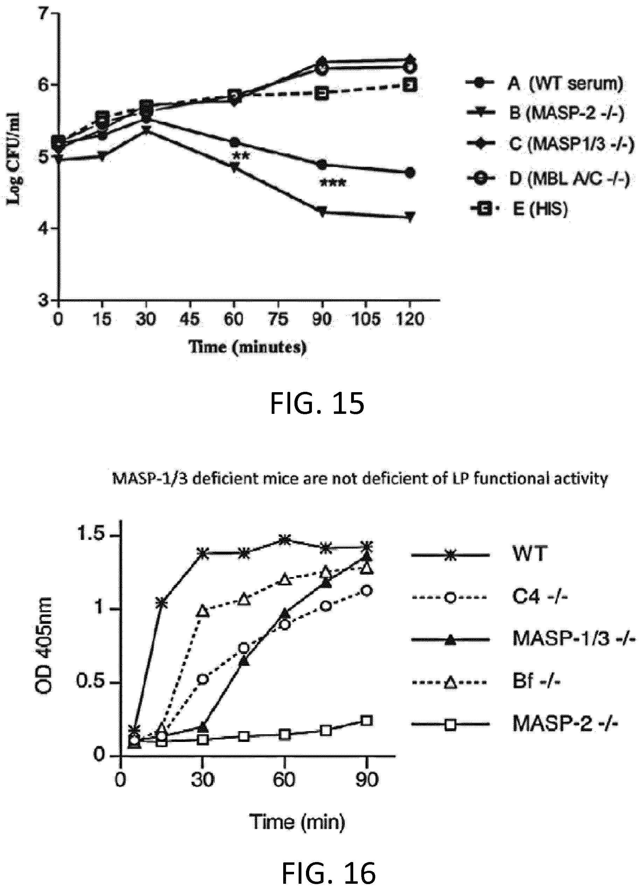

FIGURE 15 graphically illustrates the log cfu/mL of viable counts of *N. meningitidis* serogroup B-MC58 recovered at different time points in the mouse sera samples shown in TABLE 9, showing that the MASP-2 -/- knockout mouse (referred to as "MASP-2 -/-") serum has a higher level of bactericidal activity for N. meningitidis than WT mouse serum, whereas in contrast, the MASP-1/3 -/- mouse serum does not have any bactericidal activity, as described in Example 3;
FIGURE 16 graphically illustrates the kinetics of C3 activation under lectin pathway-specific conditions (1% plasma) in WT, C4-/-, MASP-1/3-/-, Factor B-/- and MASP-2-/- mouse sera, as described in Example 4;

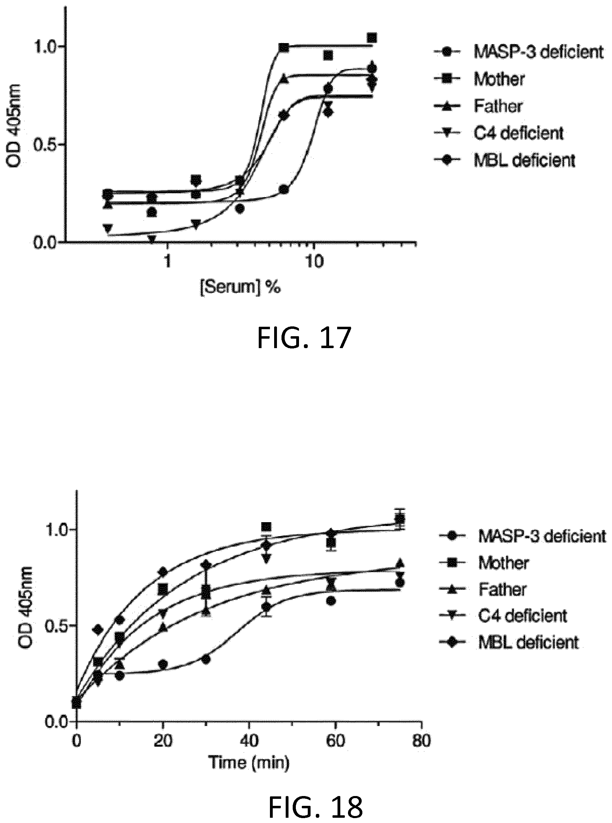

FIGURE 17 graphically illustrates the level of alternative pathway-driven (AP-driven) C3b deposition on zymosan-coated microtiter plates under "traditional" alternative pathway-specific (AP-specific) conditions (*i.e.* BBS/EGTA/Mg⁺⁺ without Ca⁺⁺) as a function of serum concentration in serum samples obtained from MASP-3-deficient, C4-deficient and MBL-deficient human subjects, as described in Example 4;
FIGURE 18 graphically illustrates the level of AP-driven C3b deposition on zymosan-coated microtiter plates under "traditional" AP-specific conditions (*i.e.,* BBS/EGTA/Mg⁺⁺ without Ca⁺⁺) as a function of time in 10% human serum samples obtained from MASP-3-deficient, C4-deficient and MBL-deficient human subjects, as described in Example 4;

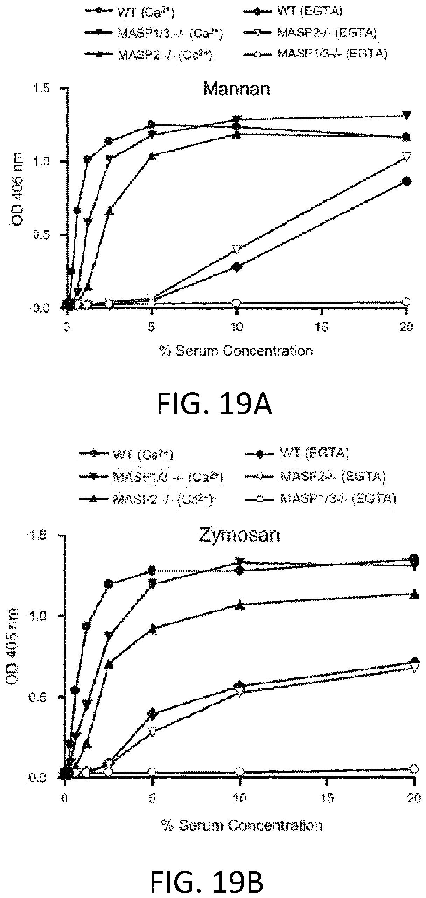

FIGURE 19A graphically illustrates the level of C3b deposition on mannan-coated microtiter plates as a function of serum concentration in serum samples obtained from WT, MASP-2-deficient, and MASP-1/3-deficient mice under "traditional" AP-specific conditions (*i.e.* BBS/EGTA/Mg⁺⁺ without Ca⁺⁺) or under physiological conditions allowing both the lectin pathway and the alternative pathway (AP) to function (BBS/Mg⁺⁺/Ca⁺⁺), as described in Example 4;
FIGURE 19B graphically illustrates the level of C3b deposition on zymosan-coated microtiter plates as a function of serum concentration in serum samples obtained from WT, MASP-2-deficient, and MASP-1/3-deficient mice under traditional AP-specific conditions (*i.e.* BBS/EGTA/Mg⁺⁺ without Ca⁺⁺) or under physiological conditions allowing both the lectin pathway and the alternative pathway to function (BBS/Mg⁺⁺/Ca⁺⁺), as described in Example 4;

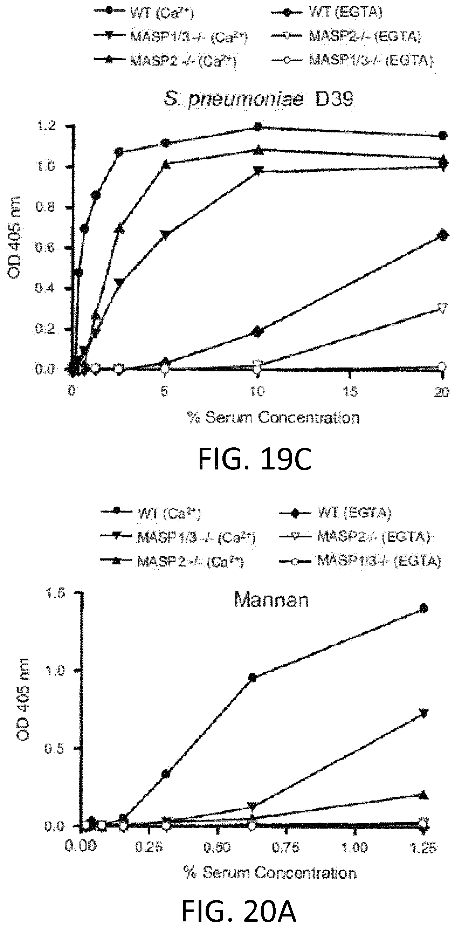

FIGURE 19C graphically illustrates the level of C3b deposition on *S. pneumoniae* D39-coated microtiter plates as a function of serum concentration in serum samples obtained from WT, MASP-2-deficient, and MASP-1/3-deficient mice under traditional AP-specific conditions (*i.e.* BBS/EGTA/Mg⁺⁺ without Ca⁺⁺) or under physiological conditions allowing both the lectin pathway and the alternative pathway to function (BBS/Mg⁺⁺/Ca⁺⁺), as described in Example 4;
FIGURE 20A graphically illustrates the results of a C3b deposition assay in highly diluted sera carried out on mannan-coated microtiter plates under traditional AP-specific conditions (*i.e.* BBS/EGTA/Mg⁺⁺ without Ca⁺⁺) or under physiological conditions allowing both the lectin pathway and the alternative pathway to function (BBS/Mg⁺⁺/Ca⁺⁺), using serum concentrations ranging from 0 % up to 1.25%, as described in Example 4;

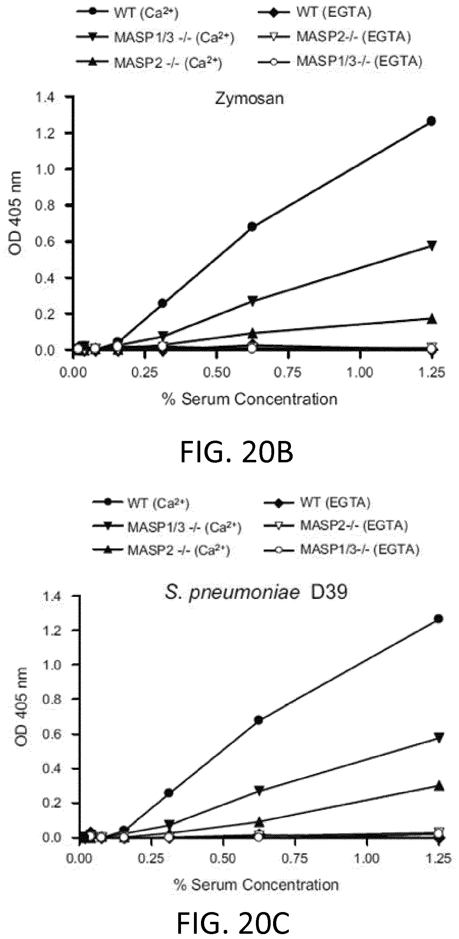

FIGURE 20B graphically illustrates the results of a C3b deposition assay carried out on zymosan-coated microtiter plates under traditional AP-specific conditions (*i.e.* BBS/EGTA/Mg⁺⁺ without Ca⁺⁺) or under physiological conditions allowing both the lectin pathway and the alternative pathway to function (BBS/Mg⁺⁺/Ca⁺⁺), using serum concentrations ranging from 0 % up to 1.25%, as described in Example 4;
FIGURE 20C graphically illustrates the results of a C3b deposition assay carried out on *S. pneumoniae* D39-coated microtiter plates under traditional AP-specific conditions *(i.e.* BBS/EGTA/Mg⁺⁺ without Ca⁺⁺) or under physiological conditions allowing both the lectin pathway and the alternative pathway to function (BBS/Mg⁺⁺/Ca⁺⁺), using serum concentrations ranging from 0 % up to 1.25%, as described in Example 4;

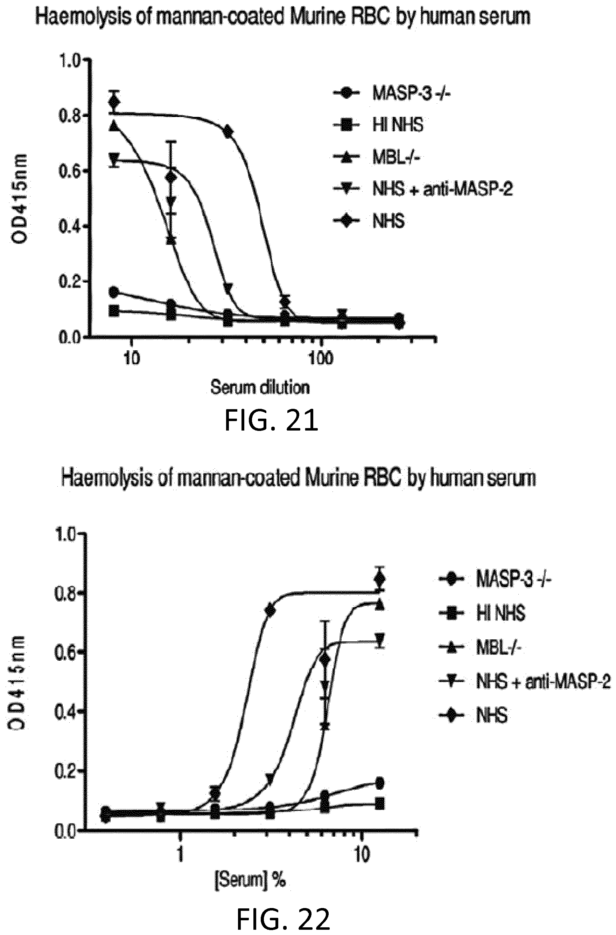

FIGURE 21 graphically illustrates the level of hemolysis (as measured by hemoglobin release of lysed mouse erythrocytes (Crry/C3-/-) into the supernatant measured by photometry) of mannan-coated murine erythrocytes by human serum under physiological conditions (*i.e.,* in the presence of Ca⁺⁺) over a range of serum dilutions in serum from MASP-3-/-, heat inactivated normal human serum (HI NHS), MBL-/-, NHS + MASP-2 monoclonal antibody and NHS control, as described in Example 5;
FIGURE 22 graphically illustrates the level of hemolysis (as measured by hemoglobin release of lysed mouse erythrocytes (Crry/C3-/-) into the supernatant measured by photometry) of mannan-coated murine erythrocytes by human serum under physiological conditions (i.e., in the presence of Ca⁺⁺) over a range of serum concentration in serum from MASP-3-/-, heat inactivated (HI) NHS, MBL-/-, NHS + MASP-2 monoclonal antibody and NHS control, as described in Example 5;

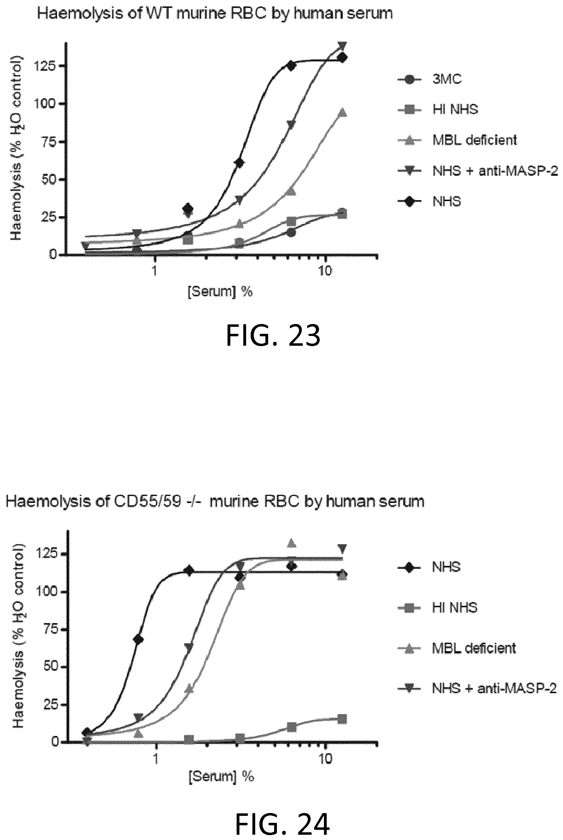

FIGURE 23 graphically illustrates the level of hemolysis (as measured by hemoglobin release of lysed WT mouse erythrocytes into the supernatant measured by photometry) of non-coated murine erythrocytes by human serum under physiological conditions (i.e., in the presence of Ca⁺⁺) over a range of serum concentrations in serum from 3MC (MASP-3-/-), heat inactivated (HI) NHS, MBL-/-, NHS + MASP-2 monoclonal antibody and NHS control, as described in Example 5;
FIGURE 24 graphically illustrates hemolysis (as measured by hemoglobin release of lysed mouse erythrocytes (CD55/59-/-) into the supernatant measured by photometry) of non-coated murine erythrocytes by human serum under physiological conditions (i.e., in the presence of Ca⁺⁺) over a range of serum concentrations in serum from heat inactivated (HI) NHS, MBL-/-, NHS + MASP-2 monoclonal antibody and NHS control, as described in Example 5;

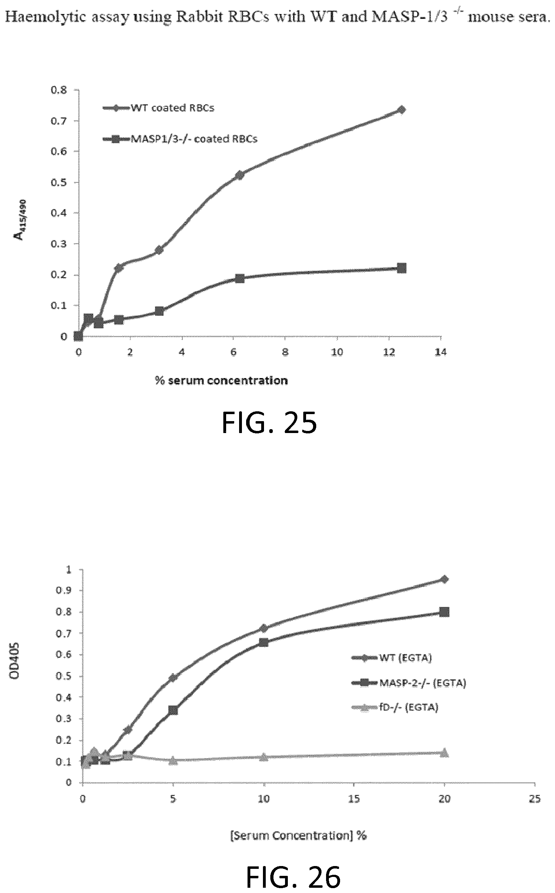

FIGURE 25 graphically illustrates hemolysis (as measured by hemoglobin release of lysed rabbit erythrocytes into the supernatant measured by photometry) of mannan-coated rabbit erythrocytes by MASP-1/3-/- mouse serum and WT control mouse serum under physiological conditions (*i.e.,* in the presence of Ca⁺⁺) over a range of serum concentrations, as described in Example 6;
FIGURE 26 graphically illustrates the level of C3b deposition (OD 405 nm) on a zymosan-coated microtiter plate as a function of serum concentration in serum samples from factor D-/-, MASP-2-/- and WT mouse sera in a C3 deposition assay carried out under AP-specific conditions, as described in Example 7;

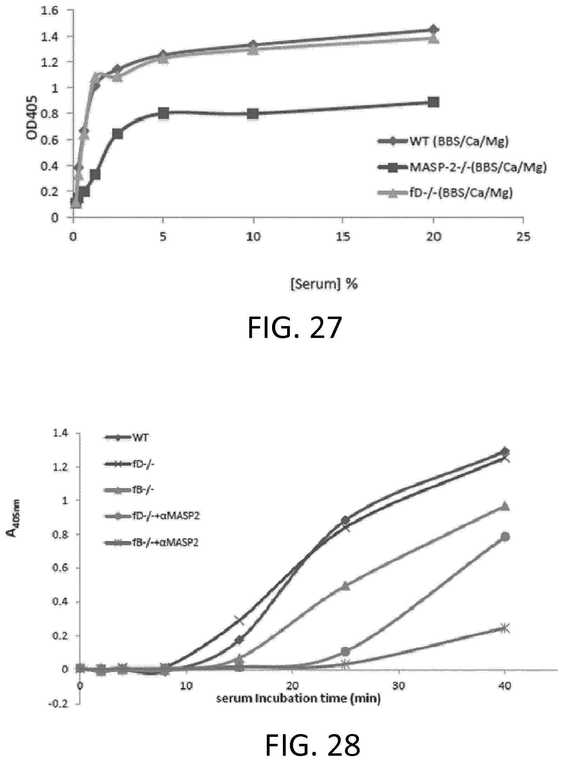

FIGURE 27 graphically illustrates the level of C3b deposition (OD 405 nm) on a zymosan-coated microtiter plate as a function of serum concentration in serum samples from factor D-/-; MASP-2-/- and WT mouse sera in a C3 deposition assay carried out under physiological conditions (in the presence of Ca⁺⁺), as described in Example 7;
FIGURE 28 graphically illustrates the level of C3b deposition (OD 405 nm) on a zymosan-coated microtiter plate as a function of serum incubation time (minutes) in mouse serum samples obtained from factor D-/-; factor B-/-; plus and minus MASP-2 monoclonal antibody in a C3b deposition assay carried out under physiological conditions (in the presence of Ca⁺⁺), as described in Example 7;

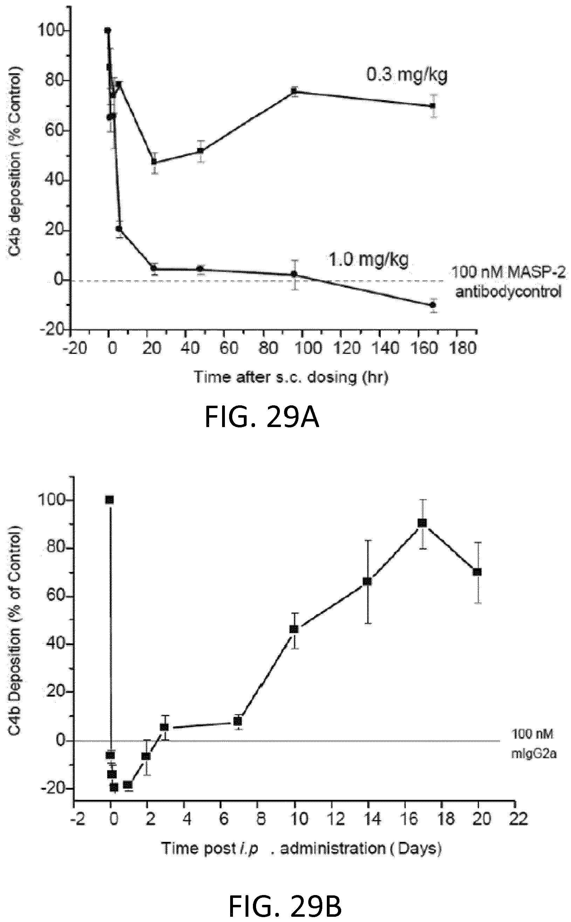

FIGURE 29A graphically illustrates lectin pathway specific C4b deposition on a zymosan-coated microtiter plate, measured *ex vivo* in undiluted serum samples taken from mice (n=3 mice/group) at various time points after subcutaneous dosing of either 0.3 mg/kg or 1.0 mg/kg of the mouse MASP-2 MoAb, as described in Example 13;
FIGURE 29B graphically illustrates the time course of lectin pathway recovery for three weeks following a single intraperitoneal administration of mouse MASP-2 MoAb at 0.6 mg/kg in mice, as described in Example 13;

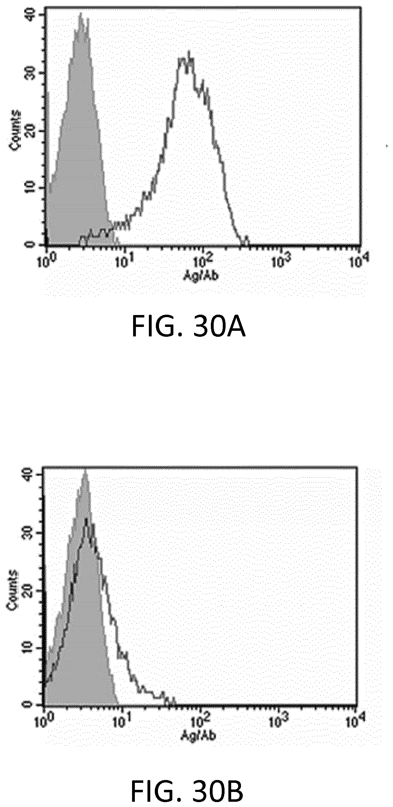

FIGURE 30A is a FACS histogram of MASP-3 antigen/antibody binding for clone M3J5, as described in Example 15;
FIGURE 30B is a FACS histogram of MASP-3 antigen/antibody binding for clone M3M1, as described in Example 15;

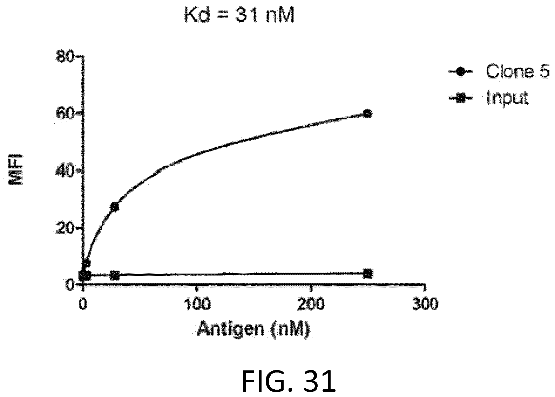

FIGURE 31 graphically illustrates a saturation binding curve of clone M3J5 (Clone 5) for the MASP-3 antigen, as described in Example 15;

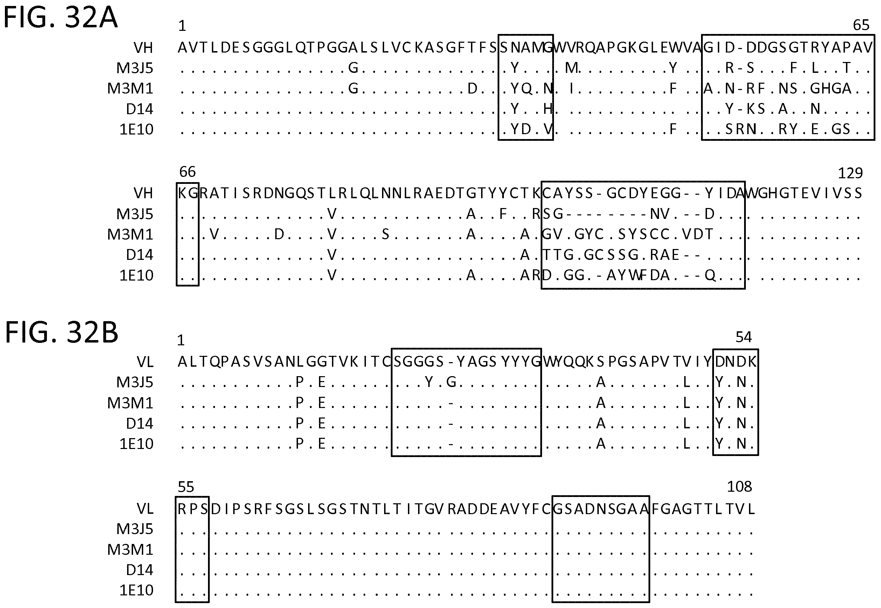

FIGURE 32A is an amino acid sequence alignment of the VH regions of M3J5, M3M1, D14 and 1E10 to the chicken DT40 VH sequence, wherein dots represent amino acid identity with the DT40 sequence and dashes indicate spaces introduced to maximize the alignment, as described in Example 15;
FIGURE 32B is an amino acid sequence alignment of the VL regions of M3J5, M3M1, D14 and 1E10 to the chicken DT40 VL sequence, wherein dots represent amino acid identity with the DT40 sequence and dashes indicate spaces introduced to maximize the alignment, as described in Example 15;

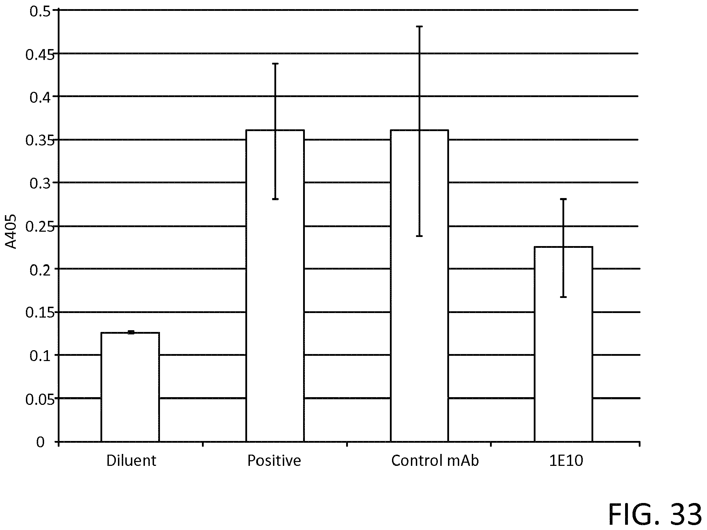

FIGURE 33 is a bar graph showing the inhibitory activity of the mAb1E10 in the Wieslab Complement System Screen, MBL Pathway in comparison to the positive serum provided with the assay kit, as well as an isotype control antibody, demonstrating that mAb1E10 partial inhibits LEA-2-dependent activation, (via inhibition of MASP-1-dependent activation of MASP-2), whereas the isotype control antibody does not, as described in Example 15;

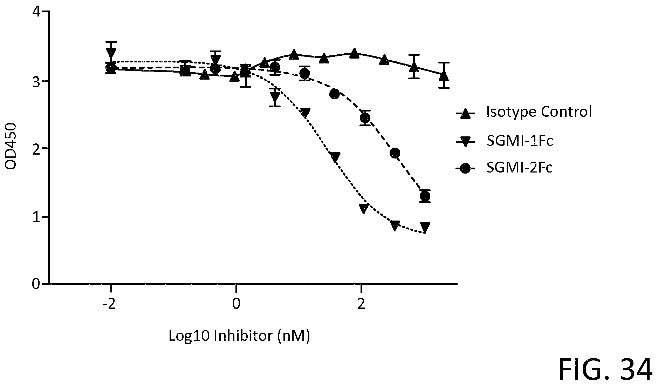

FIGURE 34 graphically illustrates the level of C3b deposition for 1% normal human serum plus isotype control, SGMI-1Fc or SGMI-2Fc over a concentration range of 0.15 to 1000 nM, demonstrating that both SGMI-1Fc and SGMI-2Fc inhibited C3b deposition from normal serum in mannan-coated ELISA wells, with IC₅₀ values of approximately 27nM and 300nM, respectively, as described in Example 16;

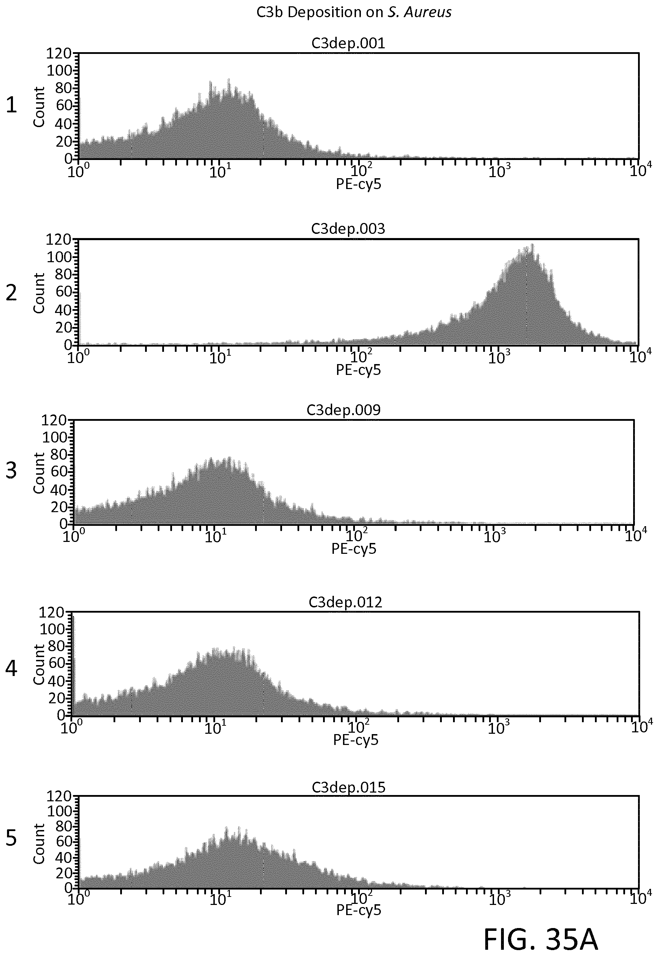

FIGURE 35A provides the results of flow cytometry analysis for C3b deposition on heat-killedS *Staphylococcus aureus*, demonstrating that in normal human serum in the presence of EDTA, which is known to inactivate the lectin and alternative pathways, no C3b deposition was observed (panel 1), in normal human serum treated with Mg⁺⁺/EGTA, alternative pathway-driven C3b deposition is observed (panel 2), and as shown in panel 3, 4 and 5, in factor B-depleted, factor D-depleted and properdin (factor P)-depleted serum, respectively, no alternative pathway driven C3b deposition is observed, as described in Example 17;

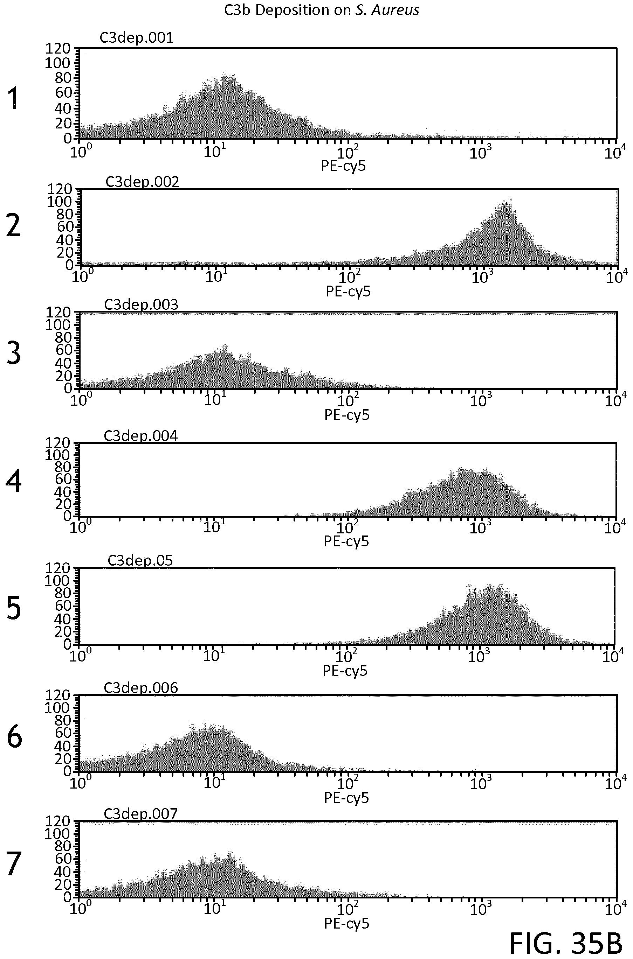

FIGURE 35B provides the results of flow cytometry analysis for C3b deposition on heat-killed *S. aureus*, demonstrating that, as in EDTA-treated normal serum (panel 1), AP-driven C3b deposition is absent in 3MC serum in the presence of Mg⁺⁺/EGTA (panel 3), whereas panels 4 and 5 show that active full length rMASP-3 (panel 4) and active rMASP-3 (CCP1-CCP2-SP) (panel 5) both restore AP-driven C3b deposition in 3MC serum to levels observed in normal serum treated with Mg⁺⁺/EGTA (panel 2), neither inactive rMASP-3 (S679A) (panel 6) nor wild type rMASP-1 (panel 7) can restore AP-driven C3b deposition in 3MC serum, as described in Example 17;

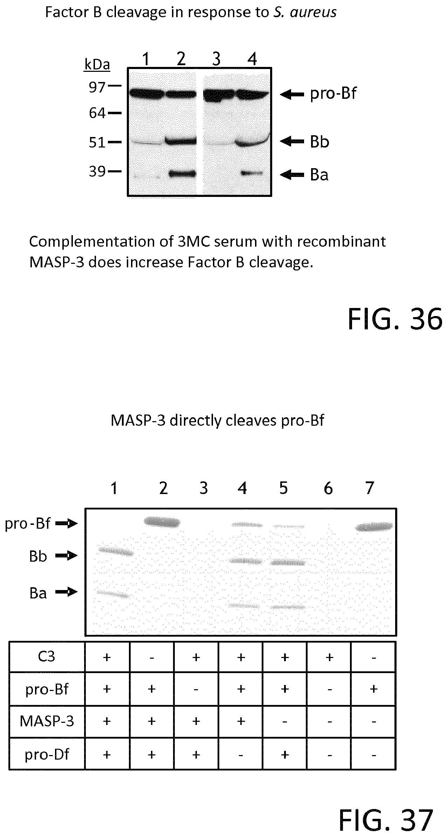

FIGURE 36 shows the results of a Western blot analysis to determine factor B cleavage in response to *S. aureus* in 3MC serum in the presence or absence of rMASP-3, demonstrating that the normal human serum in the presence of EDTA (negative control, lane 1) demonstrates very little Factor B cleavage relative to normal human serum in the presence of Mg⁺⁺/EGTA, shown in lane 2 (positive control), as further shown in lane 3, 3MC serum demonstrates very little Factor B cleavage in the presence of Mg⁺⁺/EGTA. However, as shown in lane 4, Factor B cleavage is restored by the addition and preincubation of full-length, recombinant MASP-3 protein to the 3MC serum, as described in Example 17;
FIGURE 37 shows Comassie staining of a protein gel in which Factor B cleavage is analyzed, demonstrating that Factor B cleavage is most optimal in the presence of C3, MASP-3 and pro-factor D (lane 1), and as shown in lanes 4 and 5, either MASP-3 or pro-factor D alone are able to mediate Factor B cleavage, as long as C3 is present, as described in Example 17;

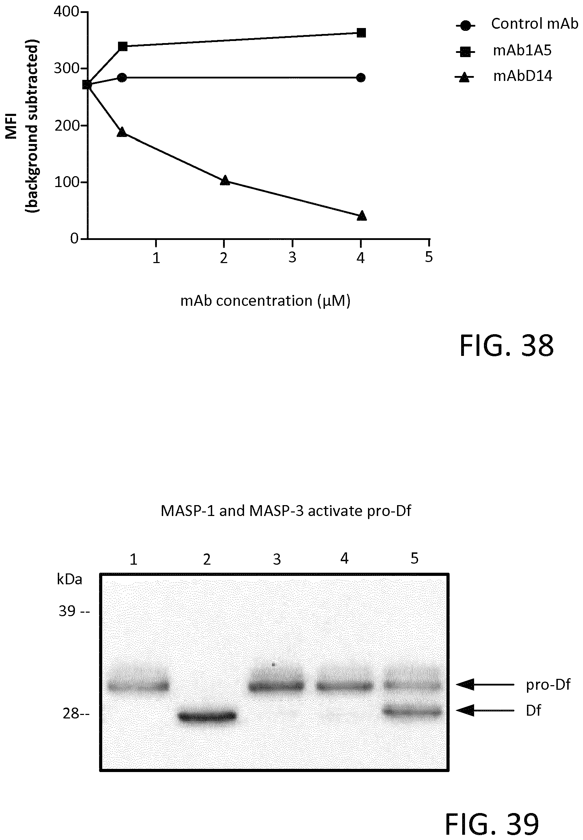

FIGURE 38 graphically illustrates the mean fluorescent intensities (MFI) of C3b staining *of S. aureus* obtained from mAbD14 (which binds MASP-3), mAb1A5 (negative control antibody) and an isotype control antibody plotted as a function of mAb concentration in 3MC serum in the presence of rMASP-3, demonstrating that mAbD14 inhibits MASP-3-dependent C3b deposition in a concentration-dependent manner, as described in Example 17;
FIGURE 39 shows Western blot analysis of pro-factor D substrate cleavage, wherein compared to pro-factor D alone (lane 1) or the inactive full length recombinant MASP-3 (S679A; lane 3) or MASP-1 (S646A; lane 4), full length wild type recombinant MASP-3 (lane2) and MASP-1 (lane 5) either completely or partially cleave pro-factor D to generate mature factor D, as described in Example 18;

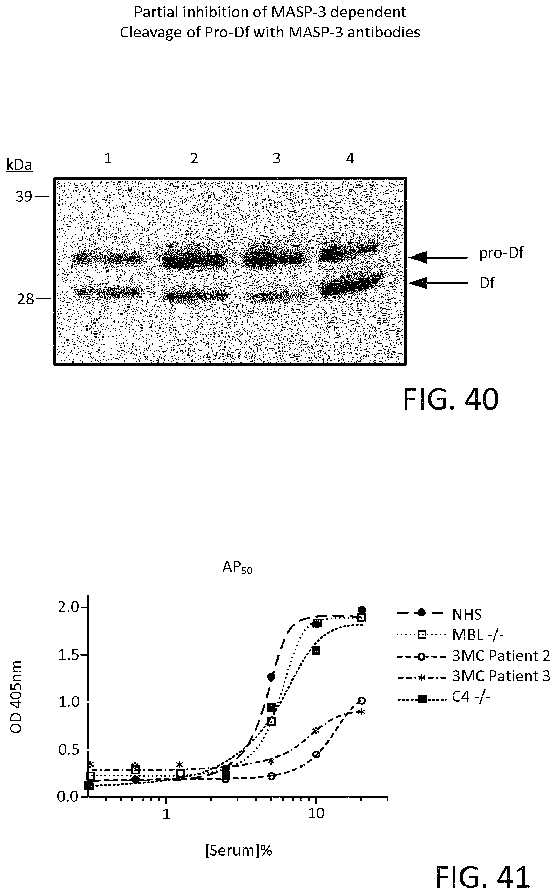

FIGURE 40 is a Western blot showing the inhibitory activity of the MASP-3 binding mAbs D14 (lane 2) and M3M1 (lane 3) on MASP-3-dependent pro-factor D cleavage in comparison to a control reaction containing only MASP-3 and pro-factor D (no mAb, lane 1), as well as a control reaction containing a mAb obtained from the DTLacO library that binds MASP-1, but not MASP-3 (lane 4), as described in Example 18;
FIGURE 41 graphically illustrates the level of AP-driven C3b deposition on zymosan-coated microtiter plates as a function of serum concentration in serum samples obtained from MASP-3-deficient (3MC), C4-deficient and MBL-deficient subjects, demonstrating that MASP-3-deficient sera from Patient 2 and Patient 3 have residual AP activity at high serum concentrations (25%, 12.5%, 6.25% serum concentrations), but a significantly higher AP₅₀ (i.e., 8.2% and 12.3% of serum needed to achieve 50% of maximum C3 deposition), as described in Example 19;

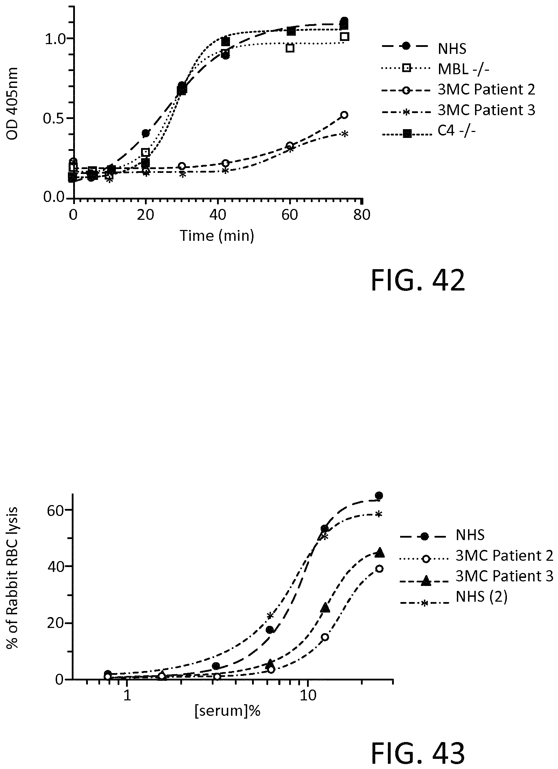

FIGURE 42 graphically illustrates the level of AP-driven C3b deposition on zymosan-coated microtiter plates under "traditional" AP-specific conditions (i.e., BBS/EGTA/Mg⁺⁺ without Ca⁺⁺) as a function of time in 10% human serum samples obtained from MASP-3 deficient, C4-deficient and MBL-deficient human subjects, as described in Example 19;
FIGURE 43 graphically illustrates the percent hemolysis (as measured by hemoglobin release of lysed rabbit erythrocytes into the supernatant measured by photometry) of mannan-coated rabbit erythrocytes over a range of serum concentrations in serum from two normal human subjects (NHS) and from two 3MC patients (Patient 2 and Patient 3), measured in the absence of Ca⁺⁺, demonstrating that MASP-3 deficiency reduces the percentage of complement-mediated lysis of mannan-coated erythrocytes as compared to normal human serum, as described in Example 19;

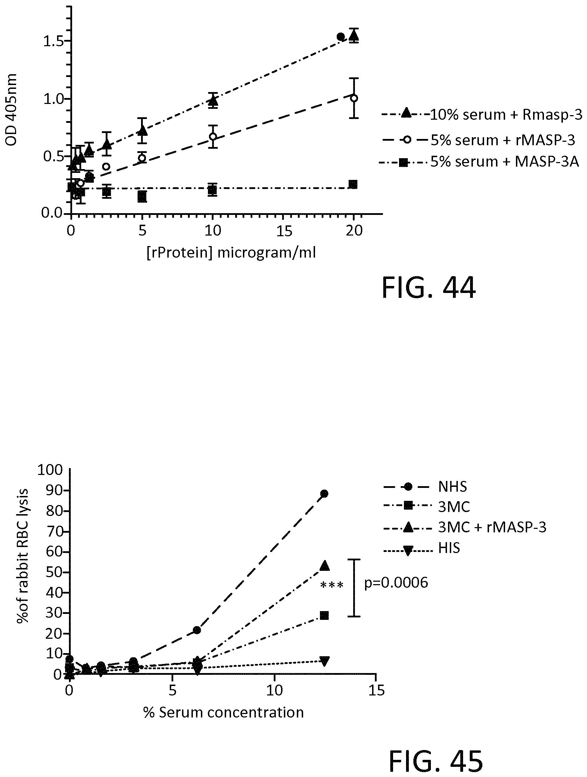

FIGURE 44 graphically illustrates the level of AP-driven C3b deposition on zymosan-coated microtiter plates as a function of the concentration of recombinant full length MASP-3 protein added to serum samples obtained from human 3MC Patient 2 (MASP-3^{-/-}), demonstrating that, compared to the negative control inactive recombinant MASP-3 (MASP-3A; S679A), active recombinant MASP-3 protein reconstitutes AP-driven C3b deposition on zymosan-coated plates in a concentration-dependent manner, as described in Example 19;
FIGURE 45 graphically illustrates the percent hemolysis (as measured by hemoglobin release of lysed rabbit erythrocytes into the supernatant measured by photometry) of mannan-coated rabbit erythrocytes over a range of serum concentrations in (1) normal human serum (NHS); (2) 3MC patient serum; (3) 3MC patient serum plus active full length recombinant MASP-3 (20 µg/ml); and (4) heat-inactivated human serum (HIS), measured in the absence of Ca⁺⁺, demonstrating that the percent lysis of rabbit erythrocytes is significantly increased in 3MC serum containing rMASP-3 as compared to the percent lysis in 3MC serum without recombinant MASP-3 (p=0.0006), as described in Example 19;

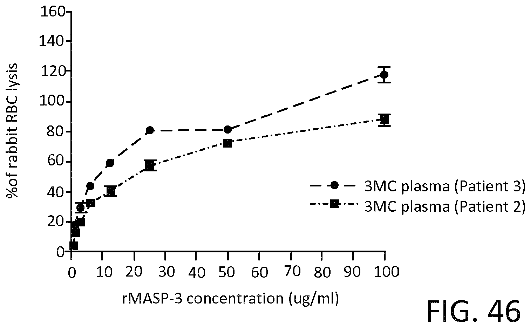

FIGURE 46 graphically illustrates the percentage of rabbit erythrocyte lysis in 7% human serum from 3MC Patient 2 and from 3MC Patient 3 containing active recombinant MASP-3 at a concentration range of 0 to 110 µg/ml (in BBS/ Mg⁺⁺/EGTA, demonstrating that the percentage of rabbit erythrocyte lysis increases with the amount of recombinant MASP-3 in a concentration-dependent manner, as described in Example 19; and

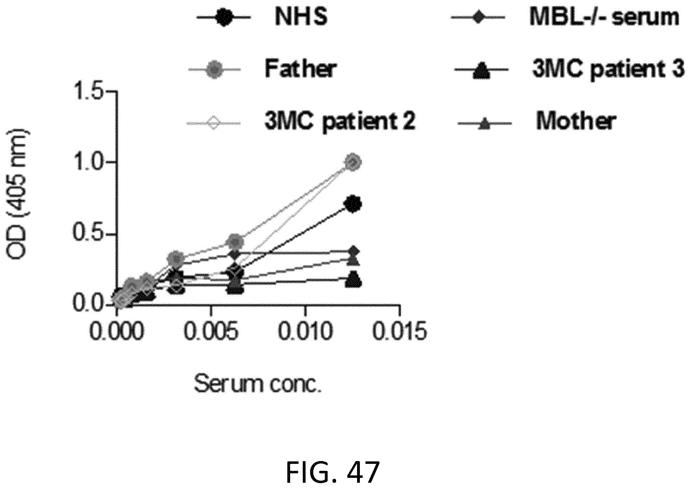

FIGURE 47 graphically illustrates the level of LEA-2-driven C3b deposition on Mannan-coated ELISA plates as a function of the concentration of human serum diluted in BBS buffer, for serum from a normal human subject (NHS), from two 3MC patients (Patient 2 and Patient 3), from the parents of Patient 3 and from a MBL-deficient subject.

### DESCRIPTION OF SEQUENCE LISTING

SEQ ID NO:1 human MAp19 cDNA
SEQ ID NO:2 human MAp19 protein (with leader)
SEQ ID NO:3 human MAp19 protein (mature)
SEQ ID NO:4 human MASP-2 cDNA
SEQ ID NO:5 human MASP-2 protein (with leader)
SEQ ID NO:6 human MASP-2 protein (mature)
SEQ ID NO:7 human MASP-3 cDNA
SEQ ID NO:8 human MASP-3 protein (w/leader)
SEQ ID NO:9 human MASP-1 cDNA
SEQ ID NO:10 human MASP-1 protein (w/leader)
SEQ ID NO:11 human MAp44 protein (w/leader)
SEQ ID NO: 12 rat MASP-2 cDNA
SEQ ID NO: 13 rat MASP-2 protein (with leader)
SEQ ID NO:14 DNA encoding 17D20_dc35VH21N11VL (OMS646) heavy chain variable region (VH) (without signal peptide)
SEQ ID NO:15 17D20_dc35VH21N11VL (OMS646) heavy chain variable region (VH) polypeptide
SEQ ID NO: 16 17N16mc heavy chain variable region (VH) polypeptide
SEQ ID NO:17 17D20_dc21N11VL (OMS644) light chain variable region (VL) polypeptide
SEQ ID NO:18 DNA encoding 17N16_dc17N9 (OMS641) light chain variable region (VL) (without signal peptide)
SEQ ID NO:19 17N16_dc17N9 (OMS641) light chain variable region (VL) polypeptide
SEQ ID NO:20: scFv daughter clone 17N16m_d17N9 full length polypeptide
SEQ ID NO:21: scFv daughter clone 17D20m_d3521N11 full length polypeptide
SEQ ID NO:22: scFv daughter clone 17N16m_d17N9 DNA encoding full length polypeptide (without signal peptide)
SEQ ID NO:23: scFv daughter clone 17D20m_d3521N11 DNA encoding full length polypeptide (without signal peptide)
SEQ ID NO:24: parent DTLacO heavy chain variable region (VH) polypeptideSEQ ID NO:25: MASP-3 specific clone M3J5 heavy chain variable region (VH) polypeptide
SEQ ID NO:26: MASP-3 specific clone M3M1 heavy chain variable region (VH) polypeptide
SEQ ID NO:27: parent DTLacO light chain variable region (VL) polypeptide
SEQ ID NO:28: MASP-3 specific clone M3J5 light chain variable region (VL) polypeptide
SEQ ID NO:29: MASP-3 specific clone M3M1 light chain variable region (VL) polypeptide
SEQ ID NO:30: MASP-3 clone D14 heavy chain variable region (VH) polypeptide
SEQ ID NO:31: MASP-3 clone D14 light chain variable region (VL) polypeptide
SEQ ID NO:32: MASP-1 clone 1E10 heavy chain variable region (VH) polypeptide
SEQ ID NO:33: MASP-1 clone 1E10 light chain variable region (VL) polypeptide
SEQ ID NO:34 SGMI-1 peptide
SEQ ID NO:35 SGMI-2 peptide
SEQ ID NO:36 human IgG1-Fc polypeptide;
SEQ ID NO:37 peptide linker #1 (12aa);
SEQ ID NO:38: peptide linker #2 (10aa);
SEQ ID NO:39: nucleic acid encoding polypeptide fusion comprising the human IL-2-signal sequence, SGMI-1, linker#1, and human IgG1-Fc;
SEQ ID NO:40: mature polypeptide fusion comprising SGMI-1, linker#1 and human IgG1-Fc (SGMI-1Fc);
SEQ ID NO:41: nucleic acid encoding polypeptide fusion comprising the human IL-2-signal sequence, SGMI-2, linker#1 and human IgG1-Fc;
SEQ ID NO:42: mature polypeptide fusion comprising SGMI-2, linker#1 and human IgG1-Fc (SGMI-2Fc).

### DETAILED DESCRIPTION

### I. DEFINITIONS

Unless specifically defined herein, all terms used herein have the same meaning as would be understood by those of ordinary skill in the art of the present disclosure. The following definitions are provided in order to provide clarity with respect to the terms as they are used in the specification and claims to describe the present disclosure.

As used herein, the lectin pathway effector arm 1 ("LEA-1") refers to lectin-dependent activation of factor B and factor D by MASP-3.

As used herein, the lectin pathway effector arm 2 ("LEA-2") refers to MASP-2-dependent complement activation.

As used herein, the term "MASP-3-dependent complement activation" comprises two components: (i) lectin MASP-3-dependent activation of factor B and factor D, encompassed in LEA-1-mediated complement activation, occurs in the presence of Ca⁺⁺, commonly leading to the conversion of C3bB to C3bBb and of pro-factor D to factor D; and (ii) lectin-independent conversion of factor B and factor D, which can occur in the absence of Ca⁺⁺, commonly leading to the conversion of C3bB to C3bBb and of pro-factor D to factor D. LEA-1-mediated complement activation and lectin-independent conversion of factor B and factor D have been determined to cause opsonization and/or lysis. While not wishing to be bound by any particular theory, it is believed that only when multiple C3b molecules associate and bind in close proximity, the C3bBb C3 convertase changes its substrate specificity and cleaves C5 as the alternative pathway C5 convertase termed C3bBb(C3b)n.

As used herein, the term "MASP-2-dependent complement activation", also referred to herein as LEA-2-mediated complement activation, comprises MASP-2 lectin-dependent activation, which occurs in the presence of Ca⁺⁺, leading to the formation of the lectin pathway C3 convertase C4b2a and upon accumulation of the C3 cleavage product C3b subsequently to the C5 convertase C4b2a(C3b)n, which has been determined to cause opsonization and/or lysis.

As used herein, the term "traditional understanding of the alternative pathway" also referred to as the "traditional alternative pathway" refers to the alternative pathway prior to the instant discovery described herein, *i.e*., complement activation that is triggered, for example, by zymosan from fungal and yeast cell walls, lipopolysaccharide (LPS) from Gram negative outer membranes, and rabbit erythrocytes, as well as from many pure polysaccharides, viruses, bacteria, animal tumor cells, parasites and damaged cells, and which has traditionally been thought to arise from spontaneous proteolytic generation of C3b from complement factor C3. As used herein, activation of the "traditional alternative pathway", also referred to herein as the "alternative pathway", is measured in Mg⁺⁺/EGTA buffer *(i.e.,* in the absence of Ca⁺⁺).

As used herein, the term "lectin pathway" refers to complement activation that occurs via the specific binding of serum and non-serum carbohydrate-binding proteins including mannan-binding lectin (MBL), CL-11 and the ficolins (H-ficolin, M-ficolin, or L-ficolin). As described herein, the inventors have discovered that the lectin pathway is driven by the two effector arms, lectin pathway effector arm 1 (LEA-1), which is now known to be MASP-3-dependent, and lectin pathway effector arm 2 (LEA-2), which is MASP-2-dependent. As used herein, activation of the lectin pathways are assessed using Ca⁺⁺ containing buffers.

As used herein, the term "classical pathway" refers to complement activation that is triggered by antibody bound to a foreign particle and requires binding of the recognition molecule C1q.

As used herein, the term "HTRA-1" refers to the serine peptidase High-temperature requirement serine protease A1.

As used herein, the term "MASP-3 inhibitory agent" refers to any agent that directly or indirectly inhibits MASP-3-dependent complement activation, including agents that bind to or directly interact with MASP-3, including MASP-3 antibodies and MASP-3 binding fragments thereof, natural and synthetic peptides, competitive substrates, small-molecules, expression inhibitors and isolated natural inhibitors, and also encompasses peptides that compete with MASP-3 for binding to another recognition molecule (e.g., MBL, CL-11, H-ficolin, M-ficolin, or L-ficolin) in the lectin pathway. In one case, the MASP-3 inhibitory agent is specific to MASP-3, and does not bind to MASP-1 or MASP-2. An inhibitory agent that directly inhibits MASP-3 can be referred to as a direct MASP-3 inhibitory agent (e.g., a MASP-3 antibody), while an inhibitory agent that indirectly inhibits MASP-3 can be referred to as an indirect MASP-3 inhibitory agent (e.g., a MASP-1 antibody that inhibits MASP-3 activation). An example of a direct MASP-3 inhibitory agent is a MASP-3 specific inhibitory agent, such as a MASP-3 inhibitory agent that specifically binds to a portion of MASP-3 (SEQ ID NO:8) with a binding affinity of at least 10 times greater than to other components in the complement system. In one case, a MASP-3 inhibitory agent indirectly inhibits MASP-3 activity, such as, for example, an inhibitor of MASP-3 activation, including an inhibitor of MASP-1-mediated MASP-3 activation (e.g., a MASP-1 antibody or MASP-1 binding fragments thereof, natural and synthetic peptides, small-molecules, expression inhibitors and isolated natural inhibitors, and also encompasses peptides that compete with MASP-1 for binding to MASP-3). In another case, a MASP-3 inhibitory agent inhibits MASP-3-mediated maturation of factor D. In another case, a MASP-3 inhibitory agent inhibits MASP-3-mediated activation of factor B. MASP-3 inhibitory agents useful in the method of the disclosure may reduce MASP-3-dependent complement activation by greater than 10%, such as greater than 20%, greater than 50%, or greater than 90%. In one case, the MASP-3 inhibitory agent reduces MASP-3-dependent complement activation by greater than 90% (i.e., resulting in MASP-3 complement activation of only 10% or less). It is expected that MASP-3 inhibition will block, in full or in part, both LEA-1-related lysis and opsonization and lectin-independent conversion of factor B and factor D-related lysis and opsonization.

As used herein, the term "MASP-1 inhibitory agent" refers to any agent that binds to or directly interacts with MASP-1 and inhibits at least one of (i) MASP-3-dependent complement activation and/or (ii) MASP-2-dependent complement activation and/or (iii) lectin-independent or lectin-dependent MASP-1-mediated maturation of factor D, wherein the lectin-dependent MASP-1 maturation of factor D involves direct activation of factor D, including MASP-1 antibodies and MASP-1 binding fragments thereof, natural and synthetic peptides, small-molecules, expression inhibitors and isolated natural inhibitors, and also encompasses peptides that compete with MASP-1 for binding to another recognition molecule (e.g., MBL, CL-11, H-ficolin, M-ficolin, or L-ficolin) in the lectin pathway. MASP-1 inhibitory agents useful in the method of the disclosure reduce MASP-3-dependent complement activation by greater than 10%, such as greater than 20%, greater than 50%, or greater than 90%. The MASP-1 inhibitory agent reduces MASP-3-dependent complement activation by greater than 90% (i.e., resulting in MASP-3 complement activation of only 10% or less). In another case, MASP-1 inhibitory agents useful in the method of the disclosure reduce MASP-2-dependent complement activation by greater than 10%, such as greater than 20%, greater than 50%, or greater than 90%. In one case, the MASP-1 inhibitory agent reduces MASP-2-dependent complement activation by greater than 90% (i.e., resulting in MASP-2 complement activation of only 10% or less).

MASP-1 inhibitory agents useful in the method of the disclosure reduce both MASP-3-dependent complement activation (LEA-1), lectin-independent conversion of factor B and factor D, and MASP-2-dependent complement activation (LEA-2) by greater than 10%, such as greater than 20%, greater than 50%, or greater than 90%. The MASP-1 inhibitory agent reduces MASP-3-dependent complement activation (LEA-1), lectin-independent conversion of factor B and factor D, and MASP-2-dependent complement activation (LEA-2) by greater than 90% (i.e., resulting in MASP-3 complement activation of only 10% or less and MASP-2 complement activation of only 10% or less).

An example of a direct MASP-1 inhibitory agent is a MASP-1-specific inhibitory agent, such as a MASP-1 inhibitory agent that specifically binds to a portion of MASP-1 (SEQ ID NO:10) with a binding affinity of at least 10 times greater than to other components in the complement system. In many instances, given that MASP-1 can activate MASP-3, and given the MASP-1 can activate MASP-2, inhibition of MASP-1 would be expected to be effective in inhibiting MASP-3 and/or MASP-2. In some instances, however, inhibition of either MASP-1 or MASP-3 or MASP-2 may be a preferred case relative to inhibition of the other MASP targets. For example, in the setting of *Staphylococcus aureus (S. aureus)* infection, MASP-3 has been shown to be activated and is responsible for *S. aureus* opsonization in the absence of MASP-1 (see Iwaki D. et al., J Immunol 187(7):3751-8 (2011)). Therefore, in the treatment of paroxysmal nocturnal hemoglobinuria (PNH), for example, it might be advantageous to directly inhibit MASP-1 rather than MASP-3, thereby reducing the potential susceptibility to *S. aureus* during LEA-1-inhibitory treatment of PNH.

As used herein, the term "MASP-2 inhibitory agent" refers to any agent that binds to or directly interacts with MASP-2 and inhibits at least one of (i) MASP-2-dependent complement activation and/or (ii) MASP-1-dependent complement activation, including MASP-2 antibodies and MASP-2 binding fragments thereof, natural and synthetic peptides, small-molecules, expression inhibitors and isolated natural inhibitors, and also encompasses peptides that compete with MASP-2 for binding to another recognition molecule (e.g., MBL, CL-11, H-ficolin, M-ficolin, or L-ficolin) in the lectin pathway. MASP-2 inhibitory agents useful in the method of the disclosure may reduce MASP-2-dependent complement activation by greater than 10%, such as greater than 20%, greater than 50%, or greater than 90%. In one case, the MASP-2 inhibitory agent reduces MASP-2-dependent complement activation by greater than 90% (i.e., resulting in MASP-2 complement activation of only 10% or less). An example of a direct MASP-2 inhibitory agent is a MASP-2-specific inhibitory agent, such as a MASP-2 inhibitory agent that specifically binds to a portion of MASP-2 (SEQ ID NO:5) with a binding affinity of at least 10 times greater than to other components in the complement system.

As used herein, the term "antibody" encompasses antibodies and antibody fragments thereof, derived from any antibody-producing mammal (e.g., mouse, rat, rabbit, and primate including human), or from a hybridoma, phage selection, recombinant expression or transgenic animals (or other methods of producing antibodies or antibody fragments"), that specifically bind to a target polypeptide, such as, for example, MASP-1, MASP-2 or MASP-3 polypeptides or portions thereof. It is not intended that the term "antibody" limited as regards to the source of the antibody or the manner in which it is made (e.g., by hybridoma, phage selection, recombinant expression, transgenic animal, peptide synthesis, etc). Exemplary antibodies include polyclonal, monoclonal and recombinant antibodies; pan-specific, multispecific antibodies (e.g., bispecific antibodies, trispecific antibodies); humanized antibodies; murine antibodies; chimeric, mouse-human, mouse-primate, primate-human monoclonal antibodies; and anti-idiotype antibodies, and may be any intact antibody or fragment thereof. As used herein, the term "antibody" encompasses not only intact polyclonal or monoclonal antibodies, but also fragments thereof (such as dAb, Fab, Fab', F(ab')₂, Fv), single chain (ScFv), synthetic variants thereof, naturally occurring variants, fusion proteins comprising an antibody portion with an antigen-binding fragment of the required specificity, humanized antibodies, chimeric antibodies, and any other modified configuration of the immunoglobulin molecule that comprises an antigen-binding site or fragment (epitope recognition site) of the required specificity.

A "monoclonal antibody" refers to a homogeneous antibody population wherein the monoclonal antibody is comprised of amino acids (naturally occurring and non-naturally occurring) that are involved in the selective binding of an epitope. Monoclonal antibodies are highly specific for the target antigen. The term "monoclonal antibody" encompasses not only intact monoclonal antibodies and full-length monoclonal antibodies, but also fragments thereof (such as Fab, Fab', F(ab')₂, Fv), single chain (ScFv), variants thereof, fusion proteins comprising an antigen-binding portion, humanized monoclonal antibodies, chimeric monoclonal antibodies, and any other modified configuration of the immunoglobulin molecule that comprises an antigen-binding fragment (epitope recognition site) of the required specificity and the ability to bind to an epitope. It is not intended to be limited as regards the source of the antibody or the manner in which it is made (*e.g.,* by hybridoma, phage selection, recombinant expression, transgenic animals, etc.). The term includes whole immunoglobulins as well as the fragments etc. described above under the definition of "antibody".

As used herein, the term "antibody fragment" refers to a portion derived from or related to a full-length antibody, such as, for example, a MASP-1, MASP-2 or MASP-3 antibody, generally including the antigen binding or variable region thereof. Illustrative examples of antibody fragments include Fab, Fab', F(ab)₂, F(ab')₂ and Fv fragments, scFv fragments, diabodies, linear antibodies, single-chain antibody molecules and multispecific antibodies formed from antibody fragments.

As used herein, a "single-chain Fv" or "scFv" antibody fragment comprises the V_{H} and V_{L} domains of an antibody, wherein these domains are present in a single polypeptide chain. Generally, the Fv polypeptide further comprises a polypeptide linker between the V_{H} and V_{L} domains, which enables the scFv to form the desired structure for antigen binding.

As used herein, a "chimeric antibody" is a recombinant protein that contains the variable domains and complementarity-determining regions derived from a non-human species (e.g., rodent) antibody, while the remainder of the antibody molecule is derived from a human antibody.

As used herein, a "humanized antibody" is a chimeric antibody that comprises a minimal sequence that conforms to specific complementarity-determining regions derived from non-human immunoglobulin that is transplanted into a human antibody framework. Humanized antibodies are typically recombinant proteins in which only the antibody complementarity-determining regions are of non-human origin (including antibodies generated from phage display or yeast).

As used herein, the term "mannan-binding lectin" ("MBL") is equivalent to mannan-binding protein ("MBP").

As used herein, the "membrane attack complex" ("MAC") refers to a complex of the terminal five complement components (C5b combined with C6, C7, C8 and C9) that inserts into and disrupts membranes (also referred to as C5b-9).

As used herein, "a subject" includes all mammals, including without limitation humans, non-human primates, dogs, cats, horses, sheep, goats, cows, rabbits, pigs and rodents.

As used herein, the amino acid residues are abbreviated as follows: alanine (Ala;A), asparagine (Asn;N), aspartic acid (Asp;D), arginine (Arg;R), cysteine (Cys;C), glutamic acid (Glu;E), glutamine (Gln;Q), glycine (Gly;G), histidine (His;H), isoleucine (Ile;I), leucine (Leu;L), lysine (Lys;K), methionine (Met;M), phenylalanine (Phe;F), proline (Pro;P), serine (Ser;S), threonine (Thr;T), tryptophan (Trp;W), tyrosine (Tyr;Y), and valine (Val;V).

In the broadest sense, the naturally occurring amino acids can be divided into groups based upon the chemical characteristic of the side chain of the respective amino acids. By "hydrophobic" amino acid is meant either Ile, Leu, Met, Phe, Trp, Tyr, Val, Ala, Cys or Pro. By "hydrophilic" amino acid is meant either Gly, Asn, Gln, Ser, Thr, Asp, Glu, Lys, Arg or His. This grouping of amino acids can be further subclassed as follows. By "uncharged hydrophilic" amino acid is meant either Ser, Thr, Asn or Gln. By "acidic" amino acid is meant either Glu or Asp. By "basic" amino acid is meant either Lys, Arg or His.

As used herein the term "conservative amino acid substitution" is illustrated by a substitution among amino acids within each of the following groups: (1) glycine, alanine, valine, leucine, and isoleucine, (2) phenylalanine, tyrosine, and tryptophan, (3) serine and threonine, (4) aspartate and glutamate, (5) glutamine and asparagine, and (6) lysine, arginine and histidine.

The term "oligonucleotide" as used herein refers to an oligomer or polymer of ribonucleic acid (RNA) or deoxyribonucleic acid (DNA) or mimetics thereof. This term also covers those oligonucleobases composed of naturally-occurring nucleotides, sugars and covalent internucleoside (backbone) linkages as well as oligonucleotides having non-naturally-occurring modifications.

As used herein, an "epitope" refers to the site on a protein (e.g., a human MASP-3 protein) that is bound by an antibody. "Overlapping epitopes" include at least one (e.g., two, three, four, five, or six) common amino acid residue(s), including linear and non-linear epitopes.

As used herein, the terms "polypeptide," "peptide," and "protein" are used interchangeably and mean any peptide-linked chain of amino acids, regardless of length or post-translational modification. The MASP proteins (MASP-1, MASP-2 or MASP-3) described herein can contain or be wild-type proteins or can be variants that have not more than 50 (e.g., not more than one, two, three, four, five, six, seven, eight, nine, ten, 12, 15, 20, 25, 30, 35, 40, or 50) conservative amino acid substitutions. Conservative substitutions typically include substitutions within the following groups: glycine and alanine; valine, isoleucine, and leucine; aspartic acid and glutamic acid; asparagine, glutamine, serine and threonine; lysine, histidine and arginine; and phenylalanine and tyrosine.

The human MASP-1 protein (set forth as SEQ ID NO:10), human MASP-2 protein (set forth as SEQ ID NO:5) and human MASP-3 protein (set forth as SEQ ID NO:8) described herein also include "peptide fragments" of the proteins, which are shorter than full-length and/or immature (pre-pro) MASP proteins, including peptide fragments of a MASP protein include terminal as well internal deletion variants of the protein. Deletion variants can lack one, two, three, four, five, six, seven, eight, nine, ten, 11, 12, 13, 14, 15, 16, 17, 18, 19, or 20 amino acid segments (of two or more amino acids) or noncontiguous single amino acids. The human MASP-1 protein can have an amino acid sequence that is, or is greater than, 70 (e.g., 71, 72, 73, 74, 75, 76, 77, 78, 79, 80, 81, 82, 83, 84, 85, 86, 87, 88, 89, 90, 91, 92, 93, 94, 95, 96, 97, 98, 99, or 100) % identical to the human MASP-1 protein having the amino acid sequence set forth in SEQ ID NO: 10.

The human MASP-3 protein can have an amino acid sequence that is, or is greater than, 70 (e.g., 71, 72, 73, 74, 75, 76, 77, 78, 79, 80, 81, 82, 83, 84, 85, 86, 87, 88, 89, 90, 91, 92, 93, 94, 95, 96, 97, 98, 99, or 100) % identical to the human MASP-3 protein having the amino acid sequence set forth in SEQ ID NO: 8.

In some cases, the human MASP-2 protein can have an amino acid sequence that is, or is greater than, 70 (e.g., 71, 72, 73, 74, 75, 76, 77, 78, 79, 80, 81, 82, 83, 84, 85, 86, 87, 88, 89, 90, 91, 92, 93, 94, 95, 96, 97, 98, 99, or 100) % identical to the human MASP-2 protein having the amino acid sequence set forth in SEQ ID NO: 5.

In some cases, peptide fragments can be at least 6 (e.g., at least 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 55, 60, 65, 70, 75, 80, 85, 90, 95, 100, 110, 120, 130, 140, 150, 160, 170, 180, 190, 200, 250, 300, 350, 400, 450, 500, or 600 or more) amino acid residues in length (e.g., at least 6 contiguous amino acid residues in any one of SEQ ID NOS: 5, 8 or 10). In some cases, an antigenic peptide fragment of a human MASP protein is fewer than 500 (e.g., fewer than 450, 400, 350, 325, 300, 275, 250, 225, 200, 190, 180, 170, 160, 150, 140, 130, 120, 110, 100, 95, 90, 85, 80, 75, 70, 65, 60, 55, 50, 49, 48, 47, 46, 45, 44, 43, 42, 41, 40, 39, 38, 37, 36, 35, 34, 33, 32, 31, 30, 29, 28, 27, 26, 25, 24, 23, 22, 21, 20, 19, 18, 17, 16, 15, 14, 13, 12, 11, 10, 9, 8, 7, or 6) amino acid residues in length (e.g., fewer than 500 contiguous amino acid residues in any one of SEQ ID NOS: 5, 8 or 10).

In some cases, in the context of generating an antibody that binds MASP-1, MASP-2 and/or MASP-3, the peptide fragments are antigenic and retain at least 10% (e.g., at least 10%, at least 15%, at least 20%, at least 25%, at least 30%, at least 35%, at least 40%, at least 50%, at least 55%, at least 60%, at least 70%, at least 80%, at least 90%, at least 95%, at least 98%, at least 99%, at least 99.5%, or 100% or more) of the ability of the full-length protein to induce an antigenic response in a mammal (see below under "Methods for Producing an Antibody").

Percent (%) amino acid sequence identity is defined as the percentage of amino acids in a candidate sequence that are identical to the amino acids in a reference sequence, after aligning the sequences and introducing gaps, if necessary, to achieve the maximum percent sequence identity. Alignment for purposes of determining percent sequence identity can be achieved in various ways that are within the skill in the art, for instance, using publicly available computer software such as BLAST, BLAST-2, ALIGN, ALIGN-2 or Megalign (DNASTAR) software. Appropriate parameters for measuring alignment, including any algorithms needed to achieve maximal alignment over the full-length of the sequences being compared can be determined by known methods.

The human MASP-1 protein (SEQ ID NO:10) is encoded by the cDNA sequence set forth as SEQ ID NO:9; the human MASP-2 protein (SEQ ID NO:5) is encoded by the cDNA sequence set forth as SEQ ID NO:4; and the human MASP-3 protein (SEQ ID NO:8) is encoded by the cDNA sequence set forth as SEQ ID NO:7. Those skilled in the art will recognize that the cDNA sequences disclosed in SEQ ID NO:9, SEQ ID NO:4 and SEQ ID NO:7 represent a single allele of human MASP-1, MASP-2 and MASP-3, respectively, and that allelic variation and alternative splicing are expected to occur. Allelic variants of the nucleotide sequences shown in SEQ ID NO:9, SEQ ID NO:4 and SEQ ID NO:7, including those containing silent mutations and those in which mutations result in amino acid sequence changes, are within the scope of the present disclosure. Allelic variants of the MASP-1, MASP-2 or MASP-3 sequence can be cloned by probing cDNA or genomic libraries from different individuals according to standard procedures, or may be identified by homology comparison search (e.g., BLAST searching) of databases containing such information.

### II. THE LECTIN PATHWAY: A NEW UNDERSTANDING

### i. Overview: the Lectin pathway has been redefined

As described herein, the inventors have made the surprising discovery that the lectin pathway of complement has two effector arms to activate complement, both driven by lectin pathway activation complexes formed of carbohydrate recognition components (MBL, CL-11 and ficolins): i) the effector arm formed by the lectin pathway-associated serine proteases MASP-1 and MASP-3, referred to herein as "lectin pathway effector arm 1" or "LEA-1"; and (ii) the MASP-2 driven activation effector arm, referred to herein as "lectin pathway effector arm 2", or "LEA-2". Both LEA-1 and LEA-2 can effect lysis and/or opsonization.

It has also been determined that lectin-independent conversion of factor B by MASP-3 and lectin-independent conversion of factor D by HTRA-1, MASP-1 and MASP-3, which both can occur in the absence of Ca⁺⁺, commonly lead to the conversion of C3bB to C3bBb and of pro-factor D to factor D. Therefore, inhibiting MASP-3 can inhibit both LEA-1 and the lectin-independent activation of factor B and/or factor D, which can result in the inhibition of lysis and/or opsonization.

**FIGURE 1** illustrates this new understanding of the pathways of complement activation. As shown in **FIGURE 1**, LEA-1 is driven by lectin-bound MASP-3, which can activate the zymogen of factor D to its active form and/or cleave the C3b- or C3b(H₂0)-bound factor B, leading to conversion of the C3bB zymogen complex into its enzymatically active form C3bBb. Activated factor D, generated by MASP-3, can also convert the C3bB or C3b(H₂0) zymogen complexes into their enzymatically active form. MASP-1 is capable of rapid self-activation, whereas MASP-3 is not. In many cases, MASP-1 is the activator of MASP-3.

While in many examples lectins (i.e., MBL, CL-11 or ficolins) can direct activity to cellular surfaces, **FIGURE 1** also outlines the lectin-independent functions of MASP-3, MASP-1, and HTRA-1 in factor B activation and/or factor D maturation. As with the lectin-associated form of MASP-3 in LEA-1, the lectin-independent form of MASP-3 is capable of mediating conversion of C3bB or C3b(H₂0) to C3bBb (see also **FIGURES 36** and 37) and pro-factor D to factor D (see **FIGURE 39**). MASP-1 (see also **FIGURE 39**) and the non-MASP-related protein HTRA-1 can also activate factor D (Stanton et al., Evidence That the HTRA1 Interactome Influences Susceptibility to Age-Related Macular Degeneration, presented at The Association for Research in Vision and Ophthalmology 2011 conference on May 4, 2011) in a manner in which no lectin component is required.

Thus, MASP-1 (via LEA-1 and lectin-independent forms), MASP-3 (via LEA-1 and lectin-independent forms), and HTRA-1 (lectin-independent only) are capable of either direct or indirect activation at one or more points along a MASP-3 - factor D - factor B axis. In doing so, they generate C3bBb, the C3 convertase of the alternative pathway, and they stimulate the production and deposition of C3b on microbial surfaces. C3b deposition plays a critical role in opsonization, labeling the surfaces of microbes for destruction by host phagocytic cells, such as macrophages. As an example herein (**FIGURE 35**), MASP-3 is critical for opsonization of *S. aureus.* C3b deposition occurs rapidly on *S. aureus* exposed to human serum in a MASP-3-dependent fashion (**FIGURE 35**).

The contributions of LEA-1 and the lectin-independent functions of MASP-3, MASP-1, or HTRA-1 are not limited to opsonization, however. As diagrammed in **FIGURE 1**, these three components can also cause cell lysis by indirect or direct activation of factor B, and the production of C3b. These components form complexes that generate the alternative pathway C5 convertase, C3bBb(C3b)ₙ. As described further herein, the requirement for MASP-3 and MBL, but not MASP-2 (and, therefore, not LEA-2 in this example), in the lysis of *N. meningitidis* (see **FIGURES 13**, **14** and **15**) demonstrates a role for LEA-1 in lysis. In summary, the opsonization results obtained from the *S. aureus* studies and the lysis results observed in the *N. meningitidis* studies support the role of LEA-1 in both processes (as diagrammed in **FIGURE 1**). Furthermore, these studies demonstrate that both opsonization and lysis can result from the conversion of C3bB or C3b(H₂0) and/or of pro-factor D to factor D; therefore, both processes can be outcomes of the lectin-independent roles of MASP-3, MASP-1, or HTRA-1. Thus, the model developed by the inventors in **FIGURE 1** supports the use of inhibitors of principally MASP-3, but also MASP-1 and/or HTRA-1, to block opsonization and/or lysis and to treat pathologies caused by dysregulation of these processes.

### 1. Lectin Pathway Effector Arm (LEA-1)

The first effector arm of the lectin pathway, LEA-1, is formed by the lectin pathway-associated serine proteases MASP-1 and MASP-3. As described herein, the inventors have now shown that, in the absence of MASP-3 and in the presence of MASP-1, the alternative pathway is not effectively activated on surface structures. These results demonstrate that MASP-3 plays a previously undisclosed role in initiating the alternative pathway, and this is confirmed using the MASP-3-deficient 3MC serum obtained from patients with the rare 3MC autosomal recessive disorder (Rooryck C, et al., Nat Genet. 43(3):197-203 (2011)) with mutations that render the serine protease domain of MASP-3 dysfunctional. Based on these novel findings, it is expected that complement activation involving the alternative pathway, as conventionally defined, is MASP-3-dependent. In fact, MASP-3, and its activation of LEA-1, may represent the hitherto elusive initiator of the alternative pathway.

As further described in Examples 1-4 herein, in MASP-2-deficient sera, the inventors observed a higher activity of lectin-dependent alternative pathway activation resulting in a higher bactericidal activity (*i.e.,* lytic activity) against *N. meningitidis.* While not wishing to be bound by any particular theory, it is believed that in absence of MASP-2, MASP-1-bearing carbohydrate recognition complexes are more likely to bind close to MASP-3-bearing carbohydrate recognition complexes to activate MASP-3. It is known that, in many instances, activation of MASP-3 is dependent on MASP-1 activity, as MASP-3 is not an auto-activating enzyme and very often requires the activity of MASP-1 to be converted from its zymogen form into its enzymatically active form. MASP-1 (like MASP-2) is an auto-activating enzyme, while MASP-3 does not auto-activate and, in many instances, needs the enzymatic activity of MASP-1 to be converted into its enzymatically active form. *See,* Zundel S, et al., J Immunol., 172(7):4342-50 (2004). In absence of MASP-2, all lectin pathway recognition complexes are either loaded with MASP-1 or MASP-3. Therefore, the absence of MASP-2 facilitates the MASP-1-mediated conversion of MASP-3 into its enzymatically active form. Once MASP-3 is activated, activated MASP-3 initiates alternative pathway activation, now referred to as "LEA-1" activation, through a MASP-3-mediated conversion of C3bB to C3bBb and/or conversion of pro-factor D to factor D. C3bBb, also referred to as the alternative pathway C3 convertase, cleaves additional C3 molecules yielding deposition of opsonic C3b molecules. If several C3b fragments bind in close proximity to the C3bBb convertase complex, this results in the formation of the alternative pathway C5 convertase C3bBb(C3b)n, which promotes formation of MAC. Additionally, C3b molecules deposited on the surface form new sites for factor B binding, which can now be cleaved by factor D and/or MASP-3 to form additional sites where alternative pathway C3 and C5 convertase complexes can be formed. This latter process is needed for effective lysis and does not require lectins once the initial C3b deposition has occurred. A recent publication (Iwaki D. et al., J Immunol 187(7):3751-8 (2011)) as well as data generated from the inventors (**FIGURE 37**) demonstrate that the alternative pathway C3 convertase zymogen complex C3bB is converted into its enzymatically active form by activated MASP-3. The inventors now have discovered that the MASP-3-mediated cleavage of factor B represents a subcomponent of the newly described LEA-1, which promotes lectin-dependent formation of the alternative pathway C3 convertase C3bBb.

### 2. Lectin Pathway Effector Arm (LEA-2)

The second effector arm of the lectin pathway, LEA-2, is formed by the lectin pathway-associated serine protease MASP-2. MASP-2 is activated upon binding of the recognition components to their respective pattern, and may also be activated by MASP-1, and subsequently cleaves the complement component C4 into C4a and C4b. After the binding of the cleavage product C4b to plasma C2, C4b-bound C2 becomes substrate of a second MASP-2-mediated cleavage step which converts C4b-bound C2 into the enzymatically active complex C4bC2a and a small C2b cleavage fragment. C4b2a is the C3-converting C3 convertase of the lectin pathway, converting the abundant plasma component C3 into C3a and C3b. C3b binds to any surface in close proximity via a thioester bond. If several C3b fragments bind in close proximity to the C3 convertase complex C4b2a, this convertase alters its specificity to convert C5 into C5b and C5a, forming the C5 convertase complex C4b2a(C3b)n. While this C5 convertase can initiate formation of MAC, this process is thought to be insufficiently effective to promote lysis on its own. Rather, the initial C3b opsonins produced by LEA-2 form the nucleus for the formation of new alternative pathway C3 convertase and C5 convertase sites, which ultimately lead to abundant MAC formation and lysis. This latter event is mediated by factor D activation of factor B associated with LEA-2-formed C3b, and hence is dependent on LEA-1 by virtue of the essential role for MASP-1 in the maturation of factor D. There is also a MASP-2-dependent C4-bypass activation route to activate C3 in the absence of C4, which plays an important role in the pathophysiology of ischemia-reperfusion injury, since C4-deficient mice are not protected from ischemia-reperfusion injury while MASP-2-deficient mice are (Schwaeble et al., PNAS, 2011 *supra*). LEA-2 is also tied to the coagulation pathway, involving the cleavage of prothrombin to thrombin (common pathway) and also the cleavage of factor XII (Hageman factor) to convert into its enzymatically active form XIIa. Factor XIIa in turn cleaves factor XI to XIa (intrinsic pathway). The intrinsic pathway activation of the clotting cascade leads to fibrin formation, which is of critical importance for thrombus formation.

**FIGURE 1** illustrates the new understanding of the lectin pathway and alternative pathway based on the results provided herein. **FIGURE 1** delineates the role of LEA-2 in both opsonization and lysis. While MASP-2 is the initiator of "downstream" C3b deposition (and resultant opsonization) in multiple lectin-dependent settings physiologically (**FIGURES 20A**, **20B**, **20C**), it also plays a role in lysis of serum-sensitive bacteria. As illustrated in **FIGURE 1**, the proposed molecular mechanism responsible for the increased bactericidal activity of MASP-2-deficient or MASP-2-depleted serum/plasma for serum-sensitive pathogens such as *N. meningitidis* is that, for the lysis of bacteria, lectin pathway recognition complexes associated with MASP-1 and MASP-3 have to bind in close proximity to each other on the bacterial surface, thereby allowing MASP-1 to cleave MASP-3. In contrast to MASP-1 and MASP-2, MASP-3 is not an auto-activating enzyme, but, in many instances, requires activation/cleavage by MASP-1 to be converted into its enzymatically active form.

As further shown in **FIGURE 1**, activated MASP-3 can then cleave C3b-bound factor B on the pathogen surface to initiate the alternative activation cascade by formation of the enzymatically active alternative pathway C3 and C5 convertases C3bBb and C3bBb(C3b)n, respectively. MASP-2-bearing lectin-pathway activation complexes have no part in the activation of MASP-3 and, in the absence of or after depletion of MASP-2, all-lectin pathway activation complexes will either be loaded with MASP-1 or MASP-3. Therefore, in the absence of MASP-2, the likelihood is markedly increased that on the microbial surface MASP-1- and MASP-3-bearing lectin-pathway activation complexes will come to sit in close proximity to each other, leading to more MASP-3 being activated and thereby leading to a higher rate of MASP-3-mediated cleavage of C3b-bound factor B to form the alternative pathway C3 and C5 convertases C3bBb and C3bBb(C3b)n on the microbial surface. This leads to the activation of the terminal activation cascades C5b-C9 that forms the Membrane Attack Complex, composed of surface-bound C5b associated with C6, C5bC6 associated with C7, C5bC6C7 associated with C8, and C5bC6C7C8, leading to the polymerization of C9 that inserts into the bacterial surface structure and forms a pore in the bacterial wall, which will lead to osmolytic killing of the complement-targeted bacterium.

The core of this novel concept is that the data provided herein clearly show that the lectin pathway activation complexes drive the following two distinct activation routes, as illustrated in **FIGURE 1**:
i) LEA-1: A MASP-3-dependent activation route that initiates and drives activation of complement by generating the alternative pathway convertase C3bBb through initial cleavage and activation of factor B on activator surfaces, which will then catalyze C3b deposition and formation of the alternative pathway convertase C3bBb. The MASP-3-driven activation route plays an essential role in the opsonization and lysis of microbes and drives the alternative pathway on the surface of bacteria, leading to optimal rates of activation to generate membrane attack complexes; and
ii) LEA-2: A MASP-2-dependent activation route leading to the formation of the lectin pathway C3 convertase C4b2a and, upon accumulation of the C3 cleavage product C3b, subsequently to the C5 convertase C4b2a(C3b)n. In the absence of complement C4, MASP-2 can form an alternative C3 convertase complex which involves C2 and clotting factor XI.

In addition to its role in lysis, the MASP-2-driven activation route plays an important role in bacterial opsonization leading to microbes being coated with covalently bound C3b and cleavage products thereof (i.e., iC3b and C3dg), which will be targeted for the uptake and killing by C3 receptor-bearing phagocytes, such as granulocytes, macrophages, monocytes, microglia cells and the reticuloendothelial system. This is the most effective route of clearance of bacteria and microorganisms that are resistant to complement lysis. These include most of the gram-positive bacteria.

In addition to LEA-1 and LEA-2, there is the potential for lectin-independent activation of factor D by MASP-3, MASP-1 and/or HTRA-1, and there is also the potential for lectin-independent activation of factor B by MASP-3.

While not wishing to be bound by any particular theory, it is believed that each of (i) LEA-1, (ii) LEA-2 and (iii) lectin-independent activation of factor B and/or factor D lead to opsonization and/or the formation of MAC with resultant lysis.

### ii. Background of MASP-1, MASP-2 and MASP-3

Three mannan-binding lectin-associated serine proteases (MASP-1, MASP-2 and MASP-3) are presently known to be associated in human serum with the mannan-binding lectin (MBL). Mannan-binding lectin is also called 'mannose-binding protein' or 'mannose-binding lectin' in the recent literature. The MBL-MASP complex plays an important role in innate immunity by virtue of the binding of MBL to carbohydrate structures present on a wide variety of microorganisms. The interaction of MBL with specific arrays of carbohydrate structures brings about the activation of the MASP proenzymes which, in turn, activate complement by cleaving the complement components C4 and C2 to form the C3 convertase C4b2b (Kawasaki et al., J. Biochem 106:483-489 (1989); Matsushita & Fujita, J. Exp Med. 176:1497-1502 (1992); Ji et al., J. Immunol 150:571-578 (1993)).

The MBL-MASP proenzyme complex was, until recently, considered to contain only one type of protease (MASP-1), but it is now clear that there are two other distinct proteases (i.e., MASP-2 and MASP-3) associated with MBL (Thiel et al., Nature 386:506-510 (1997); Dahl et al., Immunity 15:127-135 (2001)), as well as an additional serum protein of 19 kDa, referred to as "MAp19" or "sMAP" (Stover et al., J. Immunol 162:3481-3490 (1999); Stover et al., J. Immunol 163:6848-6859 (1999); Takahashi et al., Int. Immunol 11:859-63 (1999)).
MAp19 is an alternatively spliced gene product of the structural gene for MASP-2 and lacks the four C-terminal domains of MASP-2, including the serine endopeptidase domain. The abundantly expressed truncated mRNA transcript encoding MAp19 is generated by an alternative splicing/polyadenylation event of the MASP-2 gene. By a similar mechanism, the MASP-1/3 gene gives rise to three major gene products, the two serine proteases MASP-1 and MASP-3 and a truncated gene product of 44 kDa referred to as "MAp44" (Degn et al., J. Immunol 183(11):7371-8 (2009); Skjoedt et al., JBiol Chem 285:8234-43 (2010)).

MASP-1 was first described as the P-100 protease component of the serum Ra-reactive factor, which is now recognized as being a complex composed of MBL plus MASP (Matsushita et al., Collectins and Innate Immunity, (1996); Ji et al., J Immunol 150:571-578 (1993). The ability of an MBL-associated endopeptidase within the MBL-MASPs complex to act on the complement components C4 and C2 in a manner apparently identical to that of the C1s enzyme within the C1q-(C1r)₂-(C1s)₂ complex of the classical pathway of complement suggests that there is a MBL-MASPs complex which is functionally analogous to the C1q-(C1r)₂-(C1s)₂ complex. The C1q-(C1r)₂-(C1s)₂ complex is activated by the interaction of C1q with the Fc regions of antibody IgG or IgM present in immune complexes. This brings about the autoactivation of the C1r proenzyme which, in turn, activates the C1s proenzyme which then acts on complement components C4 and C2.

The stoichiometry of the MBL-MASPs complex differs from the one found for the C1q-(C1r)₂-(C1s)₂ complex in that different MBL oligomers appear to associate with different proportions of MASP-1/MAp19 or MASP-2/MASP-3 (Dahl et al., Immunity 15:127-135 (2001). The majority of MASPs and MAp19 found in serum are not complexed with MBL (Thiel et al., J Immunol 165:878-887 (2000)) and may associate in part with ficolins, a recently described group of lectins having a fibrinogen-like domain able to bind to N-acetylglucosamine residues on microbial surfaces (Le et al., FEBS Lett 425:367 (1998); Sugimoto et al., J. Biol Chem 273:20721 (1998)). Among these, human L-ficolin, H-ficolin and M-ficolin associate with MASPs as well as with MAp19 and may activate the lectin pathway upon binding to the specific carbohydrate structures recognized by ficolins (Matsushita et al., J Immunol 164:2281-2284 (2000); Matsushita et al., J Immunol 168:3502-3506 (2002)). In addition to the ficolins and MBL, an MBL-like lectin collectin, called CL-11, has been identified as a lectin pathway recognition molecule (Hansen et al. J Immunol 185:6096-6104 (2010); Schwaeble et al. PNAS 108:7523-7528 (2011)). There is overwhelming evidence underlining the physiological importance of these alternative carbohydrate recognition molecules and it is therefore important to understand that MBL is not the only recognition component of the lectin activation pathway and that MBL deficiency is not to be mistaken for lectin-pathway deficiency. The existence of possibly an array of alternative carbohydrate-recognition complexes structurally related to MBL may broaden the spectrum of microbial structures that initiate a direct response of the innate immune system via activation of complement.

All lectin pathway recognition molecules are characterized by a specific MASPs-binding motif within their collagen-homologous stalk region (Wallis et al. J. Biol Chem 279:14065-14073 (2004)). The MASP-binding site in MBLs, CL-11 and ficolins is characterized by a distinct motif within this domain: Hyp-Gly-Lys-Xaa-Gly-Pro, where Hyp is hydroxyproline and Xaa is generally an aliphatic residue. Point mutations in this sequence disrupt MASP binding.

### 1. Respective structures, sequences, chromosomal localization and splice variants

**FIGURE 2** is a schematic diagram illustrating the domain structure of the MASP-2 polypeptide (SEQ ID NO:5) and MAp19 polypeptide (SEQ ID NO:2) and the exons encoding the same. **FIGURE 3** is a schematic diagram illustrating the domain structure of the MASP-1 polypeptide (SEQ ID NO:10), MASP-3 polypeptide (SEQ ID NO:8) and MAp44 polypeptide (SEQ ID NO: 11) and the exons encoding the same. As shown in **FIGURES 2** **and** **3**, the serine proteases MASP-1, MASP-2 and MASP-3 consist of six distinct domains arranged as found in C1r and C1s; *i.e.*, (I) an N-terminal C1r/C1s/sea urchin VEGF/bone morphogenic protein (or CUBI) domain; (II) an epidermal growth factor (EGF)-like domain; (III) a second CUB domain (CUBII); (IV and V) two complement control protein (CCP1 and CCP2) domains; and (VI) a serine protease (SP) domain.

The cDNA-derived amino acid sequences of human and mouse MASP-1 (Sato et al., Int Immunol 6:665-669 (1994); Takada et al., Biochem Biophys Res Commun 196:1003-1009 (1993); Takayama et al., J. Immunol 152:2308-2316 (1994)), human, mouse, and rat MASP-2 (Thiel et al., Nature 386:506-510 (1997); Endo et al., J Immunol 161:4924-30 (1998); Stover et al., J. Immunol 162:3481-3490 (1999); Stover et al., J. Immunol 163:6848-6859 (1999)), as well as human MASP-3 (Dahl et al., Immunity 15:127-135 (2001)) indicate that these proteases are serine peptidases having the characteristic triad of His, Asp and Ser residues within their putative catalytic domains (Genbank Accession numbers: human MASP-1: BAA04477.1; mouse MASP-1: BAA03944; rat MASP-1: AJ457084; Human MASP-3:AAK84071; mouse MASP-3: AB049755, as accessed on Genbank on 2/15/2012).

As further shown in **FIGURES 2** and **3**, upon conversion of the zymogen to the active form, the heavy chain (alpha, or A chain) and light chain (beta, or B chain) are split to yield a disulphide-linked A-chain and a smaller B-chain representing the serine protease domain. The single-chain proenzyme MASP-1 is activated (like proenzyme C1r and C1s) by cleavage of an Arg-Ile bond located between the second CCP domain (domain V) and the serine protease domain (domain VI). Proenzymes MASP-2 and MASP-3 are considered to be activated in a similar fashion to that of MASP-1. Each MASP protein forms homodimers and is individually associated with MBL and the ficolins in a Ca⁺⁺-dependent manner.

### 2. MASP-1/3

The human MASP-1 polypeptide (SEQ ID NO:10) and MASP-3 polypeptide (SEQ ID NO:8) arise from one structural gene (Dahl et al., Immunity 15:127-135 (2001), which has been mapped to the 3q27-28 region of the long arm of chromosome 3 (Takada et al., Genomics 25:757-759 (1995)). The MASP-3 and MASP-1 mRNA transcripts are generated from the primary transcript by an alternative splicing/polyadenylation process. The MASP-3 translation product is composed of an alpha chain, which is common to both MASP-1 and MASP-3, and a beta chain (the serine protease domain), which is unique to MASP-3. As shown in **FIGURE 3**, the human MASP-1 gene encompasses 18 exons. The human MASP-1 cDNA (set forth as SEQ ID NO:9) is encoded by exons 2, 3, 4, 5, 6, 7, 8, 10, 11, 13, 14, 15, 16, 17 and 18. As further shown in **FIGURE 3**, the human MASP 3 gene encompasses twelve exons. The human MASP-3 cDNA (set forth as SEQ ID NO:7) is encoded by exons 2, 3, 4, 5, 6, 7, 8, 10, 11 and 12. An alternative splice results in a protein termed MBL-associated protein 44 ("MAp44)," (set forth as SEQ ID NO:11), arising from exons 2, 3, 4, 5, 6, 7, 8 and 9.

The human MASP-1 polypeptide (SEQ ID NO: 10 from Genbank BAA04477.1) has 699 amino acid residues, which includes a leader peptide of 19 residues. When the leader peptide is omitted, the calculated molecular mass of MASP-1 is 76,976 Da. As shown in **FIGURE 3**, the MASP-1 amino acid sequence contains four N-linked glycosylation sites. The domains of the human MASP-1 protein (with reference to SEQ ID NO:10) are shown in **FIGURE 3** and include an N-terminal C1r/C1s/sea urchin VEFG/bone morphogenic protein (CUBI) domain (aa 25-137 of SEQ ID NO:10), an epidermal growth factor-like domain (aa 139-181 of SEQ ID NO:10), a second CUB domain (CUBII) (aa 185-296 of SEQ ID NO:10), as well as a tandem of complement control protein (CCP1 aa 301-363 and CCP2 aa 367-432 of SEQ ID NO:10) domains and a serine protease domain (aa 449-694 of SEQ ID NO:10).

The human MASP-3 polypeptide (SEQ ID NO:8, from Genbank AAK84071) has 728 amino acid residues, which includes a leader peptide of 19 residues. When the leader peptides are omitted, the calculated molecular mass of MASP-3 is 81,873 Da. As shown in **FIGURE 3**, there are seven N -linked glycosylation sites in MASP-3. The domains of the human MASP-3 protein (with reference to SEQ ID NO:8) are shown in **FIGURE 3** and include an N-terminal C1r/C1s/sea urchin VEGF/bone morphogenic protein (CUBI) domain (aa 25-137 of SEQ ID NO:8), an epidermal growth factor-like domain (aa 139-181 of SEQ ID NO:8), a second CUB domain (CUBII) (aa 185-296 of SEQ ID NO:8), as well as a tandem of complement control protein (CCP1 aa 301-363 and CCP2 aa 367-432 of SEQ ID NO:8) domains and a serine protease domain (aa 450-711 of SEQ ID NO:8).

The MASP-3 translation product is composed of an alpha chain (heavy chain), containing the CUB-1-EGF-CUB-2-CCP-1-CCP-2 domains (alpha chain: aa 1-448 of SEQ ID NO:8) which is common to both MASP-1 and MASP-3, and a light chain (beta chain: aa 449-728 of SEQ ID NO:8), containing the serine protease domain, which is unique to MASP-3 and MASP-1.

### 3. MASP-2

The human MASP-2 gene is located on chromosome 1p36.3-2 (Stover et al., Cytogenet and Cell Genet 84:148-149 (1999) and encompasses twelve exons, as shown in **FIGURE 2**. MASP-2 (SEQ ID NO:5) and MAp19 (SEQ ID NO:2) are encoded by transcripts of a single structural gene generated by alternative splicing/polyadenylation (Stover et al., Genes and Immunity 2:119-127 (2001)). The human MASP-2 cDNA (SEQ ID NO:4) is encoded by exons 2, 3, 4, 6, 7, 8, 9, 10, 11 and 12. The 20 kDa protein termed MBL-associated protein 19 ("MAp19", also referred to as "sMAP") (SEQ ID NO:2), encoded by (SEQ ID NO:1) arises from exons 2, 3, 4 and 5. MAp19 is a nonenzymatic protein containing the N-terminal CUB1-EGF region of MASP-2 with four additional residues (EQSL) derived from exon 5 as shown in **FIGURE 2**.

The MASP-2 polypeptide (SEQ ID NO:5) has 686 amino acid residues, which includes a leader peptide of 15 residues that is cleaved off after secretion, resulting in the mature form of human MASP-2 (SEQ ID NO:6). As shown in **FIGURE 2**, the MASP-2 amino acid sequence does not contain any N-linked glycosylation sites. The MASP-2 polypeptide exhibits a molecular structure similar to MASP-1, MASP-3, and C1r and C1s, the proteases of the C1 complement system. The domains of the human MASP-2 protein (numbered with reference to SEQ ID NO:5) are shown in **FIGURE 2** and include an N-terminal C1r/C1s/sea urchin VEGF/bone morphogenic protein (CUBI) domain (aa 24-136 of SEQ ID NO:5), an epidermal growth factor-like domain (aa 138-180 of SEQ ID NO:5), a second CUB domain (CUBII) (aa 184-295 of SEQ ID NO:5), as well as a tandem of complement control protein (CCP1 aa 300-359 and CCP2 aa 364-431 of SEQ ID NO:5) domains and a serine protease domain (aa 445-682 of SEQ ID NO:5).

As shown in **FIGURE 2**, the MASP-2 polypeptide has an alpha chain (heavy chain) containing the CUB-1-EGF-CUB-2-CCP-1-CCP-2 domains (alpha chain: aa 1-443 of SEQ ID NO:5) and a beta chain (light chain) containing the serine protease domain (beta chain: aa 444-686). The CUB-1, EGF and CUB-2 domains are required for dimerization and the CUB-1, EGF, CUB-2 and CCP-1 domains contain the binding site for MBP. As described in Wallis et al., J. Biol Chem 279:14065-14073 (2004), each MASP-2 dimer binds to two MBL subunits.

### 4. Comparison of MASP-1, MASP-2 and MASP-3 amino acid sequences

**FIGURE 4** is an amino acid alignment of the protein sequences of MASP-1 (SEQ ID NO:10), MASP-2 (SEQ ID NO:6) and MASP-3 (SEQ ID NO:8), showing the CUBI, EGF, CUBII, CCP1, CCP2 domains and conserved catalytic triad residues (H, D, S) in the serine protease (SP) domains. The symbol "." indicates an identical amino acid sequence.

**FIGURE 5** is an amino acid alignment of the alpha chain sequences, including the CUBI-EGF-CUBII-CCP1-CCP2, of MASP-1 (alpha chain: aa 1-447 of SEQ ID NO:10) MASP-2 (alpha chain: aa 1-443 of SEQ ID NO:5) and MASP-3 (alpha chain: aa 1-448 of SEQ ID NO:8). There are numerous patches of identity in the CUBI, EGF, and CUBII domains, as indicated by dotted boxes in **FIGURE 5**. The CCP2 and CCP2 domains are indicated by the dark shaded boxes. The overall percent identity between the alpha chains of human MASP1/3 and human MASP-2 is provided below in TABLE 1.

**FIGURE 6** is an amino acid alignment of the beta chain sequences (including the serine protease domains) of MASP-1 (beta chain: aa 448-699 of SEQ ID NO: 10), MASP-2 (beta chain: aa 444-686 of SEQ ID NO:5) and MASP-3 (beta chain: aa 449-728 of SEQ ID NO:8). **FIGURE 7A** shows a pairwise amino acid alignment between the beta chain sequences of MASP-1 (beta chain: aa 448-699 of SEQ ID NO: 10) and MASP-2 (beta chain: aa 444-686 of SEQ ID NO:5). **FIGURE 7B** shows a pairwise amino acid alignment between the beta chain sequences of MASP-1 (beta chain: aa 448-699 of SEQ ID NO: 10) and MASP-3 (beta chain: aa 449-728 of SEQ ID NO:8). **FIGURE 7C** shows a pairwise amino acid alignment between the beta chain sequences of MASP-2 (beta chain: aa 444-686 of SEQ ID NO:5) and MASP-3 (beta chain: aa 449-728 of SEQ ID NO:8). The regions of identity in **FIGURES 5-7** are shown as dotted boxes surrounding the identical amino acids (shown as "." Symbol).

The percent identity between the alpha and beta chains of the human MASP-1, MASP-2 and MASP-3 proteins is provided in **TABLE 1** below.

**TABLE 1: Percent Identity between human MASP proteins**

| | **% Identity Between A Chains** | | | **% Identity Between B Chains** | | |
|---|---|---|---|---|---|---|
| | MASP-1 | MASP-2 | MASP-3 | MASP-1 | MASP-2 | MASP-3 |
| MASP-1 | 100% | 45.6% | 98% | 100% | 27% | 27% |
| MASP-2 | 45.6% | 100% | 45.4% | 27% | 100% | 28.6% |
| MASP-3 | 98% | 45.4% | 100% | 27% | 28.6% | 100% |

With regard to the alpha chains (heavy chains), as indicated above in **TABLE 1**, the MASP-1 and MASP-3 alpha chains are identical (except for the 15 amino acid sequence at 3' end). The overall % identity between the MASP-2 and MASP-3 alpha chain is 45.4%, with numerous patches of identity in the CUBI-EGF-CUBII domains, as shown in **FIGURE 5****.**

With regard to the beta chains (light chains), the overall percent identity between the three beta chains is low, in the range of 27% to 28%. However, although overall identity between the three B-chains is low, there are numerous patches of identity, as shown in **FIGURE 6**. As further shown in **FIGURES 7A-C****,** identical patches of sequence are more broadly distributed between MASP-2 and 3 than between 1 and 2 or 1 and 3.

All the cysteine residues present in MASP-2, MASP-3, C1r and C1s align with equivalent residues in MASP-1; however, MASP-1 has two cysteine residues (at positions 465 and 481 in the L chain) that are not found in the MASP-2, MASP-3, C1r and C1s. These two cysteine residues in MASP-1 are in the expected positions used to form the 'histidine-loop' disulfide bridge as found in trypsin and chymotrypsin. This suggests that MASP-2, MASP-3, C1r, and C1s may have evolved, by gene duplication and divergence, from MASP-1 (Nonaka & Miyazawa, Genome Biology 3 Reviews 1001.1-1001.5 (2001)).

### 5. Respective biological functions/activities, including relevant human genetic data

The role of the MBL/Ficolin-MASPs complexes in innate immunity is mediated via the calcium-dependent binding of the C-type lectin domains (present in the MBL molecule) or via the binding of the fibrinogen-like domains (present in the ficolin molecule) to carbohydrate structures found on yeast, bacteria, viruses, and fungi. This recognition phase brings about the activation of the proenzyme MASP-2, which then mimics the action of the activated C1s within the C1q-(C1r)₂-(C1s)₂ complex by cleaving C4 and C2 to form the C3 convertase C4b2b. This allows deposition of C4b and C3b on target pathogens and thus promotes killing and clearance through phagocytosis.

Evidence in the recent literature suggests that the lectin pathway activation complex only requires the activity of MASP-2 to cleave C4 and C2: i) the reconstitution of a minimal lectin-pathway activation complex using recombinant MBL and recombinantly expressed MASP-2 appears to be sufficient to effectively cleave both C4 and C2 *in vitro* (Vorup-Jensen et al., J. Immunol 165:2093-2100 (2000); Rossi et al., J Biol Chem 276:40880-40887 (2001); Ambrus et al., J Immunol 170:1374-1382 (2003); Gál et al, J Biol Chem 280:33435-33444 (2005)); while ii) the serum of mice with a gene-targeted deficiency of MASP-2 is devoid of any lectin pathway functional activity (Schwaeble et al., PNAS 108:7523-7528 (2011)). Recently, a genetically determined deficiency of MASP-2 was described (Stengaard-Pedersen et al., New Eng. J. Med. 349:554-560, (2003)). The mutation of a single nucleotide leads to an Asp-Gly exchange in the CUB1 domain and renders MASP-2 incapable of binding to MBL.

In addition, the functional characterization of sera of mice deficient of both MASP-1 and MASP-3 shows that lectin pathway activity is slower, but not absent when comparing sera of wild-type and MASP-1/MASP-3 knockout (MASP-1/3^{-/-}) mice under physiological conditions (Takahashi et al., J. Immunol 180:6132-6138 (2008); Schwaeble et al., PNAS (2011)). These studies suggest that in contrast to the classical pathway effector endopeptidase C1s, activation of MASP-2 does not essentially involve or require the activity of any of the other MBL-associated serine endopeptidases (*i.e.,* MASP-1 or MASP-3) and that the proteolytic activity of MASP-2 suffices to translate binding of the carbohydrate recognition molecules of the lectin pathway *(i.e.,* MBL, ficolins or CL-11) into complement activation. However, more recent studies have demonstrated that while MASP-2 does have the capacity to autoactivate, the catalytic rate of MASP-1 activation of the MASP-2 zymogen exceeds that of MASP-2 cleavage of its own zymogen form by about 85,000 fold (Héja et al., PNAS 106:10498-503 (2011); Megyeri et al., J. Biol. Chem. 288(13):8922-34 (2013)). Therefore, it is likely that the primary activator of MASP-2 in physiological settings is MASP-1. As judged by the size of the fragments of C4 generated, and the functional C3 convertase activity generated, it seems likely that the activated MASP-2 cleaves C4 and C2 in an identical manner to that carried out by activated C1s, *i.e,.* at a single arginyl bond (Arg76 Ala77) within the alpha-chain of C4 and at a single arginyl bond (Arg223 Lys224) within the proenzyme chain of C2. It has also been reported that the mouse MASP (in the form of the mouse MBL-MASP complex designated Ra-reactive factor) can, unlike C1s, cleave the alpha-chain of complement component C3 to yield the biologically active fragments C3a and C3b (Ogata et al, J. Immunol 154:2351-2357 (1995)). If this were to take place in the human system, it would require the cleavage of a single arginyl bond (Arg77 Ser78) within the alpha-chain of C3. Activated MASP-2, like activated C1s, is unable to cleave complement component C5. The proteolytic activities of MASP-1 and MASP-2 are inhibited by C1-Inhibitor (Matsushita et al., J Immunol 165:2637-2642 (2000) whereas C1-Inhibitor does not react with MASP-3 (Dahl et al., Immunity 15:127-135 (2001); Zundel et al., J Immunol 172:4342-4350 (2004)).

The biological functions of MASP-1 and MASP-3 have been slow to emerge. The substrate specificity and the physiological role of MASP-1 have been a subject of debate since its discovery. Numerous potential substrates have been identified during the recent years. It was suggested that MASP-1 can cleave native C3 slowly and this direct cleavage of C3 may initiate the complement cascade perhaps with the contribution of the alternative pathway (Matsushita et al., J Immunol 165:2637-2642 (2000)). Later it was shown that recombinant MASP-1 cleaves the inactive (thioester hydrolyzed) form of C3 which is unproductive in terms of initiating the complement cascade (Ambrus et al., J Immunol 170:1374-1382 (2003)). The lack of lectin pathway activity in the serum dilutions of MASP-2-deficient mice unequivocally proved that the MASP-1-driven C3-bypass mechanism does not exist (Schwaeble et al., PNAS 108:7523-7528 (2011)). The complement components that are cleaved by MASP-1 with considerable efficiency are C2 (Rossi et al., J Biol Chem 276:40880-40887 (2001); Ambrus et al., J Immunol 170:1374-1382 (2003)) and the zymogen form of factor D (Takahashi et al., J Exp Med 207:29-37 (2010)). As for the ability of MASP-1 to cleave C2, it is plausible therefore that MASP-1 can augment the C3-convertase (C4b2a)-forming ability of MASP-2 via C2 cleavage. This suggestion is supported by the observation that the activity of the lectin pathway is diminished in MASP-1-depleted human serum and in the serum of MASP-1/3-deficient mice (Takahashi et al., J Immunol 180:6132-6138 (2008)), which observation also suggests that MASP-1 has a role in activating MASP-2. Moreover, while every C4b deposited by MBL-MASPs complex can form C4b2a convertase, only one out of four C4b deposited by the classical pathway C1 complex can do the same (Rawal et al., J Biol Chem 283 (12):7853-63 (2008)).

MASP-1 also cleaves MASP-2 and MASP-3 (Megyeri M., et al, J Biol Chem. 2013 Mar 29;288(13):8922-34). Recent experiments suggest that although MASP-2 can autoactivate, MASP-1 is the primary activator of zymogen MASP-2. The activation of MASP-2 was delayed in the serum of MASP-1 knockout mice (Takahashi et al., J Immunol 180: 6132-6138 (2008)) and a similar result was obtained when the activity of MASP-1 was blocked by a specific inhibitor in normal human serum (Kocsis et al., J Immunol 185(7):4169-78 (2010)). Moreover, Degn et al. (J. Immunol. 189(8): 3957-69 (2012)) found MASP-1 to be critical for MASP-2 activation and subsequent C4 cleavage in human serum. The catalytic rate for the conversion of zymogen MASP-2 to active MASP-2 is more than 85,000-fold greater than the rate by which MASP-2 can autoactivate (Megyeri et al., J. Biol. Chem. 288:8922-8934 (2013); Héja et al., J. Biol. Chem. 287(24):20290-300 (2012); Héja et al., PNAS 109:10498-503 (2012)).

Recent discoveries have also linked MASP-1 to the alternative pathway. MASP-1 can convert zymogen factor D into its enzymatically active form (**FIGURE 39****;** Takahashi et al., J Exp Med 207:29-37 (2010)). Furthermore, MASP-1 activates the zymogen form of MASP-3 (Megyeri et al., J. Biol. Chem. 288:8922-8934 (2013); Degn et al. J. Immunol. 189(8): 3957-69 (2012)), which itself can activate zymogen factor D (**FIGURE 39**) as well as cleave factor B, another essential component of the alternative pathway, to its active form (Iwaki et al., J. Immunol. 187:3751-58 (2011)). The conversion of pro-factor D and pro-factor B, however, is likely to be independent of the activation state of LEA-2 and may occur through non-complex-bound MASP-1.

Several lines of evidence indicate that MASP-1 is a thrombin-like enzyme and is important in activation of the coagulation pathway. MASP-1 can cleave several substrates of thrombin including fibrinogen (Hajela K. et al., Immunobiology 205(4-5):467-75 (2002)), factor XIII (Krarup et al., Biochim Biophys Acta 1784(9):1294-1300 (2008)) and protease-activated receptor 4 (PAR4) (Megyeri et al., J Immunol 183(5):3409-16 (2009)). Moreover, antithrombin in the presence of heparin is a more efficient inhibitor of MASP-1 than C1-inhibitor (Dobó et al., J Immunol 183:1207-1214 (2009)). The connection between the complement and the coagulation pathway is also underlined by the observation that MASP-2 is able to activate prothrombin (Krarup A. et al., PLoS One 2(7):e623 (2007)). Limited coagulation represents an ancient type of innate immunity when the spreading of invading pathogens is prevented by the fibrin clot. The releasing fibrinopeptide B has proinflammatory activity. The MASP-1-mediated cleavage of PAR4 activates the endothelial cells-initiating inflammatory reaction (Megyeri et al., J Immunol 183(5):3409-16 (2009)).

MASP-3 has no proteolytic activity towards C4, C2 or C3 substrates. Conversely, MASP-3 was reported to act as an inhibitor of the lectin pathway (Dahl et al., Immunity 15:127-135 (2001)). This conclusion may have come about because in contrast to MASP-1 and MASP-2, MASP-3 is not an autoactivating enzyme (Zundel S. et al., J Immunol 172:4342-4350 (2004); Megyeri et al., J. Biol. Chem. 288:8922-8934 (2013).

Recently, evidence for possible physiological functions of MASP-1 and MASP-3 emerged from transgenic mouse studies using a mouse strain with a combined MASP-1 and MASP-3 deficiency. While MASP-1/3-knockout mice have a functional lectin pathway (Schwaeble et al., PNAS 108:7523-7528 (2011)), they appear to lack alternative pathway activity (Takahashi et al., JEM 207(1):29-37 (2010)). Lack of alternative pathway activity appears to be due to a processing defect of complement factor D, which is necessary for alternative pathway activity. In MASP-1/3 knockout mice, all factor D is circulating as a proteolytically inactive pro-form, whereas in the serum of normal mice, substantially all of factor D is in the active form. Biochemical analysis suggested that MASP-1 may be able to convert complement factor D from its zymogen form into its enzymatically active form (**FIGURE 39**; Takahashi et al., JEM 207(1):29-37 (2010)). MASP-3 also cleaves pro-factor D zymogen and produce active factor D *in vitro* (**FIGURE 39**; Takahashi et al., JEM 207(1):29-37 (2010)). Factor D is present as an active enzyme in circulation in normal individuals, and MASP-1 and MASP-3, as well as HTRA-1, may be responsible for this activation. Furthermore, mice with combined MBL and ficolin deficiencies still produce normal levels of factor D and have a fully functional alternative pathway. Thus, these physiological functions of MASP-1 and MASP-3 do not necessarily involve lectins, and are thus unrelated to the lectin pathway. Recombinant mouse and human MASP-3 also appear to cleave factor B and support C3 deposition on *S. aureus in vitro* (**FIGURE 36**; Iwaki D. et al., J Immunol 187(7):3751-8 (2011)).

An unexpected physiological role for MASP-3 has emerged from recent studies of patients with 3MC syndrome (previously designated the Carnevale, Mingarelli, Malpuech, and Michels syndrome; OMIM # 257920). These patients display severe developmental abnormalities, including cleft palate, cleft lip, cranial malformations and mental retardation. Genetic analysis identified 3MC patients that were homozygous for a dysfunctional MASP-3 gene (Rooryck et al., Nat Genet. 43(3):197-203 (2011)). Another group of 3MC patients was found to be homozygous for a mutation in the MASP-1 gene that leads to the absence of functional MASP-1 and MASP-3 proteins. Yet another group of 3MC patients lacked a functional CL-11 gene. (Rooryck et al., Nat Genet. 43(3):197-203 (2011)). Thus, the CL-11 MASP-3 axis appears to play a role during embryonic development. The molecular mechanisms of this developmental pathway are unclear. It is unlikely, however, to be mediated by a conventional complement-driven process since individuals with deficiencies of common complement components C3 do not develop this syndrome. Thus, prior to the discovery of the instant inventors, as described herein, a functional role for MASP-3 in lectin-dependent complement activation was previously not established.

The structures of the catalytic fragment of MASP-1 and MASP-2 have been determined by X-ray crystallography. Structural comparison of MASP-1 protease domain with those of other complement proteases revealed the basis of its relaxed substrate specificity (Dobó et al., J. Immunol 183:1207-1214 (2009)). While the accessibility of the substrate binding groove of MASP-2 is restricted by surface loops (Harmat et al., J Mol Biol 342:1533-1546 (2004)), MASP-1 has an open substrate binding pocket which resembles that of trypsin rather than other complement proteases. A thrombin-like property of the MASP-1 structure is the unusually large 60 amino acid loop (loop B) which may interact with substrates. Another interesting feature of the MASP-1 structure is the internal salt bridge between the S1 Asp189 and Arg224. A similar salt bridge can be found in the substrate binding pocket of factor D, which can regulate its protease activity. C1s and MASP-2 have almost identical substrate specificities. Surprisingly, some of the eight surface loops of MASP-2, which determine the substrate specificities, have quite different conformations compared to those of C1s. This means that the two functionally related enzymes interact with the same substrates in a different manner. The structure of zymogen MASP-2 shows an inactive protease domain with disrupted oxyanion hole and substrate binding pocket (Gál et al., J Biol Chem 280:33435-33444 (2005)). Surprisingly, zymogen MASP-2 shows considerable activity on a large protein substrate, C4. It is likely that the structure of zymogen MASP-2 is quite flexible, enabling the transition between the inactive and the active forms. This flexibility, which is reflected in the structure, may play a role in the autoactivation process.

Northern blot analysis indicates that liver is the major source of MASP-1 and MASP-2 mRNA. Using a 5' specific cDNA probe for MASP-1, major MASP-1 transcript was seen at 4.8 kb and a minor one at approximately 3.4 kb, both present in human and mouse liver (Stover et al., Genes Immunity 4:374-84 (2003)). MASP-2 mRNA (2.6 kb) and MAp19 mRNA (1.0 kb) are abundantly expressed in liver tissue. MASP-3 is expressed in the liver, and also in many other tissues, including neuronal tissue (Lynch N. J. et al., J Immunol 174:4998-5006 (2005)).

A patient with a history of infections and chronic inflammatory disease was found to have a mutated form of MASP-2 that fails to form an active MBL-MASP complex (Stengaard-Pedersen et al., N Engl J Med 349:554-560 (2003)). Some investigators have determined that deficiency of MBL leads to a tendency to frequent infections in childhood (Super et al., Lancet 2:1236-1239 (1989); Garred et al., Lancet 346:941-943 (1995) and a decreased resistance to HIV infection (Nielsen et al., Clin Exp Immunol 100:219-222 (1995); Garred et al., Mol Immunol 33 (suppl 1):8 (1996)). However, other studies have not demonstrated a significant correlation of low MBL levels with increased infections (Egli et al., PLoS One. 8(1):e51983(2013); Ruskamp et al., J Infect Dis. 198(11):1707-13 (2008); Israëls et al., Arch Dis Child Fetal Neonatal Ed. 95(6):F452-61 (2010)). While the literature is mixed, deficiency, or non-utilization, of MASP may have an adverse effect on an individual's ability to mount immediate, non-antibody-dependent defense against certain pathogens.

### iii. Supporting data for the new understanding, underscoring traditional assay conditions that are devoid of Ca⁺⁺ and results obtained using a more physiological set of conditions that include Ca⁺⁺.

Several independent lines of strong experimental evidence are provided herein pointing to the conclusion that the lectin pathway activation route of complement activates complement via two independent effector mechanisms: i) LEA-2: a MASP-2-driven path that mediates complement-driven opsonisation, chemotaxis (Schwaeble et al., PNAS 108:7523-7528 (2011)), and cell lysis, and ii) LEA-1: a novel MASP-3-dependent activation route that initiates complement activation by generating the alternative pathway convertase C3bBb through cleavage and activation of factor B on activator surfaces, which will then catalyze C3b deposition and formation of the alternative pathway convertase C3bBb, which can result in cell lysis as well as microbial opsonization. In addition, as described herein, separate lectin-independent activation of factor B and/or factor D by MASP-1, MASP-3, or HTRA-1, or a combination of any the three, can also lead to complement activation via the alternative pathway.

A lectin pathway-dependent MASP-3-driven activation of the alternative pathway appears to contribute to the well-established factor D-mediated cleavage of C3b-bound factor B to achieve optimal activation rates for complement-dependent lysis through the terminal activation cascade to lyse bacterial cells through the formation of C5b-9 membrane attack complexes (MAC) on the cellular surface (**FIGURES 14-15**). This rate-limited event appears to require optimal coordination as it is defective in the absence of MASP-3 functional activity as well as in the absence of factor D functional activity. As described in Examples 1-4 herein, the inventors discovered this MASP-3-dependent lectin pathway function when studying the phenotype of MASP-2 deficiency and MASP-2 inhibition in experimental mouse models of *N. menigitidis* infection. Gene-targeted, MASP-2-deficient mice and wild-type mice treated with antibody-based MASP-2 inhibitors were highly resistant to experimental *N. meningitidis* infection (see **FIGURES 8-12**). When the infectious dose was adjusted to give approximately 60% mortality in the wild-type littermates, all of the MASP-2-deficient or MASP-2-depleted mice cleared the infection and survived (see **FIGURE 8** and **FIGURE 12**). This extremely high degree of resistance was reflected in a significant increase of serum bactericidal activity in MASP-2-deficient or MASP-2-depleted mouse serum. Further experiments showed that this bactericidal activity was dependent on alternative pathway-driven bacterial lysis. Mouse sera deficient of factor B, or factor D, or C3 showed no bactericidal activity towards N. *meningitidis*, indicating that the alternative pathway is essential for driving the terminal activation cascade. A surprising result was that mouse sera deficient of MBL-A and MBL-C (both being the lectin-pathway recognition molecules that recognize *N. meningitidis*) as well as mouse sera deficient of the lectin pathway-associated serine proteases MASP-1 and MASP-3 had lost all bacteriolytic activity towards *N. meningitidis* (**FIGURE 15**). A recent paper (Takahashi M. et al., JEM 207: 29-37 (2010)) and work presented herein (**FIGURE 39**) demonstrate that MASP-1 can convert the zymogen form of factor D into its enzymatically active form and may in part explain the loss of lytic activity through the absence of enzymatically active factor D in these sera. This does not explain the lack of bactericidal activity in MBL-deficient mice since these mice have normal enzymatically active factor D (Banda et al., Mol Imunol 49(1-2):281-9 (2011)). Remarkably, when testing human sera from patients with the rare 3MC autosomal recessive disorder (Rooryck C, et al., Nat Genet. 43(3):197-203) with mutations that render the serine protease domain of MASP-3 dysfunctional, no bactericidal activity against *N. meningitidis* was detectable (n.b.: These sera have MASP-1 and factor D, but no MASP-3).

The hypothesis that human serum requires lectin pathway-mediated MASP-3-dependent activity to develop bactericidal activity is further supported by the observation that MBL-deficient human sera also fail to lyse *N. meningitidis* (**FIGURES 13-14**). MBL is the only human lectin-pathway recognition molecule that binds to this pathogen. Since MASP-3 does not auto-activate, the inventors hypothesize that the higher bacteriolytic activity in MASP-2-deficient sera could be explained by a favored activation of MASP-3 through MASP-1 since, in the absence of MASP-2, all lectin-pathway activation complexes that bind to the bacterial surface will be loaded with either MASP-1 or MASP-3. Since activated MASP-3 cleaves both factor D (**FIGURE 39**) and factor B to generate their respective enzymatically active forms *in vitro* (**FIGURE 37** and Iwaki D., et al., J. Immunol.187(7):3751-3758 (2011)), the most likely function of MASP-3 is to facilitate the formation of the alternative pathway C3 convertase (i.e., C3bBb).

While the data for the lectin-dependent role are compelling, multiple experiments suggest that MASP-3 and MASP-1 are not necessarily obligated to function in a complex with lectin molecules. Experiments such as that shown in **FIGURE 35B** demonstrate the ability of MASP-3 to activate the alternative pathway (as demonstrated by C3b deposition *on S. aureus*) under conditions (i.e., the presence of EGTA) in which complexes with lectin would not be present. **FIGURE 35A** demonstrates that deposition under these conditions is dependent upon factor B, factor D, and factor P, all critical components of the alternative pathway. Addtionally, factor D activation by MASP-3 and MASP-1 (**FIGURE 39**), and factor B activation by MASP-3 (**FIGURE 37**) can occur *in vitro* in the absence of lectin. Finally, hemolysis studies of mouse erythrocytes in the presence of human serum demonstrate a clear role for both MBL and MASP-3 for cell lysis. However, the deficiency of MBL does not completely reproduce the severity of the deficiency of MASP-3, in contrast to what would be expected if all functional MASP-3 were complexed with MBL. Thus, the inventors do not wish to be constrained by the notion that all of the roles for MASP-3 (and MASP-1) demonstrated herein can be attributed solely to function associated with lectin.

The identification of the two effector arms of the lectin pathway, as well as the possible lectin-independent functions of MASP-1, MASP-3, and HTRA-1, represent novel opportunities for therapeutic interventions to effectively treat defined human pathologies caused by excessive complement activation in the presence of microbial pathogens or altered host cells or metabolic deposits. As described herein, the inventors have now discovered that in the absence of MASP-3 and in the presence of MASP-1, the alternative pathway is not activated on surface structures (see **FIGURES 17-18**, **35B**, **41-42**, **45-46**). Since the alternative pathway is important in driving the rate-limiting events leading to bacterial lysis as well as cell lysis (Mathieson PW, et al., J Exp Med 177(6):1827-3 (1993)), our results demonstrate that activated MASP-3 plays an important role in the lytic activity of complement. As shown in **FIGURES 14-15**, **21-23**, **43-44**, and **46-47**, in serum of 3MC patients lacking MASP-3 but not MASP-1, the lytic terminal activation cascade of complement is defective. The data shown in **FIGURES 14** and **15** demonstrate a loss of bacteriolytic activity in absence of MASP-3 and/or MASP-1/MASP-3 functional activity. Likewise, the loss of hemolytic activity in MASP-3-deficient human serum (**FIGURES 21-23**, **43-44** and **46-47**), coupled with the ability to reconstitute hemolysis by adding recombinant MASP-3 (**FIGURES 46-47**), strongly supports the conclusion that activation of the alternative pathway on target surfaces (which is essential to drive complement-mediated lysis) depends on the presence of activated MASP-3. Based on the new understanding of the lectin pathway detailed above, alternative pathway activation of target surfaces is thus dependent upon LEA-1, and/or lectin-independent activation of factor B and/or factor D, which is also mediated by MASP-3, and therefore, agents that block MASP-3-dependent complement activation will prevent alternative pathway activation on target surfaces.

The disclosure of the essential role of MASP-3-dependent initiation of alternative pathway activation implies that the alternative pathway is not an independent, stand-alone pathway of complement activation as described in essentially all current medical textbooks and recent review articles on complement. The current and widely held scientific view is that the alternative pathway is activated on the surface of certain particulate targets (microbes, zymosan, and rabbit erythrocytes) through the amplification of spontaneous "tick-over" C3 activation. However, the absence of any alternative pathway activation in sera of MASP-1 and MASP-3 double-deficient mice and human 3MC patient serum on both zymosan-coated plates and two different bacteria (*N. meningitidis* and *S. aureus*), and the reduction of hemolysis of erythrocytes in MASP-3-deficient sera from human and mouse indicate that initiation of alternative pathway activation on these surfaces requires functional MASP-3. The required role for MASP-3 may be either lectin-dependent or - independent, and leads to formation of the alternative pathway C3 convertase and C5 convertase complexes, i.e. C3bBb and C3bBb(C3b)n, respectively. Thus, the inventors here disclose the existence of a previously elusive initiation routes for the alternative pathway. This initiation route is dependent upon (i) LEA-1, a newly discovered activation arm of the lectin pathway, and/or (ii) lectin-independent roles of the proteins MASP-3, MASP-1, and HTRA-1.

### III. THE ROLE OF MASP-2 AND MASP-3 IN PAROXYSMAL NOCTURNAL HEMOGLOBINURIA AND THERAPEUTIC METHODS USING MASP-2 AND MASP-3 INHIBITORY AGENTS

### i. Overview of PNH

Paroxysmal nocturnal hemoglobinuria (PNH), sometimes also referred to as Marchiafava-Micheli syndrome, is an acquired, potentially life-threatening disease of the blood. PNH may develop on its own, referred to as "primary PNH" or in the context of other bone marrow disorders such as aplastic anemia, referred to as "secondary PNH." The majority of cases are primary PNH. PNH is characterized by complement-induced destruction of red blood cells (hemolysis), low red blood cell counts (anemia), thrombosis and bone marrow failure. Laboratory findings in PNH show changes consistent with intravascular hemolytic anemia: low hemoglobin, raised lactate dehydrogenase, raised reticulocyte counts (immature red cells released by the bone marrow to replace the destroyed cells), raised bilirubin (a breakdown product of hemoglobin), in the absence of autoreactive RBC-binding antibodies as a possible cause.

The hallmark of PNH is the chronic complement-mediated hemolysis caused by the unregulated activation of terminal complement components, including the membrane attack complex, on the surface of circulating RBCs. PNH RBCs are subject to uncontrolled complement activation and hemolysis due to the absence of the complement regulators CD55 and CD59 on their surface (Lindorfer, M.A., et al., Blood 115(11):2283-91 (2010), Risitano, et al., Mini-Reviews in Medicinal Chemistry, 11:528-535 (2011)). CD55 and CD59 are abundantly expressed on normal RBCs and control complement activation. CD55 acts as a negative regulator of the alternative pathway, inhibiting the assembly of the alternative pathway C3 convertase (C3bBb) complex and accelerating the decay of preformed convertase, thus blocking the formation of the membrane attack complex (MAC). CD59 inhibits the complement membrane attack complex directly by binding the C5b678 complex and preventing C9 from binding and polymerizing.

While hemolysis and anemia are the dominant clinical features of PNH, the disease is a complex hematologic disorder that further includes thrombosis and bone marrow failure as part of the clinical findings (Risitano et al, Mini Reviews in Med Chem11:528-535 (2011)). At the molecular level, PNH is caused by the abnormal clonal expansion of hematopoietic stem cells lacking a functional PIG A gene. PIG A is an X-linked gene encoding a glycosyl-phosphatidyl inositol transferase required for the stable surface expression of GPI-anchored class A glycoproteins, including CD55 and CD59. For reasons that are presently under investigation, hematopoietic stem cells with a dysfunctional PIG A gene that arise as the result of spontaneous somatic mutations can undergo clonal expansion to the point where their progeny constitute a significant portion of the peripheral hematopoietic cell pool. While both erythrocyte and lymphocyte progeny of the mutant stem cell clone lack CD55 and CD59, only the RBCs undergo overt lysis after they enter the circulation.

Current treatment for PNH includes blood transfusion for anemia, anticoagulation for thrombosis and the use of the monoclonal antibody eculizumab (Soliris®), which protects blood cells against immune destruction by inhibiting the complement system (Hillmen P. et al., N. Engl. J. Med. 350(6):552-559 (2004)). Eculizumab (Soliris®) is a humanized monoclonal antibody that targets the complement component C5, blocking its cleavage by C5 convertases, thereby preventing the production of C5a and the assembly of MAC. Treatment of PNH patients with eculizumab has resulted in a reduction of intravascular hemolysis, as measured by lactate dehydrogenase (LDH), leading to hemoglobin stabilization and transfusion independence in about half of the patients (Risitano et al, Mini-Reviews in Medicinal Chemistry, 11(6) (2011)). While nearly all patients undergoing therapy with eculizumab achieve normal or almost normal LDH levels (due to control of intravascular hemolysis), only about one third of the patients reach a hemoglobin value about 11gr/dL, and the remaining patients on eculizumab continue to exhibit moderate to severe (i.e., transfusion-dependent) anemia, in about equal proportions (Risitano A.M. et al., Blood 113:4094-100 (2009)). As described in Risitano et al., Mini-Reviews in Medicinal Chemistry 11:528-535 (2011), it was demonstrated that PNH patients on eculizumab contained large amounts of C3 fragments bound to their PNH erythrocytes (while untreated patients did not). This finding lead to the recognition that in Soliris treated PNH patients, PNH RBCs that are no longer hemolyzed due to C5 blockade now can accumulate copious amounts of membrane-bound C3 fragments, which operate as opsonins, resulting in their entrapment in the reticuloendothelial cells through specific C3 receptors and subsequent extravascular hemolysis. Thus, while preventing intravascular hemolysis and the resulting sequelae, eculizumab therapy simply diverts the disposition of these RBCs from intravascular to extravascular hemolysis, resulting in substantial residual untreated anemia in many patients (Risitano A.M. et al., Blood 113:4094-100 (2009)). Therefore, therapeutic strategies in addition to the use of eculizumab are needed for those patients developing C3-fragment-mediated extravascular hemolysis, because they continue to require red cell transfusions. Such C3 fragment targeting approaches have demonstrated utility in experimental systems (Lindorfer et al., Blood 115:2283-91, 2010).

### ii. Complement-initiating mechanisms in PNH

The causal link between defective surface expression of the negative complement regulators CD55 and CD59 in PNH, combined with the effectiveness of eculizumab in preventing intravascular hemolysis, clearly define PNH as a condition mediated by the complement system. While this paradigm is widely accepted, the nature of the events initiating complement activation, and the complement activation pathway(s) involved remain unresolved. Because CD55 and CD59 negatively regulate the terminal amplification steps in the complement cascade common to all complement initiation pathways, deficiency of these molecules will lead to exaggerated formation and membrane integration of membrane attack complexes, regardless of whether complement activation is initiated by the lectin pathway, by the classical pathway or by spontaneous turnover of the alternative pathway. Thus, in PNH patients, any complement activation events that lead to C3b deposition on the RBC surface can trigger subsequent amplification and pathological hemolysis (intravascular and/or extravascular) and precipitate a hemolytic crisis. A clear mechanistic understanding of the molecular events triggering hemolytic crisis in PNH patients has remained elusive. Because no complement initiating event is overtly evident in PNH patients undergoing a hemolytic crisis, the prevailing view is that complement activation in PNH may occur spontaneously owing to low level "tick-over" activation of the alternative pathway, which is subsequently magnified by inappropriate control of terminal complement activation due to lack of CD55 and CD59.

However, it is important to note that in its natural history, PNH usually develops or is exacerbated after certain events, such as an infection or an injury (Risitano, Biologics 2:205-222 (2008)), which have been shown to trigger complement activation. This complement activation response is not dependent on prior immunity of the host towards the inciting pathogen, and hence likely does not involve the classical pathway. Rather, it appears that this complement activation response is initiated by lectin binding to foreign or "altered self" carbohydrate patterns expressed on the surface of microbial agents or damaged host tissue. Thus, the events precipitating hemolytic crisis in PNH are tightly linked to complement activation initiated via lectins. This makes it very likely that lectin activation pathways provide the initiating trigger that ultimately leads to hemolysis in PNH patients.

Using well-defined pathogens that activate complement via lectins as experimental models to dissect the activation cascades at the molecular level, we demonstrate that, depending on the inciting microbe, complement activation can be initiated by either LEA-2 or LEA-1, leading to opsonization and/or lysis. This same principle of dual responses (i.e., opsonization and/or lysis) to lectin initiation events will likely also apply to other types of infectious agents, or to complement activation by lectins following tissue injury to the host, or other lectin-driven complement activation events that may precipitate PNH. On the basis of this duality in the lectin pathway, we infer that LEA-2- and/or LEA-1-initiated complement activation in PNH patients promotes opsonization and/or lysis of RBCs with C3b and subsequent extravascular and intravascular hemolysis. Therefore, in the setting of PNH, inhibition of both LEA-1 and LEA-2 would be expected to address both intravascular and extravascular hemolysis, providing a significant advantage over the C5 inhibitor eculizumab.

It has been determined that exposure to *S. pneumoniae* preferentially triggers lectin-dependent activation of LEA-2, which leads to opsonization of this microbe with C3b. Since *S. pneumonia* is resistant to MAC-mediated lysis, its clearance from circulation occurs through opsonisation with C3b. This opsonization and subsequent removal from circulation is LEA-2-dependent, as indicated by compromised bacterial control in MASP-2-deficient mice and in mice treated with MASP-2 monoclonal antibodies (PLOS Pathog., 8: e1002793. (2012)).

In exploring the role of LEA-2 in the innate host responses to microbial agents, we tested additional pathogens. A dramatically different outcome was observed when *Neisseria meningitidis* was studied as a model organism. *N. meningitidis* also activates complement via lectins, and complement activation is required for containment of *N. meningitidis* infections in the naive host. However, LEA-2 plays no host protective functional role in this response: As shown in **FIGURES 8** and **9****,** blockade of LEA-2 through genetic ablation of MASP-2 does not reduce survival following infection with N. *meningitidis.* To the contrary, LEA-2 blockade by MASP-2 ablation significantly improved survival **(****FIGURES 8** and **9****)** as well as illness scores **(****FIGURE 11****)** in these studies. LEA-2 blockade by administration of MASP-2 antibody yielded the same result **(****FIGURE 12****),** eliminating secondary or compensatory effects in the knockout-mouse strain as a possible cause. These favorable outcomes in LEA-2-ablated animals were associated with a more rapid elimination of *N. meningitidis* from the blood **(****FIGURE 10****).** Also, as described herein, incubation of *N. meningitidis* with normal human serum killed *N. meningitidis* **(****FIGURE 13****).** Addition of a functional monoclonal antibody specific for human MASP-2 that blocks LEA-2, but not administration of an isotype control monoclonal antibody, may enhance this killing response. Yet, this process depends on lectins and at least a partially functional complement system, as MBL-deficient human serum or heat-inactivated human serum was unable to kill *N. meningitidis* **(****FIGURE 13****).** Collectively, these novel findings suggest that *N. meningitidis* infections in the presence of a functional complement system are controlled by a lectin-dependent but LEA-2-independent pathway of complement activation.

The hypothesis that LEA-1 may be the complement pathway responsible for lectin-dependent killing of *N. meningitidis* was tested using a serum specimen from a 3MC patient. This patient was homozygous for a nonsense mutation in exon 12 of the MASP-1/3 gene. As a result, this patient lacked a functional MASP-3 protein, but was otherwise complement sufficient (exon 12 is specific for the MASP-3 transcript; the mutation has no effect on MASP-1 function or expression levels) (see Nat Genet 43(3):197-203 (2011)). Normal human serum efficiently kills *N. meningitidis,* but heat-inactivated serum deficient in MBL (one of the recognition molecules for the Lectin pathway) and MASP-3-deficient serum were unable to kill *N. meningitidis* **(****FIGURE 14****).** Thus, LEA-1 appears to mediate *N. meningitidis* killing. This finding was confirmed using serum samples from knockout mouse strains. While complement containing normal mouse serum readily killed *N*. *meningitidis,* MBL-deficient or MASP-1/3-deficient mouse serum was as ineffective as heat-inactivated serum that lacks functional complement **(****FIGURE 15****).** Conversely, MASP-2-deficient serum exhibited efficient killing of *N. meningitidis.*

These findings provide evidence for a hitherto unknown duality in the lectin pathway by revealing the existence of separate LEA-2 and LEA-1 pathways of lectin-dependent complement activation. In the examples detailed above, LEA-2 and LEA-1 are non-redundant and mediate distinct, functional outcomes. The data suggest that certain types of lectin pathway activators (including, but not limited to *S. pneumonia*) preferentially initiate complement activation via LEA-2 leading to opsonization, while others (exemplified by *N. meningitidis*) preferentially initiate complement activation via LEA-1 and promote cytolytic processes. The data do not, however, necessarily limit LEA-2 to opsonization and LEA-1 to cytolytic processes, as both pathways in other settings can mediate opsonization and/or lysis.

In the context of lectin-dependent complement activation by *N. meningitidis,* LEA-2 and LEA-1 arms appear to compete with each other, as blockade of LEA-2 enhanced LEA-1-dependent lytic destruction of the organism *in vitro* **(****FIGURE 15****).** As detailed above, this finding can be explained by the increased likelihood of lectin MASP-1 complexes residing in close proximity to lectin MASP-3 complexes in the absence of MASP-2, which will enhance LEA-1 activation and thus promote more effective lysis of *N. meningitides.* Because lysis of *N. meningitidis* is the main protective mechanism in the naive host, blockade of LEA-2 *in vivo* increases *N. meningitidis* clearance and leads to enhanced killing.

While the examples discussed above illustrate opposing effects of LEA-2 and LEA-1 with respect to outcomes following infection with *N. meningitidis,* there may be other settings where both LEA-2 and LEA-1 may synergize to produce a certain outcome. As detailed below, in other situations of pathological complement activation via lectins such as those present in PNH, LEA-2- and LEA-1-driven complement activation may cooperate in a synergistic manner to contribute to the overall pathology of PNH. In addition, as described herein, MASP-3 also contributes to the lectin-independent conversion of factor B and factor D, which can occur in the absence of Ca⁺⁺, commonly leading to the conversion of C3bB to C3bBb and of pro-factor D to factor D, which may further contribute to the pathology of PNH.

### iii. Biology and expected functional activity in PNH

This section describes the inhibitory effects of LEA-2 and LEA-1 blockade on hemolysis in an *in vitro* model of PNH. The findings support the utility of LEA-2-blocking agents (including, but not limited to, antibodies that bind to and block the function of MASP-2) and LEA-1-blocking agents (including, but not limited to, antibodies that bind to and block the function of MASP-1-mediated activation of MASP-3, MASP-3, or both) to treat subjects suffering from one or more aspects of PNH, and also the use of inhibitors of LEA-2 and/or LEA-1, and/or MASP-3-dependent, lectin-independent complement activation (including MASP-2 inhibitors, MASP-3 inhibitors, and dual- or bispecific MASP-2/MASP-3 or MASP-1/MASP-2 inhibitors, and pan-specific MASP-1/MASP-2/MASP-3 inhibitors) to ameliorate the effects of C3-fragment-mediated extravascular hemolysis in PNH patients undergoing therapy with a C5-inhibitor such as eculizumab.

### iv. MASP-2 inhibitors to block opsonization and extravascular hemolysis of PNH RBCs through the reticuloendothelial systems

As detailed above, PNH patients become anemic owing to two distinct mechanisms of RBC clearance from circulation: intravascular hemolysis via activation of the membrane attack complex (MAC), and extravascular hemolysis following opsonization with C3b and subsequent clearance following complement receptor binding and uptake by the reticuloendothelial system. The intravascular hemolysis is largely prevented when a patient is treated with eculizumab. Because eculizumab blocks the terminal lytic effector mechanism that occurs downstream of both the complement-initiating activation event as well as the ensuing opsonization, eculizumab does not block extravascular hemolysis (Risitano A.M. et al., Blood 113:4094-100 (2009)). Instead, RBCs that would have undergone hemolysis in untreated PNH patients now can accumulate activated C3b proteins on their surface, which augments uptake by the reticuloendothelial system and enhances their extravascular hemolysis. Thus, eculizumab treatment effectively diverts RBC disposition from intravascular hemolysis to potential extravascular hemolysis. As a result, some eculizumab-treated PNH patients remain anemic. It follows that agents that block complement activation upstream and prevent the opsonization of PNH RBCs may be particularly suitable to block the extravascular hemolysis occasionally seen with eculizumab.

The microbial data presented here suggest that LEA-2 is often the dominant route for lectin-dependent opsonization. Furthermore, when lectin-dependent opsonization (measured as C3b deposition) was assessed on three prototypic lectin activation surfaces (mannan, **FIGURE 19A****;** zymosan, **FIGURE 19B****,** and *S. pneumonia;* **FIGURE 19C****),** LEA-2 appears to be the dominant route for lectin-dependent opsonization under physiologic conditions (i.e., in the presence of Ca⁺⁺ wherein all complement pathways are operational). Under these experimental conditions, MASP-2-deficient serum (which lacks LEA-2) is substantially less effective in opsonizing the test surfaces than WT serum. MASP-1/3-deficient serum (which lacks LEA-1) is also compromised, though this effect is much less pronounced as compared to serum lacking LEA-2. The relative magnitude of the contributions of LEA-2 and LEA-1 to lectin-driven opsonization is further illustrated in **FIGURES 20A - 20C****.** While the alternative pathway of complement has been reported to support opsonization of lectin activating surfaces in the absence of lectin pathway or classical pathway (Selander et al., J Clin Invest 116(5): 1425-1434 (2006)), the alternative pathway in isolation (measured under Ca⁺⁺-free assay conditions) appears substantially less effective than the LEA-2- and LEA-1-initiated processes described herein. By extrapolation, these data suggest that opsonization of PNH RBCs may also be preferentially initiated by LEA-2 and, to a lesser extent, by LEA-1 (possibly amplified by the alternative pathway amplification loop), rather than the result of lectin-independent alternative pathway activation. Therefore, LEA-2 inhibitors may be expected to be most effective at limiting opsonization and preventing extravascular hemolysis in PNH. However, recognition of the fact that lectins other than MBL, such as ficolins, bind to non-carbohydrate structures such as acetylated proteins, and that MASP-3 preferentially associates with H-ficolin (Skjoedt et al., Immunobiol. 215:921-931, 2010), leaves open the possibility of a significant role for LEA-1 in PNH-associated RBC opsonization as well. Therefore, LEA-1 inhibitors are expected to have additional anti-opsonization effects, and the combination of LEA-1 and LEA-2 inhibitors is expected to be optimal and mediate the most robust treatment benefit in limiting opsonization and extravascular hemolysis in PNH patients. This concept is further supported by opsonization data shown in **FIGURE 28****:** factor D-deficient mouse serum (which lacks the ability to activate the alternative pathway in fluid phase but has a functional classical pathway as well as functional LEA-1 and LEA-2 pathways) shows no deficit in opsonization compared to WT serum. Factor B-deficient serum, which lacks LEA-1, shows reduced opsonization while factor D-deficient serum treated with MASP-2 monoclonal antibody to block LEA-2-mediated complement activation yields a more robust suppression of opsonization **(****FIGURE 28****).** Importantly, addition of MASP-2 monoclonal antibody to factor B-deficient serum suppressed opsonization more effectively than either MASP-2 blockade or factor D blockade alone. Thus, LEA-2 and LEA-1 act additively or synergistically to promote opsonization, and a crossreactive or bispecific LEA-1/LEA-2 inhibitor is expected to be most effective at blocking opsonization and extravascular hemolysis in PNH.

### v. Role of MASP-3 inhibitors in PNH

Using an *in vitro* model of PNH, we demonstrated that complement activation and the resulting hemolysis in PNH are indeed initiated by LEA-2 and/or LEA-1 activation, and that it is not an independent function of the alternative pathway. These studies used mannan-sensitized RBCs of various mouse stains, including RBCs from Crry-deficient mice (an important negative regulator of the terminal complement pathway in mice) as well as RBCs from CD55/CD59-deficient mice, which lack the same complement regulators that are absent in PNH patients). When mannan-sensitized Crry-deficient RBCs were exposed to complement-sufficient human serum, the RBCs effectively hemolysed at a serum concentration of 3% **(****FIGURE 21** and **22****)** while complement-deficient serum (HI: heat-inactivated) was not hemolytic. Remarkably, complement-sufficient serum where LEA-2 was blocked by addition of MASP-2 antibody had reduced hemolytic activity, and 6% serum was needed for effective hemolysis. Similar observations were made when CD55/CD59-deficient RBCs were tested **(****FIGURE 24****).** Complement-sufficient human serum supplemented with MASP-2 monoclonal antibody (i.e., serum where LEA-2 is suppressed) was about two-fold less effective than untreated serum in supporting hemolysis. Furthermore, higher concentrations of LEA-2-blocked serum (i.e., treated with antiMASP-2 monoclonal antibody) were needed to promote effective hemolysis of untreated WT RBCs compared to untreated serum **(****FIGURE 23****).**

Even more surprisingly, serum from a 3MC patient homozygous for a dysfunctional MASP-3 protein (and hence lacking LEA-1) was completely unable to hemolyze mannan-sensitized Crry-deficient RBCs **(****FIGURE 22** and **FIGURE 23****).** A similar outcome was observed when unsensitized normal RBCs were used: As shown in **FIGURE 23****,** LEA-1-defective serum isolated from a 3MC patient was completely ineffective at mediating hemolysis. Collectively, these data indicate that whereas LEA-2 contributes significantly to the intravascular hemolysis response, LEA-1 is the predominant complement-initiating pathway leading to hemolysis. Thus, while LEA-2 blocking agents are expected to significantly reduce intravascular hemolysis of RBCs in PNH patients, LEA-1 blocking agents are expected to have a more profound effect and largely eliminate complement-driven hemolysis.

It should be noted that the serum of the LEA-1-deficient 3MC patient used in this study possessed a diminished but functional alternative pathway when tested under conventional alternative pathway assay conditions **(****FIGURE 17****).** This finding suggests that LEA-1 makes a greater contribution to hemolysis than alternative pathway activity as conventionally defined in this experimental setting of PNH. By inference, it is implied that LEA-1-blocking agents will be at least as effective as agents blocking other aspects of the alternative pathway in preventing or treating intravascular hemolysis in PNH patients.

### vi. Role of MASP-2 inhibitors in PNH

The data presented herein suggest the following pathogenic mechanisms for anemia in PNH: intravascular hemolysis due to unregulated activation of terminal complement components and lysis of RBC by formation of MAC, which is initiated predominantly, though not exclusively, by LEA-1, and extravascular hemolysis caused by opsonization of RBCs by C3b, which appears to be initiated predominately by LEA-2. While a discernible role for LEA-2 in initiating complement activation and promoting MAC formation and hemolysis is apparent, this process appears substantially less effective than LEA-1-initiated complement activation leading to hemolysis. Thus, LEA-2-blocking agents are expected to significantly reduce intravascular hemolysis in PNH patients, though this therapeutic activity is expected to be only partial. By comparison, a more substantial reduction in intravascular hemolysis in PNH patients is expected for LEA-1-blocking agents.

Extravascular hemolysis, a less dramatic, yet equally important mechanism of RBC destruction that leads to anemia in PNH, is primarily the result of opsonization by C3b, which appears to be predominantly mediated by LEA-2. Thus, LEA-2-blocking agents may be expected to preferentially block RBC opsonization and the ensuing extravascular hemolysis in PNH. This unique therapeutic activity of LEA-2-blocking agents is expected to provide a significant treatment benefit to all PNH patients as no treatment currently exists for those PNH patients who experience this pathogenic process.

### vii. LEA-2 inhibitors as adjunct treatment to LEA-1 inhibitors or terminal complement blocking agents

The data presented herein detail two pathogenic mechanisms for RBC clearance and anemia in PNH which can be targeted, separately or in combination, by distinct classes of therapeutic agents: the intravascular hemolysis initiated predominantly, though not exclusively, by LEA-1 and thus expected to be effectively prevented by a LEA-1-blocking agent, and extravascular hemolysis due to C3b opsonization driven predominantly by LEA-2, and thus effectively prevented by a LEA-2-blocking agent.

It is well documented that both intravascular and extravascular mechanisms of hemolysis lead to anemia in PNH patients (Risitano et al., Blood 113:4094-4100 (2009)). Therefore, it is expected that a LEA-1-blocking agent that prevents intravascular hemolysis in combination with a LEA-2 blocking agent that primarily prevents extravascular hemolysis will be more effective than either agent alone in preventing the anemia that develops in PNH patients. In fact, the combination of LEA-1- and LEA-2-blocking agents is expected to prevent all relevant mechanisms of complement initiation in PNH and thus block all symptoms of anemia in PNH.

It is also known that C5-blocking agents (such as eculizumab) effectively block intravascular hemolysis but do not interfere with opsonization. This leaves some anti-C5-treated PNH patients with substantial residual anemia due to extravascular hemolysis mediated by LEA-2 that remains untreated. Therefore, it is expected that a C5-blocking agent (such as eculizumab) that prevents intravascular hemolysis in combination with a LEA-2 blocking agent that reduces extravascular hemolysis will be more effective than either agent alone in preventing the anemia that develops in PNH patients.

Other agents that block the terminal amplification loop of the complement system leading to C5 activation and MAC deposition (including, but not limited to agents that block properdin, factor B or factor D or enhance the inhibitory activity of factor I, factor H or other complement inhibitory factors) are also expected to inhibit intravascular hemolysis. However, these agents are not expected to interfere with LEA-2-mediated opsonization in PNH patients. This leaves some PNH patients treated with such agents with substantial residual anemia due to extravascular hemolysis mediated by LEA-2 that remains untreated. Therefore, it is expected that treatment with such agents that prevent intravascular hemolysis in combination with a LEA-2-blocking agent that minimizes extravascular hemolysis will be more effective than either agent alone in preventing the anemia that develops in PNH patients. In fact, the combination of such agents and a LEA-2 blocking agent is expected to prevent all relevant mechanisms of RBC destruction in PNH and thus block all symptoms of anemia in PNH.

### viii. Use of LEA-1 and LEA-2 multiple, bispecific or pan-specific antibodies to treat PNH

As detailed above, the use of a combination of pharmacologic agents that individually block LEA-1 and LEA-2, and thus in combination block all complement activation events that mediate the intravascular as well as the extravascular hemolysis, is expected to provide the best clinical outcome for PNH patients. This outcome can be achieved for example, by co-administration of an antibody that has LEA-1-blocking activity together with an antibody that has LEA-2-blocking activity. In some cases, LEA-1- and LEA-2-blocking activities are combined into a single molecular entity, and that such entity with combined LEA-1- and LEA-2-blocking activity will effectively block intravascular as well as the extravascular hemolysis and prevent anemia in PNH. Such an entity may comprise or consist of a bispecific antibody where one antigen-combining site specifically recognizes MASP-1 and blocks LEA-1 and diminishes LEA-2 and the second antigen-combining site specifically recognizes MASP-2 and further blocks LEA-2. Alternatively, such an entity may consist of a bispecific monoclonal antibody where one antigen-combining site specifically recognizes MASP-3 and thus blocks LEA-1 and the second antigen-combining site specifically recognizes MASP-2 and blocks LEA-2. Such an entity may optimally consist of a bispecific monoclonal antibody where one antigen-combining site specifically recognizes both MASP-1 and MASP-3 and thus blocks LEA-1 and diminishes LEA-2 while the second antigen-combining site specifically recognized MASP-2 and further blocks LEA-2. Based on the similarities in the overall protein sequence and architecture, it can also be envisioned that a conventional antibody with two identical binding sites can be developed that specifically binds to MASP-1 and to MASP-2 and to MASP-3 in a functional manner, thus achieving functional blockade of LEA-1 and LEA-2. Such an antibody with pan-MASP inhibitory activity is expected to block both the intravascular as well as the extravascular hemolysis and thus effectively treat the anemia in PNH patients.

### IV. MASP INHIBITORY AGENTS

With the recognition that the lectin pathway of complement is composed of two major complement activation arms, LEA-1 and LEA-2, and that there also is a lectin-independent complement activation arm, comes the realization that it would be highly desirable to specifically inhibit one or more of these effector arms that cause a pathology associated with PNH without completely shutting down the immune defense capabilities of complement (i.e., leaving the classical pathway intact). This would leave the C1q-dependent complement activation system intact to handle immune complex processing and to aid in host defense against infection.

### i. Compositions for inhibiting LEA-1-mediated complement activation

As described herein, the inventors have unexpectedly discovered that activation of LEA-1, leading to lysis, is MASP-3-dependent. As further described herein, under physiological conditions, MASP-3-dependent LEA-1 activation also contributes to opsonization, thereby providing an additive effect with LEA-2-mediated complement activation. As demonstrated in Example 7, in the presence of Ca⁺⁺, factor D is not required, as MASP-3 can drive activation of LEA-1 in factor D^{-/-} sera. MASP-3, MASP-1, and HTRA-1 are able to convert pro-factor D to active factor D. Likewise, MASP-3 activation appears, in many instances, to be dependent on MASP-1, since MASP-3 (in contrast to MASP-1 and MASP-2) is not an auto-activating enzyme and is incapable of converting into its active form without the help of MASP-1 (Zundel, S. et al., J.Immunol. 172: 4342-4350 (2004); Megyeri et al., J. Biol. Chem. 288:8922-8934 (2013). As MASP-3 does not autoactivate and, in many instances, requires the activity of MASP-1 to be converted into its enzymatically active form, the MASP-3-mediated activation of the alternative pathway C3 convertase C3Bb can either be inhibited by targeting the MASP-3 zymogen or already-activated MASP-3, or by targeting MASP-1-mediated activation of MASP-3, or both, since, in many instances, in the absence of MASP-1 functional activity, MASP-3 remains in its zymogen form and is not capable of driving LEA-1 through direct formation of the alternative pathway C3 convertase (C3bBb).

The preferred protein component to target in the development of therapeutic agents to specifically inhibit LEA-1 is an inhibitor of MASP-3 (including inhibitors of MASP-1-mediated MASP-3 activation (e.g., a MASP-1 inhibitor that inhibits MASP-3 activation)).

In accordance with the foregoing, the disclosure provides methods of inhibiting the adverse effects of LEA-1 (i.e., hemolysis and opsonization) in a subject suffering from PNH, or at risk for developing PNH, comprising administering to the subject a pharmaceutical composition comprising an amount of a MASP-3 inhibitory agent effective to inhibit MASP-3-dependent complement activation and a pharmaceutically acceptable carrier.

MASP-3 inhibitory agents are administered in an amount effective to inhibit MASP-3-dependent complement activation in a living subject suffering from, or at risk for developing, PNH. Representative MASP-3 inhibitory agents include: molecules that inhibit the biological activity of MASP-3, including molecules that inhibit at least one or more of the following: lectin MASP-3-dependent activation of factor B, lectin MASP-3-dependent activation of pro-factor D, MASP-3-dependent, lectin-independent activation of factor B, and MASP-3-dependent, lectin-independent activation of pro-factor D (such as small-molecule inhibitors, MASP-3 antibodies and fragments thereof, or blocking peptides which interact with MASP-3 or interfere with a protein-protein interaction), and molecules that decrease the expression of MASP-3 (such as MASP-3 antisense nucleic acid molecules, MASP-3 specific RNAi molecules and MASP-3 ribozymes). A MASP-3 inhibitory agent may effectively block MASP-3 protein-to-protein interactions, interfere with MASP-3 dimerization or assembly, block Ca⁺⁺ binding, interfere with the MASP-3 serine protease active site, or reduce MASP-3 protein expression, thereby preventing MASP-3 from activating LEA-1-mediated, or lectin-independent, complement activation. The MASP-3 inhibitory agents can be used alone as a primary therapy or in combination with other therapeutics as an adjuvant therapy to enhance the therapeutic benefits of other medical treatments, as further described herein.

In one case, the MASP-3 inhibitory agent specifically binds to a portion of MASP-3 (SEQ ID NO:8) with a binding affinity of at least 10 times greater than to other components in the complement system. In another case, a MASP-3 inhibitory agent specifically binds to a portion of MASP-3 (SEQ ID NO:8) with a binding affinity of at least 100 times greater than to other components in the complement system. In one case, the MASP-3 inhibitory agent specifically binds to the serine protease domain of MASP-3 (aa 450-711 of SEQ ID NO:8) and inhibits MASP-3-dependent complement activation, with the proviso that the inhibitory agent does not bind to the serine protease domain of MASP-1 (SEQ ID NO:10), and it does not bind to the serine protease domain of MASP-2 (SEQ ID NO:5). In one case, the MASP-3 inhibitory agent is a MASP-3 monoclonal antibody, or fragment thereof, that specifically binds to MASP-3.

In another case, the MASP-3 inhibitory agent specifically binds to a portion of MASP-1 (SEQ ID NO:10) with a binding affinity of at least 10 times greater than to other components in the complement system, and inhibits MASP-1-mediated activation of MASP-3. In another case, the MASP-3 inhibitory agent specifically binds to a portion of MASP-1 (SEQ ID NO:10) with a binding affinity of at least 100 times greater than to other components (i.e., polypeptides, or fragments thereof) in the complement system, and inhibits MASP-1-mediated activation of MASP-3. In some cases, the MASP-3 inhibitory agent specifically binds to the serine protease domain of MASP-1 (aa 449-694 of SEQ ID NO:10) and inhibits MASP-1-mediated activation of MASP-3, with the proviso that the inhibitory agent does not bind to the serine protease domain of MASP-2 (SEQ ID NO:5), and it does not bind to the serine protease domain of MASP-3 (SEQ ID NO:8). In one case, the MASP-3 inhibitory agent is a MASP-1 monoclonal antibody, or fragment thereof, that specifically binds to MASP-1 and inhibits MASP-1-mediated activation of MASP-3. In some cases, the MASP-3 inhibitory agent that binds to MASP-1 inhibits MASP-1-mediated activation of MASP-3 and further inhibits MASP-1-mediated maturation of factor D.

In another case, the MASP-3 inhibitory agent binds to a portion of MASP-3 (SEQ ID ON:8) and also binds to a portion of MASP-1 (SEQ ID NO:10), with the proviso that the inhibitory agent does not bind to MASP-2 (SEQ ID NO:5), or MAp19 (SEQ ID NO:3). In one case, the MASP-3 inhibitory agent binds to a portion of MASP-3 (SEQ ID ON:8) and also binds to a portion of MASP-1 (SEQ ID NO: 10), with the proviso that the inhibitory agent does not bind to MASP-2 (SEQ ID NO:5) or MAp19 (SEQ ID NO:3). In one case, the MASP-3 inhibitory agent binds to a portion of MASP-3 (SEQ ID ON:8) and also binds to a portion of MASP-1 (SEQ ID NO:10), with the proviso that the inhibitory agent does not bind to MASP-2 (SEQ ID NO:5), MAp19 (SEQ ID NO:3), or MAp44 (SEQ ID NO:11), thereby providing allowing for a lower effective dose for inhibiting MASP-3-dependent complement activation due to the lack of binding to MAp44, which is present at a high concentration in human serum.

In one case, the MASP-3 inhibitory agent is a MASP-1/MASP-3 dual inhibitory agent that binds to an epitope within the amino acid region that is conserved between MASP-1 and MASP-3, such as the CUBI-CCP2 domain (aa 25-432 of SEQ ID NO:10), as illustrated in **FIGURES 3-5****.** In one case, the MASP-3 inhibitory agent is a MASP-1/MASP-3 dual inhibitory agent that binds to an epitope within the amino acid region that is conserved between MASP-1 and MASP-3, with the proviso that the inhibitory agent does not bind to MAp44, such as the CCP domain (aa 367-432 of SEQ ID NO:10). In another case, the MASP-3 inhibitory agent is a bispecific inhibitory agent, such as a bispecific monoclonal antibody, that specifically binds to an epitope on the MASP-3 protein (SEQ ID NO:8) and an epitope on the MASP-1 protein (SEQ ID NO:10). In some cases, the MASP-3 inhibitory agent is a bispecific monoclonal antibody that binds to the serine protease domain of MASP-1 (aa 449-694 of SEQ ID NO: 10) and also binds to a domain in the serine protease of MASP-3 (aa 450-711 of SEQ ID NO:8).

The binding affinity of the MASP-3 inhibitory agents can be determined using a suitable binding assay.

The inhibition of MASP-3-dependent complement activation is characterized by at least one of the following changes in a component of the complement system that occurs as a result of administration of a MASP-3 inhibitory agent in accordance with the methods of the disclosure: the inhibition of LEA-1-mediated complement activation (inhibition of hemolysis and/or opsonization); inhibition of lectin-independent conversion of factor B; inhibition of lectin-independent conversion of factor D, inhibition of MASP-3 serine protease substrate-specific cleavage, the reduction of hemolysis (measured, for example as described in Example 5) or the reduction of C3 cleavage and C3b deposition (measured, for example, as described in Example 4 and Example 11).

In some cases, the MASP-3 inhibitory agents selectively inhibit MASP-3-dependent complement activation (i.e., LEA-1-mediated complement activation and/or lectin-independent conversion of factor B and/or lectin-independent conversion of factor D), leaving the C1q-dependent complement activation system functionally intact.

In some cases, the MASP-3 inhibitory agents are antibodies, or fragments thereof, including MASP-3 antibodies and MASP-3 binding fragments thereof, MASP-1 antibodies and fragments thereof, natural and synthetic peptides, or small-molecules. In some cases, the MASP-3 inhibitory agents are small-molecule protease inhibitors that are selective for MASP-1, or selective for MASP-3, or selective for MASP-1 and MASP-3.

### ii. Compositions for inhibiting activation of LEA-2

As described herein, LEA-2-mediated complement activation is MASP-2-dependent, leading to opsonization and/or lysis. Therefore, the preferred protein component to target in the development of therapeutic agents to specifically inhibit the LEA-2 lectin-dependent complement system is MASP-2. Several proteins have been shown to bind to, or interact with MASP-2 through protein-to-protein interactions. For example, MASP-2 is known to bind to, and form calcium-dependent complexes with, the lectin proteins MBL, H-ficolin and L-ficolin and collectin-11. Ma Y., et al., J Innate Immun. Epub Dec 4 (2012). Each MASP-2/lectin complex has been shown to activate complement through the MASP-2-dependent cleavage of proteins C4 and C2 (Ikeda, K., et al., J. Biol. Chem. 262:7451-7454, (1987); Matsushita, M., et al., J. Exp. Med. 176:1497-2284, (2000); Matsushita, M., et al., J. Immunol. 168:3502-3506, (2002)). Studies have shown that the CUB1-EGF domains of MASP-2 are essential for the association of MASP-2 with MBL (Thielens, N.M., et al., J. Immunol. 166:5068, (2001)). It has also been shown that the CUB1EGFCUBII domains mediate dimerization of MASP-2, which is required for formation of an active MBL complex (Wallis, R., et al., J. Biol. Chem. 275:30962-30969, 2000). Therefore, MASP-2 inhibitory agents can be identified that bind to or interfere with MASP-2 target regions known to be important for MASP-2-dependent complement activation.

In accordance with the foregoing, the disclosure provides methods of inhibiting the adverse effects of LEA-2-mediated complement activation in a subject suffering from PNH, or at risk for developing PNH, comprising administering to the subject a pharmaceutical composition comprising an amount of a MASP-2 inhibitory agent effective to inhibit MASP-2-dependent complement activation and a pharmaceutically acceptable carrier.

MASP-2 inhibitory agents are administered in an amount effective to inhibit MASP-2-dependent LEA-2 in a living subject suffering from, or at risk for developing PNH. Representative MASP-2 inhibitory agents include: molecules that inhibit the biological activity of MASP-2 (such as small-molecule inhibitors, MASP-2 antibodies or blocking peptides that interact with MASP-2 or interfere with a protein-protein interaction), and molecules that decrease the expression of MASP-2 (such as MASP-2 antisense nucleic acid molecules, MASP-2 specific RNAi molecules and MASP-2 ribozymes), thereby preventing MASP-2 from activating LEA-2.

A MASP-2 inhibitory agent may effectively block MASP-2 protein-to-protein interactions, interfere with MASP-2 dimerization or assembly, block Ca⁺⁺ binding, interfere with the MASP-2 serine protease active site, or may reduce MASP-2 protein expression, thereby preventing MASP-2 from activating LEA-2. The MASP-2 inhibitory agents can be used alone as a primary therapy or in combination with other therapeutics as an adjuvant therapy to enhance the therapeutic benefits of other medical treatments, as further described herein.

In one case, the MASP-2 inhibitory agent specifically binds to a portion of MASP-2 (SEQ ID NO:5) with a binding affinity of at least 10 times greater than to other antigens in the complement system. In another case, the MASP-2 inhibitory agent specifically binds to a portion of MASP-2 (SEQ ID NO:5) with a binding affinity of at least 100 times greater than to other antigens in the complement system. In one case, the MASP-2 inhibitory agent specifically binds to at least one of (i) the CCP1-CCP2 domain (aa 300-431 of SEQ ID NO:5) or the serine protease domain of MASP-2 (aa 445-682 of SEQ ID NO:5) and inhibits MASP-2-dependent complement activation, with the proviso that the inhibitory agent does not bind to the serine protease domain of MASP-1 (SEQ ID NO:10), and it does not bind to the serine protease domain of MASP-3 (SEQ ID NO:8). In one case, the MASP-2 inhibitory agent is a MASP-2 monoclonal antibody, or fragment thereof that specifically binds to MASP-2.

The binding affinity of the MASP-2 inhibitory agent can be determined using a suitable binding assay.

The inhibition of MASP-2-dependent complement activation is characterized by at least one of the following changes in a component of the complement system that occurs as a result of administration of a MASP-2 inhibitory agent in accordance with the methods of the disclosure: the inhibition of the generation or production of MASP-2-dependent complement-activation-system products C4b, C3a, C5a and/or C5b-9 (MAC) (measured, for example, as described in Example 2 of US Patent No. 7,919,094), the reduction of C4 cleavage and C4b deposition (measured, for example as described in Example 8 or Example 9), or the reduction of C3 cleavage and C3b deposition (measured, for example, as described in Example 11).

In some cases, the MASP-2 inhibitory agents selectively inhibit MASP-2 complement activation (i.e., LEA-2), leaving the C1q-dependent complement activation system functionally intact.

In some cases, the MASP-2 inhibitory agents are antibodies, or fragments thereof, including MASP-2 antibodies and MASP-2 binding fragments thereof, natural and synthetic peptides, or small-molecules. In some cases, the MASP-2 inhibitory agents are small-molecule protease inhibitors that are selective for MASP-2.

### iii. Compositions for inhibiting LEA-1-mediated complement activation and LEA-2-mediated complement activation

In another aspect, the disclosure provides methods for inhibiting the adverse effects of LEA-1 and inhibiting the adverse effects of LEA-2 in a subject suffering from one or more aspects of PNH, or at risk for developing PNH.

In one case, this aspect of the disclosure is directed to a method of increasing the survival of red blood cells in a subject suffering from PNH, comprising administering to the subject a composition comprising an amount of at least one of a MASP-1 inhibitory agent and/or a MASP-3 inhibitory agent effective to increase the survival of red blood cells.

In one case, the composition comprises a MASP-1 inhibitory agent. In one case, the MASP-1 inhibitory agent inhibits MASP-3-mediated complement activation and also inhibits MASP-2-mediated complement activation.

In one case, the composition comprises a MASP-3 inhibitory agent. In one case, the MASP-3 inhibitory agent inhibits at least one of: lectin MASP-3-dependent activation of factor B; lectin MASP-3-dependent activation of factor D; MASP-3-dependent, lectin-independent activation of factor B; and/or MASP-3-dependent, lectin-independent, activation of factor D.

In one case, the composition comprises a MASP-1 inhibitory agent and a MASP-3 inhibitory agent.

In some cases, the method further comprises administering to the subject a composition comprising a MASP-2 inhibitory agent.

In another case, this aspect of the disclosure comprises administering to a subject suffering from PNH a pharmaceutical composition comprising an amount of a MASP-2 inhibitory agent effective to inhibit MASP-2-dependent complement activation and an amount of a MASP-3 inhibitory agent effective to inhibit MASP-3-dependent complement activation and a pharmaceutically acceptable carrier.

In some cases, the composition comprises a single agent that inhibits both LEA-1 and LEA-2 (i.e., a dual MASP-2/MASP-3 inhibitory agent, a dual MASP-1/MASP-2 inhibitory agent, a bispecific MASP-2/MASP-3 inhibitory agent, a bispecific MASP-1/MASP-2 inhibitory agent, or a pan-MASP-1/2/3 inhibitory agent or a trispecific MASP-1/2/3 inhibitory agent). In some cases, the composition comprises a combination of LEA-1 and LEA-2 inhibitory agents, for example, a combination of dual inhibitory agents plus a single inhibitory agent, a combination of bispecific inhibitory agents plus a single inhibitory agent, or a combination of any of the MASP-1, MASP-2 and/or MASP-3 inhibitory agents as described herein that in combination inhibit both LEA-1 and LEA-2, as further described herein.

In one case provided is a pharmaceutical composition for inhibiting both LEA-1 and LEA-2, comprising at least one MASP-3 inhibitory agent and at least one MASP-2 inhibitory agent and a pharmaceutically acceptable carrier. The pharmaceutical composition comprises a combination of a first molecule that is a MASP-3 inhibitory agent and a second molecule that is a MASP-2 inhibitory agent. In another case, the pharmaceutical composition comprises a single molecular entity that includes activity as a MASP-3 inhibitory agent and activity as a MASP-2 inhibitory agent (i.e., an inhibitory agent that inhibits both MASP-2-mediated LEA-2 activation and MASP-3-mediated LEA-1 activation). In one case, the inhibitory agent is a MASP-2/MASP-3 dual inhibitory agent that binds to an epitope within an amino acid region that is conserved between MASP-2 (SEQ ID NO:5) and MASP-3 (SEQ ID NO:8), such as the serine protease domain, for example the N-terminal region of the beta chain (e.g., the first 150 aa of the N-terminal region of the beta chain of SEQ ID NO5 and SEQ ID NO:8:), as shown in **FIGURES 4****,** **6** and **7C****.** In one case, the inhibitory agent is a bispecific inhibitory agent, such as a bispecific monoclonal antibody, that specifically binds to an epitope on the MASP-2 protein (SEQ ID NO:5) and an epitope on the MASP-3 protein (SEQ ID NO:8). In some cases, the inhibitory agent is a bispecific monoclonal antibody that binds to at least one of the CCP1-CCP2 domain of MASP-2 (aa 300-431 of SEQ ID NO:5) or the serine protease domain of MASP-2 (aa 445-682 of SEQ ID NO:5) and also binds to an epitope in the serine protease of MASP-3 (aa 450-711 of SEQ ID NO:8).

In another case, the disclosure provides a composition for inhibiting both LEA-1 and LEA-2, comprising an inhibitory agent that inhibits both MASP-2-mediated LEA-2 activation and MASP-1-mediated activation of MASP-3, thereby inhibiting MASP-3-mediated LEA-1 activation (and optionally also inhibiting the MASP-1-mediated maturation of factor D). In one case, the inhibitory agent is a MASP-1/MASP-2 dual inhibitory agent that binds to an epitope within an amino acid region that is conserved between MASP-1 (SEQ ID NO:10) and MASP-2 (SEQ ID NO:5), such as the serine protease domain, as shown in **FIGURES 4****,** **6** and **7A**. In one case, the inhibitory agent is a bispecific inhibitory agent, such as a bispecific monoclonal antibody, that specifically binds to an epitope on the MASP-1 protein (SEQ ID NO:10) and an epitope on the MASP-2 protein (SEQ ID NO:5). In some cases, the inhibitory agent is a bispecific monoclonal antibody that binds to the serine protease domain of MASP-1 (aa 449-694 of SEQ ID NO: 10) and also binds to at least one of the CCP1-CCP2 domain of MASP-2 (aa 300-431 of SEQ ID NO:5) or the serine protease domain of MASP-2 (aa 445-682 of SEQ ID NO:5).

In another case, the disclosure provides a composition for inhibiting both LEA-1 and LEA-2, comprising an inhibitory agent that inhibits MASP-2-mediated LEA-2 activation, MASP-3-mediated LEA-1 activation by directly binding to MASP-3 and also inhibits MASP-1-mediated activation of MASP-3, thereby inhibiting MASP-3-mediated LEA-1 activation (and optionally also inhibiting the MASP-1-mediated maturation of factor D). In one case, the inhibitory agent is a pan-MASP inhibitor that binds to an amino acid region that is conserved between MASP-1 (SEQ ID NO:10), MASP-2 (SEQ ID NO:5) and MASP-3 (SEQ ID NO:8), for example a conserved region in the CUBI-EGF-CUB2 domain, as shown in **FIGURES 4** and **5****.** As illustrated in **FIGURES 4** and **5****,** there are numerous patches of identity shared between MASP-1, MASP-2 and MASP-3 in the CUBI-EGF-CUBII domains, thereby allowing for the generation of pan-specific MASP antibodies. In some cases, the pan-specific MASP antibody can bind to an epitope within the CUB2 domain of MASP-1 (aa 185-296 of SEQ ID NO:10), MASP-2 (aa 184-295 of SEQ ID NO:5) and MASP-3 (aa 185-296 of SEQ ID NO:8). It is noted that a pan-specific MASP inhibitor that binds to CUBI-EGF of MASP-1, MASP-2 and MASP-3 would also bind to MAp19 and MAp44, therefore the effective therapeutic dosage of such an inhibitor would be adjusted to a higher level to compensate for this binding. It is further noted that a pan-specific MASP inhibitor that binds to the CUBII domain of MASP-1, MASP-2 and MASP-3 would also bind to MAp44, therefore the effective therapeutic dosage of such an inhibitor would be adjusted to a higher level to compensate for this binding.

In one case, the inhibitory agent is a trispecific MASP-1/2/3 inhibitor that binds to an epitope on the MASP-1 protein (SEQ ID NO:10), an epitope on the MASP-2 protein (SEQ ID NO:5) and an epitope on the MASP-3 protein (SEQ ID NO:8). In some cases, the inhibitory agent is a trispecific monoclonal antibody that binds to the serine protease domain of MASP-1 (aa 449-694 of SEQ ID NO:10), binds to at least one of the CCP1-CCP2 domain of MASP-2 (aa 300-431 of SEQ ID NO:5) or the serine protease domain of MASP-2 (aa 445-682 of SEQ ID NO:5) and also binds to an epitope in the serine protease of MASP-3 (aa 450-711 of SEQ ID NO:8).

Exemplary inhibitory agents for inhibiting LEA-1, LEA-2 or LEA-1 and LEA-2 are described below in **TABLE 2.**

**TABLE 2: MASP Inhibitory Agents**

| **Type of MASP inhibitor** | **Inhibitor Binding domain(s)** | **Cross-reactivity*** | **Assay for inhibitory activity** | **Therapeutic Utility** |
|---|---|---|---|---|
| **MASP-3 specific** | MASP-3 serine protease domain (aa 450-711 of SEQ ID NO:8) | Binds to MASP-3; not to MASP-1; MASP-2; MAp44 or MAp19 | Inhibition of MASP-3 serine protease substrate-specific cleavage; LEA-1 inhibition, assay for inhibition of factor D activation; inhibition of hemolysis of non-human RBCs by human serum | Inhibit LEA-1-mediated complement activation (inhibit lysis and opsonization) |
| **MASP-2 specific** | MASP-2 CCP1-CCP2 domain (aa 300-431 of SEQ ID NO:5); or MASP-2 serine protease domain (aa 445-682 of SEQ ID NO:5) | Binds to MASP-2; not to MASP-1; MASP-3; MAP 19 or MAp44 | Inhibition of MASP-2-specific protease substrate-specific cleavage, LEA-2 inhibition | Inhibit LEA-2-mediated complement activation (inhibit opsonization and/or lysis) |
| **MASP-1 specific** | **MASP**-**1** serine protease domain (aa449-694 of SEQ ID NO: 10) | Binds to MASP-1; not to MASP-2, MASP-3, MAp44 or MAp19 | Inhibition of MASP-1-specific protease substrate-specific cleavage; LEA-1 and LEA2 inhibition, Assay for inhibition of factor D activation; assay for restoration of AP-1 activity in factor D depleted serum supplemented with pro-factor D | Inhibit LEA-1 and LEA-2mediated complement activation (inhibit lysis and/or opsonization) |
| **MASP-2/MASP-3 dual inhibitor (one antibody binds to conserved region)** | Region of serine protease domain conserved between | Binds MASP-2 and MASP-3; not MASP-1, MAp44, or MAp19. | Assay for MASP-2- and MASP-3 protease substrate-specific cleavage, inhibition of | Inhibit LEA-1 and LEA-2-mediated complement activation |
| | MASP-2 and MASP-3, especially the N-terminal region of beta chain (first 150aa) | | LEA-1 and LEA-2 | (inhibit lysis and/or opsonization) |
| **MASP-1/3 dual inhibitor that excludes MAp44** | MASP-1/3 CCP2 domain (aa 367-432 of SEQ ID NO:10) | Binds **MASP**-**1** and MASP-3; not MAp44, MASP-2, or MAp19 | Assay for MASP-3 and **MASP**-**1** protease substrate-specific cleavage and inhibition of factor D activation, LEA-1 and LEA-2 inhibition | Inhibit LEA-1- and LEA-2-mediated complement activation (inhibit lysis and/or opsonization) |
| **MASP-1/3 dual inhibitor that includes MAp44** | MASP-1/3 CUBI-CCP1 domain (aa25-363 of SEQ ID NO: 10) | Binds MASP-1, MASP-3, and MAp44; not MASP-2 or MAp19 | Assay for MASP-3 and **MASP**-**1** protease substrate-specific cleavage and inhibition of factor D activation, LEA-1 and LEA-2 inhibition | Inhibit LEA-1- and LEA-2-mediated complement activation (inhibit lysis and/or opsonization) |
| **MASP-1/2 dual inhibitor** | Region of serine protease domain conserved between MASP-1 and MASP-2 | Binds MASP-1 and MASP-2; not MASP-3, MAp19 or MAp44 | Assay for inhibition of MASP-1 and MASP-2 serine protease substrate-specific cleavage; LEA-1 and LEA-2 inhibition | Inhibit LEA-1-and LEA-2-mediated complement activation (inhibit lysis and/or opsonization) |
| **MASP-1/2/3 pan inhibitor** | Conserved region of CUB1-EGF-CUB2, especially CUB2 domain (common interaction site) | In addition to MASP-1/2/3 would bind to MAp44, and possibly Map 19 | Assay for MASP-1-, MASP-2- and MASP-3-specific protease substrate-specific cleavage and inhibition of factor D activation; inhibition of LEA-1 and LEA-2 | Inhibit LEA-1-and LEA-2-mediated complement activation (inhibit lysis and/or opsonization) |
| MASP-2/MASP-3 **bispecific** inhibitor | MASP-2-specific binding to CCP1-CCP2 (aa 300-431 of SEQ ID NO:5); or MASP-2 serine protease domain (aa 445-682 of SEQ ID NO:5) and MASP-3-specific binding to serine protease domain (aa 450-711 of SEQ ID NO:8) | Binds MASP-2 and MASP-3; not MASP-1, MAp44 or MAp19 | Assay for MASP-2- and MASP-3-specific protease substrate-specific cleavage, inhibition of LEA-1 and LEA-2 | Inhibit LEA-1-and LEA-2-mediated complement activation (inhibit lysis and/or opsonization) |
| **MASP**-**1**/**MASP**-**2 bispecific inhibitor** | **MASP**-**1** serine protease domain (aa449-694 of SEQ ID NO:10), and MASP-2-specific binding to CCP1-CCP2 (aa 300-431 of SEQ ID NO:5); or MASP-2 serine protease domain (aa 445-682 of SEQ ID NO:5) | Binds to MASP-1 and to MASP-2; not MASP-3, MAp19 or MAp44 | Assay for inhibition of MASP-1- and MASP-2-specific serine protease substrate-specific cleavage; LEA-1 and LEA-2 inhibition | Inhibit LEA-1-and LEA-2-mediated complement activation (inhibit lysis and/or opsonization) |
| **MASP**-**1**/**MASP**-**3 bispecific** | MASP-1 serine protease domain (aa449-694 of SEQ ID NO:10) and MASP-3-specific | Binds to MASP-1 and MASP-3; not to MASP-2, MAp44 or MAp19 | Assay for MASP-1 and MASP-3-protease substrate-specificcleavage and inhibition of factor D activation, LEA-1 | Inhibit LEA-1-and LEA-2-mediated complement activation (inhibit lysis and/or opsonization) |
| | binding to serine protease domain (aa 450-711 of SEQ ID NO:8) | | and LEA-2 inhibition | |
| **MASP**-**1**/**MASP-2/MASP-3 trispecific** | **MASP**-**1** serine protease domain, MASP-2 serine protease domain or CCP-CCP2 domain and MASP-3 serine protease domain | Binds to MASP-1 and MASP-2 and MASP-3; not MAp19 or MAp44 | Assay for MASP-1-, MASP-2- and MASP-3-protease substrate-specific cleavage and inhibition of factor D activation, inhibition of LEA-1 and LEA-2 | Inhibit LEA-1- and LEA-2-mediated complement activation (inhibit lysis and/or opsonization) |

| | | | | |
|---|---|---|---|---|
| *With regard to cross-reactivity column as set forth in **TABLE 2,** the designated MASP inhibitor binds to the inhibitor binding domain with a binding affinity of at least 10 times greater (e.g., at least 20 times, at least 50 times or at least 100 times greater) than to the other complement components (*i.e.*, polypeptides or fragments thereof) listed as "not" binding. | | | | |

In some cases, the composition comprises a combination of LEA-1 and LEA-2 inhibitory agents, for example, a combination of single inhibitory agents as described above and shown in **TABLE 2.** For example, the composition comprises a combination of a MASP-1 antibody and a MASP-2 antibody. In one case, the composition comprises a combination of a MASP-1 antibody and a MASP-3 antibody. In one case, the composition comprises a combination of a MASP-2 antibody and a MASP-3 antibody. In one case, the composition comprises a combination of a MASP-1, and MASP-2 and a MASP-3 antibody. In some cases, the methods of the disclosure comprise administration of a single composition comprising a combination of inhibitory agents. In other cases, the methods of the disclosure comprise co-administering separate compositions.

In some cases, the compositions comprise a combination of a dual inhibitory agent plus a single inhibitory agent (i.e., a MASP-2/3 dual inhibitor plus a MASP-1 inhibitor; a MASP-1/3 dual inhibitor plus a MASP-2 inhibitor; or a MASP-1/2 dual inhibitor plus a MASP-3 inhibitor). The methods of the disclosure comprise co-administering separate compositions comprising a dual inhibitor and a single inhibitor.

In some cases, the compositions comprise a combination of a bispecific inhibitory agent plus a single inhibitory agent (i.e., a MASP-2/3 bispecific inhibitor plus a MASP-1 inhibitor; a MASP-1/3 bispecific inhibitor plus a MASP-2 inhibitor; or a MASP-1/2 bispecific inhibitor plus a MASP-3 inhibitor). The methods of the disclosure comprise co-administering separate compositions comprising a bispecific inhibitor and a single inhibitor.

It is noted that MASP-3 inhibitory agents and/or MASP-2 inhibitory agents and/or MASP-1 inhibitory agents would be used to clear the target protein from the plasma as compared to a C5 antibody which must localize to the site of action.

### V. MASP ANTIBODIES

The MASP inhibitory agent comprises a MASP antibody (e.g., a MASP-1, MASP-2 or MASP-3 antibody) that inhibits at least one of the LEA-1 and/or LEA-2 complement activation pathways. The MASP antibodies useful in this aspect of the disclosure include polyclonal, monoclonal or recombinant antibodies derived from any antibody producing mammal and may be multispecific (i.e., bispecific or trispecific), chimeric, humanized, fully human, anti-idiotype, and antibody fragments. Antibody fragments include Fab, Fab', F(ab)₂, F(ab')₂, Fv fragments, scFv fragments and single-chain antibodies as further described herein.

MASP antibodies can be screened for the ability to inhibit the LEA-1 or LEA-2-dependent complement activation system using the assays described herein. Several MASP-1, MASP-2 and MASP-3 antibodies have been described in the literature and some have been newly generated, some of which are listed below in **TABLE 3.** These exemplary MASP antibodies can be screened for the ability to inhibit the LEA-1- and/or LEA-2-dependent complement activation system using the assays described herein. For example, as described in Examples 11-13 herein, anti-rat MASP-2 Fab2 antibodies have been identified that block MASP-2-dependent complement activation. As further described in Example 14, fully human MASP-2 scFv antibodies have been identified that block MASP-2-dependent complement activation. As further described in Example 15, MASP-3 antibodies have been generated. Once a MASP antibody is identified that functions as an inhibitor of LEA-1 or LEA-2, it can be used in a pharmaceutical composition as descrbed herein, and it can also be used to generate bispecific and trispecific inhibitory agents, as set forth in **TABLE 2** and further described herein (see e.g., Example 8).

**TABLE 3: MASP-1, MASP-2 and MASP-3 SPECIFIC ANTIBODIES**

| TARGET | ANTIGEN | ANTIBODY TYPE | REFERENCE |
|---|---|---|---|
| MASP-2 | Recombinant MASP-2 | Rat Polyclonal | Peterson, S.V., et al., Mol. Immunol. 37:803-811,2000 |
| MASP-2 | Recombinant human CCP1/2-SP fragment (MoAb 8B5) | Rat MoAb (subclass IgG1) | Moller-Kristensen, M., et al., J. of Immunol. Methods 282:159-167, 2003 |
| MASP-2 | Recombinant human MAp 19 (MoAb 6G12) (cross-reacts with MASP-2) | Rat MoAb (subclass IgG1) | Moller-Kristensen, M., et al., J. of Immunol. Methods 282:159-167, 2003 |
| MASP-2 | hMASP-2 | Mouse MoAb (S/P) Mouse MoAb (N-term) | Peterson, S.V., et al., *Mol. Immunol.* 35:409, April 1998 |
| MASP-2 | hMASP-2 (CCP1-CCP2-SP domain | rat MoAb: Nimoab101, produced by hybridoma cell line 03050904 (ECACC) | WO 2004/106384 |
| MASP-2 | hMASP-2 (full-length histagged) | murine MoAbs: | WO 2004/106384 |
| | | NimoAb104, produced by hybridoma cell line M0545YM035 (DSMZ) | |
| | | NimoAb108, produced by hybridoma cell line M0545YM029 (DSMZ) | |
| | | NimoAb109 produced by hybridoma cell line M0545YM046 (DSMZ) | |
| | | NimoAb110 produced by hybridoma cell line M0545YM048 (DSMZ) | |
| MASP-2 | Rat MASP-2 (full-length) | MASP-2 Fab2 antibody fragments | Examples 11-12 |
| MASP-2 | hMASP-2 (full-length) | Fully human scFv clones | Example 14 |
| MASP-1 | hMASP-1 (full-length) | Mouse MoAbs: | Terai I. et al., Clin Exp Immunol 110:317-323 (1997); MoAb1E2: Commercially available from Hycult Biotech Cat#HM2092 MoAb2B 11: commercially available from Hycult Biotech: Cat#HM2093 |
| | | MoaAbs1E2 and 2B11 produced by hybridoma line 1 E2 and 2B 11 (do not cross-react with MASP-2). Both abs recognize the heavy chain common to both MASP-1 and MASP-3 | |
| MASP-1 | hMASP-1 (full-length) | Mouse MoAb 4C2 | Endo M. et al., Nephrol Dial Transplant 13:1984-1990 (1998) |
| MASP-1 | hMASP-1 (full-length) | MASP-1 chicken abs | Example 15 |
| MASP-3 | hMASP-3 (full-length) | Mouse MoAbs: | Skjoedt et al., Immunobiology 215(11):921-31 (2010) |
| | | MoAb-7D8;MoAb-7B7;MoAb-8B3; and MoAb-5H3 MoAb-7D8 and mAb-5H3 are MASP-3-specific, others cross-react with MASP-1 | |
| MASP-3 | hMASP-3 (full-length) | Rat MoAb 38:12-3, Does not recognize MASP-1 | Moller-Kristensen et al., Int Immunol 19:141 (2007); Commercially available from Hycult Biotech: Cat #HM2216 |
| MASP-3 | hMASP-3 (full-length) | MASP-3 chicken abs | Example 15 |

### i. MASP antibodies with reduced effector function

In some cases, the MASP antibodies described herein have reduced effector function in order to reduce inflammation that may arise from the activation of the classical complement pathway. The ability of IgG molecules to trigger the classical complement pathway has been shown to reside within the Fc portion of the molecule (Duncan, A.R., et al., Nature 332:738-740 (1988)). IgG molecules in which the Fc portion of the molecule has been removed by enzymatic cleavage are devoid of this effector function (see Harlow, Antibodies: A Laboratory Manual, Cold Spring Harbor Laboratory, New York, 1988). Accordingly, antibodies with reduced effector function can be generated as the result of lacking the Fc portion of the molecule by having a genetically engineered Fc sequence that minimizes effector function, or being of either the human IgG₂ or IgG₄ isotype.

Antibodies with reduced effector function can be produced by standard molecular biological manipulation of the Fc portion of the IgG heavy chains as described in Jolliffe et al., Int'l Rev. Immunol. 10:241-250, (1993), and Rodrigues et al., J. Immunol. 151:6954-6961, (1998). Antibodies with reduced effector function also include human IgG2 and IgG4 isotypes that have a reduced ability to activate complement and/or interact with Fc receptors (Ravetch, J.V., et al., Annu. Rev. Immunol. 9:457-492, (1991); Isaacs, J.D., et al., J. Immunol. 148:3062-3071, 1992; van de Winkel, J.G., et al., Immunol. Today 14:215-221, (1993)). Humanized or fully human antibodies specific to human MASP-1, MASP-2 or MASP-3 (including dual, pan, bispecific or trispecific antibodies) comprised of IgG2 or IgG4 isotypes can be produced by one of several methods known to one of ordinary skilled in the art, as described in Vaughan, T.J., et al., Nature Biotechnical 16:535-539, (1998).

### ii. Production of MASP antibodies

MASP-1, MASP-2 or MASP-3 antibodies can be produced using MASP-1, MASP-2 or MASP-3 polypeptides (e.g., full-length MASP-1, MASP-1 or MASP-3) or using antigenic MASP-1, 2 or 3 epitope-bearing peptides (e.g., a portion of the MASP-2 polypeptide). Immunogenic peptides may be as small as five amino acid residues. For example, the MASP-2 polypeptide including the entire amino acid sequence of SEQ ID NO:5 may be used to induce MASP-2 antibodies useful in the method of the disclosure. Particular MASP domains known to be involved in protein-protein interactions, such as the CUBI, and CUBI-EGF domains, as well as the region encompassing the serine-protease active site, for example, as set forth in **TABLE 2,** may be expressed as recombinant polypeptides using methods well known in the art and used as antigens. In addition, peptides comprising a portion of at least 6 amino acids of the MASP-1 polypeptide (SEQ ID NO:10), or of the MASP-2 polypeptide (SEQ ID NO:5) or of the MASP-3 polypeptide (SEQ ID NO:8) are also useful to induce MASP-1, MASP-2 or MASP-3 antibodies, respectively. The MASP peptides and polypeptides used to raise antibodies may be isolated as natural polypeptides, or recombinant or synthetic peptides and catalytically inactive recombinant polypeptides. Antigens useful for producing MASP antibodies also include fusion polypeptides, such as fusions of a MASP polypeptide or a portion thereof with an immunoglobulin polypeptide or with maltose-binding protein. The polypeptide immunogen may be a full-length molecule or a portion thereof. If the polypeptide portion is hapten-like, such portion may be advantageously joined or linked to a macromolecular carrier (such as keyhole limpet hemocyanin (KLH), bovine serum albumin (BSA) or tetanus toxoid) for immunization.

### iii. Polyclonal antibodies

Polyclonal antibodies against MASP-1, MASP-2 or MASP-3 can be prepared by immunizing an animal with MASP-1, MASP-2 or MASP-3 polypeptide or an immunogenic portion thereof using methods well known to those of ordinary skill in the art. See, for example, Green et al., "Production of Polyclonal Antisera," in Immunochemical Protocols (Manson, ed.). The immunogenicity of a MASP polypeptide can be increased through the use of an adjuvant, including mineral gels, such as aluminum hydroxide or Freund's adjuvant (complete or incomplete), surface active substances such as lysolecithin, pluronic polyols, polyanions, oil emulsions, KLH and dinitrophenol. Polyclonal antibodies are typically raised in animals such as horses, cows, dogs, chicken, rats, mice, rabbits, guinea pigs, goats, or sheep. Alternatively, a MASP antibody useful in the present invention may also be derived from a subhuman primate. General techniques for raising diagnostically and therapeutically useful antibodies in baboons may be found, for example, in Goldenberg et al., International Patent Publication No. WO 91/11465, and in Losman, M.J., et al., Int. J. Cancer 46:310, (1990). Sera containing immunologically active antibodies are then produced from the blood of such immunized animals using standard procedures well known in the art.

### iv. Monoclonal antibodies

In some cases, the LEA-2 inhibitory agent is a MASP-2 monoclonal antibody and/or the LEA-1 inhibitory agent is a MASP-3 monoclonal antibody or a MASP-1 monoclonal antibody. As described above, in some cases, MASP-1, MASP-2 and MASP-3 monoclonal antibodies are highly specific, being directed against a single MASP-1, MASP-2 or MASP-3 epitope. As used herein, the modifier "monoclonal" indicates the character of the antibody as being obtained from a substantially homogenous population of antibodies, and is not to be construed as requiring production of the antibody by any particular method. Monoclonal antibodies can be obtained using any technique that provides for the production of antibody molecules by continuous cell lines in culture, such as the hybridoma method described by Kohler, G., et al., Nature 256:495, (1975), or they may be made by recombinant DNA methods (see, e.g., U.S. Patent No. 4,816,567 to Cabilly). Monoclonal antibodies may also be isolated from phage antibody libraries using the techniques described in Clackson, T., et al., Nature 352:624-628, (1991), and Marks, J.D., et al., J. Mol. Biol. 222:581-597, (1991). Such antibodies can be of any immunoglobulin class including IgG, IgM, IgE, IgA, IgD and any subclass thereof.

For example, monoclonal antibodies can be obtained by injecting a suitable mammal (e.g., a BALB/c mouse) with a composition comprising a MASP-1 polypeptide, a MASP-2 polypeptide or a MASP-3 polypeptide, or portion thereof. After a predetermined period of time, splenocytes are removed from the mouse and suspended in a cell culture medium. The splenocytes are then fused with an immortal cell line to form a hybridoma. The formed hybridomas are grown in cell culture and screened for their ability to produce a monoclonal antibody against MASP-1, MASP-2 or MASP-3. (See also Current Protocols in Immunology, Vol. 1., John Wiley & Sons, pages 2.5.1-2.6.7, 1991.)

Human monoclonal antibodies may be obtained through the use of transgenic mice that have been engineered to produce specific human antibodies in response to antigenic challenge. In this technique, elements of the human immunoglobulin heavy and light chain locus are introduced into strains of mice derived from embryonic stem cell lines that contain targeted disruptions of the endogenous immunoglobulin heavy chain and light chain loci. The transgenic mice can synthesize human antibodies specific for human antigens, such as the MASP-2 antigens described herein, and the mice can be used to produce human MASP-2 antibody-secreting hybridomas by fusing B-cells from such animals to suitable myeloma cell lines using conventional Kohler-Milstein technology. Methods for obtaining human antibodies from transgenic mice are described, for example, by Green, L.L., et al., Nature Genet. 7:13, 1994; Lonberg, N., et al., Nature 368:856, 1994; and Taylor, L.D., et al., Int. Immun. 6:579, 1994.

Monoclonal antibodies can be isolated and purified from hybridoma cultures by a variety of well-established techniques. Such isolation techniques include affinity chromatography with Protein-A Sepharose, size-exclusion chromatography, and ion-exchange chromatography (see, for example, Coligan at pages 2.7.1-2.7.12 and pages 2.9.1-2.9.3; Baines et al., "Purification of Immunoglobulin G (IgG)," in Methods in Molecular Biology, The Humana Press, Inc., Vol. 10, pages 79-104, 1992).

Once produced, polyclonal, monoclonal or phage-derived antibodies are first tested for specific MASP-1, MASP-2 or MASP-3 binding or, where desired, dual MASP-1/3, MASP-2/3 or MASP-1/2 binding. Methods for determining whether an antibody binds to a protein antigen and/or the affinity for an antibody to a protein antigen are known in the art. For example, the binding of an antibody to a protein antigen can be detected and/or quantified using a variety of techniques such as, but not limited to, Western blot, dot blot, plasmon surface resonance method (e.g., BIAcore system; Pharmacia Biosensor AB, Uppsala, Sweden and Piscataway, NJ), or enzyme-linked immunosorbent assays (ELISA). See, e.g., Harlow and Lane (1988) "Antibodies: A Laboratory Manual" Cold Spring Harbor Laboratory Press, Cold Spring Harbor, N. Y.; Benny K. C. Lo (2004) "Antibody Engineering: Methods and Protocols," Humana Press (ISBN: 1588290921); Borrebaek (1992) "Antibody Engineering, A Practical Guide," W.H. Freeman and Co., NY; Borrebaek (1995) "Antibody Engineering," 2nd Edition, Oxford University Press, NY, Oxford; Johne et al. (1993), Immunol. Meth. 160:191-198; Jonsson et al. (1993) Ann. Biol. Clin. 51: 19-26; and Jonsson et al. (1991) Biotechniques 11:620-627. See also, U.S. Patent No. 6,355,245.

The affinity of MASP monoclonal antibodies can be readily determined by one of ordinary skill in the art (see, e.g., Scatchard, A., NY Acad. Sci. 51:660-672, 1949). The MASP-1, MASP-2 or MASP-3 monoclonal antibodies useful for the methods of the disclosure bind to MASP-1, MASP-2, or MASP-3 with a binding affinity of <100 nM, preferably <10 nM and most preferably <2 nM.

Once antibodies are identified that specifically bind to MASP-1, MASP-2 or MASP-3, the MASP-1, MASP-2 or MASP-3 antibodies are tested for the ability to function as a LEA-1 inhibitory agent or a LEA-2 inhibitory agent in one of several functional assays, for example as described in **TABLE 2.** For example, antibodies identified that specifically bind to MASP-2 are tested for the ability to function as a LEA-2 inhibitory agent in one of several assays, such as, for example, as described in **TABLE 2** (e.g., a lectin-specific C4 cleavage assay (such as the assay described in Example 8 or Example 9), or a C3b deposition assay (such as the assay described in Example 4 or Example 11)). As a further example, antibodies identified that specifically bind to MASP-1 or MASP-3 are tested for the ability to function as a LEA-1 inhibitory agent in one of several assays, such as, for example, as described in **TABLE 2** (e.g., the reduction of hemolysis, measured, for example as described in Example 5, or the reduction of C3 cleavage and C3b deposition, measured, for example, as described in Example 4 and Example 11).

### v. Chimeric/humanized antibodies

Monoclonal antibodies useful in the method of the disclosure include chimeric antibodies in which a portion of the heavy and/or light chain is identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass, as well as fragments of such antibodies (U.S. Patent No. 4,816,567, to Cabilly; and Morrison, S.L., et al., Proc. Nat'l Acad. Sci. USA 81:6851-6855, (1984)).

One form of a chimeric antibody useful in the disclosure is a humanized monoclonal MASP-1, MASP-2 or MASP-3 antibody. Humanized forms of non-human (e.g., murine) antibodies are chimeric antibodies, which contain minimal sequence derived from non-human immunoglobulin. Humanized monoclonal antibodies are produced by transferring the non-human (e.g., mouse) complementarity determining regions (CDR), from the heavy and light variable chains of the mouse immunoglobulin into a human variable domain. Typically, residues of human antibodies are then substituted in the framework regions of the non-human counterparts. Furthermore, humanized antibodies may comprise residues that are not found in the recipient antibody or in the donor antibody. These modifications are made to further refine antibody performance. In general, the humanized antibody will comprise substantially all of at least one, and typically two, variable domains in which all or substantially all of the hypervariable loops correspond to those of a non-human immunoglobulin and all or substantially all of the Fv framework regions are those of a human immunoglobulin sequence. The humanized antibody optionally also will comprise at least a portion of an immunoglobulin constant region (Fc), typically that of a human immunoglobulin. For further details, see Jones, P.T., et al., Nature 321:522-525, (1986); Reichmann, L., et al., Nature 332:323-329, (1988); and Presta, Curr. Op. Struct. Biol. 2:593-596, (1992).

The humanized antibodies useful in the disclosure include human monoclonal antibodies including at least a MASP-1, MASP-2, or MASP-3 binding CDR3 region. In addition, the Fc portions may be replaced so as to produce IgA or IgM as well as human IgG antibodies. Such humanized antibodies will have particular clinical utility because they will specifically recognize human MASP-1, MASP-2 or MASP-3 but will not evoke an immune response in humans against the antibody itself. Consequently, they are better suited for *in vivo* administration in humans, especially when repeated or long-term administration is necessary.

Techniques for producing humanized monoclonal antibodies are also described, for example, by Jones, P.T., et al., Mature 321:522, (1986); Carter, P., et al., Proc. Nat'l. Acad. Sci. USA 89:4285, (1992); Sandhu, J.S., Crit. Rev. Biotech. 12:437, (1992); Singer, I.I., et al., J. Immun. 150:2844, (1993); Sudhir (ed.), Antibody Engineering Protocols, Humana Press, Inc., (1995); Kelley, "Engineering Therapeutic Antibodies," in Protein Engineering: Principles and Practice, Cleland et al. (eds.), John Wiley & Sons, Inc., pages 399-434, (1996); and by U.S. Patent No. 5,693,762, to Queen, 1997. In addition, there are commercial entities that will synthesize humanized antibodies from specific murine antibody regions, such as Protein Design Labs (Mountain View, CA).

### vi. Recombinant antibodies

MASP-1, MASP-2 or MASP-3 antibodies can also be made using recombinant methods. For example, human antibodies can be made using human immunoglobulin expression libraries (available for example, from Stratagene, Corp., La Jolla, CA) to produce fragments of human antibodies (V_{H}, V_{L}, Fv, Factor D, Fab or F(ab')₂). These fragments are then used to construct whole human antibodies using techniques similar to those for producing chimeric antibodies.

### vii. Anti-idiotype antibodies

Once MASP-1, MASP-2 or MASP-3 antibodies are identified with the desired inhibitory activity, these_antibodies can be used to generate anti-idiotype antibodies that resemble a portion of MASP-1, MASP-2 or MASP-3 using techniques that are well known in the art. See, e.g., Greenspan, N.S., et al., FASEB J. 7:437, (1993). For example, antibodies that bind to MASP-2 and competitively inhibit a MASP-2 protein interaction required for complement activation can be used to generate anti-idiotypes that resemble the MBL binding site on MASP-2 protein and therefore bind and neutralize a binding ligand of MASP-2 such as, for example, MBL.

### viii. Immunoglobulin fragments

The MASP-2 and MASP-3 inhibitory agents useful in the method of the disclosure encompass not only intact immunoglobulin molecules but also the well known fragments including Fab, Fab', F(ab)₂, F(ab')₂ and Fv fragments, scFv fragments, diabodies, linear antibodies, single-chain antibody molecules and multispecific (e.g., bispecific and trispecific) antibodies formed from antibody fragments.

It is well known in the art that only a small portion of an antibody molecule, the paratope, is involved in the binding of the antibody to its epitope (see, e.g., Clark, W.R., The Experimental Foundations of Modern Immunology, Wiley & Sons, Inc., NY, 1986). The pFc' and Fc regions of the antibody are effectors of the classical complement pathway but are not involved in antigen binding. An antibody from which the pFc' region has been enzymatically cleaved, or which has been produced without the pFc' region, is designated an F(ab')₂ fragment and retains both of the antigen binding sites of an intact antibody. An isolated F(ab')₂ fragment is referred to as a bivalent monoclonal fragment because of its two antigen binding sites. Similarly, an antibody from which the Fc region has been enzymatically cleaved, or which has been produced without the Fc region, is designated a Fab fragment, and retains one of the antigen binding sites of an intact antibody molecule.

Antibody fragments can be obtained by proteolytic hydrolysis, such as by pepsin or papain digestion of whole antibodies by conventional methods. For example, antibody fragments can be produced by enzymatic cleavage of antibodies with pepsin to provide a 5S fragment denoted F(ab')₂. This fragment can be further cleaved using a thiol reducing agent to produce 3.5S Fab' monovalent fragments. Optionally, the cleavage reaction can be performed using a blocking group for the sulfhydryl groups that result from cleavage of disulfide linkages. As an alternative, an enzymatic cleavage using pepsin produces two monovalent Fab fragments and an Fc fragment directly. These methods are described, for example, U.S. Patent No. 4,331,647 to Goldenberg; Nisonoff, A., et al., Arch. Biochem. Biophys. 89:230, (1960); Porter, R.R., Biochem. J. 73:119, (1959); Edelman, et al., in Methods in Enzymology 1:422, Academic Press, (1967); and by Coligan at pages 2.8.1-2.8.10 and 2.10.-2.10.4.

In some cases, the use of antibody fragments lacking the Fc region are preferred to avoid activation of the classical complement pathway which is initiated upon binding Fc to the Fcγ receptor. There are several methods by which one can produce a monoclonal antibody that avoids Fcγ receptor interactions. For example, the Fc region of a monoclonal antibody can be removed chemically using partial digestion by proteolytic enzymes (such as ficin digestion), thereby generating, for example, antigen-binding antibody fragments such as Fab or F(ab)₂ fragments (Mariani, M., et al., Mol. Immunol. 28:69-71, (1991)). Alternatively, the human γ4 IgG isotype, which does not bind Fcγ receptors, can be used during construction of a humanized antibody as described herein. Antibodies, single chain antibodies and antigen-binding domains that lack the Fc domain can also be engineered using recombinant techniques described herein.

### ix. Single-chain antibody fragments

Alternatively, one can create single peptide chain binding molecules specific for MASP-1, MASP-2 or MASP-3 in which the heavy and light chain Fv regions are connected. The Fv fragments may be connected by a peptide linker to form a single-chain antigen binding protein (scFv). These single-chain antigen binding proteins are prepared by constructing a structural gene comprising DNA sequences encoding the V_{H} and V_{L} domains which are connected by an oligonucleotide. The structural gene is inserted into an expression vector, which is subsequently introduced into a host cell, such as *E. coli.* The recombinant host cells synthesize a single polypeptide chain with a linker peptide bridging the two V domains. Methods for producing scFvs are described for example, by Whitlow, et al., "Methods: A Companion to Methods in Enzymology" 2:97, (1991); Bird, et al., Science 242:423, (1988); U.S. Patent No. 4,946,778, to Ladner; Pack, P., et al., Bio/Technology 11:1271, (1993).

As an illustrative example, a MASP-3-specific scFv can be obtained by exposing lymphocytes to MASP-3 polypeptide *in vitro* and selecting antibody display libraries in phage or similar vectors (for example, through the use of immobilized or labeled MASP-3 protein or peptide). Genes encoding polypeptides having potential MASP-3 polypeptide binding domains can be obtained by screening random peptide libraries displayed on phage or on bacteria such as *E. coli.* These random peptide display libraries can be used to screen for peptides which interact with MASP-3. Techniques for creating and screening such random peptide display libraries are well known in the art (U.S. Patent No. 5,223,409, to Lardner; U.S. Patent No. 4,946,778, to Ladner; U.S. Patent No. 5,403,484, to Lardner; U.S. Patent No. 5,571,698, to Lardner; and Kay et al., Phage Display of Peptides and Proteins Academic Press, Inc., 1996) and random peptide display libraries and kits for screening such libraries are available commercially, for instance from CLONTECH Laboratories, Inc. (Palo Alto, Calif.), Invitrogen Inc. (San Diego, Calif.), New England Biolabs, Inc. (Beverly, Mass.), and Pharmacia LKB Biotechnology Inc. (Piscataway, N.J.).

Another form of a MASP-3 antibody fragment useful in this aspect of the disclosure is a peptide coding for a single complementarity-determining region (CDR) that binds to an epitope on a MASP-3 antigen and inhibits MASP-3-dependent complement activation (i.e., LEA-1). Another form of a MASP-1 antibody fragment useful in this aspect of the disclosure is a peptide coding for a single complementarity-determining region (CDR) that binds to an epitope on a MASP-1 antigen and inhibits MASP-3-dependent complement activation (i.e., LEA-1). Another form of a MASP-2 antibody fragment useful in this aspect of the disclosure is a peptide coding for a single complementarity-determining region (CDR) that binds to an epitope on a MASP-2 antigen and inhibits MASP-2-dependent complement activation (i.e., LEA-2).

CDR peptides ("minimal recognition units") can be obtained by constructing genes encoding the CDR of an antibody of interest. Such genes are prepared, for example, by using the polymerase chain reaction to synthesize the variable region from RNA of antibody-producing cells (see, for example, Larrick et al., Methods: A Companion to Methods in Enzymology 2:106, (1991); Courtenay-Luck, "Genetic Manipulation of Monoclonal Antibodies," in Monoclonal Antibodies: Production, Engineering and Clinical Application, Ritter et al. (eds.), page 166, Cambridge University Press, (1995); and Ward et al., "Genetic Manipulation and Expression of Antibodies," in Monoclonal Antibodies: Principles and Applications, Birch et al. (eds.), page 137, Wiley-Liss, Inc., 1995).

The MASP antibodies described herein are administered to a subject in need thereof to inhibit LEA-1, LEA-2 or a combination of LEA-1 and LEA-2 complement activation. In some cases, the MASP inhibitory agent is a high-affinity human or humanized monoclonal MASP-1, MASP-2 or MASP-3 antibody with reduced effector function.

### x. Bispecific antibodies

The MASP-2 and MASP-3 inhibitory agents useful in the method of the disclosure encompass multispecific (i.e., bispecific and trispecific) antibodies. Bispecific antibodies are monoclonal, preferably human or humanized, antibodies that have binding specificities for at least two different antigens. As described above and shown in **TABLE 2,** in one case, the method comprises the use of a bispecific antibody comprising a binding specificity for MASP-2 (e.g., binding to at least one of CCP1-CCP2 or serine protease domain of MASP-2) and a binding specificity for MASP-3 (e.g., binding to the serine protease domain of MASP-3). In another case, the method comprises the use of a bispecific antibody comprising a binding specificity for MASP-1 (e.g., binding to the serine protease domain of MASP-1) and a binding specificity for MASP-2 (e.g., binding to at least one of CCP1-CCP2 or serine protease domain of MASP-2). In another case, the method comprises the use of a bispecific antibody comprising a binding specificity for MASP-1 (e.g., binding to the serine protease domain of MASP-1) and a binding specificity for MASP-3 (e.g., binding to the serine protease domain of MASP-3). In another case, the method comprises the use of a trispecific antibody comprising a binding specificity for MASP-1 (e.g., binding to the serine protease domain of MASP-1), a binding specificity for MASP-2 (e.g., binding to at least one of CCP1-CCP2 or serine protease domain of MASP-2) and a binding specificity for MASP-3 (e.g., binding to the serine protease domain of MASP-3).

Methods for making bispecific antibodies are within the purview of those skilled in the art. Traditionally, the recombinant production of bispecific antibodies is based on the co-expression of two immunoglobulin heavy-chain/light-chain pairs, where the two heavy chains have different specificities (Milstein and Cuello, Nature 305:537-539 (1983)). Antibody variable domains with the desired binding specificities (antibody-antigen combining sites) can be fused to immunoglobulin constant domain sequences. The fusion preferably is with an immunoglobulin heavy-chain constant domain, including at least part of the hinge, C_{H}2, and C_{H}3 regions. DNAs encoding the immunoglobulin heavy-chain fusions and, if desired, the immunoglobulin light chain, are inserted into separate expression vectors, and are co-transfected into a suitable host organism. For further details of illustrative currently known methods for generating bispecific antibodies see, e.g., Suresh et al., Methods in Enzymology 121:210 (1986); WO96/27011; Brennan et al., Science 229:81 (1985); Shalaby et al., J. Exp. Med. 175:217-225 (1992); Kostelny et al., J. Immunol. 148(5):1547-1553 (1992); Hollinger et al. Proc. Natl. Acad. Sci USA 90:6444-6448 (1993); Gruber et al., J. Immunol. 152:5368 (1994); and Tutt et al., J. Immunol. 147:60 (1991). Bispecific antibodies also include cross-linked or heteroconjugate antibodies. Heteroconjugate antibodies may be made using any convenient cross -linking methods. Suitable crosslinking agents are well known in the art, and are disclosed in U.S. Pat. No. 4,676,980, along with a number of cross-linking techniques.

Various techniques for making and isolating bispecific antibody fragments directly from recombinant cell culture have also been described. For example, bispecific antibodies have been produced using leucine zippers. (See, e.g., Kostelny et al. J.Immunol. 148(5):1547-1553 (1992)). The "diabody" technology described by Hollinger et al. Proc. Natl. Acad. Sci USA 90:6444-6448 (1993), has provided an alternative mechanism for making bispecific antibody fragments. The fragments comprise a heavy-chain variable domain (VH) connected to a light-chain variable domain (VL) by a linker which is too short to allow pairing between the two domains on the same chain. Accordingly, the VH and VL domains of one fragment are forced to pair with the complementary VL and VH domains of another fragment, thereby forming two antigen-binding sites. Bispecific diabodies, as opposed to bispecific whole antibodies, may also be particularly useful because they can be readily constructed and expressed in E. coli. Diabodies (and many other polypeptides such as antibody fragments) of appropriate binding specificities can be readily selected using phage display (WO94/13804) from libraries. If one arm of the diabody is to be kept constant, for instance, with a specificity directed against antigen X, then a library can be made where the other arm is varied and an antibody of appropriate specificity selected.

Another strategy for making bispecific antibody fragments by the use of single-chain Fv (scFv) dimers has also been reported. (See, e.g., Gruber et al. J. Immunol., 152:5368 (1994)). Alternatively, the antibodies can be "linear antibodies" as described in, e.g., Zapata et al., Protein Eng. 8(10):1057-1062 (1995). Briefly described, these antibodies comprise a pair of tandem Factor D segments (V_{H}-C_{H}I-V_{H}-C_{H}I) which form a pair of antigen binding regions. Linear antibodies can be bispecific or monospecific. The methods of the disclosure also embrace the use of variant forms of bispecific antibodies such as the tetravalent dual variable domain immunoglobulin (DVD-Ig) molecules described in Wu et al., Nat Biotechnol 25:1290-1297 (2007). The DVD-Ig molecules are designed such that two different light chain variable domains (VL) from two different parent antibodies are linked in tandem directly or via a short linker by recombinant DNA techniques, followed by the light chain constant domain. Methods for generating DVD-Ig molecules from two parent antibodies are further described in, e.g., WO08/024188 and WO07/024715.

### VI. NON-PEPTIDE INHIBITORS

In some cases, the MASP-3 or MASP-2 inhibitory agent is a MASP-3 or a MASP-2 or a MASP-1 inhibitory peptide or a non-peptide inhibitor of MASP-3, or of MASP-2 or of MASP-1. Non-peptide MASP inhibitory agents may be administered to the subject systemically, such as by intra-arterial, intravenous, intramuscular, subcutaneous or other parenteral administration, or by oral administration. The MASP inhibitory agent may be administered periodically over an extended period of time for treatment or control of a chronic condition, or may be by single or repeated administration in the period before, during or following acute trauma or injury.

### VII. PHARMACEUTICAL COMPOSITIONS AND DELIVERY METHODS

### Dosing

In another aspect, the disclosure provides compositions for inhibiting the adverse effects of MASP-3-dependent complement activation in a subject suffering from a hemolytic disease, such as PNH, comprising administering to the subject a composition comprising an amount of a MASP-3 inhibitory agent effective to inhibit MASP-3-dependent complement activation and a pharmaceutically acceptable carrier. In some cases, the method further comprises administering a composition comprising a MASP-2 inhibitory agent. The MASP-3 and MASP-2 inhibitory agents can be administered to a subject in need thereof, at therapeutically effective doses to treat or ameliorate conditions associated with MASP-3-dependent complement activation (LEA-1), and optionally also MASP-2-dependent complement activation (LEA-2). A therapeutically effective dose refers to the amount of the MASP-3 inhibitory agent, or a combination of a MASP-3 inhibitory agent and a MASP-2 inhibitory agent sufficient to result in amelioration of symptoms of the condition.

Toxicity and therapeutic efficacy of MASP-3 and MASP-2 inhibitory agents can be determined by standard pharmaceutical procedures employing experimental animal models. Using such animal models, the NOAEL (no observed adverse effect level) and the MED (the minimally effective dose) can be determined using standard methods. The dose ratio between NOAEL and MED effects is the therapeutic ratio, which is expressed as the ratio NOAEL/MED. MASP-3 inhibitory agents and MASP-2 inhibitory agents that exhibit large therapeutic ratios or indices are most preferred. The data obtained from the cell culture assays and animal studies can be used in formulating a range of dosages for use in humans. The dosage of the MASP-3 inhibitory agent and MASP-2 inhibitory agent preferably lies within a range of circulating concentrations that include the MED with little or no toxicity. The dosage may vary within this range depending upon the dosage form employed and the route of administration utilized.

For any compound formulation, the therapeutically effective dose can be estimated using animal models. For example, a dose may be formulated in an animal model to achieve a circulating plasma concentration range that includes the MED. Quantitative levels of the MASP-3 inhibitory agent or MASP-2 inhibitory agent in plasma may also be measured, for example, by high performance liquid chromatography.

In addition to toxicity studies, effective dosage may also be estimated based on the amount of target MASP protein present in a living subject and the binding affinity of the MASP-3 or MASP-2 inhibitory agent.

It has been reported that MASP-1 levels in normal human subjects is present in serum in levels in the range of from 1.48 to 12.83 µg/mL (Terai I. et al, Clin Exp Immunol 110:317-323 (1997); Theil et al., Clin. Exp. Immunol. 169:38 (2012)). The mean serum MASP-3 concentrations in normal human subjects has been reported to be in the range of about 2.0 to 12.9 µg/mL (Skjoedt M et al., Immunobiology 215(11):921-31 (2010); Degn et al., J. Immunol Methods, 361-37 (2010); Csuka et al., Mol. Immunol. 54:271 (2013). It has been shown that MASP-2 levels in normal human subjects is present in serum in low levels in the range of 500 ng/mL, and MASP-2 levels in a particular subject can be determined using a quantitative assay for MASP-2 described in Moller-Kristensen M., et al., J. Immunol. Methods 282:159-167 (2003) and Csuka et al., Mol. Immunol. 54:271 (2013).

Generally, the dosage of administered compositions comprising MASP-3 inhibitory agents or MASP-2 inhibitory agents varies depending on such factors as the subject's age, weight, height, sex, general medical condition, and previous medical history. As an illustration, MASP-3 inhibitory agents or MASP-2 inhibitory agents (such as MASP-3 antibodies, MASP-1 antibodies or MASP-2 antibodies), can be administered in dosage ranges from about 0.010 to 100.0 mg/kg, preferably 0.010 to 10 mg/kg, preferably 0.010 to 1.0 mg/kg, more preferably 0.010 to 0.1 mg/kg of the subject body weight. In some cases, MASP-2 inhibitory agents (such as MASP-2 antibodies) are administered in dosage ranges from about preferably 0.010 to 10 mg/kg, preferably 0.010 to 1.0 mg/kg, more preferably 0.010 to 0.1 mg/kg of the subject body weight. In some cases, MASP-1 inhibitory agents (such as MASP-1 antibodies) or MASP-3 inhibitory agents (such as MASP-3 antibodies) are administered in dosage ranges from about 0.010 to 100.0 mg/kg, preferably 0.010 to 10 mg/kg, preferably 0.010 to 1.0 mg/kg, more preferably 0.010 to 0.1 mg/kg of the subject body weight.

Therapeutic efficacy of MASP-3 inhibitory compositions, optionally in combination with MASP-2 inhibitory compositions, or of MASP-1 inhibitory compositions, optionally in combination with MASP-2 inhibitory compositions, and methods of the present disclosure in a given subject, and appropriate dosages, can be determined in accordance with complement assays well known to those of skill in the art. Complement generates numerous specific products. During the last decade, sensitive and specific assays have been developed and are available commercially for most of these activation products, including the small activation fragments C3a, C4a, and C5a and the large activation fragments iC3b, C4d, Bb, and sC5b-9. Most of these assays utilize monoclonal antibodies that react with new antigens (neoantigens) exposed on the fragment, but not on the native proteins from which they are formed, making these assays very simple and specific. Most rely on ELISA technology, although radioimmunoassay is still sometimes used for C3a and C5a. These latter assays measure both the unprocessed fragments and their 'desArg' fragments, which are the major forms found in the circulation. Unprocessed fragments and C5a_{desArg} are rapidly cleared by binding to cell surface receptors and are hence present in very low concentrations, whereas C3a_{desArg} does not bind to cells and accumulates in plasma. Measurement of C3a provides a sensitive, pathway-independent indicator of complement activation. Alternative pathway activation can be assessed by measuring the Bb fragment and/or measurement of factor D activation. Detection of the fluid-phase product of membrane attack pathway activation, sC5b-9, provides evidence that complement is being activated to completion. Because both the lectin and classical pathways generate the same activation products, C4a and C4d, measurement of these two fragments does not provide any information about which of these two pathways has generated the activation products.

The inhibition of MASP-3-dependent complement activation is characterized by at least one of the following changes in a component of the complement system that occurs as a result of administration of a MASP-3 inhibitory agent in accordance with the methods of the disclosure: the inhibition of LEA-1-mediated complement activation (inhibition of hemolysis and opsonization); inhibition of MASP-3 serine protease substrate-specific cleavage, the reduction of hemolysis (measured, for example as described in Example 5) or the reduction of C3 cleavage and C3b deposition (measured, for example, as described in Example 4 or Example 11).

The inhibition of MASP-2-dependent complement activation is characterized by at least one of the following changes in a component of the complement system that occurs as a result of administration of a MASP-2 inhibitory agent in accordance with the methods of the disclosure: the inhibition of the generation or production of MASP-2-dependent complement activation system products C4b, C3a, C5a and/or C5b-9 (MAC) (measured, for example, as described in measured, for example, as described in Example 2 of US Patent No. 7,919,094), the reduction of C4 cleavage and C4b deposition (measured, for example as described in Example 8 or Example 9), or the reduction of C3 cleavage and C3b deposition (measured, for example, as described in Example 11).

### i. Pharmaceutical carriers and delivery vehicles

In general, the MASP-3 inhibitory agent compositions and the MASP-2 inhibitory agent compositions of the present disclosure, or compositions comprising a combination of MASP-2 and MASP-3 inhibitory agents, may be combined with any other selected therapeutic agents, are suitably contained in a pharmaceutically acceptable carrier. The carrier is non-toxic, biocompatible and is selected so as not to detrimentally affect the biological activity of the MASP-3 inhibitory agent or the MASP-2 inhibitory agent (and any other therapeutic agents combined therewith). Exemplary pharmaceutically acceptable carriers for peptides are described in U.S. Patent No. 5,211,657 to Yamada. The MASP antibodies useful in the invention, as described herein, may be formulated into preparations in solid, semi-solid, gel, liquid or gaseous forms such as tablets, capsules, powders, granules, ointments, solutions, depositories, inhalants and injections allowing for oral, parenteral or surgical administration. The disclosure also contemplates local administration of the compositions by coating medical devices and the like.

Suitable carriers for parenteral delivery via injectable, infusion or irrigation and topical delivery include distilled water, physiological phosphate-buffered saline, normal or lactated Ringer's solutions, dextrose solution, Hank's solution, or propanediol. In addition, sterile, fixed oils may be employed as a solvent or suspending medium. For this purpose any biocompatible oil may be employed including synthetic mono- or diglycerides. In addition, fatty acids such as oleic acid find use in the preparation of injectables. The carrier and agent may be compounded as a liquid, suspension, polymerizable or non-polymerizable gel, paste or salve.

The carrier may also comprise a delivery vehicle to sustain (i.e., extend, delay or regulate) the delivery of the agent(s) or to enhance the delivery, uptake, stability or pharmacokinetics of the therapeutic agent(s). Such a delivery vehicle may include, by way of non-limiting example, microparticles, microspheres, nanospheres or nanoparticles composed of proteins, liposomes, carbohydrates, synthetic organic compounds, inorganic compounds, polymeric or copolymeric hydrogels and polymeric micelles. Suitable hydrogel and micelle delivery systems include the PEO:PHB:PEO copolymers and copolymer/cyclodextrin complexes disclosed in WO 2004/009664 A2 and the PEO and PEO/cyclodextrin complexes disclosed in U.S. Patent Application Publication No. 2002/0019369 A1. Such hydrogels may be injected locally at the site of intended action, or subcutaneously or intramuscularly to form a sustained release depot.

Compositions of the present invention may be formulated for delivery subcutaneously, intra-muscularly, intravenously, intra-arterially or as an inhalant.

For intra-articular delivery, the MASP-3 inhibitory agent or the MASP-2 inhibitory agent may be carried in above-described liquid or gel carriers that are injectable, above-described sustained-release delivery vehicles that are injectable, or a hyaluronic acid or hyaluronic acid derivative.

For oral administration of non-peptidergic agents, the MASP-3 inhibitory agent or MASP-2 inhibitory agent may be carried in an inert filler or diluent such as sucrose, cornstarch, or cellulose.

For topical administration, the MASP-3 inhibitory agent or MASP-2 inhibitory agent may be carried in ointment, lotion, cream, gel, drop, suppository, spray, liquid or powder, or in gel or microcapsular delivery systems via a transdermal patch.

Various nasal and pulmonary delivery systems, including aerosols, metered-dose inhalers, dry powder inhalers, and nebulizers, are being developed and may suitably be adapted for delivery of the present invention in an aerosol, inhalant, or nebulized delivery vehicle, respectively.

For intrathecal (IT) or intracerebroventricular (ICV) delivery, appropriately sterile delivery systems (e.g., liquids; gels, suspensions, etc.) can be used to administer the present disclosure.

The compositions of the present disclosure may also include biocompatible excipients, such as dispersing or wetting agents, suspending agents, diluents, buffers, penetration enhancers, emulsifiers, binders, thickeners, flavoring agents (for oral administration).

### ii. Pharmaceutical carriers for antibodies and peptides

More specifically with respect to MASP antibodies, as described herein, exemplary formulations can be parenterally administered as injectable dosages of a solution or suspension of the compound in a physiologically acceptable diluent with a pharmaceutical carrier that can be a sterile liquid such as water, oils, saline, glycerol or ethanol. Additionally, auxiliary substances such as wetting or emulsifying agents, surfactants, pH buffering substances and the like can be present in compositions comprising MASP antibodies. Additional components of pharmaceutical compositions include petroleum (such as of animal, vegetable or synthetic origin), for example, soybean oil and mineral oil. In general, glycols such as propylene glycol or polyethylene glycol are preferred liquid carriers for injectable solutions.

The MASP antibodies can also be administered in the form of a depot injection or implant preparation that can be formulated in such a manner as to permit a sustained or pulsatile release of the active agents.

### VIII. MODES OF ADMINISTRATION

The pharmaceutical compositions comprising the MASP-3 inhibitory agents or MASP-2 inhibitory agents may be administered in a number of ways depending on whether a local or systemic mode of administration is most appropriate for the condition being treated. Further, the compositions of the present disclosure can be delivered by coating or incorporating the compositions on or into an implantable medical device.

### i. Systemic delivery

As used herein, the terms "systemic delivery" and "systemic administration" are intended to include but are not limited to oral and parenteral routes including intramuscular (IM), subcutaneous, intravenous (IV), intraarterial, inhalational, sublingual, buccal, topical, transdermal, nasal, rectal, vaginal and other routes of administration that effectively result in dispersement of the delivered agent to a single or multiple sites of intended therapeutic action. Preferred routes of systemic delivery for the present compositions include intravenous, intramuscular, subcutaneous, intraarterial and inhalational. It will be appreciated that the exact systemic administration route for selected agents utilized in particular compositions of the present disclosure will be determined in part to account for the agent's susceptibility to metabolic transformation pathways associated with a given route of administration. For example, peptidergic agents may be most suitably administered by routes other than oral.

The MASP inhibitory antibodies, as described herein, can be delivered into a subject in need thereof by any suitable means. Methods of delivery of MASP antibodies and polypeptides include administration by oral, pulmonary, parenteral (e.g., intramuscular, intraperitoneal, intravenous (IV) or subcutaneous injection), inhalation (such as via a fine powder formulation), transdermal, nasal, vaginal, rectal, or sublingual routes of administration, and can be formulated in dosage forms appropriate for each route of administration.

By way of representative example, MASP inhibitory antibodies and peptides can be introduced into a living body by application to a bodily membrane capable of absorbing the polypeptides, for example the nasal, gastrointestinal and rectal membranes. The polypeptides are typically applied to the absorptive membrane in conjunction with a permeation enhancer. (See, e.g., Lee, V.H.L., Crit. Rev. Ther. Drug Carrier Sys. 5:69, (1988); Lee, V.H.L., J. Controlled Release 13:213, (1990); Lee, V.H.L., Ed., Peptide and Protein Drug Delivers, Marcel Dekker, New York (1991); DeBoer, A.G., et al., J. Controller Release 13:241, (1990). For example, STDHF is a synthetic derivative of fusidic acid, a steroidal surfactant that is similar in structure to the bile salts, and has been used as a permeation enhancer for nasal delivery. (Lee, W.A., Biopharm. 22, Nov./Dec. 1990.)

The MASP inhibitory antibodies as described herein may be introduced in association with another molecule, such as a lipid, to protect the polypeptides from enzymatic degradation. For example, the covalent attachment of polymers, especially polyethylene glycol (PEG), has been used to protect certain proteins from enzymatic hydrolysis in the body and thus prolong half-life (Fuertges, F., et al., J. Controller Release 11:139, (1990)). Many polymer systems have been reported for protein delivery (Bae, Y.H., et al., J. Controlled Release 9:271, (1989); Hori, R., et al., Pharm. Res. 6:813, (1989); Yamakawa, I., et al., J. Pharm. Sci. 79:505, (1990); Yoshihiro, I., et al., J. Controlled Release 10:195, (1989); Asano, M., et al., J. Controlled Release 9:111, (1989); Rosenblatt, J., et al., J. Controlled Release 9:195, (1989); Makino, K., J. Controlled Release 12:235, (1990); Takakura, Y., et al., J. Pharm. Sci. 78:117, (1989); Takakura, Y., et al., J. Pharm. Sci. 78:219, (1989)).

Recently, liposomes have been developed with improved serum stability and circulation half-times (see, e.g., U.S. Patent No. 5,741,516, to Webb). Furthermore, various methods of liposome and liposome-like preparations as potential drug carriers have been reviewed (see, e.g., U.S. Patent No. 5,567,434, to Szoka; U.S. Patent No. 5,552,157, to Yagi; U.S. Patent No. 5,565,213, to Nakamori; U.S. Patent No. 5,738,868, to Shinkarenko; and U.S. Patent No. 5,795,587, to Gao).

For transdermal applications, the MASP inhibitory antibodies, as described herein, may be combined with other suitable ingredients, such as carriers and/or adjuvants. There are no limitations on the nature of such other ingredients, except that they must be pharmaceutically acceptable for their intended administration, and cannot degrade the activity of the active ingredients of the composition. Examples of suitable vehicles include ointments, creams, gels, or suspensions, with or without purified collagen. The MASP inhibitory antibodies may also be impregnated into transdermal patches, plasters, and bandages, preferably in liquid or semi-liquid form.

The compositions of the present invention may be systemically administered on a periodic basis at intervals determined to maintain a desired level of therapeutic effect. For example, compositions may be administered, such as by subcutaneous injection, every two to four weeks or at less frequent intervals. The dosage regimen will be determined by the physician considering various factors that may influence the action of the combination of agents. These factors will include the extent of progress of the condition being treated, the patient's age, sex and weight, and other clinical factors. The dosage for each individual agent will vary as a function of the MASP-3 inhibitory agent or the MASP-2 inhibitory agent that is included in the composition, as well as the presence and nature of any drug delivery vehicle (e.g., a sustained release delivery vehicle). In addition, the dosage quantity may be adjusted to account for variation in the frequency of administration and the pharmacokinetic behavior of the delivered agent(s).

### ii. Local delivery

As used herein, the term "local" encompasses application of a drug in or around a site of intended localized action, and may include for example topical delivery to the skin or other affected tissues, ophthalmic delivery, intrathecal (IT), intracerebroventricular (ICV), intra-articular, intracavity, intracranial or intravesicular administration, placement or irrigation. Local administration may be preferred to enable administration of a lower dose, to avoid systemic side effects, and for more accurate control of the timing of delivery and concentration of the active agents at the site of local delivery. Local administration provides a known concentration at the target site, regardless of interpatient variability in metabolism, blood flow, etc. Improved dosage control is also provided by the direct mode of delivery.

Local delivery of a MASP-3 inhibitory agent or a MASP-2 inhibitory agent may be achieved in the context of surgical methods for treating a disease or condition, such as for example during procedures such as arterial bypass surgery, atherectomy, laser procedures, ultrasonic procedures, balloon angioplasty and stent placement. For example, a MASP-3 inhibitory agent or a MASP-2 inhibitory agent can be administered to a subject in conjunction with a balloon angioplasty procedure. A balloon angioplasty procedure involves inserting a catheter having a deflated balloon into an artery. The deflated balloon is positioned in proximity to the atherosclerotic plaque and is inflated such that the plaque is compressed against the vascular wall. As a result, the balloon surface is in contact with the layer of vascular endothelial cells on the surface of the blood vessel. The MASP-3 inhibitory agent or MASP-2 inhibitory agent may be attached to the balloon angioplasty catheter in a manner that permits release of the agent at the site of the atherosclerotic plaque. The agent may be attached to the balloon catheter in accordance with standard procedures known in the art. For example, the agent may be stored in a compartment of the balloon catheter until the balloon is inflated, at which point it is released into the local environment. Alternatively, the agent may be impregnated on the balloon surface, such that it contacts the cells of the arterial wall as the balloon is inflated. The agent may also be delivered in a perforated balloon catheter such as those disclosed in Flugelman, M.Y., et al., Circulation 85:1110-1117, (1992). See also published PCT Application WO 95/23161 for an exemplary procedure for attaching a therapeutic protein to a balloon angioplasty catheter. Likewise, the MASP-3 inhibitory agent or MASP-2 inhibitory agent may be included in a gel or polymeric coating applied to a stent, or may be incorporated into the material of the stent, such that the stent elutes the MASP-3 inhibitory agent or MASP-2 inhibitory agent after vascular placement.

MASP-3 inhibitory agents or MASP-2 inhibitory agents used in the treatment of arthritides and other musculoskeletal disorders may be locally delivered by intra-articular injection. Such compositions may suitably include a sustained release delivery vehicle. As a further example of instances in which local delivery may be desired, MASP-2 inhibitory compositions used in the treatment of urogenital conditions may be suitably instilled intravesically or within another urogenital structure.

### IX. TREATMENT REGIMENS

In prophylactic applications, the pharmaceutical compositions are administered to a subject susceptible to, or otherwise at risk of, PNH in an amount sufficient to eliminate or reduce the risk of developing symptoms of the condition. In therapeutic applications, the pharmaceutical compositions are administered to a subject suspected of, or already suffering from, PNH in a therapeutically effective amount sufficient to relieve, or at least partially reduce, the symptoms of the condition.

In one case, the subject's red blood cells are opsonized by fragments of C3 in the absence of the composition, and administration of the composition to the subject increases the survival of red blood cells in the subject. In one case, the subject exhibits one or more symptoms in the absence of the composition selected from the group consisting of (i) below normal levels of hemoglobin, (ii) below normal levels of platelets; (iii) above normal levels of reticulocytes, and (iv) above normal levels of bilirubin, and administration of the composition to the subject improves at least one or more of the symptoms, resulting in (i) increased, normal, or nearly normal levels of hemoglobin (ii) increased, normal or nearly normal levels of platelets, (iii) decreased, normal or nearly normal levels of reticulocytes, and/or (iv) decreased, normal or nearly normal levels of bilirubin.

In both prophylactic and therapeutic regimens, compositions comprising MASP-3 inhibitory agents and optionally MASP-2 inhibitory agents may be administered in several dosages until a sufficient therapeutic outcome has been achieved in the subject. The MASP-3 and/or MASP-2 inhibitory agent comprises a MASP-1 antibody, a MASP-2 antibody or a MASP-3 antibody, which suitably may be administered to an adult patient (e.g., an average adult weight of 70 kg) in a dosage of from 0.1 mg to 10,000 mg, more suitably from 1.0 mg to 5,000 mg, more suitably 10.0 mg to 2,000 mg, more suitably 10.0 mg to 1,000 mg and still more suitably from 50.0 mg to 500 mg, or 10 to 200 mg. For pediatric patients, dosage can be adjusted in proportion to the patient's weight.

Application of the MASP-3 inhibitory compositions and optional MASP-2 inhibitory compositions of the present disclosure may be carried out by a single administration of the composition (e.g., a single composition comprising MASP-2 and MASP-3 inhibitory agents, or bispecific or dual inhibitory agents, or co-administration of separate compositions), or a limited sequence of administrations, for treatment of PNH. Alternatively, the composition may be administered at periodic intervals such as daily, biweekly, weekly, every other week, monthly or bimonthly over an extended period of time for treatment of PNH.

In some cases, a first composition comprising at least one MASP-3 inhibitory agent and a second composition comprising at least one MASP-2 inhibitory agent are administered to a subject suffering from PNH. In one case, the first composition comprising at least one MASP-3 inhibitory agent and a second composition comprising at least one MASP-2 inhibitory agent are administered simultaneously (i.e., within a time separation of no more than about 15 minutes or less, such as no more than any of 10, 5 or 1 minute). In one case, the first composition comprising at least one MASP-3 inhibitory agent and a second composition comprising at least one MASP-2 inhibitory agent are administered sequentially (i.e., the first composition is administered either prior to or after the administration of the second composition, wherein the time separation of administration is more than 15 minutes). In some cases, the first composition comprising at least one MASP-3 inhibitory agent and a second composition comprising at least one MASP-2 inhibitory agent are administered concurrently (i.e., the administration period of the first composition overlaps with the administration of the second composition). For example, in some cases, the first composition and/or the second composition are administered for a period of at least one, two, three or four weeks or longer. In one case, at least one MASP-3 inhibitory agent and at least one MASP-2 inhibitory agent are combined in a unit dosage form. In one case, a first composition comprising at least one MASP-3 inhibitory agent and a second composition comprising at least one MASP-2 inhibitory agent are packaged together in a kit for use in treatment of PNH.

In some cases, the subject suffering from PNH has previously undergone, or is currently undergoing treatment with a terminal complement inhibitor that inhibits cleavage of complement protein C5. In some cases, the method comprises administering to the subject a composition of the invention comprising a MASP-3 and optionally a MASP-2 inhibitor and further administering to the subject a terminal complement inhibitor that inhibits cleavage of complement protein C5. In some cases, the terminal complement inhibitor is a humanized anti-C5 antibody or antigen-binding fragment thereof. In some cases, the terminal complement inhibitor is eculizumab.

### X. EXAMPLES

The following examples merely illustrate the best mode now contemplated for practicing the disclosure.

### EXAMPLE 1

This Example demonstrates that MASP-2 deficient mice are protected from *Neisseria meningitidis* induced mortality after infection with either *N. meningitidis* serogroup A or *N. meningitidis* serogroup B.

### Methods:

MASP-2 knockout mice (MASP-2 KO mice) were generated as described in Example 1 of US 7,919,094. 10-week-old MASP-2 KO mice (n=10) and wild-type (WT) C57/BL6 mice (n=10) were inoculated by intraperitoneal (i.p.) injection with a dosage of 2.6 x 10⁷ CFU of *N. meningitidis* serogroup A Z2491 in a volume of 100 µl. The infective dose was administered to mice in conjunction with iron dextran at a final concentration of 400 mg/kg. Survival of the mice after infection was monitored over a 72-hour time period.

In a separate experiment, 10-week-old MASP-2 KO mice (n=10) and WT C57/BL6 mice (n=10) were inoculated by i.p. injection with a dosage of 6 x 10⁶ CFU of *N. meningitidis* serogroup B strain MC58 in a volume of 100 µl. The infective dose was administered to mice in conjunction with iron dextran at a final dose of 400 mg/kg. Survival of the mice after infection was monitored over a 72-hour time period. An illness score was also determined for the WT and MASP-2 KO mice during the 72-hour time period after infection, based on the illness scoring parameters described below in **TABLE 4,** which is based on the scheme of Fransen et al. (2010) with slight modifications.

**TABLE 4: Illness Scoring associated with clinical signs in infected mice**

| **Signs** | **Score** |
|---|---|
| Normal | 0 |
| Slightly ruffled fur | 1 |
| Ruffled fur, slow and sticky eyes | 2 |
| Ruffled fur, lethargic and eyes shut | 3 |
| Very sick and no movement after stimulation | 4 |
| Dead | 5 |

Blood samples were taken from the mice at hourly intervals after infection and analyzed to determine the serum level (log cfu/mL) of *N. meningitidis* in order to verify infection and determine the rate of clearance of the bacteria from the serum.

### Results:

**FIGURE 8** is a Kaplan-Meyer plot graphically illustrating the percent survival of MASP-2 KO and WT mice after administration of an infective dose of 2.6 x 10⁷ cfu *of N. meningitidis* serogroup A Z2491. As shown in **FIGURE 8****, 100%** of the MASP-2 KO mice survived throughout the 72-hour period after infection. In contrast, only 80% of the WT mice (*p*=0.012) were still alive 24 hours after infection, and only 50% of the WT mice were still alive at 72 hours after infection. These results demonstrate that MASP-2-deficient mice are protected from *N. meningitidis* serogroup A Z2491-induced mortality.

**FIGURE 9** is a Kaplan-Meyer plot graphically illustrating the percent survival of MASP-2 KO and WT mice after administration of an infective dose of 6 x 10⁶ cfu *of N. meningitidis* serogroup B strain MC58. As shown in **FIGURE 9****,** 90% of the MASP-2 KO mice survived throughout the 72-hour period after infection. In contrast, only 20% of the WT mice (*p*=0.0022) were still alive 24 hours after infection. These results demonstrate that MASP-2-deficient mice are protected from *N. meningitidis* serogroup B strain MC58-induced mortality.

**FIGURE 10** graphically illustrates the log cfu/mL of *N. meningitidis* serogroup B strain MC58 recovered at different time points in blood samples taken from the MASP-2 KO and WT mice after i.p. infection with 6x10⁶ cfu of *N. meningitidis* serogroup B strain MC58 (n=3 at different time points for both groups of mice). The results are expressed as Means±SEM. As shown in **FIGURE 10****,** in WT mice the level of *N. meningitidis* in the blood reached a peak of about 6.0 log cfu/mL at 24 hours after infection and dropped to about 4.0 log cfu/mL by 36 hours after infection. In contrast, in the MASP-2 KO mice, the level of *N. meningitidis* reached a peak of about 4.0 log cfu/mL at 12 hours after infection and dropped to about 1.0 log cfu/mL by 36 hours after infection (the symbol "*" indicates p<0.05; the symbol "**" indicates p=0.0043). These results demonstrate that although the MASP-2 KO mice were infected with the same dose of *N. meningitidis* serogroup B strain MC58 as the WT mice, the MASP-2 KO mice have enhanced clearance of bacteraemia as compared to WT.

**FIGURE 11** graphically illustrates the average illness score of MASP-2 KO and WT mice at 3, 6, 12 and 24 hours after infection with 6x10⁶ cfu of *N. meningitidis* serogroup B strain MC58. As shown in **FIGURE 11****,** the MASP-2-deficient mice showed high resistance to the infection, with much lower illness scores at 6 hours (symbol "*" indicates p=0.0411), 12 hours (symbol "**" indicates p=0.0049) and 24 hours (symbol "***" indicates p=0.0049) after infection, as compared to WT mice. The results in **FIGURE 11** are expressed as means±SEM.

In summary, the results in this Example demonstrate that MASP-2-deficient mice are protected from *N. meningitides-*induced mortality after infection with either *N. meningitidis* serogroup A or *N. meningitidis* serogroup B.

### EXAMPLE 2

This Example demonstrates that the administration of MASP-2 antibody after infection with *N. meningitidis* increases the survival of mice infected with *N. meningitidis.*

### Background/Rationale:

As described in Example 24 of US Patent 7,919,094, rat MASP-2 protein was utilized to pan a Fab phage display library, from which Fab2 #11 was identified as a functionally active antibody. Full-length antibodies of the rat IgG2c and mouse IgG2a isotypes were generated from Fab2 #11. The full-length MASP-2 antibody of the mouse IgG2a isotype was characterized for pharmacodynamic parameters (as described in Example 38 of US Patent 7,919,094).

In this Example, the mouse MASP-2 full-length antibody derived from Fab2 #11 was analyzed in the mouse model *of N. meningitidis* infection.

### Methods:

The mouse IgG2a full-length MASP-2 antibody isotype derived from Fab2 #11, generated as described above, was tested in the mouse model *of N. meningitidis* infection as follows.

### 1. Administration of mouse-MASP-2 monoclonal antibodies (MoAb) after infection

9-week-old C57/BL6 Charles River mice were treated with inhibitory mouse MASP-2 antibody (1.0 mg/kg) (n=12) or control isotype antibody (n=10) at 3 hours after i.p. injection with a high dose (4x10⁶ cfu) of *N. meningitidis* serogroup B strain MC58.

### Results:

**FIGURE 12** is a Kaplan-Meyer plot graphically illustrating the percent survival of mice after administration of an infective dose of 4x10⁶ cfu *of N. meningitidis* serogroup B strain MC58, followed by administration 3 hours post-infection of either inhibitory MASP-2 antibody (1.0 mg/kg) or control isotype antibody. As shown in **FIGURE 12****,** 90% of the mice treated with MASP-2 antibody survived throughout the 72-hour period after infection. In contrast, only 50% of the mice treated with isotype control antibody survived throughout the 72-hour period after infection. The symbol "*" indicates p=0.0301, as determined by comparison of the two survival curves.

These results demonstrate that administration of a MASP-2 antibody is effective to treat and improve survival in subjects infected with N. *meningitidis.*

As demonstrated herein, the use of MASP-2 antibody in the treatment of a subject infected with *N. meningitidis* is effective when administered within 3 hours post-infection, and is expected to be effective within 24 hours to 48 hours after infection. Meningococcal disease (either meningococcemia or meningitis) is a medical emergency, and therapy will typically be initiated immediately if meningococcal disease is suspected (i.e., before *N*. *meningitidis* is positively identified as the etiological agent).

In view of the results in the MASP-2 KO mouse demonstrated in EXAMPLE 1, it is believed that administration of MASP-2 antibody prior to infection with *N. meningitidis* would also be effective to prevent or ameliorate the severity of infection.

### EXAMPLE 3

This Example demonstrates the complement-dependent killing of *N. meningitidis* in human sera is MASP-3-dependent.

### Rationale:

Patients with decreased serum levels of functional MBL display increased susceptibility to recurrent bacterial and fungal infections (Kilpatrick et al., Biochim Biophys Acta 1572:401-413 (2002)). It is known that *N. meningitidis* is recognized by MBL, and it has been shown that MBL-deficient sera do not lyse *N. meningitidis.*

In view of the results described in Examples 1 and 2, a series of experiments were carried out to determine the efficacy of administration of MASP-2 antibody to treat *N*. *meningitidis* infection in complement-deficient and control human sera. Experiments were carried out in a high concentration of serum (20%) in order to preserve the complement pathway.

### Methods:

### 1. Serum bactericidal activity in various complement-deficient human sera and in human sera treated with human MASP-2 antibody

The following complement-deficient human sera and control human sera were used in this experiment:

**TABLE 5: Human serum samples tested (as shown in FIGURE 13)**

| **Sample** | **Serum type** |
|---|---|
| **A** | Normal human sera (NHS) + human MASP-2 Ab |
| **B** | NHS + isotype control Ab |
| **C** | MBL -/- human serum |
| **D** | NHS |
| **E** | Heat-Inactivated (HI) NHS |

A recombinant antibody against human MASP-2 was isolated from a combinatorial Antibody Library (Knappik, A., et al., J. Mol. Biol. 296:57-86 (2000)), using recombinant human MASP-2A as an antigen (Chen, C.B. and Wallis, J. Biol. Chem. 276:25894-25902 (2001)). An anti-human scFv fragment that potently inhibited lectin pathway-mediated activation of C4 and C3 in human plasma (IC50∼20 nM) was identified and converted to a full-length human IgG4 antibody.

*N. meningitidis* serogroup B-MC58 was incubated with the different sera show in **TABLE 5,** each at a serum concentration of 20%, with or without the addition of inhibitory human MASP-2 antibody (3 µg in 100 µl total volume) at 37°C with shaking. Samples were taken at the following time points: 0-, 30-, 60- and 90-minute intervals, plated out and then viable counts were determined. Heat-inactivated human serum was used as a negative control.

### Results:

**FIGURE 13** graphically illustrates the log cfu/mL of viable counts of *N. meningitidis* serogroup B-MC58 recovered at different time points in the human sera samples shown in **TABLE 5. TABLE 6** provides the Student's t-test results for **FIGURE 13****.**

**TABLE 6: Student's t-test Results for FIGURE 13 (time point 60 minutes)**

| | **Mean Diff. (Log)** | **Significant? P<0.05?** | **P value summary** |
|---|---|---|---|
| AvsB | -0.3678 | Yes | ***(0.0002) |
| AvsC | -1.1053 | Yes | ***(p<0.0001) |
| AvsD | -0.2111 | Yes | **(0.0012) |
| CvsD | 1.9 | Yes | ***(p<0.0001) |

As shown in **FIGURE 13** and **TABLE 6,** complement-dependent killing of *N. meningitidis* in human 20% serum was significantly enhanced by the addition of the human MASP-2 inhibitory antibody.

### 2. Serum bactericidal activity in various complement deficient human sera

The following complement-deficient human sera and control human sera were used in this experiment:

**TABLE 7: Human serum samples tested (as shown in FIGURE 14)**

| **Sample** | **Serum Type** |
|---|---|
| A | Normal human serum (NHS) |
| B | Heat-inactivated NHS |
| C | MBL -/- |
| D | MASP-3 -/- (MASP-1 +) |

| | |
|---|---|
| Note: The MASP-3 -/- (MASP-1 +) serum in sample D was taken from a subject with 3MC syndrome, which is a unifying term for the overlapping Carnevale, Mingarelli, Malpuech and Michels syndromes. As further described in Example 4, the mutations in exon 12 of the MASP-1/3 gene render the serine protease domain of MASP-3, but not MASP-1 dysfunctional. It is also known that factor D is intact in 3MC serum. | |

*N. meningitidis* serogroup B-MC58 was incubated with different complement-deficient human sera, each at a serum concentration of20%, at 37°C with shaking. Samples were taken at the following time points: 0-, 15-, 30-, 45-, 60-, 90- and 120-minute intervals, plated out and then viable counts were determined. Heat-inactivated human serum was used as a negative control.

### Results:

**FIGURE 14** graphically illustrates the log cfu/mL of viable counts of *N. meningitidis* serogroup B-MC58 recovered at different time points in the human sera samples shown in **TABLE 7.** As shown in **FIGURE 14****,** the WT (NHS) serum has the highest level of bactericidal activity for *N. meningitidis.* In contrast, the MBL -/- and MASP-3 -/- (which is MASP-1-sufficient) human sera do not have any bactericidal activity. These results indicate that complement-dependent killing *of N. meningitidis* in human 20% (v/v) serum is MASP-3- and MBL-dependent. **TABLE 8** provides the Student's t-test results for **FIGURE 14****.**

**TABLE 8: Student's t-test Results for FIGURE 14**

| **Comparison** | **Time Point (min)** | **Mean Diff. (Log)** | **Significant? P<0.05?** | **P value Summary** |
|---|---|---|---|---|
| AvsB | 60 | -0.8325 | Yes | ***(p<0.0001) |
| AvsB | 90 | -1.600 | Yes | ***(p<0.0001) |
| AvsC | 60 | -1.1489 | Yes | ***(p<0.0001) |
| AvsC | 90 | -1.822 | Yes | ***(p<0.0001) |
| AvsD | 60 | -1.323 | Yes | ***(0.0005) |
| A vs D | 90 | -2.185 | Yes | ***(p<0.0001) |

In summary, the results shown in **FIGURE 14** and **TABLE 8** demonstrate that complement-dependent killing *of N. meningitidis* in 20% human serum is MASP-3- and MBL-dependent.

### 3. Complement-dependent killing of N. meningitidis in 20% (v/v) mouse sera deficient of MASP-2, MASP-1/3 or MBL A/C.

The following complement-deficient mouse sera and control mouse sera were used in this experiment:

**TABLE 9: Mouse serum samples tested (as shown in FIGURE 15)**

| **Sample** | **Serum Type** |
|---|---|
| A | WT |
| B | MASP-2 -/- |
| C | MASP-1/3 -/- |
| D | MBL A/C -/- |
| E | WT heat-inactivated (HIS) |

*N. meningitidis* serogroup B-MC58 was incubated with different complement-deficient mouse sera, each at a serum concentration of20%, at 37°C with shaking. Samples were taken at the following time points: 0-, 15-, 30-, 60-, 90- and 120-minute intervals, plated out and then viable counts were determined. Heat-inactivated human serum was used as a negative control.

### Results:

**FIGURE 15** graphically illustrates the log cfu/mL of viable counts of *N. meningitidis* serogroup B-MC58 recovered at different time points in the mouse serum samples shown in **TABLE 9.** As shown in **FIGURE 15****,** the MASP-2 -/- mouse sera have a higher level of bactericidal activity for *N. meningitidis* than WT mouse sera. In contrast, the MASP-1/3 -/- mouse sera do not have any bactericidal activity. The symbol "**" indicates p=0.0058, the symbol "***" indicates p=0.001. **TABLE 10** provides the Student's t-test results for **FIGURE 15****.**

**TABLE 10: Student's t-test Results for FIGURE 15**

| **Comparison** | **Time point** | **Mean Diff. (LOG)** | **Significant? (p<0.05)?** | **P value summary** |
|---|---|---|---|---|
| A vs. B | 60 min. | 0.39 | yes | ** (0.0058) |
| A vs. B | 90 min. | 0.6741 | yes | *** (0.001) |

In summary, the results in this Example demonstrate that MASP-2 -/- serum has a higher level of bactericidal activity for *N. meningitidis* than WT serum and that complement-dependent killing *of N. meningitidis* in 20% serum is MASP-3- and MBL-dependent.

### EXAMPLE 4

This Example describes a series of experiments that were carried out to determine the mechanism of the MASP-3-dependent resistance to *N. meningitidis* infection observed in MASP-2 KO mice, as described in Examples 1-3.

### Rationale:

In order to determine the mechanism of MASP-3-dependent resistance to *N. meningitidis* infection observed in MASP-2 KO mice (described in Examples 1-3 above), a series of experiments were carried out as follows.

### 1. MASP-1/3-deficient mice are not deficient of lectin pathway functional activity (also referred to as "LEA-2")

### Methods:

In order to determine whether MASP-1/3-deficient mice are deficient of lectin pathway functional activity (also referred to as LEA-2), an assay was carried out to measure the kinetics of C3 convertase activity in plasma from various complement-deficient mouse strains tested under lectin activation pathway-specific assay conditions (1% plasma), as described in Schwaeble W. et al., PNAS vol 108(18):7523-7528 (2011).

Plasma was tested from WT, C4-/-, MASP-1/3-/-; Factor B-/-, and MASP-2-/- mice as follows.

To measure C3 activation, microtiter plates were coated with mannan (1 µg/well), zymosan (1 µg/well) in coating buffer (15 mM Na₂Co₃, 35 mM NaHCO₃), or immune complexes, generated *in situ* by coating with 1% human serum albumin (HSA) in coating buffer then adding sheep anti-HAS serum (2 µg/mL) in TBS (10mM Tris, 140 mM NaCl, pH 7.4) with 0.05% Tween 20 and 5 mM Ca⁺⁺. Plates were blocked with 0.1% HSA in TBS and washed three times with TBS/Tween20/ Ca⁺⁺. Plasma samples were diluted in 4 mM barbital, 145 mM NaCl, 2 mM CaCl₂, 1 mM MgCl₂, pH 7.4, added to the plates and incubated for 1.5 h at 37°C. After washing, bound C3b was detected using rabbit anti-human C3c (Dako), followed by alkaline phosphatase-conjugated goat anti-rabbit IgG and p-nitrophenyl phosphate.

### Results:

The kinetics of C3 activation (as measured by C3b deposition on mannan-coated plates with 1% serum) under lectin pathway-specific conditions is shown in **FIGURE 16****.** No C3 cleavage was seen in MASP-2-/- plasma. Factor B-/- (Factor B -/-) plasma cleaved C3 at half the rate of WT plasma, likely due to the loss of the amplification loop. A significant delay in the lectin pathway-dependent conversion of C3 to C3b was seen in C4-/- (T_{1/2}=33min) as well as in MASP-1/3-/- deficient plasma (T_{1/2}=49 min). This delay of C3 activation in MASP-1/3 -/- plasma has been shown to be MASP-1- rather than MASP-3-dependent. (See Takahashi M. et al., J Immunol 180:6132-6138 (2008)). These results demonstrate that MASP-1/3-deficient mice are not deficient of lectin pathway functional activity (also referred to as "LEA-2").

### 2. Effect of hereditary MASP-3 deficiency on alternative pathway activation.

### Rationale:

The effect of hereditary MASP-3 deficiency on alternative pathway activation was determined by testing serum of a MASP-3-deficient patient with 3MC syndrome caused by a frame-shift mutation in the exon encoding the serine protease of MASP-3. The 3MC syndrome is a unifying term for the overlapping Carneavale, Mingarelli, Malpuech and Michels syndromes. These rare autosomal recessive disorders exhibit a spectrum of developmental features, including characteristic facial dysmorphism, cleft lip and/or palate, craniosynostosis, learning disability and genital, limb and vesicorenal abnormalities. Rooryck et al., Mature Genetics 43:197-203 (2011) studied 11 families with 3MC syndrome and identified two mutated genes, COLEC11 and MASP-1. The mutations in the MASP-1 gene render the exon encoding the serine protease domain of MASP-3, but not the exons encoding the serine protease of MASP-1, dysfunctional. Therefore, 3MC patients with mutations in the exon encoding the serine protease of MASP-3 are deficient of MASP-3 but sufficient in MASP-1.

### Methods:

MASP-3-deficient serum was obtained from a 3MC patient, the mother and father of the 3MC patient (both heterozygous for the allele bearing a mutation that renders the exon encoding the MASP-3 serine protease domain dysfunctional), as well as from a C4-deficient patient (deficient in both human C4 genes) and an MBL-deficient subject. An alternative pathway assay was carried out under traditional AP-specific conditions (BBS/ Mg⁺⁺/EGTA, without Ca⁺⁺, wherein BBS = barbital buffered saline containing sucrose), as described in Bitter-Suermann et al., Eur. J. Immunol 11:291-295 (1981)), on zymosan-coated microtiter plates at serum concentrations ranging from 0.5 to 25% and C3b deposition was measured over time.

### Results:

**FIGURE 17** graphically illustrates the level of alternative pathway-driven C3b deposition on zymosan-coated microtiter plates as a function of serum concentration in serum samples obtained from MASP-3-deficient, C4-deficient and MBL-deficient subjects. As shown in **FIGURE 17****,** MASP-3-deficient patient serum has residual alternative pathway (AP) activity at high serum concentrations (25%, 12.5%, 6.25% serum concentrations), but a significantly higher AP₅₀ (i.e.. 9.8% of serum needed to achieve 50% of maximum C3 deposition).

**FIGURE 18** graphically illustrates the level of alternative pathway-driven C3b deposition on zymosan-coated microtiter plates under "traditional" alternative pathway-specific (AP-specific) conditions (*i.e.,* BBS/EGTA/Mg⁺⁺ without Ca⁺⁺) as a function of time in 10% human serum samples obtained from MASP-3-deficient, C4-deficient and MBL-deficient human subjects.

**TABLE 11** below summarizes the AP₅₀ results shown in **FIGURE 17** and the half-times for C3b deposition shown in **FIGURE 18****.**

**TABLE 11: Summary of Results shown in FIGURES 17 and 18**

| **Serum type** | **AP₅₀ (%)** | **T_{1/2} (min)** |
|---|---|---|
| MASP-3-deficient (3MC patient) | 9.8 | 37.4 |
| Mother of 3MC patient (heterozygous) | 4.3 | 17.2 |
| Father of 3MC patient (heterozygous) | 4.3 | 20.9 |
| C4-deficient | 4.0 | 11.6 |
| MBL-deficient | 4.8 | 11.0 |

| | | |
|---|---|---|
| Note: In BBS/ Mg⁺⁺/EGTA buffer, the lectin pathway-mediated effects are deficient due to absence of Ca⁺⁺ in this buffer. | | |

While not wishing to be bound by any particular theory, it is believed that the lower alternative pathway activity observed in the MASP-3-deficient serum is caused because the 3MC patient has active factor D in his serum, and since this patient still expresses MASP-1 and HTRA1, conversion of pro-factor D is still able to occur in the absence of MASP-3, although at a lower level

### 3. Measurement of C3b deposition on mannan, zymosan and S. pneumonia D39 in mouse sera deficient of MASP-2 or MASP-1/3.

### Methods:

C3b deposition was measured on mannan, zymosan and *S. pneumonia* D39-coated microtiter plates using mouse serum concentrations ranging from 0% to 20% obtained from MASP-2-/-, MASP-1/3-/- and WT mice. The C3b deposition assays were carried out under either "traditional" alternative pathway-specific conditions (*i*.*e*. BBS/EGTA/Mg⁺⁺ without Ca⁺⁺), or under physiological conditions allowing both the lectin pathway and the alternative pathway to function (*i.e.,* BBS/Mg⁺⁺/Ca⁺⁺).

### Results:

**FIGURE 19A** graphically illustrates the level of C3b deposition on mannan-coated microtiter plates as a function of serum concentration in serum samples obtained from WT, MASP-2-deficient, and MASP-1/3-deficient mice under traditional alternative pathway-specific conditions (*i.e.,* BBS/EGTA/Mg⁺⁺ without Ca⁺⁺), or under physiological conditions allowing both the lectin pathway and the alternative pathway to function (BBS/Mg⁺⁺/Ca⁺⁺). **FIGURE 19B** graphically illustrates the level of C3b deposition on zymosan-coated microtiter plates as a function of serum concentration in serum samples from WT, MASP-2-deficient, and MASP-1/3-deficient mice under traditional AP-specific conditions (*i.e.,* BBS/EGTA/Mg⁺⁺ without Ca⁺⁺), or under physiological conditions allowing both the lectin pathway and the alternative pathway to function (BBS/Mg⁺⁺/Ca⁺⁺). **FIGURE 19C** graphically illustrates the level of C3b deposition on *S. pneumoniae* D39-coated microtiter plates as a function of serum concentration in serum samples from WT, MASP-2-deficient, and MASP-1/3-deficient mice under traditional AP-specific conditions (*i.e*., BBS/EGTA/Mg⁺⁺ without Ca⁺⁺), or under physiological conditions allowing both the lectin pathway and the alternative pathway to function (BBS/Mg⁺⁺/Ca⁺⁺).

**FIGURE 20A** graphically illustrates the results of a C3b deposition assay in highly diluted sera carried out on mannan-coated microtiter plates under traditional AP-specific conditions (*i.e.* BBS/EGTA/Mg⁺⁺ without Ca⁺⁺) or under physiological conditions allowing both the lectin pathway and the alternative pathway to function (BBS/Mg⁺⁺/Ca⁺⁺), using serum concentrations ranging from 0 % up to 1.25%. **FIGURE 20B** graphically illustrates the results of a C3b deposition assay carried out on zymosan-coated microtiter plates under traditional AP-specific conditions *(i.e.* BBS/EGTA/Mg⁺⁺ without Ca⁺⁺) or under physiological conditions allowing both the lectin pathway and the alternative pathway to function (BBS/EGTA/Mg⁺⁺/Ca⁺⁺), using serum concentrations ranging from 0 % up to 1.25%. **FIGURE 20C** graphically illustrates the results of a C3b deposition assay carried out on *S. pneumoniae* D39-coated microtiter plates under traditional AP-specific conditions (*i.e.* BBS/EGTA/Mg⁺⁺ without Ca⁺⁺) or under physiological conditions allowing both the lectin pathway and the alternative pathway to function (BBS/EGTA/Mg⁺⁺/Ca⁺⁺), using serum concentrations ranging from 0 % up to 1.25%.

As shown in **FIGURES 20A-C****,** C3b deposition assays were also carried out under traditional alternative pathway-specific conditions (*i.e*. BBS/EGTA/Mg⁺⁺ without Ca⁺⁺) or under physiological conditions allowing both the lectin pathway and the alternative pathway to function (BBS/Mg⁺⁺/Ca⁺⁺), using higher dilutions ranging from 0 % up to 1.25% serum on mannan-coated plates **(****FIGURE 20A****);** zymosan-coated plates **(****FIGURE 20B****)** and *S. pneumoniae* D39-coated plates **(****FIGURE 20C****).** The alternative pathway tails off under higher serum dilutions, so the activity observed in the MASP-1/3-deficient serum in the presence of Ca⁺⁺ is MASP-2-mediated LP activity, and the activity in MASP-2-deficient serum in the presence of Ca⁺⁺ is MASP-1/3-mediated residual activation of the AP.

### Discussion:

The results described in this Example demonstrate that a MASP-2 inhibitor (or MASP-2 KO) provides significant protection from *N. meningitidis* infection by promoting MASP-3-driven alternative pathway activation. The results of the mouse serum bacteriolysis assays and the human serum bacteriolysis assays further show, by monitoring the serum bactericidal activity against *N. meningitidis,* that bactericidal activity against *N. meningitidis* is absent in MBL-deficient (mouse MBL A and MBL C double-deficient and human MBL-deficient sera).

**FIGURE 1** illustrates the new understanding of the lectin pathway and alternative pathway based on the results provided herein. **FIGURE 1** delineates the role of LEA-2 in both opsonization and lysis. While MASP-2 is the initiator of "downstream" C3b deposition (and resultant opsonization) in multiple lectin-dependent settings physiologically **(****FIGURE 20A****,** **20B, 20C****),** it also plays a role in lysis of serum-sensitive bacteria. As illustrated in **FIGURE 1****,** the proposed molecular mechanism responsible for the increased bactericidal activity of MASP-2-deficient or MASP-2-depleted serum/plasma for serum-sensitive pathogens such as *N. meningitidis* is that, for the lysis of bacteria, lectin pathway recognition complexes associated with MASP-1 and MASP-3 have to bind in close proximity to each other on the bacterial surface, thereby allowing MASP-1 to cleave MASP-3. In contrast to MASP-1 and MASP-2, MASP-3 is not an auto-activating enzyme, but, in many instances, requires activation/cleavage by MASP-1 to be converted into its enzymatically active form.

As further shown in **FIGURE 1****,** activated MASP-3 can then cleave C3b-bound factor B on the pathogen surface to initiate the alternative pathway activation cascade by formation of the enzymatically active alternative pathway C3 and C5 convertase C3bBb and C3bBb(C3b)n, respectively. MASP-2-bearing lectin-pathway activation complexes have no part in the activation of MASP-3 and, in the absence or after depletion of MASP-2, all-lectin pathway activation complexes will either be loaded with MASP-1 or MASP-3. Therefore, in the absence of MASP-2, the likelihood is markedly increased that on the microbial surface MASP-1 and MASP-3-bearing lectin-pathway activation complexes will come to sit in close proximity to each other, leading to more MASP-3 being activated and thereby leading to a higher rate of MASP-3-mediated cleavage of C3b-bound factor B to form the alternative pathway C3 and C5 convertases C3bBb and C3bBb(C3b)n on the microbial surface. This leads to the activation of the terminal activation cascades C5b-C9 that forms the Membrane Attack Complex, composed of surface-bound C5b associated with C6, C5bC6 associated with C7, C5bC6C7 associated with C8, and C5bC6C7C8, leading to the polymerization of C9 that inserts into the bacterial surface structure and forms a pore in the bacterial wall, which will lead to osmolytic killing of the complement-targeted bacterium.

The core of this novel concept is that the data provided herein clearly show that the lectin-pathway activation complexes drive the following two distinct activation routes, as illustrated in **FIGURE 1****:**

### EXAMPLE 5

This Example demonstrates the inhibitory effect of MASP-2 deficiency and/or MASP-3 deficiency on lysis of red blood cells from blood samples obtained from a mouse model of paroxysmal nocturnal hemoglobinuria (PNH).

### Background/Rationale:

Paroxysmal nocturnal hemoglobinuria (PNH), also referred to as Marchiafava-Micheli syndrome, is an acquired, potentially life-threatening disease of the blood, characterized by complement-induced intravascular hemolytic anemia. The hallmark of PNH is the chronic complement-mediated intravascular hemolysis that is a consequence of unregulated activation of the alternative pathway of complement due to the absence of the complement regulators CD55 and CD59 on PNH erythrocytes, with subsequent hemoglobinuria and anemia. Lindorfer, M.A., et al., Blood 115(11) (2010), Risitano, A.M, Mini-Reviews in Medicinal Chemistry, 11:528-535 (2011). Anemia in PNH is due to destruction of red blood cells in the bloodstream. Symptoms of PNH include red urine, due to appearance of hemoglobin in the urine, back pain, fatigue, shortness of breath and thrombosis. PNH may develop on its own, referred to as "primary PNH" or in the context of other bone marrow disorders such as aplastic anemia, referred to as "secondary PNH". Treatment for PNH includes blood transfusion for anemia, anticoagulation for thrombosis and the use of the monoclonal antibody eculizumab (Soliris®), which protects blood cells against immune destruction by inhibiting the complement system (Hillmen P. et al., N. Engl. J. Med. 350(6):552-9 (2004)). Eculizumab (Soliris®) is a humanized monoclonal antibody that targets the complement component C5, blocking its cleavage by C5 convertases, thereby preventing the production of C5a and the assembly of MAC. Treatment of PNH patients with eculizumab has resulted in a reduction of intravascular hemolysis, as measured by lactate dehydrogenase (LDH), leading to hemoglobin stabilization and transfusion independence in about half of the patients (Hillmen P, et al., Mini-Reviews in Medicinal Chemistry, vol 11(6) (2011)). While nearly all patients undergoing therapy with eculizumab achieve normal or almost normal LDH levels (due to control of intravascular hemolysis), only about one third of the patients reach a hemoglobin value about 11gr/dL, and the remaining patients on eculizumab continue to exhibit moderate to severe (*i.e.,* transfusion-dependent) anemia, in about equal proportions (Risitano A.M. et al., Blood 113:4094-100 (2009)). As described in Risitano et al., Mini-Reviews in Medicinal Chemistry 11:528-535 (2011), it was demonstrated that PNH patients on eculizumab contained C3 fragments bound to a substantial portion of their PNH erythrocytes (while untreated patients did not), leading to the conclusion that membrane-bound C3 fragments work as opsonins on PNH erythrocytes, resulting in their entrapment in the reticuloendothelial cells through specific C3 receptors and subsequent extravascular hemolysis. Therefore, therapeutic strategies in addition to the use of eculizumab are needed for those patients developing C3 fragment-mediated extravascular hemolysis because they continue to require red cell transfusions.

This Example describes methods to assess the effect of MASP-2- and MASP-3-deficient serum on lysis of red blood cells from blood samples obtained from a mouse model of PNH and demonstrates the efficacy of MASP-2 inhibition and/or MASP-3 inhibition to treat subjects suffering from PNH, and also supports the use of inhibitors of MASP-2 and/or inhibitors of MASP-3 (including dual or bispecific MASP-2/MASP-3 inhibitors) to ameliorate the effects of C3 fragment-mediated extravascular hemolysis in PNH subjects undergoing therapy with a C5 inhibitor such as eculizumab.

### Methods:

### PNH animal model:

Blood samples were obtained from gene-targeted mice with deficiencies of Crry and C3 (Crry/C3-/-) and CD55/CD59-deficient mice. These mice are missing the respective surface complement regulators on their erythrocytes and these erythrocytes are, therefore, susceptible to spontaneous complement autolysis as are PNH human blood cells.

In order to sensitize these erythrocytes even more, these cells were used with and without coating by mannan and then tested for hemolysis in WT C56/BL6 plasma, MBL null plasma, MASP-2 -/- plasma, MASP-1/3 -/- plasma, human NHS, human MBL -/plasma, and NHS treated with human MASP-2 antibody.

### 1. Hemolysis assay of Crry/C3 and CD55/CD59 double-deficient murine erythrocytes in MASP-2-deficient/depleted sera and controls

### Day 1. Preparation of murine RBC (± mannan coating).

Materials included: fresh mouse blood, BBS/Mg^{++/} Ca⁺⁺ (4.4 mM barbituric acid, 1.8 mM sodium barbitone, 145 mM NaCl, pH 7.4, 5mM Mg⁺⁺, 5mM Ca⁺⁺), chromium chloride, CrCl₃·6H₂O (0.5mg/mL in BBS/Mg⁺⁺/ Ca⁺⁺) and mannan, 100 µg/mL in BBS/Mg⁺⁺/Ca⁺⁺.

Whole blood (2 mL) was spun down for 1-2 min at 2000xg in a refrigerated centrifuge at 4°C. The plasma and buffy coat were aspirated off. The sample was then washed 3x by re-suspending RBC pellet in 2 mL ice-cold BBS/gelatin/Mg⁺⁺/Ca⁺⁺ and repeating centrifugation step. After the third wash, the pellet was re-suspended in 4 mL BBS/Mg⁺⁺/Ca⁺⁺. A 2 mL aliquot of the RBC was set aside as an uncoated control. To the remaining 2 mL, 2 mL CrCl3 and 2 mL mannan were added and the sample was incubated with gentle mixing at RT for 5min. The reaction was terminated by adding 7.5 mL BBS/gelatin/Mg⁺⁺/Ca⁺⁺. The sample was spun down as above, re-suspended in 2 mL BBS/gelatin/Mg⁺⁺/Ca⁺⁺ and washed a further two times as above, then stored at 4°C.

### Day 2. Hemolysis assay

Materials included BBS/gelatin/Mg⁺⁺/Ca⁺⁺ (as above), test sera, 96-well roundbottomed and flat-bottomed plates and a spectrophotometer that reads 96-well plates at 410-414 nm.

The concentration of the RBC was first determined and the cells were adjusted to 10⁹/mL, and stored at this concentration. Before use, the cells were diluted in assay buffer to 10⁸/mL, and then 100 ul per well was used. Hemolysis was measured at 410-414 nm (allowing for greater sensitivity than 541nm). Dilutions of test sera were prepared in ice-cold BBS/gelatin/Mg⁺⁺/Ca⁺⁺. 100µl of each serum dilution was pipetted into roundbottomed plate. 100 µl of appropriately diluted RBC preparation was added (i.e., 10⁸/mL), incubated at 37°C for about 1 hour, and observed for lysis. (The plates may be photographed at this point.) The plate was then spun down at maximum speed for 5 minutes. 100 µl of the fluid phase was aspirated, transferred to flat-bottom plates, and the OD was recorded at 410-414 nm. The RBC pellets were retained (these can be subsequently lysed with water to obtain an inverse result).

### Experiment #1

Fresh blood was obtained from CD55/CD59 double-deficient mice and blood of Crry/C3 double-deficient mice and erythrocytes were prepared as described in detail in the above protocol. The cells were split and half of the cells were coated with mannan and the other half were left untreated, adjusting the final concentration to 10⁸/mL, of which 100 µl was used in the hemolysis assay, which was carried out as described above.

### Results of Experiment #1: The lectin pathway is involved in erythrocyte lysis in the PNH animal model

In an initial experiment, it was determined that non-coated WT mouse erythrocytes were not lysed in any mouse serum. It was further determined that mannan-coated Crry-/- mouse erythrocytes were slowly lysed (more than 3 hours at 37 degrees) in WT mouse serum, but they were not lysed in MBL null serum. (Data not shown).

It was determined that mannan-coated Crry-/- mouse erythrocytes were rapidly lysed in human serum but not in heat-inactivated NHS. Importantly, mannan-coated Crry-/- mouse erythrocytes were lysed in NHS diluted down to 1/640 (i.e., 1/40, 1/80, 1/160, 1/320 and 1/640 dilutions all lysed). (Data not shown). In this dilution, the alternative pathway does not work (AP functional activity is significantly reduced below 8% serum concentration).

### Conclusions from Experiment #1

Mannan-coated Crry-/- mouse erythrocytes are very well lysed in highly diluted human serum with MBL but not in that without MBL. The efficient lysis in every serum concentration tested implies that the alternative pathway is not involved or needed for this lysis. The inability of MBL-deficient mouse serum and human serum to lyse the mannan-coated Crry-/- mouse erythrocytes indicates that the classical pathway also has nothing to do with the lysis observed. As lectin pathway recognition molecules are required (i.e., MBL), this lysis is mediated by the lectin pathway.

### Experiment #2

Fresh blood was obtained from the Crry/C3 and CD55/CD59 double-deficient mice and mannan-coated Crry-/- mouse erythrocytes were analyzed in the haemolysis assay as described above in the presence of the following human serum: MASP-3 -/-; MBL null; WT; NHS pretreated with human MASP-2 antibody; and heat-inactivated NHS as a control.

### Results of Experiment #2: MAST-2 inhibitors and MASP-3 deficiency prevents erythrocyte lysis in PNH animal model

With the mannan-coated Crry-/- mouse erythrocytes, NHS was incubated in the dilutions diluted down to 1/640 (i.e., 1/40, 1/80, 1/160, 1/320 and 1/640), human MBL-/- serum, human MASP-3-deficient serum (from 3MC patient), and NHS pretreated with MASP-2 mAb, and heat-inactivated NHS as a control.

The ELISA microtiter plate was spun down and the non-lysed erythrocytes were collected on the bottom of the round-well plate. The supernatant of each well was collected and the amount of hemoglobin released from the lysed erythrocytes was measured by reading the OD415 nm in an ELISA reader.

It was observed that MASP-3-/- serum did not lyse mannan-coated mouse erythrocytes at all. In the control heat-inactivated NHS (negative control), as expected, no lysis was observed. MBL-/- human serum lysed mannan-coated mouse erythrocytes at 1/8 and 1/16 dilutions. MASP-2-antibody-pretreated NHS lysed mannan-coated mouse erythrocytes at 1/8 and 1/16 dilutions while WT human serum lysed mannan-coated mouse erythrocytes down to dilutions of 1/32.

**FIGURE 21** graphically illustrates hemolysis (as measured by hemoglobin release of lysed mouse erythrocytes (Crry/C3-/-) into the supernatant measured by photometry) of mannan-coated murine erythrocytes by human serum over a range of serum dilutions in serum from MASP-3-/-, heat-inactivated (HI) NHS, MBL-/-, NHS pretreated with MASP-2 antibody, and NHS control.

**FIGURE 22** graphically illustrates hemolysis (as measured by hemoglobin release of lysed mouse erythrocytes (Crry/C3-/-) into the supernatant measured by photometry) of mannan-coated murine erythrocytes by human serum over a range of serum concentration in serum from MASP-3-/-, heat-inactivated (HI) NHS, MBL-/-, NHS pretreated with MASP-2 antibody, and NHS control.

From the results shown in **FIGURE 21** and **22****,** it is demonstrated that inhibiting MASP-3 will prevent any complement-mediated lysis of sensitized erythrocytes with deficient protection from autologous complement activation. MASP-2 inhibition with MASP-2 antibody significantly shifted the CH₅₀ and was protective to some extent, but MASP-3 inhibition was more effective.

### Experiment #3

Non-coated Crry-/- mouse erythrocytes obtained from fresh blood from the Crry/C3 and CD55/CD59 double-deficient mice were analyzed in the hemolysis assay as described above in the presence of the following sera: MASP-3-/-; MBL-/-; WT; NHS pretreated with human MASP-2 antibody, and heat-inactivated NHS as a control.

### Results:

**FIGURE 23** graphically illustrates hemolysis (as measured by hemoglobin release of lysed WT mouse erythrocytes into the supernatant measured by photometry) of non-coated murine erythrocytes over a range of serum concentrations in human sera from a 3MC (MASP-3-/-) patient, heat inactivated (HI) NHS, MBL-/-, NHS pretreated with MASP-2 antibody, and NHS control. As shown in **FIGURE 23** and summarized in **TABLE 12,** it is demonstrated that inhibiting MASP-3 inhibits complement-mediated lysis of non-sensitized WT mouse erythrocytes.

**FIGURE 24** graphically illustrates hemolysis (as measured by hemoglobin release of lysed mouse erythrocytes (CD55/59 -/-) into the supernatant measured by photometry) of non-coated murine erythrocytes by human serum over a range of serum concentrations in human sera from heat-inactivated (HI) NHS, MBL-/-, NHS pretreated with MASP-2 antibody, and NHS control. As shown in **FIGURE 24** and summarized in **TABLE 12,** it is demonstrated that inhibiting MASP-2 was protective to a limited extent.

**TABLE 12: CH₅₀ values expressed as serum concentrations**

| **Serum** | **WT** | **CD55/59 -/-** |
|---|---|---|
| 3MC patient | No lysis | No lysis |
| Heat-inactivated NHS | No lysis | No lysis |
| MBL AO/XX donor (MBL deficient) | 7.2% | 2.1% |
| NHS + MASP-2 antibody | 5.4% | 1.5% |
| NHS | 3.1% | 0.73% |

| | | |
|---|---|---|
| Note: "CH₅₀" is the point at which complement-mediated hemolysis reachs 50%. | | |

In summary, the results in this Example demonstrate that inhibiting MASP-3 prevents any complement lysis of sensitized and non-sensitized erythrocytes with deficient protection from autologous complement activation. MASP-2 inhibition also is protective to some extent. Therefore, MASP-2 and MASP-3 inhibitors alone or in combination (*i.e*., co-administered, administered sequentially) or MASP-2/MASP-3 bispecific or dual inhibitors may be used to treat subjects suffering from PNH, and may also be used to ameliorate (*i.e.,* inhibit, prevent or reduce the severity of) extravascular hemolysis in PNH patients undergoing treatment with a C5 inhibitor such as eculizumab (Soliris®).

### EXAMPLE 6

This Example describes a hemolysis assay testing mannan-coated rabbit erythrocytes for lysis in the presence of WT or MASP-1/3-/- mouse sera.

### Methods:

### 1. Hemolysis assay of rabbit RBC (mannan coated) in mouse MASP-1/3-deficient sera and WT control sera

### Day 1. Preparation of rabbit RBC.

Materials included: fresh rabbit blood, BBS/ Mg⁺⁺/Ca⁺⁺ (4.4 mM barbituric acid, 1.8 mM sodium barbitone, 145 mM NaCl, pH 7.4, 5 mM Mg⁺⁺, 5 mM Ca⁺⁺), BBS/ Mg⁺⁺/Ca⁺⁺with 0.1% gelatin, chromium chloride contained in buffer; i.e., CrCl₃.6 H₂O (0.5 mg /mL in BBS/ Mg⁺⁺/Ca⁺⁺) and mannan, 100 µg/mL in BBS/ Mg⁺⁺/Ca⁺⁺.
1. Rabbit whole blood (2 mL) was split into two 1.5 mL eppendorf tubes and centrifuged for 3 minutes at 8000 rpm (approximately 5.9 rcf) in a refrigerated eppendorf centrifuge at 4°C. The RBC pellet was washed three times after re-suspending in ice-cold BBS/Mg⁺⁺/Ca⁺⁺. After the third wash, the pellet was re-suspended in 4 mL BBS/Mg⁺⁺/Ca⁺⁺. Two mL of this aliquot were added to a 15-mL falcon tube to be used as the uncoated control. The remaining 2 mL of the RBCs aliquot were diluted in 2 mL of CrCl₃ buffer, 2 mL of the mannan solution were added and the suspension was incubated at room temperature for 5 minutes with gentle mixing. The reaction was terminated by adding 7.5 mL of BBS/0.1% gelatin/Mg⁺⁺/Ca⁺⁺ to the mixture. The erythrocytes were pelleted and the RBCs were washed twice with BBS/0.1% gelatin/Mg⁺⁺/Ca⁺⁺ as described above. The RBCs suspension was stored in BBS/0.1% gelatin/ Mg⁺⁺/Ca⁺⁺ at 4°C.
2. 100 µl of suspended RBCs were diluted with 1.4 mL water and spun down at 8000 rpm (approximately 5.9 rcf) for 3 minutes and the OD of the supernatant was adjusted to 0.7 at 541nm (an OD of 0.7 at 541nm corresponds to approximately 10⁹ erythrocytes/mL).
3. The re-suspended RBCs were diluted with BBS/0.1% gelatin/Mg⁺⁺/Ca⁺⁺ to a concentration of 10⁸ /mL.
4. Dilutions of the test sera were prepared in ice-cold BBS/gelatin/ Mg⁺⁺/Ca⁺⁺ and 100 µl of each serum dilution were pipetted into the corresponding well of round-bottom plate. 100 µl of appropriately diluted RBC (108/mL) were added to each well. As a control for complete lysis, purified water (100 µL) was mixed with the diluted RBC (100 µL) to cause 100% lysis, while BBS/0.1% gelatin/ Mg⁺⁺/Ca⁺⁺ without serum (100 µL) was used as a negative control. The plate was then incubated for 1 hour at 37°C.
5. The round-bottom plate was centrifuged at 3250 rpm for 5 minutes. The supernatant from each well (100 µL) was transferred into the corresponding wells of a flat-bottom plate and OD was read in an ELISA reader at 415-490nm. Results are reported as the ratio of the OD at 415 nm to that at 49 0nm.

### Results:

**FIGURE 25** graphically illustrates hemolysis (as measured by hemoglobin release of lysed rabbit erythrocytes into the supernatant measured by photometry) of mannan-coated rabbit erythrocytes by mouse serum over a range of serum concentrations in serum from MASP-1/3-/- and WT control. As shown in **FIGURE 25****,** it is demonstrated that inhibiting MASP-3 prevents complement-mediated lysis of mannan-coated WT rabbit erythrocytes. These results further support the use of MASP-3 inhibitors for the treatment of one or more aspects of PNH as described in Example 5.

### EXAMPLE 7

This Example demonstrates that the alternative pathway is activated in factor D-deficient serum in the presence of Ca⁺⁺.

### Experiment #1: C3b deposition assay under alternative pathway specific conditions

### Methods:

A C3b deposition assay on a zymosan-coated microtiter plate was carried out under alternative pathway-specific conditions (BBS/EGTA/Mg⁺⁺, no Ca⁺⁺) using increasing dilutions of the following mouse sera: factor D-/-; MASP- -/-; and WT.

### Results:

**FIGURE 26** graphically illustrates the level of C3b deposition (OD 405 nm) as a function of serum concentration in serum samples from factor D-/-, MASP-2 -/-; and WT mice sera in a C3 deposition assay carried out under alternative pathway specific conditions. As shown in **FIGURE 26****,** under these conditions, factor D -/- mouse serum does not activate C3 at all and the alternative pathway is not working. MASP-2-/- serum shows alternative pathway activation at a similar rate as WT serum. These results confirm that, in the absence of Ca⁺⁺, factor D is required for C3b deposition. This is consistent with the evidence that MASP-3 cannot be converted into its enzymatically active form under these conditions because the interactions of MASP-1, the MASP-3 activating enzyme, and MASP-3 with their respective carbohydrate recognition components is Ca⁺⁺-dependent.

### Experiment #2: C3b deposition assay under physiological conditions Methods:

A C3b deposition assay was carried under physiological conditions (BBS/Ca⁺⁺/Mg⁺⁺) (allowing both the LP and AP to function) using increasing dilutions of the following mouse sera: factor D -/-; MASP-2 -/-; and WT.

### Results:

**FIGURE 27** graphically illustrates the level of C3b deposition (OD 405 nm) as a function of serum concentration using samples of sera from factor D-/-; MASP-2-/-; and WT mice in a C3b deposition assay carried out under physiological conditions (in the presence of Ca⁺⁺). As shown in **FIGURE 27****,** factor D-/- mouse serum activates C3 via both the lectin and the alternative pathway with no difference as compared to WT serum through the serum dilutions indicated. MASP- -/- serum shows the turnover of C3 in lower serum dilutions by the alternative pathway only (i.e., MASP-3-driven alternative pathway activation). These results indicate that in the presence of Ca⁺⁺, factor D is not required given that MASP-3 can drive alternative pathway activity.

### Experiment #3: C3b Deposition Assay Using Mouse Sera deficient in factor B or factor D in the presence or absence of MASP-2 mAb

### Methods:

A C3b deposition assay was carried out under physiological conditions (BBS/Ca⁺⁺/Mg⁺⁺) on mannan coated microtiter plates as follows:
1. Micro-titer ELISA plates were coated overnight at 4°C with mannan (1 µg/mL) in coating buffer (15 mM Na₂CO₃, 35 mM NaHCo₃, 0.02% sodium azide, pH 9.6).
2. The next day, residual protein binding sites were blocked for 2 hours at room temperature with 250 µl/well with 0.1 % HSA in BBS (4 mM barbital, 145 mM NaCl, 2 mM CaCl₂, 1 mM MgCl₂, pH 7.4).
3. Plates were washed three times with wash buffer (TBS with 0.05% Tween 20 and 5 mM CaCl₂).
4. 1:10 diluted serum samples in BBS were added to the wells at the specified time points. Wells receiving only buffer were used as negative controls. The plate was incubated at 37°C for up to 40 minutes.
5. The plates were then washed 3 times with the wash buffer.
6. Then 100 µl of rabbit anti-human C3c (Dako) diluted 1:5000 in washing buffer was added to the wells and plates were incubated for 90 minutes at 37°C.
7. After being washed for three times with washing buffer, 100 µl of alkaline phosphatase-conjugated anti-rabbit diluted 1:5000 in washing buffer was added to the wells followed by incubation for 90 minutes at room temperature.
8. After washing, alkaline phosphatase was detected by adding 100 µl of substrate solution.
9. After incubation for 15 minutes, the optical density was measured at OD 405 nm.

### Results:

**FIGURE 28** graphically illustrates the level of C3b deposition (OD 405 nm) as a function of serum incubation time (minutes) in mouse serum samples obtained from factor D-/- or factor B-/- mice in the presence or absence of MASP-2 mAb in a C3b deposition assay carried out under physiological conditions (in the presence of Ca⁺⁺). As shown in **FIGURE 28****,** there is no difference in the amount of C3b deposition in WT and factor D/- serum, providing strong support for the conclusion that MASP-3 can initiate alternative pathway activation, even in the absence of factor D. The observed signal is thought to be due to both lectin pathway and alternative pathway activation. As further shown in **FIGURE 28****,** factor D-/- plus MASP-2 mAb shows MASP-3-mediated alternative pathway activation only. The factor B-/- plus MASP-2 mAb was background only (data not shown). Heat-inactivated serum was used as the background control value, which was identical to factor D-/- and factor B-/- with MASP-2 (data not shown).

In summary, the results in this Example demonstrate that factor D is only essential under non-physiological conditions (i.e. when testing for alternative pathway activation in BBS/EGTA/ Mg⁺⁺ in the absence of Ca⁺⁺). In contrast, when testing for alternative pathway activation under physiological conditions (in the presence of Ca⁺⁺), which allows the alternative pathway to be activated via MASP-3, factor D-deficient serum is not at all deficient in alternative pathway activity as compared to the WT control. Therefore, under physiological conditions, factor D is redundant, in that the initiation of alternative pathway activation is driven by MASP-3. These results support the conclusion that the lectin pathway directs AP activation through a MASP-3-dependent activation event.

### EXAMPLE 8

This example describes exemplary methods for producing murine monoclonal antibodies against human MASP-1, MASP-2 or MASP-3 polypeptides, and for generating dual, bispecific or pan-specific MASP antibodies.

### 1. Methods for generating MASP antibodies

Male A/J mice (Harlan, Houston, Tex.), 8 to 12 weeks of age, are injected subcutaneously with 100 µg human full-length polypeptides: rMASP-1 (SEQ ID NO:10), rMASP-2 (SEQ ID NO:5) or rMASP-3 (SEQ ID NO:8), or antigen fragments thereof, for example as set forth in **TABLE 2,** in complete Freund's adjuvant (Difco Laboratories, Detroit, Mich.) in 200 µl of phosphate buffered saline (PBS) pH 7.4. Two weeks later, themice are injected subcutaneously with 50 µg of the same human polypeptide in incomplete Freund's adjuvant. At the sixth week, the mice are injected with 50 µg of the same human polypeptide in PBS and are fused 4 days later.

For each fusion, single-cell suspensions are prepared from the spleen of an immunized mouse and used for fusion with Sp2/0 myeloma cells. 5x10⁸ of the Sp2/0 and 5x10⁸ spleen cells are fused in a medium containing 50% polyethylene glycol (M.W. 1450) (Kodak, Rochester, N.Y.) and 5% dimethylsulfoxide (Sigma Chemical Co., St. Louis, Mo.). The cells are then adjusted to a concentration of 1.5x10⁵ spleen cells per 200 µl of the suspension in Iscove medium (Gibco, Grand Island, N.Y.), supplemented with 10% fetal bovine serum, 100 units/mL of penicillin, 100 µg/mL of streptomycin, 0.1 mM hypoxanthine, 0.4 µM aminopterin and 16 µM thymidine. Two hundred microliters of the cell suspension are added to each well contained in roughly twenty 96-well microculture plates. After about ten days, culture supernatants are withdrawn for screening for reactivity with the target purified antigen (MASP-1, MASP-2 or MASP-3, or the antigen fragment from **TABLE 2)** in an ELISA assay.

**ELISA Assay** (described with reference to MASP-2): Wells of Immulon 2 (Dynatech Laboratories, Chantilly, Va.) microtest plates are coated by adding 50 µl of purified hMASP-2 at 50 ng/mL overnight at room temperature. The low concentration of MASP-2 used for coating enables the selection of high-affinity antibodies. After the coating solution is removed by flicking the plate, 200 µl of BLOTTO (non-fat dry milk) in PBS is added to each well for one hour to block the non-specific sites. An hour later, the wells are then washed with a buffer PBST (PBS containing 0.05% Tween 20). The culture supernatants from each fusion well (50 uL) are mixed with 50 µl of BLOTTO and then added to individual MASP-2-coated wells of the microtest plates. After one hour of incubation, the wells are washed with PBST and antibody binding to MASP-2 is detected by adding horseradish peroxidase (HRP)-conjugated goat anti-mouse IgG (Fc specific) (Jackson ImmunoResearch Laboratories, West Grove, Pa.). The HRP-conjugated anti-mouse IgG is diluted appropriately in BLOTTO to provide an appropriate signal to noise ratio, and added to each sample-containing well. After washing, the bound HRP-conjugated antibody is detected with the peroxidase substrate solution. Peroxidase substrate solution containing 0.1% 3,3,5,5 tetramethyl benzidine (Sigma, St. Louis, Mo.) and 0.0003% hydrogen peroxide (Sigma) is added to the wells for color development for 30 minutes. The reaction is terminated by addition of 50 µl of 2M H₂SO₄ per well and the optical density at 450 nm of the reaction mixture is measured with a BioTek ELISA Reader (BioTek Instruments, Winooski, Vt.).

**Binding Assay** (described with reference to MASP-2):
Culture supernatants that test positive in the MASP-2 ELISA assay described above can be tested in a binding assay to determine the binding affinity that the MASP-2 inhibitory agents have for MASP-2. A similar assay can also be used to determine if the inhibitory agents bind to other antigens in the complement system.

Polystyrene microtiter plate wells (96-well medium binding plates, Coming Costar, Cambridge, MA) are coated with MASP-2 (20 ng/100 µl/well, Advanced Research Technology, San Diego, CA) in phosphate-buffered saline (PBS) pH 7.4 overnight at 4°C. After aspirating the MASP-2 solution, wells are blocked with PBS containing 1% bovine serum albumin (BSA; Sigma Chemical) for 2 hours at room temperature. Wells without MASP-2 coating serve as the background controls. Aliquots of hybridoma supernatants or purified MASP-2 MoAbs, at varying concentrations in BSA PBS blocking solution, are added to the wells. Following a two-hour incubation at room temperature, the wells are extensively rinsed with PBS. MASP-2-bound MASP-2 MoAb is detected by the addition of peroxidase-conjugated goat anti-mouse IgG (Sigma Chemical) in blocking solution, which is allowed to incubate for 1hour at room temperature. The plate is rinsed again thoroughly with PBS, and 100 µl of 3,3',5,5'-tetramethyl benzidine (TMB) substrate (Kirkegaard and Perry Laboratories, Gaithersburg, MD) is added. The reaction of TMB is quenched by the addition of 100 µl of 1M phosphoric acid, and the plate is read at 450 nm in a microplate reader (SPECTRA MAX 250, Molecular Devices, Sunnyvale, CA).

The culture supernatants from the positive wells are then tested for the ability to inhibit complement activation in a functional assay such as the C4 cleavage assay as described herein (Example 9). The cells in positive wells are then cloned by limiting dilution. The MoAbs are tested again for reactivity with hMASP-2 in an ELISA assay as described above. The selected hybridomas are grown in spinner flasks and the spent culture supernatant collected for antibody purification by protein A affinity chromatography.

MASP-2 antibodies may be assayed for LEA-2 inhibitory activity in a C4 cleavage assay, for example as described in Example 9.

While the ELISA and Binding Assay above are described with reference to MASP-2, it will be understood by those of skill in the art that the same ELISA and binding assays may be carried out using MASP-1 or MASP-3 polypeptides and antigen fragments thereof (e.g., as described in **TABLE 2).** MASP-3 antibodies may be assayed for inhibition of MASP-3 serine protease cleavage of a MASP-3 substrate and for LEA-1 inhibitory activity in a C3b deposition assay, for example as described in Example 4, in a hemolysis assay as described in Example 5. MASP-1 antibodies may be assayed for inhibition of MASP-1 serine protease cleavage of a MASP-1 substrate, for inhibition of MASP-3 activation, and for LEA-1 inhibitory activity in a C3b deposition assay, for example as described in Example 4, and in a hemolysis assay as described in Example 5.

### 2. Methods for Generating Dual-MASP antibodies

MASP-2/3 dual inhibitory antibodies: As shown in **FIGURES 4****,** **6** and **7C****,** there are regions conserved between MASP-2 and MASP-3 in the serine protease domain, encoded by the beta chain of SEQ ID NO:5 and SEQ ID NO:8. Therefore, a dual MASP-2/3 antibody can be generated using an antigen comprising or consisting of the serine protease domain of MASP-2 (or MASP-3), such as the beta chain of SEQ ID NO:5 (or SEQ ID NO:8) to generate a monoclonal antibody as described above, or alternatively, these antigen(s) may be used to screen a phage library for clones that specifically bind to these antigen(s), followed by screening for dual binding to MASP-3 (or MASP-1). The dual MASP-2/3 antibodies are then screened for inhibitory activity in a functional assay, for example as described in **TABLE 2.**

MASP-1/3 dual inhibitory antibodies: As shown in **FIGURES 3-5****,** MASP-1 and MASP-3 share an identical conserved region in the CUBI-CCP2 domain (aa 25-432 of SEQ ID NO:10), which is also shared by MAp44. As shown in **FIGURE 3****,** MAp44 does not contain a CCP2 domain. Therefore, a dual MASP-1/3 antibody inclusive of MAp44 is generated using an antigen comprising or consisting of the CUBI-CCP-2 domain of MASP-1 (or MASP-3) to generate a monoclonal antibody as described above, or alternatively, this antigen is used to screen a phage library for clones that specifically bind to this antigen, followed by screening for dual binding to MASP-3 (or MASP-1). A dual MASP-1/3 antibody exclusive of MAp44 is generated in a similar manner, using an antigen comprising or consisting of the CCP2 domain of MASP-1 (or MASP-3). The dual MASP-1/3 antibodies are then screened for inhibitory activity in a functional assay, for example as described in **TABLE 2.**

MASP-1/2 dual inhibitory antibodies: As shown in **FIGURES 4****,** **6** and **7A**, the serine protease domain of MASP-1 and MASP-2 contains regions that are conserved. Therefore, a dual MASP-1/2 antibody is generated using an antigen comprising or consisting of the serine protease domain of MASP-1 (or MASP-2) to generate a monoclonal antibody as described above, or alternatively, this antigen is used to screen a phage library for clones that specifically bind to this antigen, followed by screening for dual binding to MASP-2 (or MASP-1). The dual MASP-1/2 antibodies are then screened for inhibitory activity in a functional assay, for example as described in **TABLE 2.**

### 3. Methods for Generating Pan-specific MASP antibodies:

Alpha Chain: Numerous patches of identity between MASP-2 and MASP-1/3 suggest that it may be possible to generate monoclonal antibodies that bind MASP-1/3 and MASP-2. In particular, most of the identity lies within the CUB1-EGF-CUB2 domains, as shown in **FIGURE 5****.** The various domains illustrated in **FIGURE 5** were identified according to Yongqing, et al., Biochemica et Biophysica Acta 1824:253-262(2012); Teillet et al., J. Biol. Chem. 283:25715-25724 (2008); and Marchler-Bauer et al., Nucleic Acids Res. 39:D225-229(2011).

Beta Chain: Numerous patches of identity between MASP-2 and MASP-1/3, as shown in **FIGURE 6****,** would allow for the generation of a pan-specific MASP-1/2/3 inhibitor.

### Methods:

Pan-specific MASP inhibitory antibodies (*i.e.,* antibodies that inhibit MASP-1, 2 and 3 activity) are generated as follows:
1. Screen a library against MASP-1/3 and MASP-2 Alpha-chain CUB1-EGF-CUB2 domains and select clones that cross-react to both MASP-1/3 and MASP-2.
2. Screen the clones for the ability to inhibit functional activity, for example as described in **TABLE 2.**
3. Use the DTLacO affinity/functionality maturation technology (Yabuki et al., PLoS ONE, 7(4):e36032 (2012)) to optimize both binding to all three proteins and inhibitory function.
4. As described in **TABLE 2,** pan-MASP inhibitors can be used to inhibit LEA-1- and LEA-2-mediated complement activation.

### 4. Methods for generating bispecific MASP-2/3 antibodies

Bispecific MASP-2/3 inhibitory antibodies are generated as follows:
1. Exemplary MASP-2 specific inhibitory antibodies that bind to the CCP1 domain and inhibit MASP-2-dependent complement activation have been identified, as described in Examples 11-14.
2. A MASP-3 specific inhibitory antibody is generated by screening a library against the MASP-3 polypeptide and identifying MASP-3 antibodies, as described in Example 15, followed by assaying the antibodies for LEA-1 inhibitory activity in a functional assay, for example as described in **TABLE 2.** Exemplary MASP-3 antibodies are described in Example 15.
3. The antigen binding region specific for MASP-2 and MASP-3 are cloned into a framework to generate a bi-specific antibody. Numerous bispecific antibody formats have been described, including immunoglobulin G-like formats as well as various fusion protein and single chain variable fragment configurations (Holmes, Nature Reviews, Drug Discovery 10:798-800 (2011), Müller and Kontermann, Biodrugs 24:89-98 (2010)). In one example, bispecific antibodies can be generated by fusing two hybridomas expressing antibodies against two distinct antigens, resulting in various heavy and light chain pairings, a percentage of which comprise a heavy and light chain specific for one antigen paired with a heavy and light chain specific for the other antigen (Milstein and Cuello, Mature 305:537-539 (1983)). A similar bispecific antibody may be generated recombinantly, by co-expressing two immunoglobulin heavy-chain/light-chain pairs, where the two heavy chains have different specificities Antibody variable domains with the desired binding specificities (antibody-antigen combining sites) (e.g. MASP-2 antibodies as described in Examples 11-14, MASP-3 antibodies as described in Example 15) can be fused to immunoglobulin constant domain sequences. The fusion preferably is with an immunoglobulin heavy-chain constant domain, including at least part of the hinge, C_{H}2, and C_{H}3 regions. DNAs encoding the immunoglobulin heavy-chain fusions and, if desired, the immunoglobulin light chain, are inserted into separate expression vectors, and are co-transfected into a suitable host organism.

In addition to paired immunoglobulin heavy and light chains, linkage of single chain variable fragments specific for two different targets is exemplified in Kipriyanov et al., J. Mol. Biol. 293:41 (1999)). In this example, a single polynucleotide expression construct is designed to encode two pairs of heavy and light chain variable regions separated by linker peptides, with each pair imparting specificity for a distinct protein target. Upon expression, the polypeptide assembles in a configuration in which the heavy and light chain pair specific for one target forms one antigen-binding surface of the protein, and the other pair forms a separate antigen-binding surface, creating a molecule termed a single chain diabody. Depending on the length of the linker between the central heavy and light chain variable region pair, the polypeptide can also be forced to dimerize, resulting in the formation of a tandem diabody.

For example, DNA encoding the following immunoglobulin polypeptides may be inserted into one or more vectors and expressed in a suitable host organism to generate the following illustrative, non-limiting examples of a bi-specific antibodies.

### MASP-2/3 bispecific antibodies

In one case, a bispecific antibody is provided that binds human MASP-2 and human MASP-3 and comprises:
(I) a MASP-2 specific binding region comprising at least one or more of the following:a) a heavy chain variable region comprising: i) a heavy chain CDR1 comprising the amino acid sequence from 31-35 of SEQ ID NO:21; and ii) a heavy chain CDR2 comprising the amino acid sequence from 50-65 of SEQ ID NO:21; and iii) a heavy chain CDR3 comprising the amino acid sequence from 95-102 of SEQ ID NO:21; and/or at least one or more of the following:b) a light chain variable region comprising: i) a light chain CDR1 comprising the amino acid sequence from 24-34 of either SEQ ID NO:25 or SEQ ID NO:27; and ii) a light chain CDR2 comprising the amino acid sequence from 50-56 of either SEQ ID NO:25 or SEQ ID NO:27; and iii) a light chain CDR3 comprising the amino acid sequence from 89-97 of either SEQ ID NO:25 or SEQ ID NO:27; and
(II) a MASP-3 specific binding region, optionally a MASP-3 specific binding region comprising at least one of a) a heavy chain variable region comprising: i) a heavy chain CDR1 comprising the amino acid sequence from 31-35 of SEQ ID NO:25 or SEQ ID NO:26; and ii) a heavy chain CDR2 comprising the amino acid sequence from 50-65 of SEQ ID NO:25 or SEQ ID NO:26; and iii) a heavy chain CDR3 comprising the amino acid sequence from 95-102 of SEQ ID NO:25 or SEQ ID NO:26; and
   b) a light chain variable region comprising: i) a light chain CDR1 comprising the amino acid sequence from 24-34 of either SEQ ID NO:28 or SEQ ID NO:29; and ii) a light chain CDR2 comprising the amino acid sequence from 50-56 of either SEQ ID NO:28 or SEQ ID NO:29; and iii) a light chain CDR3 comprising the amino acid sequence from 89-97 of either SEQ ID NO:28 or SEQ ID NO:29.

### MASP-1/2 bispecific antibodies

In one case, a bispecific antibody is provided that binds human MASP-1 and human MASP-2 and comprises:
(I) a MASP-2 specific binding region comprising at least one or more of the following:a) a heavy-chain variable region comprising: i) a heavy-chain CDR1 comprising the amino acid sequence from 31-35 of SEQ ID NO:21; and ii) a heavy-chain CDR2 comprising the amino acid sequence from 50-65 of SEQ ID NO:21; and iii) a heavy-chain CDR3 comprising the amino acid sequence from 95-102 of SEQ ID NO:21; and/or at least one or more of the following:b) a light-chain variable region comprising: i) a light-chain CDR1 comprising the amino acid sequence from 24-34 of either SEQ ID NO:25 or SEQ ID NO:27; and ii) a light chain CDR2 comprising the amino acid sequence from 50-56 of either SEQ ID NO:25 or SEQ ID NO:27; and iii) a light-chain CDR3 comprising the amino acid sequence from 89-97 of either SEQ ID NO:25 or SEQ ID NO:27; and
(II) a MASP-1 specific binding region.
   4. Testing for functional inhibitory activity against MASP-2 and/or MASP-3 is carried out, for example as described in **TABLE 2,** and as further described herein.

### EXAMPLE 9

This example describes an *in vitro* C4 cleavage assay used as a functional screen to identify MASP-2 inhibitory agents capable of blocking MASP-2-dependent complement activation via L-ficolin/P35, H-ficolin, M-ficolin or mannan.

**C4 Cleavage Assay**: A C4 cleavage assay has been described by Petersen, S.V., et al., J. Immunol. Methods 257:107, 2001, which measures lectin pathway activation resulting from lipoteichoic acid (LTA) on *S. aureus,* which binds to L-ficolin.

**Reagents:** Formalin-fixed *S. aureus* (DSM20233) is prepared as follows: bacteria is grown overnight at 37°C in tryptic soy blood medium, washed three times with PBS, then fixed for 1 hour at room temperature in PBS/0.5% formalin, and washed a further three times with PBS, before being resuspended in coating buffer (15 mM Na₂CO₃, 35 mM NaHCO₃, pH 9.6).

**Assay:** The wells of a Nunc MaxiSorb microtiter plate (Nalgene Nunc International, Rochester, NY) are coated with: 100 µl of formalin-fixed *S. aureus* DSM20233 (OD₅₅₀ = 0.5) in coating buffer with 1 µg of L-ficolin in coating buffer. After overnight incubation, wells are blocked with 0.1% human serum albumin (HSA) in TBS (10 mM Tris-HCl, 140 mM NaCl, pH 7.4), then are washed with TBS containing 0.05% Tween 20 and 5 mM CaCl₂ (wash buffer). Human serum samples are diluted in 20 mM Tris-HCl, 1 M NaCl, 10 mM CaCl₂, 0.05% Triton X-100, 0.1% HSA, pH 7.4, which prevents activation of endogenous C4 and dissociates the C1 complex (composed of C1q, C1r and C1s). MASP-2 inhibitory agents, including MASP-2 MoAbs, are added to the serum samples in varying concentrations. The diluted samples are added to the plate and incubated overnight at 4°C. After 24 hours, the plates are washed thoroughly with wash buffer, then 0.1 µg of purified human C4 (obtained as described in Dodds, A.W., Methods Enzymol. 223:46, 1993) in 100 µl of 4 mM barbital, 145 mM NaCl, 2 mM CaCl₂, 1 mM MgCl₂, pH 7.4 is added to each well. After 1.5 hours at 37°C, the plates are washed again and C4b deposition is detected using alkaline phosphatase-conjugated chicken anti-human C4c (obtained from Immunsystem, Uppsala, Sweden) and measured using the colorimetric substrate p-nitrophenyl phosphate.

**C4 Assay on mannan:** The assay described above is adapted to measure lectin pathway activation via MBL by coating the plate with LSP and mannan prior to adding serum mixed with various MASP-2 inhibitory agents.

**C4 assay on H-ficolin (Hakata Ag):** The assay described above is adapted to measure lectin pathway activation via H-ficolin by coating the plate with LPS and H-ficolin prior to adding serum mixed with various MASP-2 inhibitory agents.

### EXAMPLE 10

The following assay is used to test whether a MASP inhibitory agent blocks the classical pathway by analyzing the effect of a MASP inhibitory agent under conditions in which the classical pathway is initiated by immune complexes.

**Methods:** To test the effect of a MASP inhibitory agent on conditions of complement activation where the classical pathway is initiated by immune complexes, triplicate 50 µl samples containing 90% NHS are incubated at 37°C in the presence of 10 µg/mL immune complex or PBS, and parallel triplicate samples (+/- immune complexes) are also included containing 200 nM anti-properdin monoclonal antibody during the 37°C incubation. After a two-hour incubation at 37°C, 13 mM EDTA is added to all samples to stop further complement activation and the samples are immediately cooled to 5°C. The samples are then stored at -70°C prior to being assayed for complement activation products (C3a and sC5b-9) using ELISA kits (Quidel, Catalog Nos. A015 and A009) following the manufacturer's instructions.

### EXAMPLE 11

This example describes the identification of high-affinity MASP-2 Fab2 antibody fragments that block MASP-2 activity.

**Background and rationale:** MASP-2 is a complex protein with many separate functional domains, including: binding site(s) for MBL and ficolins, a serine protease catalytic site, a binding site for proteolytic substrate C2, a binding site for proteolytic substrate C4, a MASP-2 cleavage site for autoactivation of MASP-2 zymogen, and two Ca⁺⁺ binding sites. Fab2 antibody fragments were identified that bind with high affinity to MASP-2, and the identified Fab2 fragments were tested in a functional assay to determine if they were able to block MASP-2 functional activity.

To block MASP-2 functional activity, an antibody or Fab2 antibody fragment must bind and interfere with a structural epitope on MASP-2 that is required for MASP-2 functional activity. Therefore, many or all of the high-affinity binding MASP-2 Fab2s may not inhibit MASP-2 functional activity unless they bind to structural epitopes on MASP-2 that are directly involved in MASP-2 functional activity.

A functional assay that measures inhibition of lectin pathway C3 convertase formation was used to evaluate the "blocking activity" of MASP-2 Fab2s. It is known that the primary physiological role of MASP-2 in the lectin pathway is to generate the next functional component of the lectin-mediated complement pathway, namely the lectin pathway C3 convertase. The lectin pathway C3 convertase is a critical enzymatic complex (C4bC2a) that proteolytically cleaves C3 into C3a and C3b. MASP-2 is not a structural component of the lectin pathway C3 convertase (C4bC2a); however, MASP-2 functional activity is required in order to generate the two protein components (C4b, C2a) that comprise the lectin pathway C3 convertase. Furthermore, all of the separate functional activities of MASP-2 listed above appear to be required in order for MASP-2 to generate the lectin pathway C3 convertase. For these reasons, a preferred assay to use in evaluating the "blocking activity" of MASP-2 Fab2s is believed to be a functional assay that measures inhibition of lectin pathway C3 convertase formation.

**Generation of High Affinity Fab2s:** A phage display library of human variable light and heavy chain antibody sequences and automated antibody selection technology for identifying Fab2s that react with selected ligands of interest was used to create high-affinity Fab2s to rat MASP-2 protein (SEQ ID NO: 13). A known amount of rat MASP-2 (∼1 mg, >85% pure) protein was utilized for antibody screening. Three rounds of amplification were utilized for selection of the antibodies with the best affinity. Approximately 250 different hits expressing antibody fragments were picked for ELISA screening. High affinity hits were subsequently sequenced to determine uniqueness of the different antibodies.

Fifty unique MASP-2 antibodies were purified and 250 µg of each purified Fab2 antibody was used for characterization of MASP-2 binding affinity and complement pathway functional testing, as described in more detail below.

### Assays used to Evaluate the Inhibitory (blocking) Activity of MASP-2 Fab2s

### 1. Assay to Measure Inhibition of Formation of Lectin Pathway C3 Convertase:

Background: The lectin pathway C3 convertase is the enzymatic complex (C4bC2a) that proteolytically cleaves C3 into the two potent proinflammatory fragments, anaphylatoxin C3a and opsonic C3b. Formation of C3 convertase appears to be a key step in the lectin pathway in terms of mediating inflammation. MASP-2 is not a structural component of the lectin pathway C3 convertase (C4bC2a); therefore MASP-2 antibodies (or Fab2) will not directly inhibit activity of preexisting C3 convertase. However, MASP-2 serine protease activity is required in order to generate the two protein components (C4b, C2a) that comprise the lectin pathway C3 convertase. Therefore, MASP-2 Fab2, which inhibits MASP-2 functional activity (i.e., blocking MASP-2 Fab2) will inhibit *de novo* formation of lectin pathway C3 convertase. C3 contains an unusual and highly reactive thioester group as part of its structure. Upon cleavage of C3 by C3 convertase in this assay, the thioester group on C3b can form a covalent bond with hydroxyl or amino groups on macromolecules immobilized on the bottom of the plastic wells via ester or amide linkages, thus facilitating detection of C3b in the ELISA assay.

Yeast mannan is a known activator of the lectin pathway. In the following method to measure formation of C3 convertase, plastic wells coated with mannan were incubated for 30 minutes at 37°C with diluted rat serum to activate the lectin pathway. The wells were then washed and assayed for C3b immobilized onto the wells using standard ELISA methods. The amount of C3b generated in this assay is a direct reflection of the *de novo* formation of lectin pathway C3 convertase. MASP-2 Fab2s at selected concentrations were tested in this assay for their ability to inhibit C3 convertase formation and consequent C3b generation.

### Methods:

96-well Costar Medium Binding plates were incubated overnight at 5°C with mannan diluted in 50 mM carbonate buffer, pH 9.5 at 1 µg/50 µl/well. After overnight incubation, each well was washed three times with 200 µl PBS. The wells were then blocked with 100 µl/well of 1% bovine serum albumin in PBS and incubated for one hour at room temperature with gentle mixing. Each well was then washed three times with 200 µl of PBS. The MASP-2 Fab2 samples were diluted to selected concentrations in Ca⁺⁺ and Mg⁺⁺ containing GVB buffer (4.0 mM barbital, 141 mM NaCl, 1.0 mM MgCl₂, 2.0 mM CaCl₂, 0.1% gelatin, pH 7.4) at 5°C. A 0.5% rat serum was added to the above samples at 5°C and 100 µl was transferred to each well. Plates were covered and incubated for 30 minutes in a 37°C waterbath to allow complement activation. The reaction was stopped by transferring the plates from the 37°C waterbath to a container containing an ice-water mix. Each well was washed five times with 200 µl with PBS-Tween 20 (0.05% Tween 20 in PBS), then washed two times with 200 µl PBS. A 100 µl/well of 1:10,000 dilution of the primary antibody (rabbit anti-human C3c, DAKO A0062) was added in PBS containing 2.0 mg/mL bovine serum albumin and incubated 1 hour at room temperature with gentle mixing. Each well was washed 5 times with 200 µl PBS. 100 µl/well of 1:10,000 dilution of the secondary antibody (peroxidase-conjugated goat anti-rabbit IgG, American Qualex A102PU) was added in PBS containing 2.0 mg/mL bovine serum albumin and incubated for one hour at room temperature on a shaker with gentle mixing. Each well was washed five times with 200 µl with PBS. 100 µl/well of the peroxidase substrate TMB (Kirkegaard & Perry Laboratories) was added and incubated at room temperature for 10 minutes. The peroxidase reaction was stopped by adding 100 Tl/well of 1.0 M H₃PO₄ and the OD₄₅₀ was measured.

### 2. Assay to Measure Inhibition of MASP-2-dependent C4 Cleavage

Background: The serine protease activity of MASP-2 is highly specific and only two protein substrates for MASP-2 have been identified; C2 and C4. Cleavage of C4 generates C4a and C4b. MASP-2 Fab2 may bind to structural epitopes on MASP-2 that are directly involved in C4 cleavage (e.g., MASP-2 binding site for C4; MASP-2 serine protease catalytic site) and thereby inhibit the C4 cleavage functional activity of MASP-2.

Yeast mannan is a known activator of the lectin pathway. In the following method to measure the C4 cleavage activity of MASP-2, plastic wells coated with mannan were incubated for 30 minutes at 37°C with diluted rat serum to activate the lectin pathway. Since the primary antibody used in this ELISA assay only recognizes human C4, the diluted rat serum was also supplemented with human C4 (1.0 µg/mL). The wells were then washed and assayed for human C4b immobilized onto the wells using standard ELISA methods. The amount of C4b generated in this assay is a measure of MASP-2-dependent C4 cleavage activity. MASP-2 Fab2 at selected concentrations was tested in this assay for ability to inhibit C4 cleavage.

**Methods:** 96-well Costar Medium Binding plates were incubated overnight at 5°C with mannan diluted in 50 mM carbonate buffer, pH 9.5 at 1.0 Tg/50 µl/well. Each well was washed 3 times with 200 µl PBS. The wells were then blocked with 100 µl/well of 1% bovine serum albumin in PBS and incubated for one hour at room temperature with gentle mixing. Each well was washed 3 times with 200 µl of PBS. MASP-2 Fab2 samples were diluted to selected concentrations in Ca⁺⁺ and Mg⁺⁺ containing GVB buffer (4.0 mM barbital, 141 mM NaCl, 1.0 mM MgCl₂, 2.0 mM CaCl₂, 0.1% gelatin, pH 7.4) at 5°C. 1.0 µg/mL human C4 (Quidel) was also included in these samples. 0.5% rat serum was added to the above samples at 5°C and 100 µl was transferred to each well. The plates were covered and incubated for 30 minutes in a 37°C waterbath to allow complement activation. The reaction was stopped by transferring the plates from the 37°C waterbath to a container containing an ice-water mix. Each well was washed 5 times with 200 µl with PBS-Tween 20 (0.05% Tween 20 in PBS), then each well was washed with 2 times with 200 µl PBS. 100 µl/well of 1:700 dilution of biotin-conjugated chicken anti-human C4c (Immunsystem AB, Uppsala, Sweden) was added in PBS containing 2.0 mg/mL bovine serum albumin (BSA) and incubated one hour at room temperature with gentle mixing. Each well was washed 5 times with 200 µl PBS. 100 µl/well of 0.1 µg/mL of peroxidase-conjugated streptavidin (Pierce Chemical #21126) was added in PBS containing 2.0 mg/mL BSA and incubated for one hour at room temperature on a shaker with gentle mixing. Each well was washed 5 x 200 µl with PBS. 100 µl/well of the peroxidase substrate TMB (Kirkegaard & Perry Laboratories) was added and incubated at room temperature for 16 min. The peroxidase reaction was stopped by adding 100 µl/well of 1.0 M H₃PO₄ and the OD₄₅₀ was measured.

### 3. Binding Assay of anti-rat MASP-2 Fab2 to 'Native' rat MASP-2

Background: MASP-2 is usually present in plasma as a MASP-2 dimer complex that also includes specific lectin molecules (mannose-binding protein (MBL) and ficolins). Therefore, if one is interested in studying the binding of MASP-2 Fab2 to the physiologically relevant form of MASP-2, it is important to develop a binding assay in which the interaction between the Fab2 and 'native' MASP-2 in plasma, rather than purified recombinant MASP-2, is used. In this binding assay, the 'native' MASP-2-MBL complex from 10% rat serum was first immobilized onto mannan-coated wells. The binding affinity of various MASP-2 Fab2s to the immobilized 'native' MASP-2 was then studied using a standard ELISA methodology.

**Methods:** 96-well Costar High Binding plates were incubated overnight at 5°C with mannan diluted in 50 mM carbonate buffer, pH 9.5 at 1 µg/50 µl/well. Each well was washed 3 times with 200 µl PBS. The wells were blocked with 100 µl/well of 0.5% nonfat dry milk in PBST (PBS with 0.05% Tween 20) and incubated for one hour at room temperature with gentle mixing. Each well was washed 3 times with 200 µl of TBS/Tween/Ca⁺⁺ Wash Buffer (Tris-buffered saline, 0.05% Tween 20, containing 5.0 mM CaCl₂, pH 7.4. 10% rat serum in High Salt Binding Buffer (20 mM Tris, 1.0 M NaCl, 10 mM CaCl₂, 0.05% Triton-X100, 0.1% (w/v) bovine serum albumin, pH 7.4) was prepared on ice. 100 µl/well was added and incubated overnight at 5°C. Wells were washed 3 times with 200 µl of TBS/Tween/Ca⁺⁺ Wash Buffer. Wells were then washed 2 times with 200 µl PBS. 100 µl/well of selected concentration of MASP-2 Fab2 diluted in Ca⁺⁺ and Mg⁺⁺ containing GVB Buffer (4.0 mM barbital, 141 mM NaCl, 1.0 mM MgCl₂, 2.0 mM CaCl₂, 0.1% gelatin, pH 7.4) was added and incubated for one hour at room temperature with gentle mixing. Each well was washed 5 times with 200 µl PBS. 100 µl/well of HRP-conjugated goat anti-Fab2 (Biogenesis Cat No 0500-0099) diluted 1:5000 in 2.0 mg/mL bovine serum albumin in PBS was added and incubated for one hour at room temperature with gentle mixing. Each well was washed 5 times with 200 µl PBS. 100 µl/well of the peroxidase substrate TMB (Kirkegaard & Perry Laboratories) was added and incubated at room temperature for 70 minutes. The peroxidase reaction was stopped by adding 100 µl/well of 1.0 M H₃PO₄ and OD₄₅₀ was measured.

### RESULTS:

Approximately 250 different Fab2s that reacted with high affinity to the rat MASP-2 protein were picked for ELISA screening. These high-affinity Fab2s were sequenced to determine the uniqueness of the different antibodies, and 50 unique MASP-2 antibodies were purified for further analysis. 250 ug of each purified Fab2 antibody was used for characterization of MASP-2 binding affinity and complement pathway functional testing. The results of this analysis are shown below in **TABLE 13.**

**TABLE 13: MASP-2 FAB2 THAT BLOCK LECTIN PATHWAY COMPLEMENT ACTIVATION**

| Fab2 antibody # | C3 Convertase (IC₅₀ (nM) | K_{d} (nM) | C4 Cleavage IC₅₀ (nM) |
|---|---|---|---|
| 88 | 0.32 | 4.1 | ND |
| 41 | 0.35 | 0.30 | 0.81 |
| 11 | 0.46 | 0.86 | <2 nM |
| 86 | 0.53 | 1.4 | ND |
| 81 | 0.54 | 2.0 | ND |
| 66 | 0.92 | 4.5 | ND |
| 57 | 0.95 | 3.6 | <2 nM |
| 40 | 1.1 | 7.2 | 0.68 |
| 58 | 1.3 | 2.6 | ND |
| 60 | 1.6 | 3.1 | ND |
| 52 | 1.6 | 5.8 | <2 nM |
| 63 | 2.0 | 6.6 | ND |
| 49 | 2.8 | 8.5 | <2 nM |
| 89 | 3.0 | 2.5 | ND |
| 71 | 3.0 | 10.5 | ND |
| 87 | 6.0 | 2.5 | ND |
| 67 | 10.0 | 7.7 | ND |

As shown above in **TABLE 13,** of the 50 MASP-2 Fab2s tested, 17 were identified as MASP-2-blocking Fab2s that potently inhibit C3 convertase formation with IC₅₀ equal to or less than 10 nM Fab2s (a 34% positive hit rate). Eight of the 17 Fab2s identified have IC₅₀s in the subnanomolar range. Furthermore, all seventeen of the MASP-2 blocking Fab2s shown in **TABLE 13** gave essentially complete inhibition of C3 convertase formation in the lectin pathway C3 convertase assay. This is an important consideration, since it is theoretically possible that a "blocking" Fab2 may only fractionally inhibit MASP-2 function even when each MASP-2 molecule is bound by the Fab2.

Although mannan is a known activator of the lectin pathway, it is theoretically possible that the presence of anti-mannan antibodies in the rat serum might also activate the classical pathway and generate C3b via the classical pathway C3 convertase. However, each of the seventeen blocking MASP-2 Fab2s listed in this example potently inhibits C3b generation (>95 %), thus demonstrating the specificity of this assay for lectin pathway C3 convertase.

Binding assays were also performed with all seventeen of the blocking Fab2s in order to calculate an apparent K_{d} for each. The results of the binding assays of anti-rat MASP-2 Fab2s to native rat MASP-2 for six of the blocking Fab2s are also shown in **TABLE 13.** Similar binding assays were also carried out for the other Fab2s, the results of which are shown in **TABLE 13.** In general, the apparent K_{d}s obtained for binding of each of the six Fab2s to 'native' MASP-2 corresponds reasonably well with the IC₅₀ for the Fab2 in the C3 convertase functional assay. There is evidence that MASP-2 undergoes a conformational change from an 'inactive' to an 'active' form upon activation of its protease activity (Feinberg et al., EMBO J 22:2348-59 (2003); Gal et al., J. Biol. Chem. 280:33435-44 (2005)). In the normal rat plasma used in the C3 convertase formation assay, MASP-2 is present primarily in the 'inactive' zymogen conformation. In contrast, in the binding assay, MASP-2 is present as part of a complex with MBL bound to immobilized mannan; therefore, the MASP-2 would be in the 'active' conformation (Petersen et al., J. Immunol Methods 257:107-16, 2001). Consequently, one would not necessarily expect an exact correspondence between the IC₅₀ and K_{d} for each of the seventeen blocking Fab2 tested in these two functional assays because, in each assay, the Fab2 would be binding a different conformational form of MASP-2. Nevertheless, with the exception of Fab2 #88, there appears to be a reasonably close correspondence between the IC₅₀ and apparent K_{d} for each of the other sixteen Fab2 tested in the two assays (see **TABLE 13).**

Several of the blocking Fab2s were evaluated for inhibition of MASP-2-mediated cleavage of C4. As shown in **TABLE 13,** all of the Fab2s tested were found to inhibit C4 cleavage with IC₅₀s similar to those obtained in the C3 convertase assay.

Although mannan is a known activator of the lectin pathway, it is theoretically possible that the presence of anti-mannan antibodies in the rat serum might also activate the classical pathway and thereby generate C4b by C1s-mediated cleavage of C4. However, several MASP-2 Fab2s have been identified that potently inhibit C4b generation (>95 %), thus demonstrating the specificity of this assay for MASP-2-mediated C4 cleavage. C4, like C3, contains an unusual and highly reactive thioester group as part of its structure. Upon cleavage of C4 by MASP-2 in this assay, the thioester group on C4b can form a covalent bond with hydroxyl or amino groups on macromolecules immobilized on the bottom of the plastic wells *via* ester or amide linkages, thus facilitating detection of C4b in the ELISA assay.

These studies clearly demonstrate the creation of high-affinity FAB2s to rat MASP-2 protein that functionally block both C4 and C3 convertase activity, thereby preventing lectin pathway activation.

### EXAMPLE 12

This Example describes the epitope mapping for several of the blocking anti-rat MASP-2 Fab2 antibodies that were generated as described in Example 11.

### Methods:

The following proteins, all with N-terminal 6X His tags were expressed in CHO cells using the pED4 vector:
rat MASP-2A, a full-length MASP-2 protein, inactivated by altering the serine at the active center to alanine (S613A);
rat MASP-2K, a full-length MASP-2 protein altered to reduce autoactivation (R424K);
CUBI-11, an N-terminal fragment of rat MASP-2 that contains the CUBI, EGF-like and CUBII domains only; and
CUBI/EGF-like, an N-terminal fragment of rat MASP-2 that contains the CUBI and EGF-like domains only.

These proteins were purified from culture supernatants by nickel-affinity chromatography, as previously described (Chen et al., J. Biol. Chem. 276:25894-02 (2001)).

A C-terminal polypeptide (CCPII-SP), containing CCPII and the serine protease domain of rat MASP-2, was expressed in *E. coli* as a thioredoxin fusion protein using pTrxFus (Invitrogen). Protein was purified from cell lysates using Thiobond affinity resin. The thioredoxin fusion partner was expressed from empty pTrxFus as a negative control.

All recombinant proteins were dialyzed into TBS buffer and their concentrations determined by measuring the OD at 280 nm.

### Dot Blot Analysis:

Serial dilutions of the five recombinant MASP-2 polypeptides described above (and the thioredoxin polypeptide as a negative control for CCPII-serine protease polypeptide) were spotted onto a nitrocellulose membrane. The amount of protein spotted ranged from 100 ng to 6.4 pg, in five-fold steps. In later experiments, the amount of protein spotted ranged from 50 ng down to 16 pg, again in five-fold steps. Membranes were blocked with 5% skimmed milk powder in TBS (blocking buffer) then incubated with 1.0 µg/mL MASP-2 Fab2s in blocking buffer (containing 5.0 mM Ca⁺⁺). Bound Fab2s were detected using HRP-conjugated anti-human Fab (AbD/Serotec; diluted 1/10,000) and an ECL detection kit (Amersham). One membrane was incubated with polyclonal rabbit-anti human MASP-2 Ab (described in Stover et al., J Immunol 163:6848-59 (1999)) as a positive control. In this case, bound Ab was detected using HRP-conjugated goat anti-rabbit IgG (Dako; diluted 1/2,000).

### MASP-2 Binding Assay:

ELISA plates were coated with 1.0 µg/well of recombinant MASP-2A or CUBI-II polypeptide in carbonate buffer (pH 9.0) overnight at 4°C. Wells were blocked with 1% BSA in TBS, then serial dilutions of the MASP-2 Fab2s were added in TBS containing 5.0 mM Ca⁺⁺. The plates were incubated for one hour at RT. After washing three times with TBS/tween/Ca⁺⁺, HRP-conjugated anti-human Fab (AbD/Serotec) diluted 1/10,000 in TBS/Ca⁺⁺ was added and the plates incubated for a further one hour at room temperature. Bound antibody was detected using a TMB peroxidase substrate kit (Biorad).

### Results:

Results of the dot blot analysis demonstrating the reactivity of the Fab2s with various MASP-2 polypeptides are provided below in **TABLE 14.** The numerical values provided in **TABLE 14** indicate the amount of spotted protein required to give approximately half-maximal signal strength. As shown, all of the polypeptides (with the exception of the thioredoxin fusion partner alone) were recognized by the positive control Ab (polyclonal anti-human MASP-2 sera, raised in rabbits).

**TABLE 14: REACTIVITY WITH VARIOUS RECOMBINANT RAT MASP-2 POLYPEPTIDES ON DOT BLOTS**

| Fab2 Antibody # | MASP-2A | CUBI-II | CUBI/EGF-like | CCPII-SP | Thioredoxin |
|---|---|---|---|---|---|
| 40 | 0.16 ng | NR | NR | 0.8 ng | NR |
| 41 | 0.16 ng | NR | NR | 0.8 ng | NR |
| 11 | 0.16 ng | NR | NR | 0.8 ng | NR |
| 49 | 0.16 ng | NR | NR | >20 ng | NR |
| 52 | 0.16 ng | NR | NR | 0.8 ng | NR |
| 57 | 0.032 ng | NR | NR | NR | NR |
| 58 | 0.4 ng | NR | NR | 2.0 ng | NR |
| 60 | 0.4 ng | 0.4 ng | NR | NR | NR |
| 63 | 0.4 ng | NR | NR | 2.0 ng | NR |
| 66 | 0.4 ng | NR | NR | 2.0 ng | NR |
| 67 | 0.4 ng | NR | NR | 2.0 ng | NR |
| 71 | 0.4 ng | NR | NR | 2.0 ng | NR |
| 81 | 0.4 ng | NR | NR | 2.0 ng | NR |
| 86 | 0.4 ng | NR | NR | 10 ng | NR |
| 87 | 0.4 ng | NR | NR | 2.0 ng | NR |
| Positive Control | <0.032 ng | 0.16 ng | 0.16 ng | <0.032 ng | NR |

| | | | | | |
|---|---|---|---|---|---|
| NR = No reaction. The positive control antibody is polyclonal anti-human MASP-2 sera, raised in rabbits. | | | | | |

All of the Fab2s reacted with MASP-2A as well as MASP-2K (data not shown). The majority of the Fab2s recognized the CCPII-SP polypeptide but not the N-terminal fragments. The two exceptions are Fab2 #60 and Fab2 #57. Fab2 #60 recognizes MASP-2A and the CUBI-II fragment, but not the CUBI/EGF-like polypeptide or the CCPII-SP polypeptide, suggesting it binds to an epitope in CUBII, or spanning the CUBII and the EGF-like domain. Fab2 # 57 recognizes MASP-2A but not any of the MASP-2 fragments tested, perhaps indicating that this Fab2 recognizes an epitope in CCP1. Fab2 #40 and #49 bound only to complete MASP-2A. In the ELISA binding assay, Fab2 #60 also bound to the CUBI-II polypeptide, albeit with a slightly lower apparent affinity (data not shown).

These finding demonstrate the identification of unique blocking Fab2s to multiple regions of the MASP-2 protein.

### EXAMPLE 13

This Example describes the pharmacodynamic analysis of representative high-affinity MASP-2 Fab2 antibodies that were identified as described in Example 11.

### Background/Rationale:

As described in **Example 11,** in order to identify high-affinity antibodies that block the rat lectin pathway, rat MASP-2 protein was utilized to pan a phage display library. This library was designed to provide for high immunological diversity and was constructed using entirely human immunoglobin gene sequences. As shown in **Example 11,** approximately 250 individual phage clones were identified that bound with high affinity to the rat MASP-2 protein by ELISA screening. Sequencing of these clones identified 50 unique MASP-2 antibody-encoding phage. Fab2 protein was expressed from these clones, purified and analyzed for MASP-2 binding affinity and lectin complement pathway functional inhibition.

As shown in **TABLE 13** of **Example 11,** 17 MASP-2 Fab2s with functional blocking activity were identified as a result of this analysis (a 34% hit rate for blocking antibodies). Functional inhibition of the lectin complement pathway by Fab2s was apparent at the level of C4 deposition, which is a direct measure of C4 cleavage by MASP-2. Importantly, inhibition was equally evident when C3 convertase activity was assessed, demonstrating functional blockade of the lectin complement pathway. The 17 MASP-2 blocking Fab2s identified as described in **Example 11** potently inhibit C3 convertase formation with IC₅₀ values equal to or less than 10 nM. Eight of the 17 Fab2s identified have IC₅₀ values in the sub-nanomolar range. Furthermore, all 17 of the MASP-2 blocking Fab2s gave essentially complete inhibition of the C3 convertase formation in the lectin pathway C3 convertase assay, as summarized in **TABLE 13** of Example 11. Moreover, each of the 17 blocking MASP-2 Fab2s shown in **TABLE 13** potently inhibit C3b generation (>95%), thus demonstrating the specificity of this assay for lectin pathway C3 convertase.

Rat IgG2c and mouse IgG2a full-length antibody isotype variants were derived from Fab2 #11. This Example describes the *in vivo* characterization of these isotypes for pharmacodynamic parameters.

### Methods:

As described in **Example 11,** rat MASP-2 protein was utilized to pan a Fab phage display library, from which Fab2 #11 was identified. Rat IgG2c and mouse IgG2a full-length antibody isotype variants were derived from Fab2 #11. Both rat IgG2c and mouse IgG2a full-length antibody isotypes were characterized *in vivo* for pharmacodynamic parameters as follows.

### In vivo study in mice:

A pharmacodynamic study was carried out in mice to investigate the effect of MASP-2 antibody dosing on the plasma lectin pathway activity *in vivo.* In this study, C4 deposition was measured *ex vivo* in a lectin pathway assay at various time points following subcutaneous (sc) and intraperitoneal (ip) administration of 0.3 mg/kg or 1.0 mg/kg of the mouse MASP-2 MoAb (mouse IgG2a full-length antibody isotype derived from Fab2#11).

**FIGURE 29A** graphically illustrates lectin pathway specific C4b deposition on a zymosan-coated microtiter plate, measured *ex vivo* in undiluted serum samples taken from mice (n=3 mice/group) at various time points after subcutaneous dosing of either 0.3 mg/kg or 1.0 mg/kg of the mouse MASP-2 MoAb. Serum samples from mice collected prior to antibody dosing served as negative controls (100% activity), while serum supplemented *in vitro* with 100 nM of the same blocking MASP-2 antibody was used as a positive control (0% activity).

The results shown in **FIGURE 29A** demonstrate a rapid and complete inhibition of C4b deposition following subcutaneous administration of 1.0 mg/kg dose of mouse MASP-2 MoAb. A partial inhibition of C4b deposition was seen following subcutaneous administration of a dose of 0.3 mg/kg of mouse MASP-2 MoAb.

The time course of lectin pathway recovery was followed for three weeks following a single ip administration of mouse MASP-2 MoAb at 0.6 mg/kg in mice. As shown in **FIGURE 29B****,** a precipitous drop in lectin pathway activity occurred after antibody dosing followed by complete lectin pathway inhibition that lasted for about 7 days after i.p. administration. Slow restoration of lectin pathway activity was observed over the second and third weeks, with complete lectin pathway restoration in the mice by 17 days following MASP-2 MoAb administration.

These results demonstrate that the mouse MASP-2 Moab derived from Fab2 #11 inhibits the lectin pathway of mice in a dose-responsive manner when delivered systemically.

### EXAMPLE 14

This example describes the identification, using phage display, of fully human scFv antibodies that bind to MASP-2 and inhibit lectin-mediated complement activation (LEA-2) while leaving the classical (C1q-dependent) pathway component of the immune system intact.

### Overview:

Fully human, high-affinity MASP-2 antibodies were identified by screening a phage display library. The variable light and heavy chain fragments of the antibodies were isolated in both a scFv format and in a full-length IgG format. The human MASP-2 antibodies are useful for inhibiting cellular injury associated with lectin pathway-mediated alternative complement pathway activation while leaving the classical (C1q-dependent) pathway component of the immune system intact. In some cases, the subject MASP-2 inhibitory antibodies have the following characteristics: (a) high affinity for human MASP-2 (e.g., a K_{D} of 10 nM or less), and (b) inhibit MASP-2-dependent complement activity in 90% human serum with an IC₅₀ of 30 nM or less.

### Methods:

### Expression of full-length catalytically inactive MASP-2:

The full-length cDNA sequence of human MASP-2 (SEQ ID NO: 4), encoding the human MASP-2 polypeptide with leader sequence (SEQ ID NO:5) was subcloned into the mammalian expression vector pCI-Neo (Promega), which drives eukaryotic expression under the control of the CMV enhancer/promoter region (described in Kaufman R.J. et al., Nucleic Acids Research 19:4485-90, 1991; Kaufman, Methods in Enzymology,185:537-66 (1991)).
In order to generate catalytically inactive human MASP-2A protein, site-directed mutagenesis was carried out as described in US2007/0172483. The PCR products were purified after agarose gel electrophoresis and band preparation and single adenosine overlaps were generated using a standard tailing procedure. The adenosine-tailed MASP-2A was then cloned into the pGEM-T easy vector and transformed into *E. coli.* The human MASP-2A was further subcloned into either of the mammalian expression vectors pED or pCI-Neo.

The MASP-2A expression construct described above was transfected into DXB1 cells using the standard calcium phosphate transfection procedure (Maniatis et al., 1989). MASP-2A was produced in serum-free medium to ensure that preparations were not contaminated with other serum proteins. Media was harvested from confluent cells every second day (four times in total). The level of recombinant MASP-2A averaged approximately 1.5 mg/liter of culture medium. The MASP-2A (Ser-Ala mutant described above) was purified by affinity chromatography on MBP-A-agarose columns

### MASP-2A ELISA on ScFv Candidate Clones identified by panning/scFv conversion and filter screening

A phage display library of human immunoglobulin light- and heavy-chain variable region sequences was subjected to antigen panning followed by automated antibody screening and selection to identify high-affinity scFv antibodies to human MASP-2 protein. Three rounds of panning the scFv phage library against HIS-tagged or biotin-tagged MASP-2A were carried out. The third round of panning was eluted first with MBL and then with TEA (alkaline). To monitor the specific enrichment of phages displaying scFv fragments against the target MASP-2A, a polyclonal phage ELISA against immobilized MASP-2A was carried out. The scFv genes from panning round 3 were cloned into a pHOG expression vector and run in a small-scale filter screening to look for specific clones against MASP-2A.

Bacterial colonies containing plasmids encoding scFv fragments from the third round of panning were picked, gridded onto nitrocellulose membranes and grown overnight on non-inducing medium to produce master plates. A total of 18,000 colonies were picked and analyzed from the third panning round, half from the competitive elution and half from the subsequent TEA elution. Panning of the scFv phagemid library against MASP-2A followed by scFv conversion and a filter screen yielded 137 positive clones. 108/137 clones were positive in an ELISA assay for MASP-2 binding (data not shown), of which 45 clones were further analyzed for the ability to block MASP-2 activity in normal human serum.

### Assay to Measure Inhibition of Formation of Lectin Pathway C3 Convertase

A functional assay that measures inhibition of lectin pathway C3 convertase formation was used to evaluate the "blocking activity" of the MASP-2 scFv candidate clones. MASP-2 serine protease activity is required in order to generate the two protein components (C4b, C2a) that comprise the lectin pathway C3 convertase. Therefore, a MASP-2 scFv that inhibits MASP-2 functional activity (i.e., a blocking MASP-2 scFv), will inhibit *de novo* formation of lectin pathway C3 convertase. C3 contains an unusual and highly reactive thioester group as part of its structure. Upon cleavage of C3 by C3 convertase in this assay, the thioester group on C3b can form a covalent bond with hydroxyl or amino groups on macromolecules immobilized on the bottom of the plastic wells via ester or amide linkages, thus facilitating detection of C3b in the ELISA assay.

Yeast mannan is a known activator of the lectin pathway. In the following method to measure formation of C3 convertase, plastic wells coated with mannan were incubated with diluted human serum to activate the lectin pathway. The wells were then washed and assayed for C3b immobilized onto the wells using standard ELISA methods. The amount of C3b generated in this assay is a direct reflection of the *de novo* formation of lectin pathway C3 convertase. MASP-2 scFv clones at selected concentrations were tested in this assay for their ability to inhibit C3 convertase formation and consequent C3b generation.

### Methods:

The 45 candidate clones identified as described above were expressed, purified and diluted to the same stock concentration, which was again diluted in Ca⁺⁺ and Mg⁺⁺ containing GVB buffer (4.0 mM barbital, 141 mM NaCl, 1.0 mM MgCl₂, 2.0 mM CaCl₂, 0.1% gelatin, pH 7.4) to assure that all clones had the same amount of buffer. The scFv clones were each tested in triplicate at the concentration of 2 µg/mL. The positive control was OMS100 Fab2 and was tested at 0.4 µg/mL. C3c formation was monitored in the presence and absence of the scFv/IgG clones.

Mannan was diluted to a concentration of 20 µg/mL (1 µg/well) in 50mM carbonate buffer (15mM Na₂CO₃ + 35mM NaHCO₃ + 1.5 mM NaN₃), pH 9.5 and coated on an ELISA plate overnight at 4°C. The next day, the mannan-coated plates were washed 3 times with 200 µl PBS. 100 µl of 1% HSA blocking solution was then added to the wells and incubated for 1 hour at room temperature. The plates were washed 3 times with 200 µl PBS, and stored on ice with 200 µl PBS until addition of the samples.

Normal human serum was diluted to 0.5% in CaMgGVB buffer, and scFv clones or the OMS100 Fab2 positive control were added in triplicates at 0.01 µg/mL; 1 µg/mL (only OMS100 control) and 10 µg/mL to this buffer and preincubated 45 minutes on ice before addition to the blocked ELISA plate. The reaction was initiated by incubation for one hour at 37°C and was stopped by transferring the plates to an ice bath. C3b deposition was detected with a Rabbit α-Mouse C3c antibody followed by Goat α-Rabbit HRP. The negative control was buffer without antibody (no antibody = maximum C3b deposition), and the positive control was buffer with EDTA (no C3b deposition). The background was determined by carrying out the same assay except that the wells were mannan-free. The background signal against plates without mannan was subtracted from the signals in the mannan-containing wells. A cut-off criterion was set at half of the activity of an irrelevant scFv clone (VZV) and buffer alone.

**Results:** Based on the cut-off criterion, a total of 13 clones were found to block the activity of MASP-2. All 13 clones producing > 50% pathway suppression were selected and sequenced, yielding 10 unique clones. All ten clones were found to have the same light chain subclass, λ3, but three different heavy chain subclasses: VH2, VH3 and VH6. In the functional assay, five out of the ten candidate scFv clones gave IC₅₀ nM values less than the 25 nM target criteria using 0.5% human serum.

To identify antibodies with improved potency, the three mother scFv clones, identified as described above, were subjected to light-chain shuffling. This process involved the generation of a combinatorial library consisting of the VH of each of the mother clones paired up with a library of naive, human lambda light chains (VL) derived from six healthy donors. This library was then screened for scFv clones with improved binding affinity and/or functionality.

**TABLE 15: Comparison of functional potency in IC₅₀ (nM) of the lead daughter clones and their respective mother clones (all in scFv format)**

| **scFv clone** | **1% human serum C3 assay (IC₅₀ nM)** | **90% human serum C3 assay (IC₅₀ nM)** | **90% human serum C4 assay (IC₅₀ nM)** |
|---|---|---|---|
| 17D20mc | 38 | nd | nd |
| 17D20m_d3521N11 | 26 | >1000 | 140 |
| 17N16mc | 68 | nd | nd |
| 17N16m_d17N9 | 48 | 15 | 230 |

Presented below are the heavy-chain variable region (VH) sequences for the mother clones and daughter clones shown above in **TABLE 15,** and listed below in **TABLES 16A-F.**

The Kabat CDRs (31-35 (H1), 50-65 (H2) and 95-102 (H3)) are bolded; and the Chothia CDRs (26-32 (H1), 52-56 (H2) and 95-101 (H3)) are underlined.

### 17D20_35VH-21N11VL heavy chain variable region (VH) (SEQ ID NO:15, encoded by SEQ ID NO: 14)

### d17N9 heavy chain variable region (VH) (SEQ ID NO: 16)

### Heavy Chain Variable Region

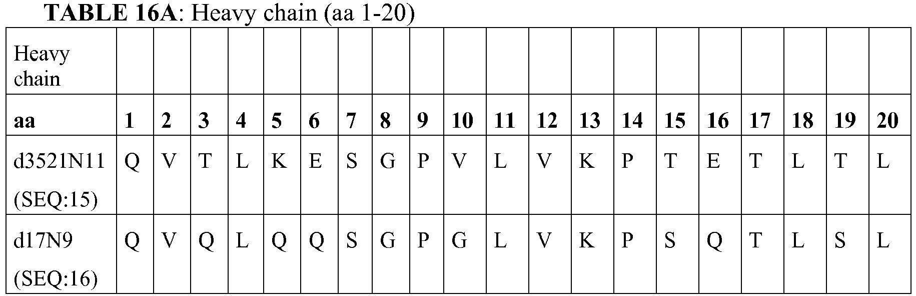

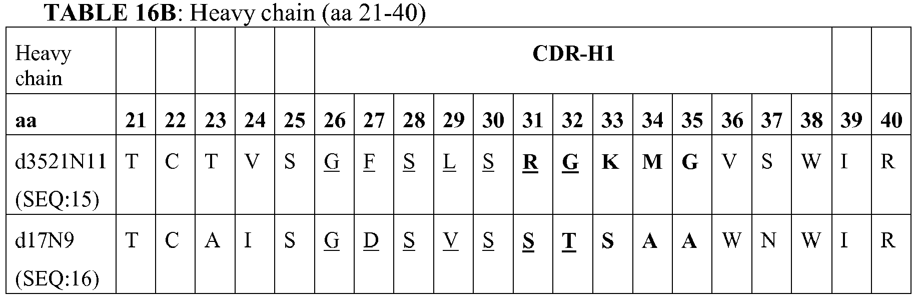

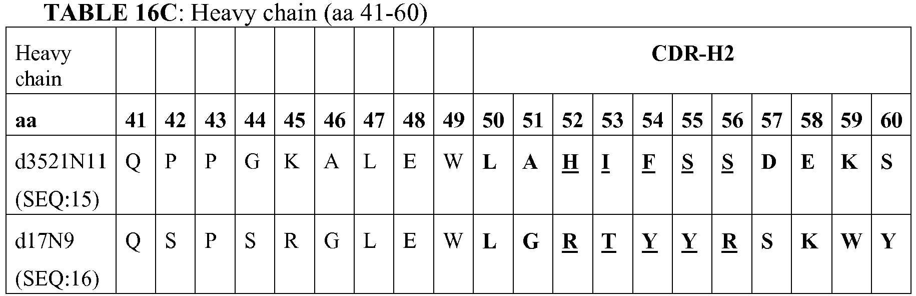

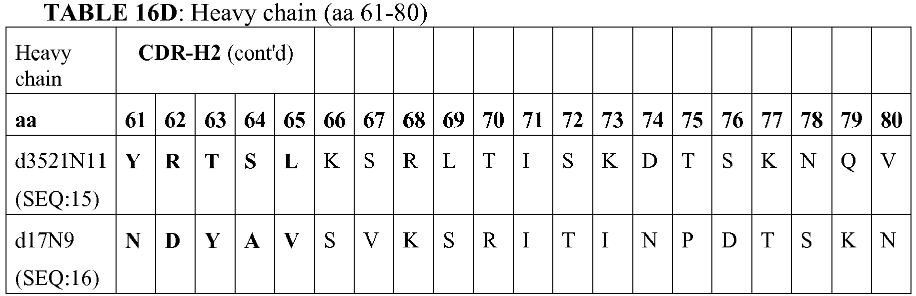

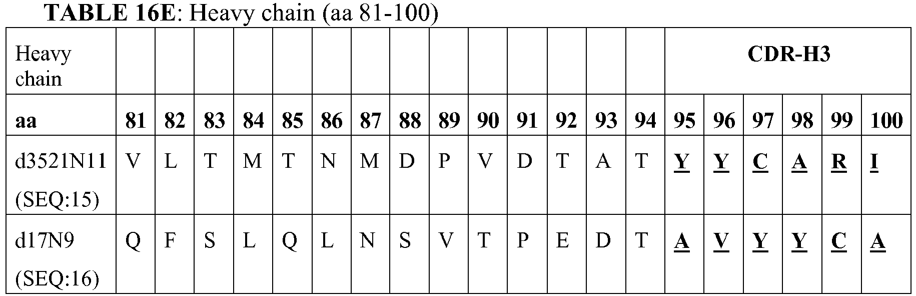

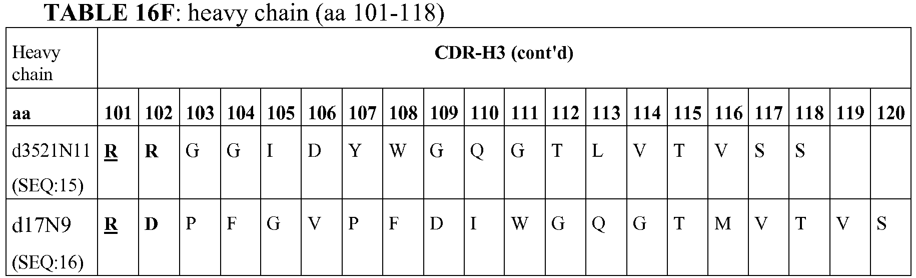

Presented below are the light-chain variable region (VL) sequences for the mother clones and daughter clones listed below in **TABLES 17A-F.**

The Kabat CDRs (24-34 (L1); 50-56 (L2); and 89-97 (L3) are bolded; and the Chothia CDRs (24-34 (L1); 50-56 (L2) and 89-97 (L3) are underlined. These regions are the same whether numbered by the Kabat or Chothia system.

### 17D20m_d3521N11 light chain variable region (VL) (SEQ ID NO:17)

### 17N16m_d17N9 light chain variable region (VL) (SEQ ID NO:19, encoded by SEQ ID NO:18)

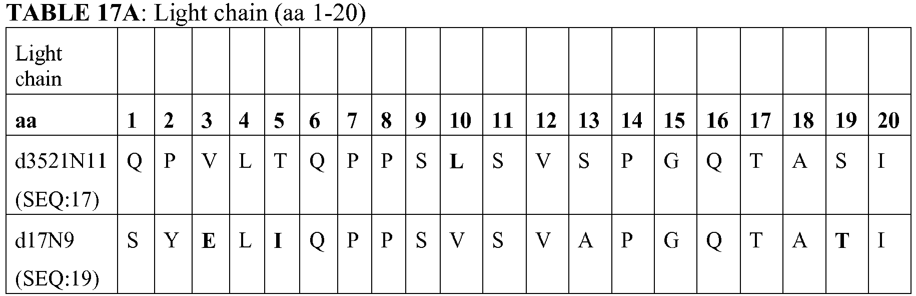

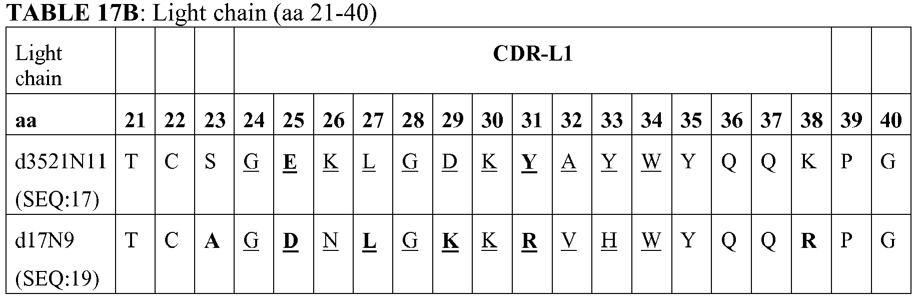

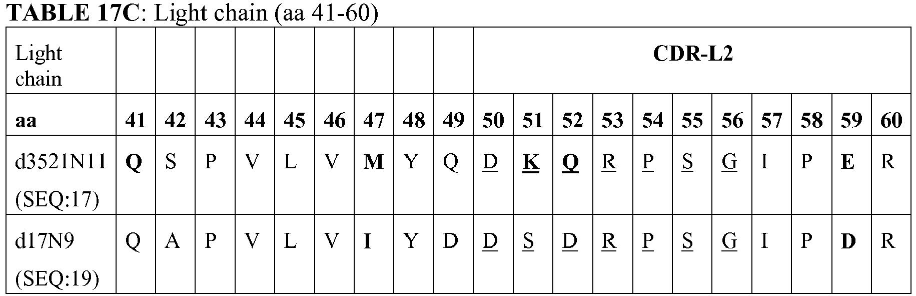

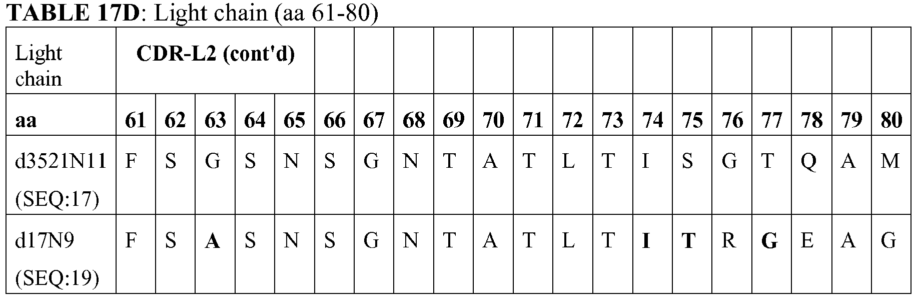

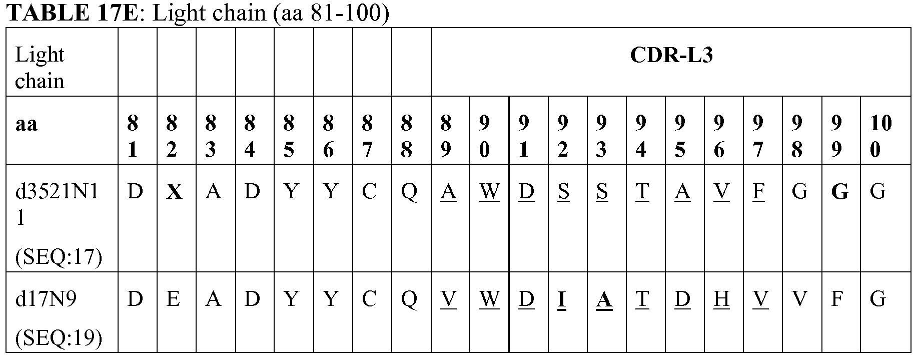

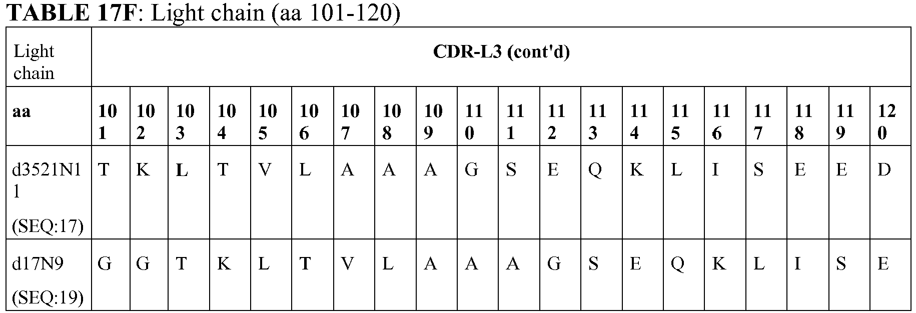

The MASP-2 antibodies OMS100 and MoAb_d3521N11VL, which have both been demonstrated to bind to human MASP-2 with high affinity and have the ability to block functional complement activity, were analyzed with regard to epitope binding by dot blot analysis. The results show that d3521N11 and OMS100 antibodies are highly specific for MASP-2 and do not bind to MASP-1/3. Neither antibody bound to MAp19 nor to MASP-2 fragments that did not contain the CCP1 domain of MASP-2, leading to the conclusion that the binding sites encompass CCP1.

Accordingly, in one case, a MASP-2 inhibitory agent for use in the compositions and methods of the claimed disclosure comprises a human antibody that binds a polypeptide consisting of human MASP-2 (SEQ ID NO:3), wherein the antibody comprises:
I)
   a) a heavy chain variable region comprising: i) a heavy chain CDR1 comprising the amino acid sequence from 31-35 of SEQ ID NO:21; and ii) a heavy chain CDR2 comprising the amino acid sequence from 50-65 of SEQ ID NO:21; and iii) a heavy chain CDR3 comprising the amino acid sequence from 95-102 of SEQ ID NO:21; and
   b) a light chain variable region comprising: i) a light chain CDR1 comprising the amino acid sequence from 24-34 of either SEQ ID NO:25 or SEQ ID NO:27; and ii) a light chain CDR2 comprising the amino acid sequence from 50-56 of either SEQ ID NO:25 or SEQ ID NO:27; and iii) a light chain CDR3 comprising the amino acid sequence from 89-97 of either SEQ ID NO:25 or SEQ ID NO:27; or II) a variant thereof that is otherwise identical to said variable domains, except for up to a combined total of 6 amino acid substitutions within said CDR regions of said heavy-chain variable region and up to a combined total of 6 amino acid substitutions within said CDR regions of said light-chain variable region, wherein the antibody or variant thereof inhibits MASP-2-dependent complement activation.

### EXAMPLE 15

This Example describes the generation of MASP-1 and MASP-3 monoclonal antibodies using an *in vitro* system comprising a modified DT40 cell line, DTLacO.

### Background/Rationale:

Antibodies against human MASP-1 and MASP-3 were generated using an *in vitro* system comprising a modified DT40 cell line, DTLacO, that permits reversible induction of diversification of a particular polypeptide, as further described in WO2009029315 and US2010093033. DT40 is a chicken B cell line that is known to constitutively mutate its heavy and light chain immunoglobulin (Ig) genes in culture. Like other B cells, this constitutive mutagenesis targets mutations to the V region of Ig genes, and thus, the CDRs of the expressed antibody molecules. Constitutive mutagenesis in DT40 cells takes place by gene conversion using as donor sequences an array of non-functional V gene segments (pseudo-V genes; ψV) situated upstream of each functional V region. Deletion of the ψV region was previously shown to cause a switch in the mechanism of diversification from gene conversion to somatic hypermutation, the mechanism commonly observed in human B cells. The DT40 chicken B cell lymphoma line has been shown to be a promising starting point for antibody evolution *ex vivo* (Cumbers, S.J. et al. Nat Biotechnol 20, 1129-1134 (2002); Seo, H. et al. Nat Biotechnol 23, 731-735 (2005)). DT40 cells proliferate robustly in culture, with an 8-10 hour doubling time (compared to 20-24 hr for human B cell lines), and they support very efficient homologous gene targeting (Buerstedde, J.M. et al. Embo J 9, 921-927 (1990)). DT40 cells command enormous potential V region sequence diversity given that they can access two distinct physiological pathways for diversification, gene conversion and somatic hypermutation, which create templated and nontemplated mutations, respectively (Maizels, N. Annu Rev Genet 39, 23-46 (2005)). Diversified heavy and light chain immunoglobulins (Igs) are expressed in the form of a cell-surface displayed IgM. Surface IgM has a bivalent form, structurally similar to an IgG molecule. Cells that display IgM with specificity for a particular antigen can be isolated by binding either immobilized soluble or membrane displayed versions of the antigen. However, utility of DT40 cells for antibody evolution has been limited in practice because - as in other transformed B cell lines - diversification occurs at less than 1% the physiological rate.

In the system used in this example, as described in WO2009029315 and US2010093033, the DT40 cells were engineered to accelerate the rate of Ig gene diversification without sacrificing the capacity for further genetic modification or the potential for both gene conversion and somatic hypermutation to contribute to mutagenesis. Two key modifications to DT40 were made to increase the rate of diversification and, consequently, the complexity of binding specificities in our library of cells. First, Ig gene diversification was put under the control of the potent *E. coli* lactose operator/repressor regulatory network. Multimers consisting of approximately 100 polymerized repeats of the potent *E. coli* lactose operator (PolyLacO) were inserted upstream of the rearranged and expressed Igλ and IgH genes by homologous gene targeting. Regulatory factors fused to lactose repressor protein (LacI) can then be tethered to the LacO regulatory elements to regulate diversification, taking advantage of the high affinity (k_{D}=10⁻¹⁴ M) of lactose repressor for operator DNA. DT40 PolyLacO-λ_{R} cells, in which PolyLacO was integrated only at Igλ, exhibited a 5-fold increase in Ig gene diversification rate relative to the parental DT40 cells prior to any engineering (Cummings, W.J. et al. PLoS Biol 5, e246 (2007)). Diversification was further elevated in cells engineered to carry PolyLacO targeted to both the Igλ and the IgH genes ("DTLacO"). DTLacO cells were demonstrated to have diversification rates 2.5- to 9.2-fold elevated relative to the 2.8% characteristic of the parental DT40 PolyLacO-λ_{R} LacI-HP1 line. Thus, targeting PolyLacO elements to both the heavy and light chain genes accelerated diversification 21.7-fold relative to the DT40 parental cell line. Tethering regulatory factors to the Ig loci not only alters the frequency of mutagenesis, but also can change the pathway of mutagenesis creating a larger collection of unique sequence changes (Cummings et al. 2007; Cummings et al. 2008). Second, a diverse collection of sequence starting points for the tethered factor-accelerated Ig gene diversification was generated. These diverse sequence starting points were added to DTLacO by targeting rearranged Ig heavy-chain variable regions, isolated from a two month old chick, to the heavy chain locus. The addition of these heavy chain variable regions created a repertoire of 10⁷ new starting points for antibody diversification. Building these new starting points into the DTLacO cell line permits the identification of clones that bind a particular target, and then rapid affinity maturation by the tethered factors. Following affinity maturation, a full-length, recombinant chimeric IgG is made by cloning the matured, rearranged heavy- and light-chain variable sequences (VH and Vλ; consisting of chicken framework regions and the complementarity determining regions or CDRs) into expression vectors containing human IgG1 and lambda constant regions. These recombinant mAbs are suitable for *in vitro* and *in vivo* applications, and they serve as the starting point for humanization.

### Methods:

### Selection for MASP-1 and MASP-3 antigen binding.

Initial selections were performed by binding DTLacO populations diversified by gene targeting to beads complexed with human MASP-1 (SEQ ID NO:10) and MASP-3 antigen (SEQ ID NO:8); and subsequent selections by FACS, using fluorescence-labeled soluble antigen (Cumbers, S.J. et al. Nat Biotechnol 20, 1129-1134 (2002); Seo, H. et al. Nat Biotechnol 23, 731-735 (2005). Because of the conserved amino acid sequence in the alpha chain that is shared between MASP-1 and MASP-3 (shown in **FIGURE 5****),** and the distinct beta chain sequences (shown in **FIGURE 6****),** separate, parallel screens for binders to MASP-1 and MASP-3 were carried out to identify MASP-1 specific mAbs, MASP-3 specific mAbs and also mAbs capable of binding to both MASP-1 and MASP-3 (dual-specific). Two forms of antigen were used to select and screen for binders. First, recombinant MASP-1 or MASP-3, either full-length or a fragment, fused to an Fc domain were bound to Dynal magnetic Protein G beads or used in FACS-based selections using a PECy5-labeled anti-human IgG(Fc) secondary antibody. Alternatively, recombinant versions of MASP-1 or MASP-3 proteins were directly labeled with Dylight flours and used for selections and screening.

### Binding and affinity.

Recombinant antibodies were generated by cloning PCR-amplified V regions into a vector that supported expression of human IgG1 in 293F cells (Yabuki et al., PLoS ONE, 7(4):e36032 (2012)). Saturation binding kinetics were determined by staining DTLacO cells expressing antibody binding MASP-1 or MASP-3 with various concentrations of fluorescent-labeled soluble antigen. Functional assays for MASP-3 specific activityincluding MASP-3-dependent C3b deposition and MASP-3-dependent factor D cleavage were carried out as described in Examples 17 and 18, respectively. A functional assay for MASP-1-specific activity, namely the inhibition of MASP-1-dependent C3b deposition was carried out as described below.

### Results:

Numerous MASP-1 and MASP-3 binding antibodies were generated using the methods described above. Binding, as demonstrated by FACS analysis, is described for the representative clones M3J5 and M3M1, which were isolated in screens for MASP-3 binders.

**FIGURE 30A** is a FACS histogram of MASP-3 antigen/antibody binding for DTLacO clone M3J5. **FIGURE 30B** is a FACS histogram of MASP-3 antigen/antibody binding for DTLacO clone M3M1. In **FIGURES 30A** and **30B** the gray filled curves are IgG1-stained negative control, and thick black curves are MASP-3-staining.

**FIGURE 31** graphically illustrates a saturation binding curve of clone M3J5 (Clone 5) for the MASP-3 antigen. As shown in **FIGURE 31****,** the apparent binding affinity of the M3J5 antibody for MASP-3 is about 31 nM.

Sequence analysis of identified clones was performed using standard methods. All clones were compared to the common (DT40) VH and VL sequences and to each other. Sequences for the two aforementioned clones, M3J5 and M3M1 are provided in an alignment with two additional representative clones, D14 and 1E10, which were identified in screens for CCP1-CCP2-SP fragments of MASP-1 and MASP-3, respectively. D14 and 1E10 bind regions common to both MASP-1 and MASP-3.

**FIGURE 32A** is an amino acid sequence alignment of the VH regions of M3J5, M3M1, D14 and 1E10 to the chicken DT40 VH sequence.

**FIGURE 32B** is an amino acid sequence alignment of the VL regions of M3J5, M3M1, D14 and 1E10 to the chicken DT40 VL sequence.

The VH and VL amino acid sequence of each clone is provided below.

### Heavy Chain Variable Region (VH) sequences

**FIGURE 32A** shows an amino acid alignment of the heavy-Chain Variable Region (VH) sequences for the parent DTLacO (SEQ ID NO: 24), the MASP-3-binding clones M3J5 (SEQ ID NO: 25), and M3M1 (SEQ ID NO: 26), and the MASP-1/MASP-3 dual binding clones D14 (SEQ ID NO:30), and 1E10.

The Kabat CDRs in the VH sequences below are located at the following amino acid positions::H1:aa 31-35; H2:aa 50-62; and H3:aa 95-102.

The Chothia CDRs in the VH sequences below are located at the following amino acid positions: H1:aa 26-32; H2: aa 52-56; and H3: aa 95-101.

### Parent DTLacO VH (SEQ ID NO:24)

### Clone M3J5 VH: (SEQ ID NO:25)

### Clone M3M1 VH: (SEQ ID NO:26)

### Clone D14 VH: (SEQ ID NO:30)

### Clone 1E10 VH: (SEQ ID NO:32)

### Light Chain Variable Regon (VL) sequences

**FIGURE 32B** shows an amino acid alignment of the light-Chain Variable Region (VL) sequences for the parent DTLacO (SEQ ID NO:27) and the MASP-3-binding clones M3J5 (SEQ ID NO:28), and M3M1 (SEQ ID NO:29), and the MASP-1/MASP-3 dual binding clones D14 (SEQ ID NO:31) and 1E10 (SEQ ID NO: 33).

### Parent DTLacO VL (SEQ ID NO:27):

### Clone M3J5 VL (SEQ ID NO:28):

### Clone M3M1 VL (SEQ ID NO:29):

### Clone D14 VL: (SEQ ID NO:31)

### Clone 1E10 VL: (SEQ ID NO:33)

### LEA-2 (MASP-2-dependent) Functional Assay

MASP-1 contributes to LEA-2 via its ability to activate MASP-2 (see **FIGURE 1****).** The Wieslab® Complement System Screen MBL assay (Euro Diagnostica, Malmö, Sweden) measures C5b-C9 deposition under conditions that isolate LEA-2-dependent activation (i.e., traditional lectin pathway activity). The assay was carried out according to the manufacturer's instructions with representative clone 1E10 tested as a final concentration of 400 nM.

**FIGURE 33** is a bar graph showing the inhibitory activity of the mAb 1E10 in comparison to the positive serum provided with the assay kit, as well as an isotype control antibody. As shown in **FIGURE 33****,** mAb 1E10 demonstrates partial inhibition of LEA-2-dependent activation (via inhibition of MASP-1-dependent activation of MASP-2), whereas the isotype control antibody does not. Stronger inhibition should be achieved by continued affinity maturation of this antibody for MASP-1 binding using the tethered factors in the DTLacO system.

LEA-1 (MASP-3-dependent) Function Assays for representative mAbs are described below in Examples 17 and 18.

### Summary of Results:

The above results showed that the DTLacO platform permitted rapid *ex vivo* discovery of MASP-1 and MASP-3 monoclonal antibodies with inhibitory properties on LEA-1 (as shown below in Examples 17 and 18) and on LEA-2 (as shown in this Example).

### EXAMPLE 16

This Example describes the generation of polypeptide inhibitors of MASP-1 and MASP-2.

### Rationale:

The generation of specific inhibitors of MASP-1 and MASP-2, termed SGMI-1 and SGMI-2, respectively, is described in Heja et al., J Biol Chem 287:20290 (2012) and Heja et al., PNAS 109:10498 (2012). SGMI-1 and SGMI-2 are each 36 amino acid peptides which were selected from a phage library of variants of the *Schistocerca gregaria* protease inhibitor 2 in which six of the eight positions of the protease binding loop were fully randomized. Subsequent *in vitro* evolution yielded mono-specific inhibitors with single digit nM K_{I} values (Heja et al., J. Biol. Chem. 287:20290, 2012). Structural studies revealed that the optimized protease binding loop forms the primary binding site that defines the specificity of the two inhibitors. The amino acid sequences of the extended secondary and internal binding regions are common to the two inhibitors and contribute to the contact interface (Heja et al., 2012. J. Biol. Chem. 287:20290). Mechanistically, both SGMI-1 and SGMI-2 block the lectin pathway of complement activation without affecting the classical or alternative pathways (Heja et al., 2012. Proc. Natl. Acad. Sci. 109:10498). The amino acid sequences of the **SGMI-1** and **SGMI-2** inhibitors are set forth below: SGMI-1-full-length: LEVTCEPGTTFKDKCNTCRCGSDGKSAFCTRKLCYQ (SEQ ID NO:34) SGMI-2-full-length: LEVTCEPGTTFKDKCNTCRCGSDGKSAVCTKLWCNQ (SEQ ID NO:35)

SGMI-1 and SGMI-2 are highly specific inhibitors of MASP-1 and MASP-2, respectively. However, as peptides they have limited potential for use in biological studies. To address these limitations, we engrafted these bioactive peptide amino acid sequences onto the amino terminus of human IgG1 Fc region to create an Fc-fusion protein,

### Methods:

To express the SGMI-IgG1 Fc fusion proteins, polynucleotides encoding the SGMI-1 (SEQ ID NO:34) and SGMI-2 (SEQ ID NO:35) peptides were synthesized (DNA 2.0) and inserted into the expression vector pFUSE-hIgG1-Fc2 (InvivoGen) between nucleotide sequences encoding the IL-2 signal sequence and the human IgG1 Fc region (SEQ ID NO:36). A flexible polypeptide linker (e.g., SEQ ID NO:37 or SEQ ID NO:38) was included between the SGMI peptide and the IgG1 Fc region.

### Flexible Polypeptide Linker Sequences:

GTGGGSGSSSRS (SEQ ID NO:37)
GTGGGSGSSS (SEQ ID NO:38)

The resulting constructs are described as follows:
A polynucleotide encoding the polypeptide fusion comprising the human IL-2 signal sequence, SGMI-1, linker and human IgG1-Fc (pFUSE-SGMI-1Fc), is set forth as SEQ ID NO:39, which encodes the mature polypeptide fusion comprising SGMI-1 (underlined), linker region (italicized) and human IgG1-Fc (together referred to as "SGMI-1Fc"), which is set forth as SEQ ID NO:40.

### SEQ ID NO:40

A polynucleotide encoding the polypeptide fusion comprising the human IL-2 signal sequence, SGMI-2, linker and human IgG1-Fc (pFUSE-SGMI-2Fc), is set forth as SEQ ID NO:41, which encodes the mature polypeptide fusion comprising SGMI-2 (underlined), linker region (italicized) and human IgG1-Fc (together referred to as "SGMI-2Fc"), which is set forth as SEQ ID NO:42:

### SEQ ID NO:42

### Production of Recombinant proteins:

Freestyle 293-F or Expi293F cells (Invitrogen) were transiently transfected according to the supplier's protocol with one of the two expression plasmids (pFUSE-SGMI-1Fc (SEQ ID NO:39) and pFUSE-SGMI-2Fc (SEQ ID NO:41). After four days of incubation at 37°C, the culture media were harvested. The Fc-fusion proteins were purified by Protein A affinity chromatography.

### Assays measuring activation of the lectin pathway.

The SGMI-1Fc and SGMI-2Fc fusion proteins were tested for the ability to inhibit deposition of C3b from 1% serum on a mannan-coated 96-well plate, which is a measure of lectin pathway activity. SGMI-1Fc and SGMI-2Fc were pre-incubated with 1% normal human serum for one hour on ice before addition to wells coated with mannan (2 µg/well). C3b deposition was measured by ELISA as described in Schwaeble et al. PNAS 108:7523, 2011.

**FIGURE 34** graphically illustrates the level of C3b deposition for 1% normal human serum plus isotype control, SGMI-1Fc or SGMI-2Fc over a concentration range of 0.15 to 1000 nM. As shown in **FIGURE 34****,** both SGMI-1Fc and SGMI-2Fc inhibited C3b deposition from normal serum in mannan-coated ELISA wells, with IC₅₀ values of approximately 27nM and 300nM, respectively.

These results demonstrate that the MASP-1 and MASP-2 inhibitory functions of the SGMI peptides are retained in the SGMI-1Fc and SGMI-2Fc fusion proteins.

### EXAMPLE 17

### Analysis of the complement pathway in 3MC serum with S. aureus

### Background/Rationale:

It was determined that MASP-3 is not activated through exposure to non-immobilized fluid-phase mannan, zymosan A or N-acetyl cysteine either in the presence or absence of normal human serum. However it was determined that recombinant and native MASP-3 are activated on the surface of heat-inactivated *S. aureus* in the presence and absence of normal human serum (NHS) or heat-inactivated human serum (HIS) (data not shown). It was also determined that C3b deposition occurs on the surface of *S. aureus* in the presence of normal human serum, and that the deposition can be monitored using a flow cytometer. Therefore, the alternative pathway (AP) response to *S. aureus* was measured as described in this Example as a means of assessing the contribution of MASP-3 to LEA-1.

### Methods:

Recombinant MASP-3: polynucleotide sequences encoding full length recombinant human MASP-3, a truncated serine protease (SP) active version of MASP-3 (CCP1-CCP2-SP), and a SP-inactivated form of MASP-3 (S679A) were cloned into the pTriEx7 mammalian expression vector (Invivogen). The resulting expression constructs encode the full length MASP-3 or the CCP1-CCP2-SP fragment with an amino-terminal Streptag and a carboxy-terminal His₆ tag. The expression constructs were transfected into Freestyle 293-F or Expi293F cells (Invitrogen) according to the protocols provided by the manufacturer. After three to four days of culture in 5% CO₂ at 37°C, recombinant proteins were purified utilizing Streptactin affinity chromatography.

Recombinant MASP-1: the full length or truncated CCP1-CCP2-SP forms of recombinant MASP-1 with or without the stabilizing R504Q (Dobo et al., J. Immunol 183:1207, 2009) or SP inactivating (S646A) mutations and bearing an amino-terminal Steptag and a carboxy-terminal His6 tag were generated as described for recombinant MASP-3 above.

### 1. C3b deposition and factor B cleavage on S. aureus in 3MC (human) serum

An initial experiment was carried out to demonstrate that the flow cytometry assay is able to detect the presence or absence of AP-driven C3b deposition (AP-C3b) as follows. Five percent of the following sera: normal human serum, factor B (Factor B)- depleted human serum, factor D-depleted human serum and properdin-depleted human serum (obtained from Complement Technology, Tyler, Texas, USA) were mixed with test antibody in either Mg⁺⁺/EGTA buffer or EDTA at 4°C overnight. Heat-killed *S. aureus* (10⁸/reaction) was added to each mixture to a total volume of 100 µL and rotated at 37°C for 40 minutes. Bacteria were washed in washing buffer, the bacterial pellet was re-suspended in washing buffer and a 80 µL aliquot of each sample was analyzed for C3b deposition on the bacterial surface, which was detected with anti-human C3c (Dako, UK) using flow cytometry.

The results of the flow cytometry detection of C3b are shown in **FIGURE 35A**. As shown in **FIGURE 35A**, panel 1, normal human serum in the presence of EDTA, which is known to inactivate the AP, no C3b deposition was observed (negative control). In normal human serum treated with Mg⁺⁺/EGTA, only lectin-independent complement pathways can function. In panel 2, Mg⁺⁺/EGTA buffer is used, therefore the AP is active, and AP-driven C3b deposition is observed (positive control). As shown in panel 3, 4 and 5, in factor B-depleted, factor D-depleted and properdin-depleted serum, respectively, no alternative pathway driven C3b deposition is observed, as expected. These results demonstrate that the assay is capable of detecting AP-dependent C3b deposition.

A C3b deposition on *S. aureus* assay was carried out as described above to assess the ability of recombinant MASP-3 to reconstitute the AP (LEA-1) in human 3MC serum, which is deficient in MASP-3 (Rooryck C, et al., Nat Genet. 43(3):197-203 (2011)). The following combinations of reagents were tested.
1. 5% normal human serum +EDTA
2. 5% normal human serum +Mg/EGTA
3. 5% human 3MC (MASP-3^{-/-}) serum + Mg^{++/}EGTA
4. 5% human 3MC (MASP-3^{-/-}) serum + Mg⁺⁺/EGTA plus active full-length rMASP-3
5. 5% human 3MC (MASP-3^{-/-}) serum + Mg⁺⁺/EGTA plus truncated active rMASP-3 (CCP1/CCP2/SP)
6. 5% human 3MC (MASP-3^{-/-}) serum + Mg⁺⁺/EGTA plus inactive rMASP-3 (S679A)
7. 5% human 3MC (MASP-3^{-/-}) serum + Mg⁺⁺/EGTA plus active full length rMASP-1

The various mixtures of 5% serum and recombinant proteins (5 µg of each) as shown above were incubated in the specified buffer conditions (either Mg⁺⁺/EGTA buffer or EDTA) at 4°C overnight. After the incubation overnight, 10⁸ heat-killed *S. aureus* were added to each mixture in a total volume of 100 µL and rotated at 37°C for 40 minutes. Bacteria were washed and re-suspended in washing buffer and an 80 µl aliquot of each sample was analyzed for C3b deposition by FACS. The remaining 20 µL aliquot of each sample was used to measure factor B cleavage by Western blot using anti-factor B antibody as described below.

The results of the flow cytometery detection of C3b are shown in **FIGURE 35B****.** Panel numbers correspond to the numbers designated for each of the reagent combination outlined above. The negative control (panel 1) and positive control (panel 2) show the absence and presence of C3b deposition, as expected. Panel 3 shows that AP-driven C3b deposition is absent in 3MC serum. Panels 4 and 5 show that active full length rMASP-3 (panel 4) and active rMASP-3 (CCP1-CCP2-SP) (panel 5) both restore AP-driven C3b deposition in 3MC serum. Panel 6 shows that inactive rMASP-3 (S679A) does not restore AP-driven C3b deposition in 3MC serum. Panel 7 shows that rMASP-1 does not restore AP-driven C3b deposition in 3MC serum.

Taken together, these results demonstrate that MASP-3 is required for AP-driven C3b deposition on *S. aureus* in human serum.

### 2. MASP-3-dependent Activation of Factor B

In order to analyze MASP-3-dependent activation of Factor B, the various mixtures of 5% serum (either normal human serum or 3MC patient serum) and recombinant proteins as shown above were assayed as described above. From each reaction mixture, 20 µL were removed and added to protein sample loading buffer. The samples were heated at 70°C for 10 minutes and loaded onto an SDS-PAGE gel. Western blot analysis was performed using a Factor B polyclonal antibody (R&D Systems). Activation of Factor B was apparent by the formation of two lower molecular weight cleavage products (Bb and Ba) derived from the higher molecular weight pro-Factor B protein.

**FIGURE 36** shows the results of a Western blot analysis to determine factor B cleavage in response to *S. aureus* in 3MC serum in the presence or absence of rMASP-3. As shown in lane 1, the normal human serum in the presence of EDTA (negative control) demonstrates very little Factor B cleavage relative to normal human serum in the presence of Mg⁺⁺/EGTA, shown in lane 2 (positive control). As shown in lane 3, 3MC serum demonstrates very little Factor B cleavage in the presence of Mg⁺⁺/EGTA. However, as shown in lane 4, Factor B cleavage is restored by the addition and pre-incubation of full-length, recombinant MASP-3 protein (5 µg) to the 3MC serum.

### 3. Assay to determine the effect of rMASP-3 on pro-factor D in factor B/C3(H2O) Cleavage

The following assay was carried out to determine the minimal requirement for MASP-3-dependent activation/cleavage of factor B.

C3(H₂O) (200ng), purified plasm factor B (20 µg), recombinant pro-factor D (200 ng) and recombinant human MASP-3 (200 ng) were mixed together in various combinations (as shown in **FIGURE 37****),** in a total volume of 100 µL in BBS/Ca⁺⁺/Mg⁺⁺ and incubated at 30°C for 30 minutes. The reaction was stopped by adding 25 uL of SDS loading dye containing 5% 2-mercaptoethanol. After boiling at 95°C for 10 minutes under shaking (300 rpm), the mixture was spun down at 1400 rpm for 5 minutes and 20 uL of the supernatant was loaded and separated on a 10% SDS gel. The gel was stained with Coomassie brilliant blue.

### Results:

**FIGURE 37** shows a Comassie-stained SDS-PAGE gel in which factor B cleavage is analyzed. As shown in lane 1, factor B cleavage is most optimal in the presence of C3, MASP-3 and pro-factor D. As shown in lane 2, C3 is absolutely required; however, as shown in lanes 4 and 5, either MASP-3 or pro-factor D are able to mediate factor B cleavage, as long as C3 is present.

### 4. Analysis of the ability of MASP-3 mAbs to inhibit MASP-3-dependent AP-driven C3b deposition

As described in this Example it was demonstrated that MASP-3 is required for AP-driven C3b deposition on *S. aureus* in human serum. Therefore, the following assay was carried out to determine if a representative MASP-3 mAb identified as described in Example 15, could inhibit activity of MASP-3. Active, recombinant MASP-3 (CCP1-CCP2-SP) fragment protein (250 ng) was pre-incubated with an isotype control mAb, mAb1A5 (control obtained from the DTLacO platform that does not bind MASP-3 or MASP-1), or mAbD14 (binds MASP-3) at three different concentrations (0.5, 2 and 4 µM) for 1 hour on ice. The enzyme-mAb mixture was exposed to 5% 3MC serum (MASP-3 deficient) and 5x10⁷ heat-killed *S. aureus* in a final reaction volume of 50 µL. The reactions were incubated at 37°C for 30 minutes, and then stained for the detection of C3b deposition. The stained bacterial cells were analyzed by a flow cytometer.

**FIGURE 38** graphically illustrates the mean fluorescent intensities (MFI) of C3b staining obtained from the three antibodies plotted as a function of mAb concentration in 3MC serum with the presence of rMASP-3. As shown in **FIGURE 38****,** mAbD14 demonstrates inhibition of C3b deposition in a concentration-dependent manner. In contrast, neither of the control mAbs inhibited C3b deposition. These results demonstrate that mAbD14 is able to inhibit MASP-3-dependent C3b deposition. Improved inhibitory activity for mAbD14 is expected following continued affinity maturation of this antibody for MASP-3 binding using the tethered factors in the DTLacO system.

### Summary of Results:

In summary, the results in this Example demonstrate a clear defect of the AP in serum deficient for MASP-3. Thus, MASP-3 has been demonstrated to make a critical contribution to the AP, using factor B activation and C3b deposition as functional endpoints. Furthermore, addition of functional, recombinant MASP-3, including the catalytically-active C-terminal portion of MASP-3 corrects the defect in factor B activation and C3b deposition in the serum from the 3MC patient. Conversely, as further demonstrated in this Example, addition of a MASP-3 antibody (e.g.,mAbD14) in 3MC serum with rMASP-3 inhibits AP-driven C3b deposition. A direct role of MASP-3 in Factor B activation, and therefore the AP, is demonstrated by the observation that recombinant MASP-3, along with C3, is sufficient to activate recombinant factor B.

### EXAMPLE 18

This Example demonstrates that MASP-1 and MASP-3 activate factor D.

### Methods:

Recombinant MASP-1 and MASP-3 were tested for their ability to cleave two different recombinant versions of pro-factor D. The first version (pro-factor D-His) lacks an N-terminal tag, but has a C-terminal His tag. Thus, this version of pro-factor D contains the 5 amino acid pro-peptide that is removed by cleavage during activation. The second version (ST-pro-factor D-His) has a Strep-TagII sequence on the N-terminus, thus increasing the cleaved N-terminal fragment to 15 amino acids. ST-pro-factor D also contains a His₆ tag at the C-terminus. The increased length of the propeptide of ST-pro-factor D-His improves the resolution between the cleaved and uncleaved forms by SDS-PAGE compared to the resolution possible with the pro-factor D-HIS form.

Recombinant MASP-1 or MASP-3 proteins (2 µg) was added to either pro-factor D-His or ST-pro-factor D-His substrates (100 ng) and incubated for 1 hour at 37°C. The reactions were electrophoresed on a 12% Bis-Tris gel to resolve pro-factor D and the active factor D cleavage product. The resolved proteins were transferred to a PVDF membrane and analyzed by Western blot by detection with a biotinylated factor D antibody (R&D Systems).

### Results:

**FIGURE 39** shows the Western blot analysis of pro-factor D substrate cleavage. As shown in **FIGURE 39****,** only full length MASP-3 (lane 2) and the MASP-1 CCP1-CCP2-SP) fragment (lane 5) cleaved ST-pro-factor D-His₆. The catalytically-inactive full length MASP-3 (S679A; lane 3) and MASP-1 (S646A; lane 3) failed to cleave either substrate. Identical results were obtained with the pro-factor D-His₆ polypeptide (not shown). The comparison of a molar excess of MASP-1 (CCP1-CCP2-SP) relative to MASP-3 suggests that MASP-3 is a more effective catalyst of pro-factor D cleavage than is MASP-1, as least under the condtions described herein.

**Conclusions:** Both MASP-1 and MASP-3 are capable of cleaving and activating factor D. This activity directly connects LEA-1 with the activation of the AP. More specifically, activation of factor D by MASP-1 or MASP-3 will lead to factor B activation, C3b deposition, and likely opsonization and/or lysis.

### 1. Assay for Inhibition of MASP-3-dependent Cleavage of pro-factor D with MASP-3 antibodies

An assay was carried out to determine the inhibitory effect of representative MASP-3 and MASP-1 mAbs, identified as described in Example 15, on MASP-3-dependent factor D cleavage as follows. Active, recombinant MASP-3 protein (80 ng) was pre-incubated with 1 µg of representative mAbs D14, M3M1 and a control antibody (which binds specifically to MASP-1, but not to MASP-3) at room temperature for 15 minutes. Pro-factor D with an N-terminal Strep-tag (ST-pro-factor D-His, 70 ng) was added and the mixture was incubated at 37°C for 75 minutes. The reactions were then electrophoresed, blotted and stained with anti-factor D as described above.

**FIGURE 40** is a Western blot showing the partial inhibitory activity of the mAbs D14 and M3M1 in comparison to a control reaction containing only MASP-3 and ST-pro-factor D-His (no mAb; lane 1), as well as a control reaction containing a mAb obtained from the DTLacO library that binds MASP-1, but not MASP-3 (lane 4). As shown in **FIGURE 40****,** in the absence of an inhibitory antibody, MASP-3 cleaves approximately 50% of pro-factor D into factor D (lane 1). The control MASP-1 specific antibody (lane 4) does not change the ratio of pro-factor D to factor D. In contrast, as shown in lanes 2 and 3, both mAb D14 and mAb M3M1 inhibit MASP-3-dependent cleavage of pro-factor D to factor D, resulting in a reduction in factor D generated.

**Conclusions:** These results demonstrate that MASP-3 mAbs D14 and M3M1 are able to inhibit MASP-3-dependent factor D cleavage. Improved inhibitory activity for mAbD14 and mAb M3M1 is expected following continued affinity maturation of these antibodies for MASP-3 binding using the tethered factors in the DTLacO system.

### EXAMPLE 19

This Example demonstrates that MASP-3 deficiency prevents complement-mediated lysis of mannan-coated WT rabbit erythrocytes.

### Background/Rationale:

As described in Examples 5 and 6 herein, the effect of MASP-2- and MASP-3-deficient serum on lysis of red blood cells from blood samples obtained from a mouse model of PNH demonstrated the efficacy of MASP-2 inhibition and/or MASP-3 inhibition to treat subjects suffering from PNH, and also supported the use of inhibitors of MASP-2 and/or inhibitors of MASP-3 (including dual or bi-specific MASP-2/MASP-3 inhibitors) to ameliorate the effects of C3 fragment-mediated extravascular hemolysis in PNH subjects undergoing therapy with a C5 inhibitor such as eculizumab.

As described in this Example, C3b deposition experiments and hemolysis experiments were carried out in MASP-3 deficient serum from additional 3MC patients, confirming the results obtained in Examples 5 and 6. In addition, experiments were carried out which demonstrated that addition of rMASP-3 to 3MC serum was able to reconstitute C3b deposition and hemolytic activity.

### Methods:

MASP-3-deficient serum was obtained from three different 3MC patients as follows:
3MC Patient 1: contains an allele bearing a mutation that renders the exon encoding the MASP-3 serine protease domain dysfunctional, supplied along with the mother and father of the 3MC patient (both heterozygous for the allele bearing a mutation that renders the exon encoding the MASP-3 serine protease domain dysfunctional),
3MC Patient 2: Has C1489T (H497Y) mutation in exon 12 of MASP-1, the exon that encodes the serine protease domain of MASP-3, resulting in nonfunctional MASP-3, but functional MASP-1 proteins.
3MC Patient 3: Has a confirmed defect in the MASP-1 gene, resulting in nonfunctional MASP-3 but functional MASP-1 proteins.

### Experiment #1: C3b Deposition Assay

An AP assay was carried out under traditional AP-specific conditions (BBS/ Mg⁺⁺/EGTA, without Ca⁺⁺, wherein BBS= barbital buffered saline containing sucrose), as described in Bitter-Suermann et al., Eur. J. Immunol 11:291-295 (1981)), on zymosan-coated microtiter plates at serum concentrations ranging from 0.5 to 25% and C3b deposition was measured over time.

### Results:

**FIGURE 41** graphically illustrates the level of AP-driven C3b deposition on zymosan-coated microtiter plates as a function of serum concentration in serum samples obtained from MASP-3-deficient (3MC), C4-deficient and MBL-deficient subjects. As shown in **FIGURE 41****,** and summarized below in **TABLE 18,** MASP-3-deficient patient sera from Patient 2 and Patient 3 have residual AP activity at high concentrations (25%, 12.5%, 6.25% serum concentrations), but a significantly higher AP₅₀ (i.e., 8.2% and 12.3% of serum needed to achieve 50% of maximum C3 deposition).

**FIGURE 42** graphically illustrates the level of AP-driven C3b deposition on zymosan-coated microtiter plates under "traditional" AP-specific conditions (i.e., BBS/EGTA/Mg⁺⁺ without Ca⁺⁺) as a function of time in 10% human serum samples obtained from MASP-3 deficient, C4-deficient and MBL-deficient human subjects.

**TABLE 18** below summarizes the AP₅₀ results shown in **FIGURE 41** and the half-times for C3b deposition shown in **FIGURE 42****.**

**TABLE 18: Summary of Results shown in FIGURES 41 and 42**

| **Serum type** | **AP₅₀ (%)** | **T_{1/2} (min)** |
|---|---|---|
| Normal | 4.5 | 26.3 |
| MBL-deficient (MBL-/-) | 5.7 | 27.5 |
| C4-deficient (C4-/-) | 5.1 | 28.6 |
| 3MC (Patient 3) | 8.2 | 58.2 |
| 3MC (Patient 2) | 12.3 | 72.4 |

| | | |
|---|---|---|
| Note: In BBS/Mg⁺⁺/EGTA buffer, the lectin pathway-mediated effects are deficient due to absence of Ca⁺⁺ in this buffer. | | |

### Experiment #2: Hemolysis assay testing mannan-coated rabbit erythrocytes for lysis in the presence of human normal or 3MC serum (in the absence of Ca⁺⁺)

### Methods:

### Preparation of rabbit RBC in the absence of Ca⁺⁺ (i.e., by using EGTA)

Rabbit whole blood (2 mL) was split into two 1.5 mL eppendorf tubes and centrifuged for 3 minutes at 8000 rpm (approximately 5.9 rcf) in a refrigerated eppendorf centrifuge at 4°C. The RBC pellet was washed three times after re-suspending in ice-cold BBS/ Mg⁺⁺/Ca⁺⁺ (4.4 mM barbituric acid, 1.8 mM sodium barbitone, 145 mM NaCl, pH 7.4, 5 mM Mg⁺⁺, 5 mM Ca⁺⁺). After the third wash, the pellet was re-suspended in 4 mL BBS/ Mg⁺⁺/Ca⁺⁺. The erythrocytes were pelleted and the RBCs were washed with BBS/0.1% gelatin/Mg⁺⁺/Ca⁺⁺ as described above. The RBCs suspension was stored in BBS/0.1% gelatin/ Mg⁺⁺/Ca⁺⁺ at 4°C. Then, 100 µL of suspended RBCs were diluted with 1.4 mL water and spun down at 8000 rpm (approximately 5.9 rcf) for 3 minutes and the OD of the supernatant was adjusted to 0.7 at 541nm (an OD of 0.7 at 541nm corresponds to approximately 10⁹ erythrocytes/ml). After that, 1 mL of the resuspended RBCs at OD 0.7 were added to 9 ml of BBS/Mg⁺⁺/EGTA in order to achieve a concentration of 10⁸ erythrocytes/ml. Dilutions of the test sera or plasma were prepared in ice-cold BBS, Mg⁺⁺, EGTA and 100 µL of each serum or plasma dilution was pipetted into the corresponding well of round-bottom plate. 100 µL of appropriately diluted RBC (10⁸ erythrocytes/ml) were added to each well. Nano-water was used to produce the positive control (100% lysis), while a dilution with BBS/Mg⁺⁺/EGTA without serum or plasma was used as a negative control. The plate was then incubated for 1 hour at 37°C. The round bottom plate was spun down at 3750 rpm for 5 minutes. Then, 100 µL of the supernatant from each well was transferred into the corresponding wells of a flat-bottom plate and OD was read at 415-490 nm.

### Results:

**FIGURE 43** graphically illustrates the percent hemolysis (as measured by hemoglobin release of lysed rabbit erythrocytes into the supernatant measured by photometry) of mannan-coated rabbit erythrocytes over a range of serum concentrations in serum from normal subjects and from two 3MC patients (Patient 2 and Patient 3), measured in the absence of Ca⁺⁺. As shown in **FIGURE 43****,** it is demonstrated that MASP-3 deficiency reduces the percentage of complement-mediated lysis of mannan-coated erythrocytes as compared to normal human serum. The differences between the two curves from the normal human serum and the two curves from the 3MC patients is significant (p=0.013, Friedman test).

**TABLE 19** below summarizes the AP₅₀ results shown in **FIGURE 43****.**

**TABLE 19: Summary of Results shown in FIGURE 43**

| **Serum type** | **AP₅₀ (%)** |
|---|---|
| Normal human serum #1 | 7.1 |
| Normal human serum #2 | 8.6 |
| 3MC Patient #2 | 11.9 |
| 3MC Patient #3 | 14.3 |

It is noted that when the serum samples shown in **TABLE 19** were pooled, the AP₅₀ value for normal human serum = 7.9 and the AP₅₀ value for 3MC serum = 12.8 (p=0.031, Wilcox matched-pairs signed rank test).

### Experiment #3: Reconstitution of human 3MC serum by recombinant MASP-3 restores AP-driven C3b deposition on zymosan coated plates

### Methods:

An AP assay was carried out under traditional AP-specific conditions (BBS/Mg⁺⁺/EGTA, without Ca⁺⁺, wherein BBS=barbital buffered saline containing sucrose), as described in Bitter-Suermann et al., Eur. J. Immunol 11:291-295 (1981)), on zymosan-coated microtiter plates in the following serum samples (1) 5% human serum from 3MC Patient #2 with full length active rMASP-3 added in at a range of 0 to 20 µg/ml; (2) 10% human serum from 3MC Patient #2 with full length active rMASP-3 added in at a range of 0 to 20 µg/ml; and (3) 5% human serum from 3MC Patient #2 with inactive rMASP-3A (S679A) added in at a range of 0 to 20 µg/ml.

### Results:

**FIGURE 44** graphically illustrates the level of AP-driven C3b deposition on zymosan-coated microtiter plates as a function of the concentration of rMASP-3 protein added to serum samples obtained from human 3MC Patient #2 (MASP-3-deficient). As shown in **FIGURE 44****,** active recombinant MASP-3 protein reconstitutes AP-driven C3b deposition on zymosan-coated plates in a concentration-dependent manner. As further shown in **FIGURE 44****,** no C3b deposition was observed in the 3MC serum containing inactive rMASP-3 (S679A).

### Experiment #4: Reconstitution of human 3MC serum by recombinant MASP-3 restores hemolytic activity in 3MC patient serum

### Methods:

A hemolytic assay was carried out using rabbit RBC using the methods described above in Experiment #2 with the following test sera at a range of 0 to 12% serum: (1) normal human serum; (2) 3MC patient serum; (3) 3MC patient serum plus active full length rMASP-3 (20 µg/ml); and (4) heat-inactivated human serum.

### Results:

**FIGURE 45** graphically illustrates the percent hemolysis (as measured by hemoglobin release of lysed rabbit erythrocytes into the supernatant measured by photometry) of mannan-coated rabbit erythrocytes over a range of serum concentrations in (1) normal human serum; (2) 3MC patient serum; (3) 3MC patient serum plus active full length rMASP-3 (20 µg/ml); and (4) heat-inactivated human serum, measured in the absence of Ca⁺⁺. As shown in **FIGURE 45****,** the percent lysis of rabbit RBC is significantly increased in 3MC serum including rMASP-3 as compared to the percent lysis in 3MC serum without rMASP-3 (p=0.0006).

**FIGURE 46** graphically illustrates the percentage of rabbit erythrocyte lysis in 7% human serum from 3MC Patient 2 and from 3MC Patient 3 containing active rMASP-3 at a concentration range of 0 to 110 µg/ml in BBS/Mg⁺⁺/EGTA. As shown in **FIGURE 46****,** the percentage of rabbit RBC lysis is restored with the amount of rMASP-3 in a concentration-dependent manner up to 100% activity.

### Experiment #5: Serum of MASP-3 deficient (CMC) patient has functional MASP-2 if MBL is present

### Methods:

A C3b deposition assay was carried out using Mannan-coated ELISA plates under to examine whether 3MC serum is deficient in LEA-2. Citrate plasma was diluted in BBS buffer in serial dilutions (starting at 1:80, 1:160, 1: 320, 1:640, 1:1280, 1:2560) and plated on Mannan-coated plates. Deposited C3b was detected using a chicken anti-human C3b assay. LEA-2 driven C3b deposition (the plasma dilutions are to high for the AP and LEA-1 to work) on Mannan-coated ELISA plates was evaluated as a function of human serum concentration in serum from a normal human subject (NHS), from two 3MC patients (Patient 2 and Patient 3), from the parents of Patient 3 and from a MBL-deficient subject.

### Results:

**FIGURE 47** graphically illustrates the level of LEA-2-driven (i.e., MASP-2-driven) C3b deposition on Mannan-coated ELISA plates as a function of the concentration of human serum diluted in BBS buffer, for serum from a normal human subject (NHS), from two 3MC patients (Patient 2 and Patient 3), from the parents of Patient 3 and from a MBL-deficient subject. These data indicate that Patient 2 is MBL sufficient. However, Patient 3 and the mother of Patient 3 are MBL deficient, and therefore their serum does not deposit C3b on Mannan via LEA-2. Replacement of MBL in these sera restores LEA-2 mediated C3b deposition in the serum of Patient 3 (who is homozygous for the SNP leading to MASP-3 deficiency) and his mother (who is heterozygous for the mutant MASP-3 allele) (data not shown). This finding demonstrates that 3MC serum is not deficient in LEA-2, but rather appears to have functional MASP-2 and functional MASP-1.

### Overall summary and Conclusions:

These results demonstrate that MASP-3 deficiency in human serum results in loss of AP activity, as manifested in reduced C3b deposition on zymosan-coated wells and reduced rabbit erythrocyte lysis. The AP can be restored in both assays by supplementing the sera with functional, recombinant human MASP-3.

## Claims

1. A composition comprising an amount of a MASP-3 inhibitory agent effective to inhibit MASP-3-dependent complement activation for use in treating a subject suffering from paroxysmal nocturnal hemoglobinuria (PNH), wherein said MASP-3 inhibitory agent is a MASP-3 monoclonal antibody or fragment thereof that specifically binds to a portion of human MASP-3 (SEQ ID NO:8).

2. A composition for use as claimed in Claim 1, wherein the composition further comprises at least one of: a MASP-1 inhibitory agent, a MASP-2 inhibitory agent, a combination of a MASP-1 inhibitory agent and a MASP-2 inhibitory agent, and/or a terminal complement inhibitor that inhibits cleavage of complement protein C5.

3. A composition for use as claimed in Claim 2, wherein the MASP-1 inhibitory agent is at least one of:
(i) a MASP-1 monoclonal antibody, or fragment thereof, that specifically binds to a portion of SEQ ID NO:10;
(ii) a MASP-1 inhibitory agent that specifically binds to a portion of MASP-1 with an affinity of at least 10 times greater than it binds to MASP-3 (SEQ ID NO:8); and/or
(iii) a MASP-1 inhibitory agent that specifically binds to the serine protease domain of MASP-1 (aa 449-694 of SEQ ID NO:10).

4. A composition for use as claimed in Claim 2, wherein the MASP-2 inhibitory agent is a MASP-2 monoclonal antibody, or fragment thereof that specifically binds to a portion of SEQ ID NO:5.

5. A composition for use as claimed in any one of Claims 1-4, wherein the composition increases the survival of red blood cells in a subject that exhibits one or more symptoms selected from the group consisting of (i) below normal levels of hemoglobin, (ii) below normal levels of platelets; (iii) above normal levels of reticulocytes, and (iv) above normal levels of bilirubin; optionally wherein the subject has previously undergone, or is currently undergoing treatment with a terminal complement inhibitor that inhibits cleavage of complement protein C5.

6. A composition for use as claimed in Claim 2, wherein the terminal complement inhibitor is a humanized anti-C5 antibody or antigen-binding fragment thereof, or wherein said inhibitor is eculizumab.

7. The composition of Claim 1 wherein the MASP-3 monoclonal antibody or fragment thereof is selected from the group consisting of a recombinant antibody, an antibody having reduced effector function, a chimeric, and a humanized or human antibody.

8. The composition of Claim 1, wherein the composition is formulated for systemic delivery, such as for delivery subcutaneously, intra-muscularly, intravenously, intra-arterially or as an inhalant.

9. A composition for use according to claim 1, comprising an amount of a MASP-3 inhibitory agent effective to increase the survival of red blood cells in a subject suffering from paroxysmal nocturnal hemoglobinuria (PNH).

10. A composition for use as claimed in Claim 9, further comprising a MASP-2 inhibitory agent.

## Patentansprüche

1. Zusammensetzung, die eine Menge eines MASP-3 hemmenden Mittels umfasst, die wirksam ist, um eine MASP-3-abhängige Komplementaktivierung zu hemmen, zur Verwendung beim Behandeln eines Patienten, der an paroxysmaler nächtlicher Hämoglobinurie (PNH) leidet, wobei das MASP-3 hemmende Mittel ein monoklonaler MASP-3-Antikörper oder ein Fragment davon ist, der/das spezifisch an einen Abschnitt humaner MASP-3 (SEQ ID NR. 8) bindet.

2. Zusammensetzung zur Verwendung nach Anspruch 1, wobei die Zusammensetzung ferner wenigstens eines der Folgenden umfasst: ein MASP-1 hemmendes Mittel, ein MASP-2 hemmendes Mittel, eine Kombination aus einem MASP-1 hemmenden Mittel und einem MASP-2 hemmenden Mittel und/oder einen terminalen Komplementinhibitor, der eine Spaltung des Komplementproteins C5 hemmt.

3. Zusammensetzung zur Verwendung nach Anspruch 2, wobei das MASP-1 hemmende Mittel wenigstens eines der Folgenden ist:
(i) ein monoklonaler MASP-1-Antikörper oder ein Fragment davon, der/das spezifisch an einen Abschnitt von SEQ ID NR. 10 bindet;
(ii) ein MASP-1 hemmendes Mittel, das mit einer Affinität, die wenigstens 10 mal größer ist als diejenige, mit der es an MASP-3 (SEQ ID NR. 8) bindet, spezifisch an einen Abschnitt von MASP-1 bindet; und/oder
(iii) ein MASP-1 hemmendes Mittel, das spezifisch an die Serinproteasedomäne von MASP-1 (aa 449-694 von SEQ ID NR. 10) bindet.

4. Zusammensetzung zur Verwendung nach Anspruch 2, wobei das MASP-2 hemmende Mittel ein monoklonaler MASP-2-Antikörper oder ein Fragment davon ist, der/das spezifisch an einen Abschnitt von SEQ ID NR. 5 bindet.

5. Zusammensetzung zur Verwendung nach einem der Ansprüche 1-4, wobei die Zusammensetzung das Überleben roter Blutkörperchen bei einem Patienten erhöht, der ein oder mehrere Symptome aufweist, die ausgewählt sind aus der Gruppe bestehend aus: (i) unternormalen Hämoglobinspiegeln, (ii) unternormalen Thrombozytenspiegeln, (iii) übernormalen Retikulozytenspiegeln und (iv) übernormalen Bilirubinspiegeln; wobei der Patient optional zuvor eine Behandlung mit einem terminalen Komplementinhibitor, der eine Spaltung des Komplementproteins C5 hemmt, absolviert hat oder aktuell eine solche Behandlung absolviert.

6. Zusammensetzung zur Verwendung nach Anspruch 2, wobei der terminale Komplementinhibitor ein humanisierter Anti-C5-Antikörper oder ein antigenbindendes Fragment davon ist oder wobei der Inhibitor Eculizumab ist.

7. Zusammensetzung nach Anspruch 1, wobei der monoklonale MASP-3-Antikörper oder das Fragment davon ausgewählt ist aus der Gruppe bestehend aus einem rekombinanten Antikörper, einem Antikörper mit reduzierter Effektorfunktion, einem chimären und einem humanisierten oder humanen Antikörper.

8. Zusammensetzung nach Anspruch 1, wobei die Zusammensetzung zur systemischen Verabreichung formuliert ist, wie etwa zur Verabreichung subkutan, intramuskulär, intravenös, intraarteriell oder als Inhalationsmittel.

9. Zusammensetzung zur Verwendung nach Anspruch 1, die eine Menge eines MASP-3 hemmenden Mittels umfasst, die wirksam ist, um das Überleben roter Blutkörperchen bei einem Patienten zu erhöhen, der an paroxysmaler nächtlicher Hämoglobinurie (PNH) leidet.

10. Zusammensetzung zur Verwendung nach Anspruch 9, die ferner ein MASP-2 hemmendes Mittel umfasst.

## Revendications

1. Composition comprenant une quantité d'agent inhibiteur de MASP-3 efficace pour inhiber l'activation du complément dépendante de MASP-3 à utiliser dans le traitement d'un sujet souffrant d'hémoglobinurie paroxystique nocturne (HPN), dans laquelle ledit agent inhibiteur de MASP-3 est un anticorps monoclonal MASP-3 ou un fragment de celui-ci qui se lie spécifiquement à une partie du MASP-3 humain (SEQ ID NO : 8).

2. Composition à utiliser selon la revendication 1, la composition comprenant en outre au moins un des éléments suivants : un agent inhibiteur de MASP-1, un agent inhibiteur de MASP-2, une combinaison d'un agent inhibiteur de MASP-1 et d'un agent inhibiteur de MASP-2, et/ou un inhibiteur de complément terminal qui inhibe le clivage d'une protéine du complément C5.

3. Composition à utiliser selon la revendication 2, dans laquelle l'agent inhibiteur de MASP-1 est au moins un des éléments suivants :
(i) un anticorps monoclonal MASP-1, ou un fragment de celui-ci, qui se lie spécifiquement à une partie de la SEQ ID NO : 10 ;
(ii) un agent inhibiteur de MASP-1 qui se lie spécifiquement à une partie de MASP-1 avec une affinité au moins 10 fois supérieure à celle de l'agent qui se lie à MASP-3 (SEQ ID NO : 8) ; et/ou
(iii) un agent inhibiteur de MASP-1 qui se lie spécifiquement au domaine sérine protéase de MASP-1 (aa 449-694 de la SEQ ID NO : 10).

4. Composition à utiliser selon la revendication 2, dans laquelle l'agent inhibiteur de MASP-2 est un anticorps monoclonal MASP-2, ou un fragment de celui-ci, qui se lie spécifiquement à une partie de la SEQ ID NO : 5.

5. Composition à utiliser selon l'une quelconque des revendications 1 à 4, la composition augmentant la survie des globules rouges chez un sujet qui présente un ou plusieurs symptômes choisis dans le groupe constitué (i) de taux d'hémoglobine inférieurs à la normale, (ii) de taux de plaquettes inférieurs à la normale ; (iii) de taux de réticulocytes supérieurs à la normale et (iv) de taux de bilirubine supérieurs à la normale ; facultativement, le sujet ayant subi préalablement, ou subissant actuellement, un traitement avec un inhibiteur de complément terminal qui inhibe le clivage d'une protéine du complément C5.

6. Composition à utiliser selon la revendication 2, dans laquelle l'inhibiteur de complément terminal est un anticorps anti-C5 humanisé ou un fragment de liaison à l'antigène de celui-ci, ou dans laquelle ledit inhibiteur est l'éculizumab.

7. Composition selon la revendication 1 dans laquelle l'anticorps monoclonal MASP-3, ou un fragment de celui-ci, est choisi dans le groupe constitué d'un anticorps recombinant, d'un anticorps ayant une fonction effectrice réduite, d'un anticorps chimérique et d'un anticorps humanisé ou humain.

8. Composition selon la revendication 1, la composition étant formulée pour une administration systémique, comme pour l'administration sous-cutanée, intramusculaire, intraveineuse, intra-artérielle ou par voie d'inhalation.

9. Composition à utiliser selon la revendication 1,
comprenant une quantité d'un agent inhibiteur de MASP-3 efficace pour augmenter la survie des globules rouges chez un sujet souffrant d'hémoglobinurie paroxystique nocturne (HPN)

10. Composition à utiliser selon la revendication 9, comprenant en outre un agent inhibiteur de MASP-2.
